# EUROPEAN PATENT SPECIFICATION

(11) **EP 2 247 740 B1**
(45) Date of publication and mention of the grant of the patent: **23.05.2012**
(21) Application number: 08786884.0
(22) Date of filing: 05.08.2008
(51) Int. Cl.: C12P 17/08, C12N 15/52, C12R 1/01

(54) **GENES ENCODING THE BIOSYNTHETIC PATHWAY FOR ETNANGIEN PRODUCTION**
FÜR DEN BIOSYNTHESEWEG FÜR DIE ETNANGIEN-PRODUKTION CODIERENDE GENE
GÈNES CODANT POUR LA VOIE BIOSYNTHÉTIQUE POUR LA PRODUCTION D'ETNANGIEN

(30) Priority: 10.08.2007 EP 07114197
(43) Date of publication of application: 10.11.2010
(73) Proprietor: Helmholtz-Zentrum für Infektionsforschung GmbH, 38124 Braunschweig (DE); Universität des Saarlandes, 66123 Saarbrücken (DE); Universität Bielefeld, 33615 Bielefeld (DE)
(72) Inventor: GERTH, Klaus, 38124 Braunschweig (DE); PERLOVA, Lena, 66119 Saarbrücken (DE); MÜLLER, Rolf, 66123 Saarbrücken (DE); SCHNEIKER, Susanne, 33615 Bielefeld (DE); PÜHLER, Alfred, 33615 Bielefeld (DE)
(74) Representative: Taruttis, Stefan Georg
(86) International application number: PCT/EP2008/060276
(87) International publication number: WO 2009/021875

(56) References cited:
- DE-B4- 19 630 980
- ACHUPP T ET AL: "A SORANGIUM CELLULOSUM (MYXOBACTERIUM) GENE CLUSTER FOR THE BIOSYNTHESIS OF THE MACROLIDE ANTIBIOTIC SORAPHEN A: CLONING, CHARACTERIZATION, AND HOMOLOGY TO POLYKETIDE SYNTHASE GENES FROM ACTINOMYCETES" JOURNAL OF BACTERIOLOGY, AMERICAN SOCIETY FOR MICROBIOLOGY, US, vol. 177, no. 13, 1 July 1995 (1995-07-01), pages 3673-3679, XP000654849 ISSN: 0021-9193
- PERLOVA ET AL: "Identification and analysis of the chivosazol biosynthetic gene cluster from the myxobacterial model strain Sorangium cellulosum So ce56" JOURNAL OF BIOTECHNOLOGY, ELSEVIER SCIENCE PUBLISHERS, AMSTERDAM, NL, vol. 121, no. 2, 24 January 2006 (2006-01-24), pages 174-191, XP005236110 ISSN: 0168-1656
- SCHNEIKER SUSANNE ET AL: "Complete genome sequence of the myxobacterium Sorangium cellulosum" NATURE BIOTECHNOLOGY, vol. 25, no. 11, November 2007 (2007-11), pages 1281-1289, XP002502475 ISSN: 1087-0156
- MENCHE DIRK ET AL: "Stereochemical Determination and Complex Biosynthetic Assembly of Etnangien, a Highly Potent RNA Polymerase Inhibitor from the Myxobacterium Sorangium cellulosum." JOURNAL OF THE AMERICAN CHEMICAL SOCIETY 29 OCT 2008, vol. 130, no. 43, 29 October 2008 (2008-10-29), pages 14234-14243, XP002502476 ISSN: 1520-5126
- PRADELLA SILKE ET AL: "Characterisation, genome size and genetic manipulation of the myxobacterium Sorangium cellulosum So ce56." ARCHIVES OF MICROBIOLOGY, vol. 178, no. 6, December 2002 (2002-12), pages 484-492, XP002502477 ISSN: 0302-8933
- IRSCHIK HERBERT ET AL: "Etnangien, a macrolide-polyene antibiotic from Sorangium cellulosum that inhibits nucleic acid polymerases." JOURNAL OF NATURAL PRODUCTS JUN 2007, vol. 70, no. 6, June 2007 (2007-06), pages 1060-1063, XP002502478 ISSN: 0163-3864
- KOPP MAREN ET AL: "Critical variations of conjugational DNA transfer into secondary metabolite multiproducing Sorangium cellulosum strains So ce12 and So ce56: development of a mariner-based transposon mutagenesis system" JOURNAL OF BIOTECHNOLOGY, ELSEVIER SCIENCE PUBLISHERS, AMSTERDAM, NL, vol. 107, no. 1, 8 January 2004 (2004-01-08), pages 29-40, XP002355734 ISSN: 0168-1656

### SEQUENCE LISTING

<110> Helmholtz-zentrum fur Infektionforschung GmbH
   Universität des Saarlandes
   Universität Bielefeld
<120> Production of Etnangien
<130> G1018PCT
<160> 61
<170> PatentIn version 3.4
<210> 1
   <211> 315
   <212> DNA
   <213> Sorangium cellulosum
<400> 1
   atgagctatt tcagaggcgt tcaacgcccg gagctcgtct ctatggaagc gattgtcaag     60
   aggtatctgt ccgagcggct cttgatcgaa ttcgacggcg aaagcggagt cgacgcagac    120
   agcgatctgt tccagctcgg cctgatggat tctcaggcct acgtcgagct catccagttt    180
   gtcgaacgtg agttcggcct gagattcacg gacgaagagc tcctgtccaa cgtggtggtc    240
   tcgctgtccg ggatcgtctc gcttgtgtcc ggtgcaatgg acgaacgcgc gcgtcagaac    300
   gcgctccggg gatga                                                     315
<210> 2
   <211> 918
   <212> DNA
   <213> Sorangium cellulosum
<400> 2
   atgaaggccc tcctgttccc cgggcaaggg gcgcaagatc cggccatgtg tgcgggggtg     60
   gtggggcacc cccggttcgc cgagcgatac ggcgtgatct gcgattcgac gcgcaccgac    120
   gtcttgcgga ggatccagga aggcgacgcc gggatcctcg atcaaaaccg gttcagctcg    180
   ctgatgaccg tgttggtcag cgcgatcttg ctcgacatgc tccgggagcg tgatgcggcg    240
   acccctgagt atttcgccgg gtacagcgtc ggtcagtggt cggccatgta cgccgcgggg    300
   atgctcacgt acgagcagac gatcgaggcg ctcgtcgagc gcgcgcgcct catggacgag    360
   tgcgtggacc gtcgccctgg cgcgatgtgc gcggtgatcg ggctccggga agcggtgctc    420
   gagcaggtgc tggccgacct gcgctcgcgg gggcatctcg tgttcatcgc gaactacaac    480
   tgcgtcgggc agtactcgat ctccggcgcc gtgccggcga tcgagctcgc cgaggaggag    540
   ctgcgagcgc accggccgaa gaagatcgtc cggctgccgg tcagcggcgc atggcactgc    600
   ccgatgctgg aggacgcgca ggtgagattc gccgaggtgc tggcgggcgt ccgcttcggg    660
   ccccgcgcgg cgccggtgct ggacaacgtc acgggggact ggctgcccga cgagcccgaa    720
   gcgctgcgcg acggcctggc gcgccacatc agccaccccg tgcagtggga gcgtgggatc    780
   aagaagctcg ctgcgctcgg gtgccgtcgg ttcgtcgagg tcgggtacgg gaatagcctt    840
   acgaagttcg ggttcttcat cgatcgacag gccgagttca gcgcattcca cggcgagggc    900
   gttgcatgtg cgggatag                                                  918
<210> 3
   <211> 1902
   <212> DNA
   <213> Sorangium cellulosum
<400> 3
   gtgcgaatgc tggcgcgcct ccgccaccgc ggccccgacg agatggggta ctacttcgac      60
   gactccgtcg cgatcggcaa cgcgcgcctc agcatcgtgg atctcgccgg cggccagcag     120
   ccgatcggcg atccgacggg ccggtactgg attgtcttca acggcgagat ctacaactac     180
   aaggagctcg gagcggagct ggccgccgcc ggccaccggt tcgcctcgag ctccgacacg     240
   gaggtgctgc ttcacgcgtg gatcgcgtgg ggcccggcgg cgttcacgcg tctgaacggc     300
   gcgttcgcgt tctgtatcta cgatcggcgc gacaaaacgc tggtgctggc gagagatcgg     360
   ttcggcaagc ggccgctcta ctacgtgcgc gacgcggaag gggtcgtctt cggctcggag     420
   ctgaagtgct tcctggagta cgagccgttc cgcttcgagc tcgaccctgg acaactcgcg     480
   tcgatcttcc gcatctggac gccgctggag gatcaatgcg ggttccgctc ggtgaggcag     540
   gtcccggccg gcgcgtacgc gcaggtcacg gggagctcgt tcgacatcgt tcactacgcg     600
   ccgctgagga tcgatcgccc cccgcccgac ctctccgagc aggaggccgc agcgcaggtg     660
   cgcgacgcgc tctcgaagag cgtgcagctc cgattgcgca gcgacgtcga ggtcggcacc     720
   tacctgagcg gggggatcga ctctgcgatc gtcgcgacgt tgatcgcgga gaactcgacc     780
   ggtagaacga agagcttctc gatccgcttc gaagaggacg agttcgacga atcgggcgac     840
   caggagctga tgtcgtcctt cctcgggacg gagcacgcat cgatccggat cgggagccag     900
   gagatcgtcg acgccttccc cgaggcgctc tggcacgccg aggtcccggt gttccggacc     960
   gcgttcgtgc cgatgtttct cctgtcccgg ctggtgaagg accacggcat caaggtggtg    1020
   ctgaccggcg agggcgcgga cgaggcgttc ctcggctacg acatcttcag agagaccctg    1080
   ctccgcgccg agtgggcgga gatggagccc gccgcgcggc agcgcaagct ctcgcgcctg    1140
   tatccgtatc tgcggcagtt cgacgagcgg aaccacgcgg cgctcgccgc gctcttcgag    1200
   cggctctcgc gcgatccgcc caccgatttc ttctcgcatg agctgcgatt cctcaattcg    1260
   cagctctcgg cgaggttgct ggcggggcgg cacgacgcgc tccggcccct gaccgagcac    1320
   gtcgcggcgc cgcacgagcg gtacgcgccc ctctcgcgcg tccagaaggc gcagtggctg    1380
   gagttcaaga cgttgctggc aggatatctg ctgtcgaccc agggcgaccg gatgagcctc    1440
   gcgcacggcg tggagaatcg ctgcccgttc ctggatccgg acgtcgtcgc gctggggtgc    1500
   gcgacgagcc tcgcgttcga cgatggcttc gacgagaaat acctgctcaa gaaggcgttc    1560
   gccgcaaagc tgcccgagcg aattctgaag aagccgaagc agccttaccg cgcgccggac    1620
   gcgagcgcgt tcctgaagcg ccagcccgac tacctctccg tgatccgatc caaggccgag    1680
   cttgagaaga tcggcgtgct ggatgtgaaa ttctgcgcgg cgttcgtcga gcggatgctc    1740
   tcgaagccgg ccgaatcgat cagccaggcg gagaatcaga catttctgtt ccttctctcg    1800
   acggcgctgc tccaccggcg cttcgtggag cgtgacgtgc cggcgccgcc gacgatcgag    1860
   ccgctgctcg tccggcggtt cgacgggagg ctcatcgcat ga                       1902
<210> 4
   <211> 15441
   <212> DNA
   <213> Sorangium cellulosum
<400> 4
   atgaaagatg agaagaagag cccggacctc ggcgggtcgg atgtcgccgt cgtcggcgtg 60
   gggggccgat tcccgcaggc tcgcgggccc gagcagctct gggagaacct gcgcagcggc 120
   cggtgcgcga tcacggcagt gccgcccggg cggtggagcg atgcggcgcg cgcgggcgag 180
   ttccgcggtg gcttcgtgga ggacgcggac tgcttcgacg ccgcgttctt caagatctcg 240
   ccgaaggagg ccgagctcat ggacccgcag cagcgaatgc tgctcgaggt cctctggcac 300
   acgctcgagg acgcgcgcgt caagccgtcg tcgctggagg gacggaagat cggcgttttc 360
   atgggggtgt gcaacgacga ctacgcggag ctcctgcacg agcacgacgc gtacgacgcc 420
   agcctctacg gctcgaccgg gacctctggc tcgatcctct ccaaccgcgt ctcgtttttc 480
   ttcgatttcc gcggaccgag cgtcacggtc gacacggcgt gctccagctc gctcgtggcc 540
   gtgcacctgg ctgcgagggc gatccgtgcg ggggaatgtg aggcggcgct cgcgggcggc 600
   gccaacctct gctggacgga gagtcgcttc ttggccttca ggagcgccgg gatgctgtcg 660
   aaggacggcg tgtgcaggac gttcgacgct cgggccaacg gctacttgcg tggcgagggc 720
   gccggcgcgg tcctcctgag gccgctccgc gatgcgcttc gcgacggtga ctacatctac 780
   ggtgtcatca aggggggagc gatcaaccac ggcggccgaa cgcgcgggtt gaccgtcacg 840
   agcccggcgc agcagcgcca gctcctgatc gacgcgtacc gcgacgccgg tgtgcacccc 900
   gacaccgtcg gctacatcga gacgcacggg accggcacgt cgctcggcga tcccatcgag 960
   ttcctcgggc tcaagcaggc gttcgaagcg ctgagagagc agggcagcga gcggcgagtg 1020
   cggcccggaa cgtgcgcgct gggcgcgctc aagaccaaca tcggccacct cgaggccgcg 1080
   gccggcgtcg ccggcctcat caaggtgctc ctgtccctga agcacggggc gatccccgga 1140
   aacctccact tcgacgagct caattcgcgg atctcgctcg acggaacgtc gttctacttc 1200
   ccgacgagca cccgcgcatg gcccgcgggc gagccgggtg acggtgcgcc gccgaggcgc 1260
   gcaggcgtga gcgcgttcgg gtacgggggc gcgtacgcgc acgtcgtcgt cgaggagcac 1320
   tgcgcgccgc cgcccgaggc gagcgcgccg gcccccggcc cccacctcgt cgcgctgtcg 1380
   gcccccacgc cggcgcggct gcgcgcctgg gcggaggcgc tcgtcgagca catcggggcg 1440
   agagcggtcg aggcgccggt tgcgctctcg gagctcgctt actccctgca gacgacgcgc 1500
   gaggagttcg aggagcgact cgcgctgatc gcggaggact ccgcggcgct gtccgagcag 1560
   ctgcgagcgt ttgtctcggg cgtcgaccct gccggttgct tccgagggaa tgtgcggacg 1620
   gcgaagggcg cgccggtgga cgcggcggtc gtcgacgcgc acatcggcgg ccgggcgctg 1680
   tcgtcgctcg cggagctctg ggtccgcggc cagtcgatcc cgtggcgcgg cctgtacgcc 1740
   ggcgccgtcc cccgcacgat cgcgctgccg ttgttctcgt tcgagcggcg caggtactgg 1800
   gtgacgcggc gtcaggtcgc ccccgcgccc gaggtctcgc ccaccgtggc gccgcctggc 1860
   ccggccgcga tcgagaagcc aagcggcatc gcgctgcgag cgcccgggga agagcggttc 1920
   gctgcgcccg cagtgtcgtc gggcgtcgaa catacggtgt cgctctcgcc cgtgggcgcc 1980
   gccgtgaacc aggcggcggt cgtcgccggg tcggccatgg gagccgcgcc gtacgggggc 2040
   ggttcaccag ccggcggggc ggcctcgtcg gccatcgcgg gcgtcgagcg ggtgaccccc 2100
   cgcgcgaccg agggcggcgt gggaggccac atgccaggaa cgacggcgac cgcacgcgac 2160
   cggcgcgcgt cgcccgtgga taacgcagcc ggcgggcgat cgctcgccgc gttggagcag 2220
   gagctcacgg ggagtctggc cgaggcgctc ttcatggacc gggcggacgt cgagcccgac 2280
   cgtcctttca tggagctcgg tctcgactcg atcgtcggcg tcgagtgggt acgggtgatc 2340
   aacacgacgt acgggctgtc gctgccggcg acgtgcgtgt acgaccatcc cacgatccgc 2400
   cgcctcgcgg cccacctgtc gtcgatcgcc ccgcgcacgg aggtatcggc ggtcgcgcac 2460
   cgccgcccgc gcggcggcgc ggccgaggct ggacgcgcgc gagcgcacga cctcggggcg 2520
   ctggaagagg ggttgacgag cagcctggcc gaggcgctct acacggaccg tgcggagatc 2580
   gatccggatc gtcccttcac ggagctcggt ctcgactcga tcgtcggcgt cgagtggata 2640
   cgggcgatca acaaagcgta cggactatcg ctgccggcga catgcgtgta cgaccacccc 2700
   acgattggcc gcctggcccg gcacctttcg ccgatgctcg gcggagcggg cgccggcccg 2760
   tcggcgcagc atggcgggga ggatgcgctg catctctcgc gcgacgatgc tgaagggcaa 2820
   cctcgcgacg cgcgcgtcgg ggcggcgcga tcgagcgagc tcgacgcgct ccaggtcgag 2880
   ctcgcaaaga gccttgcgga cgcgctctac atggaacccg gcgacgtcga cgtggaccgt 2940
   ccgttcgtgg atctggggct cgactcgatc gtgggcgtcg agtggacacg ggcgatcaac 3000
   aaggcgtacg gcgtgtccct gccggcgacg cgcctctacg accactccac cgttcgacgg 3060
   ctcgcggcgc acctctcggg cgagctcgat gtgcgtgcag ccgcggggag acctgtcgcg 3120
   accgagcgag agcgccccgc tctcgggggc gacattgcag cgcgtcccgc gcgcgagccc 3180
   gaggcgcgcg ccccgcgccc ggcacctgag catgaaggca tcgcggttat cgggatgtcg 3240
   ggggcgtttc cgaaggcgag ggacctggac gagttctggg agaacatcgc gcgcggcgtc 3300
   gactgcgtga cggagatccc gccggatcgc tgggcgatcg cggagcacta cgacccgagg 3360
   ccggacgccc cgcgcaagac ctacagcaag tggatggggg tgctcgacga cgtggatcgg 3420
   ttcgatccgc tgttcttcgg catcacgccg acggaagccg agctgatgga tccccagcaa 3480
   cggctgttcc tcgagcactg ctggagctgc atcgagtcgg ccgggatcga tccgcagcgg 3540
   ctctcgggaa gccgctgcgg ggtctacgtt ggctgcatgc cgggcgacta cgtccgggtg 3600
   ggcgacggca ccgatctcac gctgcactgg ctgatgggca gctcgtcatc gatcctcgct 3660
   gcgcggatct cgtactgctt gaacctgaag gggccgtgcc tcgcgattga cacggcctgc 3720
   tcgtcgtcgc tcgtcgcgat cgtcgaggcg tgcgactcgc tgctgctcgg gcggagtgat 3780
   ctcgccctcg cgggcggcgt gagcgtcgcc gtgcggccgg agacccatat catcggcagc 3840
   ggcgcgggga tgctctcccc gcgcgggcgc tgcttcacgt tcgacgcgcg cgccgacggc 3900
   ttcgtgccgg cggagtgcgt cggcgtcgtc ctcttgaagc ggctcgccga cgcggtgcgg 3960
   gacggggaca tcatcgccgg ggtccttcgt ggatgggcgc tgaaccagga tgggcggacc 4020
   aacggcatca ccgcgcccag cgtgacctcc caagccgcgc tgcagaagca ggtgtacgac 4080
   catttcggga tcgaccccga gaccatctcc ctcgtggagg cgcacggcac cggcaccaag 4140
   ctaggcgacc cgatcgaggt gcaggcgctg accgagtcgt tccgagcgta caccgacgag 4200
   cgggagtact gcgcgctcgg ctcggtgaag agcaacatcg gtcacgggct cgccggcgcc 4260
   ggcgttgccg gcgtgctcaa ggtcctgctc gcgcttcgac acgaacggct gccgccgacg 4320
   atccactacg cgacgcggaa cgagcacatc accttcgagg gaagcccatt ctacgtcaac 4380
   accgagctcc gtccgtgggc gcggcgggcg gggtcgccgc ggcgcgctgc gatcaactcg 4440
   ttcggcttca gcgggacgaa cgcgcacgtc gtgatcgagg aggcgccgcc gcgctcgggc 4500
   gaggtgacgt tcgcggaggc gggtctgccg gtgctgatcg tcctctcggc gaagaggccg 4560
   gagcgcctcg aggcgtacgc tgcgaagctc gcgcgatggc ttgaacggca gccggcgctc 4620
   gacctgaccg cccttgccta cacgctccag atcgggcgag aggcgatggc ctgccgggcg 4680
   gcgttcgtgg ccgacggccg gcgccggatc gtcgacgtcc tcggcgagct ggcgcgaggt 4740
   gtccgcgctc ccggggtctt catggggcag gcgaagcgcc gcggcgagac gggggcgccg 4800
   tccgcgagcg atgcagccac gggccggctg ctcggtgccc gcgcgcgcga ggagcggctg 4860
   agcgagctgg gcgagctctg ggtgcaagga acgacgatcg cgtgggatgc gctgtaccag 4920
   gatcgtacgc ccgcccgcct gagcattccg acctatccgt tcgcgaggga gcggtattgg 4980
   ttcccgttgc ccggcggcac ggcgtctgcc ccgctcctct tcgagccacg ctgggacgcg 5040
   aaggaggcgg ccggcgatgc gcgcgagggc gaccgcgccg cggcgcggtt cgcgaggcgc 5100
   tgcgtcctcg tggagccggc gtacgaagac gcggcgcgcg cgctggcgag cgcctgcccc 5160
   gggaccgaga tcgaggcgct gcgagtccac ggcggcacgc tggcagatcg cttcgcgggg 5220
   cgtgtcgagc aggtgttcga gcgcgtccgc gagctcctgc gcgatcggct cgacgggcga 5280
   gcgctgctcc aggtcgtggt gagccaccag ggcgaggggg cggaggcgct cgcgggggtc 5340
   tccggtctgc tgaagacggc gagccgggag aaccccgacc tcgtcgcgca gctcgtccag 5400
   atcgcctcgc cgatgcgggc cgacgagctg gcagcgaggt tgcggagcga cgcggcggcg 5460
   agcgacgatc gggtgattcg ctatcgcgag ggcggccggt acgtcgcgac cgtggccgcg 5520
   ctcgcaccgg cgccggacga cggcgctcgg gcgccgggcg cgcaggccgg ctggaagagc 5580
   gatggcgtgt acctgatcgc cggcggaggc ggcggcatcg ggcggatcgt cgcgcgcgac 5640
   atcgtcgagc acgccgacgg ggcgacggtg ctcctcgcgg gacgctcgcc gctcaccggc 5700
   gagctgcgcg ccgcgctcga tgcactgcgc gcggagggcg agcggcggcg ggcgcgcgtc 5760
   gagtacctga ccatggacgt cgcggacgtc gccgcggtgg aggggcgcgt tcgagacatc 5820
   ctcgagcgat acggccgtct gagcggcgtc attcacgcgg caggcgtgct ccgcgacggc 5880
   ttcatcgttc gaaagcagac gagcgacctg catgccgtgc tcgcgccgaa ggtgcgtggc 5940
   gtcgtccacc tcgatcgcgc gaccgctcgt ctcccgctcg acttcttcgc gatcttctcc 6000
   tcgatcgctg gcgtcttcgg aaacgtgggg caggcggact acgcggcggc gaacggcttc 6060
   ctggacgggt acgccgccta ccgcgatcgg ctggtcgcgg acggggagcg ccgcgggcgg 6120
   acgatcgcga ttgcgtggcc cctgtgggcg gacggcggga tgcacgtgga tcccgtcacc 6180
   gccgaggcgc tcgatcgagc cgggctcgcg ccgctctccg ccgcgcaagg gctcgccgcg 6240
   cttcacgagg cgctcgcgtc gagccgtgcg cgtgtcgtcg tcctcgcagg tgggatcgag 6300
   cggctgcaag ccatgggggt ggcgacgggt ccggcgcccg ctccggcgac gcggagcgcc 6360
   gcggcgacgg ccgcgcaggc ggtgcgcgag gcggtgccga tcaccgcggc gcgcgacgcc 6420
   gcggcgatcg atccgcgact cctcgaagag aagacgctgc gcaggctccg cgggctcttt 6480
   gctgacgtca cgaagctgcc gtcgagccag atcgatccag aggagtcgct ggagctgtat 6540
   gggatcgact cgctcctcat catgcgcctg aacgaggcgc tgtcgaaggt gttcggcgac 6600
   atctcgaaga cgctctggtt cgagtaccag acgctcgccg cgctcgccgg ccacctgagc 6660
   gcgcgtcacg ccgccgcgtg cgcgcgctgg accggaatgg atcgcctcgc cgacgctccg 6720
   gtccacgcga acgagccggg cggcgccatg gccccgctgg acgcgggggc gcgacgcgac 6780
   gaggcgcctc tgccgcgaag cgaggaggct gccggggcgc ggccggcgcg cggccccatc 6840
   gcgatcatcg gcctcagcgg ccggtacccg caggcggagg acgtcggcga gttctgggag 6900
   cacctcaagg ccgggcggag ctgcactcgc gagatacccg ccgatcgctg gtcgctcgac 6960
   ggtttttacg agcccgaccc cgaacaggca gtgatggagg ggaagagcta cggcaagtgg 7020
   ggcggtttcc tcgacggctt cgccgacttc gatccgctct tcttcaatgt ctcgccgcgc 7080
   gagtcgatga acatcgatcc gcaggagcgg ctcttcatcg agtgctgctg gagcgtgctc 7140
   gaggacgccg gctacacgcg cgacctgatc gcgtcgcagc acggggggcg ggtcggcgtc 7200
   ttcgccggga tcacgaagac cggcttcgat ctccacggcc cggagatgcg cctccacgac 7260
   cgacagagct ttccgcacac gtcgttcggc tcggtggcca atcgcgtctc ctatctgctg 7320
   aacctgcgcg gcccgagcat gccgatcgac acgatgtgct cgtcgtcgct gacggcgatc 7380
   cacgaggcgt gcgagcacct gctgcgcgac gagtgcgaga tcgcgatcgc cggcggcgtg 7440
   aatctttacg tgcacccgtc gacgtacgtc gggctgtccg cgcagcggat gctctcccgg 7500
   accgggcact gcaagagctt cggcgaaggg ggcgacggct tcgtccccgg cgagtgcgtc 7560
   ggggcggtgc tgctcaagcc gctgcagcgg gcgattgacg acggtgatca catctacgcc 7620
   gtggtcaagg ggacgagcat caaccacggt ggcaagacga acggctacac cgtgccgaat 7680
   ccggtggcgc agcgcgagct gatcctgagc gcgctcgcca gggcgggggt cgacgcgcgc 7740
   gcggtgagct acgtggaggc gcatgggacg gggacggagc tcggggatcc catcgagctg 7800
   gcgggcctga cgcaggcgtt ccgagcctgg acggaggagc gagaatactg cgcgatcggg 7860
   tctgtgaagt cgaacatcgg gcactgcgag agcgcggctg ggatagcggg tgtgacgaag 7920
   gtggtgctgc agatgaagca cggggtgctg gtcccgtcgc tgcattcgga gcggctcaac 7980
   ccgcacatcg acttcgagaa cagcccattc ttcgtgcagc gtgagctgtc ggcgtggcgg 8040
   cggccggtgg tgaaggtgga tgggaaagag cgagagcatc cgcgcatcgc cggggtctcg 8100
   tcgttcgggg cgggcggggc caatgcgcac gtggtgctcg aggagtatgt ggagcgcgcg 8160
   ggcgagcggg gtgcgctcgc gagcggagcg gggcgaggcg cggtggcggt ggtgctgtcg 8220
   gcgcggagcg cggaggcgct gcgtcggcag gcgagccggc tgagacgagc gctggagcgg 8280
   ggggagtacg gggaagggga tctggaggac atcgcgtaca cgctgcagac ggggcgagag 8340
   gcgatggagc accgtctcgg gttcatcgcc tccaacctgg acgaggtgaa gtcgaagctg 8400
   tcggcgtacg agcgaggcgc aggtgagctc gaggagttct ggcaagggga ggtgaagcga 8460
   gagaaggagg tgctttcgct gctcgggtcg gacgaagagc tgcaggaggc gatcagcaag 8520
   tgggcattgc gcggcaagta cgcgcggctc ttggagctgt gggtcaaggg gcttgctttc 8580
   gactggtcgc gagtgcacgc ggggcgcagg gcgaggcgag tgtcgctgcc gacgtatccg 8640
   ttcgagaggg agcgatactg gctgccgtcg tcgaggggga ggtctgggtc ggcgggagcg 8700
   ggtgcggggc gtattcaccc gctggtgcat gcaaacacct cgagcctgtc ggaggagcgg 8760
   ttcacgtcgg agttcacggg gcaagagccg ttcttcgcgg accacgtggt ggatgaggag 8820
   cgggtgctgc ctggggtcgc gtacctggag atggcgcggc gtgcggtgca ggaggcggtg 8880
   ggaggcgagg gggaggtagg cgagcacggg atcgagctgc atcacgtggt gtggtcgcag 8940
   ccgatcgtgc agcgaggtgc ggaggccgtg cgtgtgcacg tgcaggtcga gagcgcggag 9000
   gggtctgagc cagccgaggg ctggacgctg cgctacgaga tctactctgg gggagcgggc 9060
   gtgtcgggcg tgtcgggcga gcgtgtggtg tcgggcgtgt cgagcgagcg tgtggtgcac 9120
   tcgcagggct acgcgcggct ggtggagaag cagcctgcgc cgcgggttga cgtgagcgag 9180
   ctgcgttcgc ggtgcgagca agagcggagc ggtgcgtcgt gttacgcggc gttcgatgcg 9240
   atgggcctgc actacgggcc gtcgcatcga gggctggtga gcgtgtggcg tggggaggag 9300
   acaggcggag agcgcttcgc gctggcgcgt gtgcagctgc ctgcgagcgc gatgtccggg 9360
   cgggaggagt atgtgcttca cccgtcggtg ctggacagcg cgctgcaagc gacgatagcg 9420
   ctgagcgatg gtcagacgct gacgctgccg ttcgcgctgg acgcggcgca ggtgttcgga 9480
   gcgagcccgc cgtctgggta cgtggtgatc cgcgaggagc gtggaggcga gaagctgacg 9540
   ctggagctgt gcgacgacgg gggtgttgtg tgcgtgcggc tggccggctt cgcgagccgg 9600
   gcgcagcacc ggagcggggt agagctcttg cggcggcgct ggcggcacag cccggtggag 9660
   cattcggcgc agagcgggca tgtgcagcga tgggtgctgc tggacagcgc ctatggctcg 9720
   caggcagcag cgctgagcga gcggctgccg ggagcccaga tcacggcgct gcgcgccagc 9780
   ggcggcgtgg cggaggagtt cgagcaggtc gtcgagcagg tgatggcgac ggtgcagacg 9840
   atcctgcggg agcggccgcg gggtgaggtg ctgctgcaga tagcgctgtc gcggggtgag 9900
   cgaggcgagc ggctcggtgc ggtatccggg ctgctgaaga gcgcgagcca ggaaaacccg 9960
   aagctcgtgg gtcaggtgct ggagcttgag gacagcgcga cgctgctgga caagctcgag 10020
   gagaacgcgg cggcgagagg cgatgtagag atccgctacc agggtgggga gcggcagacg 10080
   ccggcgctgg aacctgtggt ggagtcgcga gcagcagcgg ccgaggtgcc gtggaagcaa 10140
   gacggcgtgt acctgatcac ggggggagcg ggcgggcttg ggcttctgtt tgcgaaggag 10200
   atcatcgacc gagcgcctgg agcgacgctg atcctgacgg ggcgctcgcg gctgagcggc 10260
   acgaagcaag cggcggtgga gtcgctgcgc ggtcgaggtt cgcgcgtgga gtacatgcag 10320
   ctcgacgtga gcgacggagc agcggtgtcg cgagcggtgg agcagctgca gtcggagcac 10380
   ggagggctga acgggctagt gcacgcggcg ggggtgctcc aggacagctt cgtggtgaac 10440
   aagacggaag cgcagtggcg cagcgtgctg gggccgaagg tgcgtggcct cgtgcacctg 10500
   gatgaggcga cgaagcatct ggcgctggac cacatggtgg tgttttcgtc ggtggcgggg 10560
   ctcgttggga atgtggggca ggcggactac gcggcggcga acgcgttcat ggcgcggttt 10620
   gcggagcagc gcagcgagct tgtgcgcgct ggccgtcgcc agggccggac ggtgtcgatc 10680
   gactggccgc tgtgggagag cggcggcatg caggtggacg cctcgaccgt gcggcggctg 10740
   cgccaaacat acggcgtgac cccgctcgcg accgagcacg ggatcgcggc gttctaccgg 10800
   accatggcga gcggcctcgc gcacgtcggc gtcgtcgcag gtgatgtggc gaagtggcgc 10860
   tcgcagcttg cggccaccca cgcgaccgcc ggcgaggggg acgcctcgca cgacgcgggc 10920
   tcgactgccg tcgcgtccga cgtcatggag gcgaaggcag cgcagctgct ccgaaaggtg 10980
   ctgtcgtcga gcttgaaggt gccgctggag cggatagagg gggacacgcc gctcgaccgc 11040
   tatgggatcg agtcggtgat ggtgatggag ctgacgcggg agcttgagaa gaccttcggt 11100
   ccgctatcga agacgctctt cttcgagcac caggacatcc ggtcgctgag ccggtatttc 11160
   gcggaggcgc atcgccgtcg gctcatcgag gtgctcggcg gcgacgacga gccggcgaag 11220
   agcgccgcgc gccggccgca ggagccggca tcggcgagcg ctccgcgcag ggaccgcctc 11280
   gtcacgcagc cgcgcggagg cgctgtcgcg cgcccgcttc ggccgcacga catcgcgatc 11340
   atcggcgtga gcgggagata ccccggcgcc gacgacctcg aagcgttctg ggagaacctc 11400
   aagtccggcc gggattgcat caccgagatc ccggaggacc gctggagctt ggaggagttc 11460
   ttcgatccgc agagggggaa gccggggaag atctatagcc gctggggcgg cttcatcgac 11520
   ggcgtcgacc ggttcgatcc gctcttcttc aacatctcgc cgcgtgaagc gcagttcatg 11580
   gatccgcagg agcggctctt ccttgagtgc gcgtacgaga cgatcgagga cgccggctac 11640
   acgcgagacg cgctgaagaa gcccgccgcg gccggcggcc cgcccggcac cgtgggcgta 11700
   ttcgtcggcg tgatgtacga ggaataccaa ctcttcggcg cgcaggcgca agtcctgggt 11760
   cagccgtttg cgctcaacgg caatccttcg tcgatcgcca accgcgtctc gtattttctc 11820
   gattgccacg ggccgagcat ggcggtggac acgatgtgct cgtcgtcgct caccgcgatt 11880
   cacctcgcct gccagagcct gctccacggc gggtgcgatc tggcgatcgc cggtggggtg 11940
   aacgtctcga ttcatccgaa caaatatctg gcgctgagcc aggggcagtt cgcgtcgagc 12000
   cggggtcgct gcgagagctt cggggaaggc ggggacggct acgtgccggc cgaaggcgtc 12060
   ggggcggtgc tgctcaagcc gctgcagcgg gcgattgatg acggtgatca catctacgcc 12120
   gtgatcaagg ggacgagcat caaccacggc ggcaagacga atggctacac cgtgccgagc 12180
   cccgtggcgc aggcgcagct cgtcgcgcag gcgctcacgg aagccaagat cgagccgcgc 12240
   gcggtgagct acgtggaggc gcatgggacg gggacggagc tcggggatcc catcgagctg 12300
   gcgggcctga cgcaggcgtt tcgtcgtcgg acggaggagc gagggtactg cgcgatcggg 12360
   tctgtgaagt cgaacatcgg gcactgcgag agcgcggctg ggatagcggg tgtgacgaag 12420
   gtggtgctgc agatgaagca cggggtgctg gtcccgtcgc tgcattcgga gcggctcaac 12480
   ccgcacatcg acttcgagaa cagcccattc ttcgtgcagc gtgagctgtc ggcgtggcgg 12540
   cggccggtgg tgaaggtgga tgggaaagag cgagagcatc cgcgcatcgc cggggtctcg 12600
   tcgttcgggg cgggcggggc caatgcgcac gtggtgctcg aggagtatgt ggagcgcgcg 12660
   ggcgagcggg gtgcgctcgc gagcggagcg gggcgaggcg cggtggcggt ggtgctgtcg 12720
   gcgcggagcg cggaggcgct gcgtcggcag gcgagccggc tgagacgagc gctggagcgg 12780
   ggggagtacg gggaagggga tctggaggac atcgcgtaca cgctgcagac ggggcgagag 12840
   gcgatggagc accgtctcgg gttcatcgcc tccaacctgg acgaggtgaa gtcgaagctg 12900
   tcggcgtacg agcgaggcgc aggtgagctc gaggagttct ggcaagggga ggtgaagcga 12960
   gagagggaga tgctctcgct gctcgggtcg gacgaagagc tgcaggaggc gatcggcaag 13020
   tgggcattgc gcggcaagta cgcgcggctc ttggagctgt gggtcaaggg gcttgctttc 13080
   gactggtcgc gagtgcacgc ggggcgcagg gcgaggcgag tgtcgctgcc gacgtatccg 13140
   ttcgagaggg agcgatactg gctgccgtcg tcgaggggga ggtctggggc ggcgggagcg 13200
   ggtgcgggag cgggtgcagg gcgtattcac ccgctggtgc atgcaaacac ctcgagcctg 13260
   tcggaggagc ggttcacgtc ggagttcacg gggcaagagc cgttcttcgc ggaccacgtg 13320
   gtggacgagg agcgggtgct gcctggggtc gcgtacctgg agatggcgcg gcgtgcggtg 13380
   caggaggcgg tgggaggcga gggggaggta ggcgagcacg ggatcgagct gcatcacgtg 13440
   gtgtggtcgc agccgatcgt gcagcgaggt gcggaggccg tgcgtgtgca cgtgcaggtc 13500
   gagagcgcgg aggggtctga gccagccgag ggctggacgc tgcgctacga gatctactca 13560
   gggggagcgg gcgtgtcggg cgagcgtgtg gtgcactcgc agggctacgc gcggctggtg 13620
   gagaagcagc cagcgccgcg ggttgacgtg agcgagctgc gttcgcggtg cgagcaagag 13680
   tggagcggtg cgtcgtgtta cgcggcgttc gatgcgatgg gcctgcacta cgggccgtcg 13740
   catcgagggc tggtgagcgt gtggcgtggg gaggagacag gcggagagcg ctttgcgctg 13800
   gcgcgtgtgc agctgcctgc gagcgcgatg tccgggcggg aggagtacgt gcttcacccg 13860
   tcggtgctgg acagcgcgct gcaagcgacg atagcgctga gcgatggtca gacgctgagg 13920
   ctgccgtttg cgctggacgc ggtggaggtg ttcggagcga gcccgccgtc cgggtacgtg 13980
   gtgatccgcg aggagcgtgg aggcgagaag ctgacgctgg agctgtgcga cgacgggggt 14040
   gttgtgtgtg tgcggctggc cggcttcgcg agccgggtgc agcaccggag cggggtagag 14100
   ctcttgcagc ggcgctggcg gcgcagcccg gtggagcatt cggcgcagag cgggcatgtg 14160
   cagcgatggg tgctgctgga cagcgcctat ggctcgcagg cagcagcgct gagcgagcgg  14220
   ctgccgggag cccagatcac ggcgctgcgc gccagcggcg gcgtggcgga ggagttcgag  14280
   cagttcgtcg agcaggtgat ggcgacggtg cagacgatcc tgcgggagcg gccgcggggt  14340
   gaggtgctgc tgcagatagc gctgtcgcgg ggtgagcgag gcgagcggct cggtgcggta  14400
   tccgggctgc tgaagagcgc gagccaggaa aacccgaagc tcgtgggtca ggtgctggag  14460
   cttgaggaca gcgcgacgct gctggacaag ctcgaggaga acgcggcggc gagaggtgat  14520
   gtagagatcc gctaccaggg tggggagcgg cagacgccgg cgctggaacc tgtggtggag  14580
   tcgcgagcag cagcggccga ggtgccgtgg aagcaagacg gcgtgtacct gatcacgggg  14640
   ggagcggggg gccttgggct tctgtttgcg aaggagatca tcgaccgagc gcctggagcg  14700
   acgctgatcc tgacggggcg ctcgcggctg agcggcacga agcaagcggc ggtggagtcg  14760
   ctgcgcggtc gaggttcgcg cgtggagtac atgcagctcg acgtgagcga cggagcagcg  14820
   gtgtcgcgag cggtggagca gctgcagtcg gagcacggag ggctgaacgg gctagtgcac  14880
   gcggcggggg tgctccagga cagcttcgtg gtgaacaaga cggaggcgca gtggcgcagc  14940
   gtgctggggc cgaaggtgcg tggcctcgtg cacctggatg aggcgacgaa gcatctggcg  15000
   ctggaccaca tggtggtgtt ttcgtcggtg gcggggctcg ttgggaatgt ggggcaggcg  15060
   gactacgcgg cggcgaacgc gttcatggcg cggtttgcgg agcagcgcag cgagcttgtg  15120
   cgcgctggcc gtcgccaggg ccggacggtg tcgatcgact ggccgctgtg ggagagcggc  15180
   ggcatgcaga tggacgcctc gaccgtgcag cggctgcgtc agacgcatgg tgtgatcccg  15240
   ctcgcgaccg agcacggcat cgcggcgttc taccggacca tggcgagcgg cctcgcacat  15300
   gtcggggtct tcgcgaccga ggcgtcacga gaagcgctca cggacgcgga cgacgactcc  15360
   acgctttaca tggagctctt cgaaacgatg gtggaaggcg tcctgtcgga ggacgagttc  15420
   cgggagtggc tcgtagcatg a                                            15441
<210> 5
   <211> 11487
   <212> DNA
   <213> Sorangium cellulosum
<400> 5
   atgacgcagg ggtcgcacga cgcggatgcg cgggacgaga tcgccgcgct gttctcgctg     60
   atcaaggagc agaagctgac gcgcgcggag gcgctggacc gccttcgcgc cgtccgcgct    120
   cgcctccgct cagagcgagc cgccgcgccg gcggccgcgc aaatcgccgc gacgcccccg    180
   tcaggcggtg cagtcgagct cgtcgaggcc gaggtcgtcg attacttccg gaagcaggtc    240
   gcatcggcgc tcaagctgcc gctcgatcgg gtcgatgccg acacacctct cgagcggtac    300
   gggatcgact cggtcgtcgt catgcacctg acgagcgcgc tggagaagca cttcggcccg    360
   ctgtcgaaga ccctgttctt cgagttcgac acgatcgacg ctgtcgcgcg gcacttcctc    420
   gagaaccacc gcgccgcgct gtcccggatc ctcgggaaaa gcgctcctag cgcgctcgac    480
   cgtccggtca gcgcgcctcc gcgcagcgcg cgtcccctcg gccgtccggt tgtggccgtg    540
   cgcaaggccg tctctgccag cgtcgattcg cctgttgcag cgccgcctgt cgcagcgccg 600
   cccgttgcag cgccgcccgt tgcagcaccg cccgttgcag cgccgcctat tgcagcgccg 660
   cctattgcag cgccgcccgt tgcggcgcct cctggcgtat ccgctcgtgg cgccggcccg 720
   tatgacatcg cgatcatcgg cgtgagcggc cgttacccgc aggcgaacga tctcggggag 780
   ttctgggaca acttgaggtc gggcaaggac tgcatcaccg agatcccgcc cgaccgctgg 840
   gactaccgcg cctatttcga cgcgcagaaa gggcggatcg gaaagagcta cagcaagtgg 900
   ggcggattta tcgacggtgt ggaccggttc gatccgctct tcttccacgt ctcgccgcgc 960
   gaggcgcagc tcatcgaccc gcaggagcgg ctcttcctgc agtgcgcgta cgagacgctc 1020
   gaggatgccg gttacacgcg cgattcgctg agcgggcccg cggtgcgcgg ccgtgtgggc 1080
   gtgttcgtcg gggtgatgta cgaggaatac cagctctacg gcgtcccttc acaaggcgcc 1140
   gagcagccgc tggtgccgag cggccgccca tcgtcgatcg cgaatcgcgt ctcgttcttc 1200
   tgcaacttcc acgggccgag cgtggcggtg gacacgatgt gctcgtcggc gctcaccgcg 1260
   atccacttcg cctgccgcag catcctcgaa ggcgagtgcg atctggcgat cgccggcggg 1320
   gtgaacgtct ccgttcaccc gaacaaatac ctcgccttga gccaggggca gttcgtttcg 1380
   agcgcggggc ggtgcgagag cttcggcgag ggaggagacg gctacgttcc tagcgaaggg 1440
   gtcggcgcgg tgctgctcaa gccgctctcc cgcgccatcg ccgaccgcga tcacatctat 1500
   ggcgtgatca aggggaccag cgtcaaccac ggcggcagga cgaacggcta ctccgtcccg 1560
   aacccgaacg cgcagagcga ggtaatcacc gacgccctgc agagggcggg catcgatccg 1620
   cgcgcgatca gctacatcga ggcgcacggg acggggacga gcctcggcga cccgatcgag 1680
   atcgccggcc tgagcaaggc gttccagaag gccagcggcg aggcgctcgt gcaaacgtgc 1740
   gcgatcggct cggtgaagtc gaacatcggc catggcgaga gcgcggccgg catcgccggc 1800
   gtgacgaagg tgctcctcca gctcgagcat cgccagctcg ccccgagcct gcacgcgcgg 1860
   accttgaacc cgcacatcga tttcgaccgc actccgttcc gcgtccagca ggagctcgca 1920
   gcgtggccgc ggccgcgcct ccgcatcgac ggcgtcgagc gcgaagtccc gcggctcgcg 1980
   ggggtgtcgg cgttcggcgc gggtggctcg aacgcccacg tgatcatcga ggagtacgtc 2040
   gcgcgcggcg aggcgccggc gccgccggcc gagctaccgg tggtgatcgt cctgtcggcg 2100
   aagagcgaag cgcagctccg cgcgcaggcg cgcaacctcg tcgcggcgat ccgccgtgag 2160
   ggctacaccg aagccgatct gccgtcgatc gcgtacactc tccaggtcgg tcgcgagccg 2220
   atggaggagc gcgccgcgac gacggtggcc agcctcgcgg cgctcgtcga gcgcctcgag 2280
   atgctgtgcg ccgagccgcc cgatgaacgc gactggacaa gagggtcggt cgagacgagc 2340
   aaggcggcga tcgcgttctt caagtcggac gacgaaggcc gcgagctgct cgcgagctgg 2400
   gtcttcaggc ggcactacga ccggatcgca gggctctggg tgaagggcct ccccttcgac 2460
   tgggagaggc tctacccggc cggggggccg gggcgcgtcc ggctgccgaa gtaccccttc 2520
   gcgaacgagc gctactgggt ggagacccgg cctgctgcgc ggccctcggg cgcggcgcgc 2580
   ctgcacccgc tcctgcacga gaacgtgtcg acggcgttcg agtcgaagtt cgtctcgacg 2640
   ttcacgggcg acgagccttt cctccgcgat caccgcgtcc ggcgcgacgc ggtgctgccc 2700
   ggcgcctgcc acctcgagat ggcgctggcg gccgctaaaa ggagcctgga cacggatcgc 2760
   gcggtcacgc tcgcgggcgt cgcgtggctc cggccgctgg ccgtggccca ggggggtcgc 2820
   atggtgcaga tcgacgtgat cgccagcagc gagacggagg cggagttcga gatccacagc 2880
   ggacccggcg acgacgcgga cgtcgtgtac tgccgcggcc tcgcgcgcgt cgtcgctgcc 2940
   gcacgtccac cggcgctcga cctctcgcgc ctgcgcgagg cgtgctcgcg cgccgagctc 3000
   gcgcccgagc gcgcgtacga ggcgctcggg gcggccgggc tcgagtacgg cgccgcgcat 3060
   cgtagcatcg aggagctccg cgtcgggagc gacgccgcgg ggcgcccgca ggtgctcgcc 3120
   cggctggcga tcccggagtg cgtcgccggc gcggcggggg agttccagct ccacccgagc 3180
   atcgtcgacg gcgcgatcca ggccgcggtc ggcctcgcgc tccccgcggg aggagcgagc 3240
   gaacgagcga gccgtcccgc gcttccctca gggctcgcgg cgctgtcgct ccacggcccg 3300
   acgccgcagc gcgcgtacgc ctggctccgc ttccgcgacg ggagcagccc cgacgacgac 3360
   gtcagggcga tcgacatcag cctatgcgac gagctgggcg aggtccgcgc gcagctcgat 3420
   gggctgaccc tgcgcgtgat gagcgacgcg ccgcgcgcag agacgatgct cctcgagccg 3480
   cgatgggccg aggagcccgc gcgcggcgcg cgtgcccacg cggccatcgc cgacgaggac 3540
   cggaggctct ttgtcgtcgg cgcgctcggc tcggagcagc gagcggcgct cgagcggcag 3600
   ctgcgcggag gcgagcttca cttcgtcgcg ccgggcaccg gctcgctcgc cgagaggtac 3660
   gagcgtgttg ccgctgcgct gctcggggtc ctccgcgcgc tcctggtgcg cgcgccagcc 3720
   cggcgtcgcc tgctgcaggt cgtcgtcgcg tcgagtgacc ccgaggaagg cgcgctctac 3780
   gccggcctca gcgcgatgtt gaaatcggcg agccgggaga acccgcacct cgtcacacag 3840
   cacatcacga tcggcgagcc ggcgtcggcg aacgggtggt tcgacgtgct cgacgacgag 3900
   gcagcgcgcc tcgacgcgga cgagatccgg taccgaggcg agcgccgcga ggtgcggcgg 3960
   ctcgtggagc tcgctcgcct cgacgccagc gacggggcgg ctcccccctg gcgcgacggc 4020
   ggggtgtact ggatcaccgg cggcctgggt gggctcgggc tcgtcgtggc gacgtcgatc 4080
   gcgcgcgcga gccgcggcgc ggcgctggtc gtcacgggcc ggtcgcctgt cgtgggctcg 4140
   gatggccagt cgaagctcgc cgagctccgc gccctcggcg cgcgggtcga gtaccggagc 4200
   gtcgacgtcg ccgaccgcgc cgccgtcgag gatctcgtcc gccatgtccg ggcgacgtac 4260
   ggccagctcg acggagtcgt ccacaccgcc ggcgtcgtcg aggacagctt cctcgtgcgg 4320
   aagacagacg acgagctgcg ccgggtgttc gccgcgaagg tggcggggat cgccaacctc 4380
   gacgaggcca cacgaggcga gcccctcgac tgcttcgtct gcttctcgtc cgtcgcaggg 4440
   gcgatgggga acgttggcca gtccgactac gcggcggcga acgctttcat ggacgcctac 4500
   gcgaggcgtc gcgccgagca ggtggagcgg ggcgcccgct cgggccgaac gatctcgatc 4560
   ggctggccgc tgtgggccga gggcgggttg cacgtcgact ccgccgtcga ggccaggctc 4620
   cggcgcgatg ggctcacccc gctcggcacc gccgccgggc tcgcagcgct ggcgcgatgc 4680
   atccgcgatc ccgggcgccc gagccacgtg gtggtcctgt tcggcgacgc gcgcgcgatc 4740
   cgcggcgcgc tcctcgcccg ctccaaccgg ccgtcgcccg gcgatcttgc ggccgcatcg 4800
   ccggcgaacg cgtccactcc ggcgatccgc gaagaggcgc tgaggcaggt gaagaagctc 4860
   ctcgccccgg tgatcaagct tccggtcgag cggatcgacg cggcggcgtc gttcgagacg 4920
   tacggcatcg aatcggtgat ggcggtcgag ctcacggatc ggctcgaggc ggtgttcggc 4980
   ccgctgtcga agacgctcct gttcgaggcg aagacggtcc gcgagctggc ggactacttc 5040
   gtcgagcacc acgcggcgac gctgggctca ctgctcggcg gggcgaccgc cccggccgcg 5100
   ccggcgacgg cggtctccgc cggagcaggg ccggcgcgcc gccgtgcgcg ccgcctcccg 5160
   tcgcgcggcc gcgctggcga ggcgcgcgag gcggccattg cgatcattgg cctgagcggc 5220
   cgctacccgc aggcgaacga tctcgcggag ttctgggaga acctgaggtc gggcaaggac 5280
   tgcattaccg agatcccgcg cgaccggtgg gaccaccgcg cctatttcga cgcggagcga 5340
   gggaagcccg gaaagagcta cagcaaatgg ggtggcttcc tggaggggat cgaccagttt 5400
   gatccgctct tcttcaacat ctcgccgcgc gaagcgcagc tcatggaccc gcaggagcgg 5460
   ctctttctcc tgtgcgcgta ccagacgctc gaggacgcgg gctacacgcg cgaagcgctg 5520
   agcgggcccg cggtccgcgg ccgcgtgggc gtgttcgccg gcgtgatgta cgaggagtat 5580
   cacctgtaca gcacgccgtc ggcggaggtc gcccagccct acatccccgg cggcaccttg 5640
   tcgtcgatcg cgaaccgcgt ctcgtacttc tgcgacttcc acgggccgag cctgacgttg 5700
   agcacgatgt gctcgtcgtc gctcaccgcg atccacctcg cctgccagag cctccagcag 5760
   ggcgaatgcg agctggcgct cgcgggcggc gtgaacgcgt cgattcaccc gaacaaatac 5820
   ctcggcctca gccaggggca gttcgtctcc agcacggggc ggtgcgagag cttcggcgca 5880
   ggcggagacg gctacgtccc cggcgagggg gtcggcgcgg tgctgctcaa gccgctctcc 5940
   cgtgcgatcg ccgaccgcga ccacatctat ggcgtgatca aggggaccag catcaatcac 6000
   ggcggcaaga cgaacggcta caccgtgcca aaccccacgg cgcagaccga cctgatcctc 6060
   gcagcgatcg agaaggcgcg ggtcgatccc cgcgcgatca gctacgtcga agcgcacggg 6120
   acggggacga gcctcggcga cccgatcgag atcgccgggc tgagcaaggc gttccaacgg 6180
   gcgagcggcg aggcgatcgt gcagacgtgc gcgatcggct cggtgaagtc gaacatcggt 6240
   cactgcgaga gcgcggcggg catcgctggc gtgacgaagg tgctgctcca gctcaagcac 6300
   cgtcagctcg tcccgagcct gcactcgcga acgctgaacc cttacatcga tttcggcagc 6360
   actccgttcc gcgtgcagca agagctcgaa gcgtggcggc ggccgcgcct gcgcgtcgac 6420
   ggcgtcgagc gcgaagcccc ccggatcgcg gcgatctctg catttggggc gggcgggtcc 6480
   aacgcgcact tgatcatcga agagcacgtc gagctcgacg acgcgccgac gtccgtcagc 6540
   gacgccccgg tggtcatcgt cctttcggcg gcgaccgagg cgcagctccg cgcgcaggcg 6600
   cgcaacctcc tcgcggcgat ccggcgcgag gggtacacgc agcgcgatct gccgtcgatc 6660
   gcgtacacgc tccaggtcgg ccgcgagccg atgcaggagc ggatggcgac gacggtgacg 6720
   acgatgtcgt cgctcctcga gcgcctcgag cgcgcgtgct cggagccgcc cgacctcggt 6780
   gactgggttc gaggcgtggc ggagggccgc ggtgagctcg tgtccgtctt cgcgcaagac 6840
   gacgatttcc gcggcctcgt tcgcgcatgg ctcgagcgag ggaagctctc caggctcgtc 6900
   gagctctggg tcaaggggct cacgttcgac tgggaccagg tgccgccggc cggcgtccgg 6960
   ccgcggcgcg tgagcctgcc gccgtacccg ttcgcgatga agaggtactg gggcccgtgg 7020
   ccggcgccga gcgcggcccc tccggcgacg gcgccgagcc ggccggcttc gcctcctacc 7080
   tcgatcgcgc tagctggcgc ggacgcggcg gtccgctccg acgcggcgag cccgccgatc 7140
   gcccggagcg cgcccaagga gcgcgccgcg gagatccgag ccgacgcggc gccgatctcg 7200
   ctcgtggcgc tcgttccgcc cgctgccatg gacaccgccg cgtcctccgc ggcgcgcagc 7260
   gcgcccgtcg ccatctcgct gagcccgctc cgcgcggagc ctgcttccgc cgccgtggct 7320
   cgccgcgctt cggacgagcc gcctgcgtcc ggcggagagc cgctgaccgg tcggttgcgc 7380
   gccagccttg cgcgcgccct gtgcacggac gaagcggacc tcgatcagga cgctgtcttc 7440
   aacgaccttg ggctggactc ggtcatcggc gtagagtggg cgcggtcgat cgagcgcgag 7500
   ctcgatgtcc ccctcaccgc gtcgacgctc tacgaccacc cgacgctcac ccggctatcg 7560
   gcgcacgtgt cgggcctgat gacgtcggcc cgcgcggacc tgcgagccgc gcccgggcca 7620
   gcgcctgagg tccccgaagc gcccgcgagc ccggcgctgc ccgcgcgtgg cgcccagcat 7680
   gagatctcga tccctgaggt cgcgccgcgc ccggtggagg agctggtggc ggggctgaca 7740
   cagacgctcg ccgccgcgct gtacgcggac gcgtccgaca tcgatccgga caggctcttc 7800
   gtcgatcacg gcgtcgactc cgtcatcagc gtcgagtggg tgcgccggat caacgcgaca 7860
   tttcacctgg atctgaaggc gacggtcctg ttcgaccacc cgacgattca ccagctcgcc 7920
   gagcacctcg cgacgctcga cgcgaccgct gtgcgagccg ccgtcgacgg tatctccgcg 7980
   gcggagcgcg ccggcgcgca cgagagcacg gcgcggcgtg aacgcgaagg cgcagccgag 8040
   cgcgaggccg cggcgcggcg tgcgcgcgaa gacgccgcag agcgccaggt cgcggcgcgc 8100
   catgagcccg aggccagctt cgaggtcgaa gcgcgccgcg cgtccgacgc ggtcgcgtgc 8160
   gtcgcagttc gcgagcgcgt catcgcggcg ggaccgggcg aggccgcgtg caggatcgcg 8220
   gtgatcggcg cgtcggggcg gtacccgaga gccggagatc tcgacgaata ctggagcaac 8280
   ctcgccaggg gcgtcgacgc cgtggcagag gtgcccgcct cgcgatggag cgtggagcgc 8340
   tactacgacc ccgccgtgcc ctccacgggg aaggtgtact gcaagtggct cgcggcgctc 8400
   gaggacgtcg accgcttcga tccgctgttc ttcaacatca gcccggcgga ggccgatctg 8460
   atggacccgc agcaccgcct gttcctcgaa gagagctaca aagccttcga ggatgcgggg 8520
   tattcgccgt cgcgcttgga cggcgcgcgc tgcggtgtct acctcggcat catggggagc 8580
   gagtacacgc agatcgtcgc gcagagcgga cgcaggagcg cgaccgcgaa cagcgccgcg 8640
   atcggcgccg ggagggtcgc atatcacctg aacctgaagg gacctgcgat tcctgtcgat 8700
   accgcgtgct cgtcgtcgct cgtcgcgatt cacctggccg tccaggcgct gcgggcgggg 8760
   gagatagaga tggcgctcgc aggcggggtc accctctacc tgactccgga cgcctatatc 8820
   gcgatgtgcg aggccggaat gctgtcgccc gacggacgtt gcaagacgtt cgacgaggcg 8880
   gcgaacggct tcgttccagg cgagggcgtc ggcgtcgtcg tgctcaagcg ccttccggac 8940
   gccgagcgcg acggcgactt cattcacggc gtcatcatcg gctctggcgt caaccaggac 9000
   ggcaagacga acggcatcac cgcaccgagc gtgcagagcc agactgcgct cctgaaagac 9060
   gtctacgcgc gcttcgggat cgatccccgg accatttcgt acgtggagac ccacggcacc 9120
   ggcacgaagc tcggcgatcc gatcgagctc gcggcgctgt cggccgcctt cggagcgcgc 9180
   gacgggcgcc gttgtgcgct cggctcggtg aagagcaacg tggggcacac caccgccgcg 9240
   gcaggcgtcg ccggtgtcca caaggtgctc gcctgcatgc agcacgggcg gctcgtcccc 9300
   acgctgcact tcacccgacc caacagccat ttctcgttcg aggattcgcc attttacgtc 9360
   aacaccgagg tgaggccctg ggagcgggac ggtgcttccc cgcggcgcgc ggcggtcagc 9420
   tcgttcggct tcagcgggac gaacgcgcac ctcgtgattg aggagtacgt cgccccgacg 9480
   cggcggcccg ggcgacgcgt cacgccgagc aaccccgcgc tcgtcgtcct ctcggccaag 9540
   accgaggcgc agctccagac gctcgcgggc cggcttcgag atgcgctctc gtcgccgcgt 9600
   tggaccgacg atgacctcct ggatatcgcg tacacgcttc gcgtggggcg cgcgcacctc 9660
   gaggagcggc tcggtatcgc cgccagctcg ctggacgcgc tgcgcagcgg cctccgcgcg 9720
   ttcctcgacg gcggcccgct ctcgccgaac ctggcgcgcg ggcgggcgaa gaacgcacgt 9780
   ccggtggggg cggcgcggcg ggagacagcg ggcggcgcca caccgacgga gcttctcgcg 9840
   cgctgggtcg acggcggcgc ctgcgcgtgg agcgagccgt acggcgacgg agccacgccg 9900
   cagcgtgtgc ggctgccgac ctatccgctc tcgaggcaga ggtgctgggt cgacagcggc 9960
   gccgcgccgg aggtggcgcc gccggtgccg ggccgcgtaa cgccgctcga gatgctcgcg 10020
   ccgcgctggg agatcgtgga gcaggcgccg gctcgaggcg ccgggctcgg accggagcgt 10080
   gggatcgccg tcctcggcgc ccggcccggc ccgctccgag atgcgctcgc ggcgcgcgcg 10140
   ccgcgcgccc gcttcgtgga cgcgccgtgc gacgcgtcga tcgatgagct cgtcgaggcg 10200
   ctgcgcgcgg ccccgatcga cacggtgatc tggctgctcc cggaggagag cgcgagctcc 10260
   gtcgcggcgg aggagcacat cgccgcgcag tccgcggggg cgctcttcgg gttccgatgg 10320
   atcaaggcgc tcctcgcgct ggggtacggc gggagaccgc tccggatcga cgtcgtgacc 10380
   tggcagacgc aaggcgtcca cggcgagctc gtgtacccca cgcacgcgag cgtgcacggc 10440
   ctgatcgggt cgctcgccaa ggagctgcgg agctggtcgg tccggctcgt ggaccttccg 10500
   ctcgccgtgg accgggcggc ccgcatcctc atcgactcac tgcacctcga ggcggacccc 10560
   gacggcgacg cgtccgcgtg gcgcgacggc cgatggtacc ggcagaggct cttgccgttc 10620
   gaggtccccg ccgagcaggc gtcggccgcc ttccgtcgcg gcggcgtcta cgtggtgatc 10680
   ggcggggccg gcgggctcgg ggaggcgctg accgagcacc tcgtccgcga gtaccacgcg 10740
   aacgtggtgt ggctcggccg ccgcgccctc gacgccacga tcgacgccaa ggcggatcgc 10800
   atctcccggc tcggcccccg gccggtgtac atccaggccg acgcggccga tgcgtcggcc 10860
   ctcgcgcgcg cccgccgcga cgtgctgtcc cgcttcgacc ggatccacgg cgtcgtgcac 10920
   ggtccgctcg tcctgagcga tcggagcctc gcccgcatgg aagagcggca gttcgaggcg 10980
   gggctccgcg cgaaggtcga taccgcggtg cggctcgcgc agacgttcgg cgaggacgac 11040
   ctcgacttcg tcgccttctt ttcctcgatg cagacgttcg tcaaggcgcc ggggcagagc 11100
   aactacgcgg ctggctgcac gttcagcgac gccttcgcgc agcgcctggg ccaggcgtgg 11160
   aagtgcccgg tcaaggtcgt gaactggggc tactggggca gcaccggcag cgtcgcggac 11220
   gagggacatc ggagacgcat ggcgggcgcg gggatcgggt cgatcgaacc tgacgagggg 11280
   atggacgcgg tggagacgct gctgcggctg cccgtccggc agctcgcggt cgtgaagtgg 11340
   atcggggcga cggagcgctc ggcggccgcc gccgtggaga cgacgccggc gccgcaggag 11400
   gggccgacga tgagagcgat gggggtcgtg gagacgacgc tggcggcgca gacggcgccc 11460
   gccatggagg caatgtgggc cgaataa 11487
<210> 6
   <211> 10887
   <212> DNA
   <213> Sorangium cellulosum
<400> 6
   gtgctcgcgc cgccgctccc cagctcggag caggacgcgg cgtacgagga gatcacgacg      60
   ctgctgctcg cgatcgtgtg gaggcagctc gacaggaccg ggctgcggct caccggcgac     120
   ggctggcgcg agtcggcgcg cgcccgcccg ctgtacgcgc gctggctcga tgagaccgag     180
   cgcctcctcc gtcgccacgg atacccggcc gcgggagcgg ccgtcggcga cctcgacgcg     240
   ctctgggcgc gctgggagcg atatcgcacg gaggggcgcg tcgagcaggc ggggctggtc     300
   aagcaagcgg acctggtcca ggcgacgctc gaggcattgc cggacatcct caccgggcgg     360
   cgccgcgcga cggatgtcct cttccccggc gcgtcgatgt cgagggtcga ggcggtctac     420
   aagaacaacc cgcaggccga ctatttcaat ggcgtgctcg cgcagcgcgt cgcgtcgctg     480
   atcgaggcgc ggcgggcgta cggccacaca gagccgctgc atatcctgga gatcggcgcg     540
   ggaaccggcg gcaccagcga ggtcgtcttc gaggcgctca gaccgctcca ggacctcgtc     600
   gggacgtaca cctacacgga cgtctcgaag gcgttcctcc tgcacgccga ggatgcctat     660
   ggcgcgggcc ggccctacct ccggtatcgg ctgctcgaca tccagcagcc gctcgaggcc     720
   caggggctct cgcttgggac ctacgatatc gtcattgcgg ccaacgtcct gcacgcgacg     780
   acagacatcc ggacgacgct gcgccacacg aaggcggcgc tcaagcgcaa cggcgcgctg     840
   ctgatcaacg agatcgtggc aaagagcctg ttcgcccacc tgaccttcgg gctcaccgag     900
   gggtggtggc tggtcgagga tccggagctg cggatcgagg ggtgcccggc gctgtcgatc     960
   gacggctgga acgcgctctt gaccgaggaa gggttcgatc gcgtccgctg gccggccgcc    1020
   gaagcgtcgg cgctgggcca gcagatcatc atggcgcgga gtgatggggt gatccgccca    1080
   gcgtcggcgc acgcgaagaa gatcgcgccg agcacggagg cgcgccgccc cgaggcgcag    1140
   gcgccggtgc cgcgggagcc ggtcaatgtg gacgacgacg cgctggccga tgaggttcgg    1200
   cgagcgatcg ccgcgcagct ttcgatcaac ctgcgcgtgc cgatctccca gattggatat    1260
   cacgatgcgt tcggcgatta cggggtcgac tcgatctcgg gcgtgcggct gatccagcag 1320
   ctcaacggcg ctttcggcat ccgcctcgag acgacgcacc tctacgattt cagctcgatc 1380
   cagcgcctag ccgagcacat gctctcggcg tggggccccg agctccgcgc gcggcgggcc 1440
   acgaacgacg gcggccgggc cgctgcgagc gagccgcggc gcgcgtcctc gatgaacggc 1500
   ggcgctgcca tcgcagcgtt cgcgagcgcg cccgacgcgt ccgggagcgc ggcgagccac 1560
   gcgcgcgagc ccgccgccgc gtcgacggtg tcctcgcagg gccacgcgcc gtcaccggag 1620
   cccgcgtacc ggcgcgagcc gatcgcagtc atcggcatga gcggccgctt cgccgaggcg 1680
   gatgatctcg acgcgctgtg gaggcacctc gcggccggcg ccgatctcgt ccgacccgtc 1740
   acgcgctggc ggctgccgga gcagaccgcc actggagagc ctttctgccg ccacggcggg 1800
   ctcttgaacg gcatcgaccg attcgatccg ctgttcttca atatctcggt cactgaagcg 1860
   acgacgatgg atccgcagca gcgcttcttc ctcgaggagg cgtggaaggc cctcgaagac 1920
   ggcggctatg cgggagacgg cctgagcggg agccgctgcg gggtctacgt cggctacaac 1980
   gtgacggact accagacgct gctcggtgag gacgcgccgc cgcaggcgat gtggggcaac 2040
   gcagggtcaa tcctgtcggc gcggatctcg tactatctga acctgcaagg gccggcgatc 2100
   acggtcgata cggcctgctc cagcgcgctc gtcgcgaccc acctcgcctg tcaggggctc 2160
   tggaaccacg agacggagat ggcgctcgcg ggcggcgcgt tcctccagac gacgcccggg 2220
   ttctacctcc tcgcggggcg tgcggggatg ctgtcgccct ccggccgctg ctacacgttc 2280
   gacgcgaggg ccgacggctt cgtccccggc gagggcgtgg gcgtcgtcct cctcaagcgg 2340
   ctgtgcgacg cgatcgccga cggcgatcac atccacggcg tgatctgcgg ctccggcgtc 2400
   aaccaggacg gcaacacgaa cgggatcacc gcgccgagcg ccctgtccca ggagcggctc 2460
   gagcggcagg tgtacgacac gttcggcatc cacgccgaga gcatccagct cgtcgaggcg 2520
   cacggcaccg gcacgaagct cggcgacccg gtcgagttcc gcgcgctgac gagcgcgttc 2580
   cgcaaagaca cgtcgaggac gcgcttctgc gccgtcgact cgatcaagac gaacatcggc 2640
   cacacggcgg ctgcggcagg gatcgccggg ctgctcaagg tgctcctcgc gctccggcat 2700
   cggcagctgc cgcccgccgt ccactacgcc cagccgaacc ccgcgatcga tttcgaggcg 2760
   agcccctttt acgtgaacac gacgctcacc gactggggct gcgacgcggg caagaagcgc 2820
   cgcgcggccg tcagctcgtt cggcttcagc ggcacgaacg cgcacctcgt cgtcgaggag 2880
   gccgacccgg tgcgccgccc ggagcgcgtc gagcgtcccg gccacctgct ggtgctctct 2940
   gcgcgcacgc aggagcagct ccggacccag gtgcgcaacc tgctcgacca cgtggcggcg 3000
   cgcccgcacg cggacctggg cgacgtgtgt tttacgctga tggtcggtcg gaagcgcctc 3060
   gcgcaccgcc tggcgtgcgt ctcgcggagc ctcgacgagc tcgtcggcgc cctgggccag 3120
   tggatcgaga gcgagagcgc gccgggcgtc ctggcggggc acttcaagga caacgagagc 3180
   cgggagcgcc cctcgctcag gcggtatggc aatcagtgca tcctcgagtg ccgccggggc 3240
   gaggtcgcgg ggcgcgacta catcgagcgg ctcgaggtcg tcggcgacct gttcgtccag 3300
   gggtacgcgc tcgactacgc ggacctcttc gcacccggcg cctaccggcg cgtgccgctc 3360
   ccgacatacc cgttcgctcg cgaggcgtac tgggtcaagg cgcgcggccg ggcaggcgcg 3420
   gcgtcggcgc tggagggggc cacccaggcg gagcgcgcga cgacgctcct cgcgctcccc 3480
   gcctggaggg cgcagcccgc tgccggggac ggcgcgcgca tgcgcgacgt cgacttcgcg 3540
   gcgcaccggg tgctcatcgc gggcgagctc ccggaggccg ctctccgtgc gctccgcgag 3600
   caactaccgc gcgcgcgctg cgaggtcgtg gcgccgcggg agggctcgct cgcgcagcgg 3660
   tacgagcgcg cggccgtggc ggccctccgg agcgtgcggc tcgcgctcga ggagggcgga 3720
   gggcgatcgt cgctcgtgca gctcgtcgtc gcgaccggcg gtgatcgccg cgaggcgtgc 3780
   ttcgccgggc tctcggggct gctgaagacg gcgagccgcg agaacccgcg gctcgcgacg 3840
   cagacgctcg agctcggcga tgcaggcgcg gtcgacgtgc tcccgcgcgc gctccttgag 3900
   gacgcgcgcg acgtcgacga gccggagctc ctctaccgcg acggccgccg gtgggtgcgg 3960
   cggctcgagc gggccgcgcg tctcgacgat cccaggacga tccaggcgcc ttggcgggac 4020
   ggcggcgtct acctgatcac cggcggcctc ggcggcctcg gctacctctt cgccacggcc 4080
   atcgcggaga gcacgcgagg ttccacgctc gtcctcgtcc accgatccgc gccgagcggc 4140
   gagcagcgag cccgcctcga cgcgctgcgg tcgctcggcg cgcgcgtgga cgaccagcga 4200
   cgcgacgtgg gcgacgcgcg ggacgtcgac gggctcatcg agcacgtacg ggcgagccac 4260
   ggcgcgctca cgggcgtcct ccacgcggcc gggacgacgc gcgacgcgct cttgccgaga 4320
   aagacgccgg aggaggtgaa cgacgtcctc cgcccgaagg tcgccggcct ggtccacctc 4380
   gacgaggcga cgcgcgacct tccgctcgac cacttcgtgt gcttctccgc gttcgccggt 4440
   gtgtacggca atccggggca agccgactac gccgcggcga acgccttcat ggacgcctat 4500
   gtcgagtatc gccgcgggct cgtcgaggag gggcgccgac acgggacgag cctcgcgatc 4560
   gcctggccgc tgtgggccga cggcgggatg aagatgaccg acgccggcaa ggcgaggatg 4620
   cacgccgctg gcctccggga gctggatacg cgcgacggcc tcgcggcttg ttccgcggcc 4680
   gtcgcgcgga agagcccgcg cgtcgtcgtg ctgaccggcg acgagggcaa gctcgaggcg 4740
   ttcttcgagc cctcctcgcc gcgcgcgccg cgccgacccg agccgaccgg cggtgtcgcc 4800
   agggaaggcg cgtcgctcct tcatgcgacg ctggcgcgcc tcaaggagct cctggccgtc 4860
   gtcacgacga tcgaggcgga gcgcatcgac ggcgccgagc cgttcgaggc gtacggcctc 4920
   gactcgatca tgatcaccca gctcaatcag cggctggagg cgatcttcgg cgcgatctcg 4980
   aagacgctct tcttcgagca ccggacgctc gagggcctgg cgcagcacct cgccgaggag 5040
   caccgcgagg cgtgtcagcg ctgggcggga ggcccgggcg acggcgcgcc ggacgatgtc 5100
   gcccgcgctg agccgcgggg gccgtcgagc gccgcgcagg aggcccgatc cggcgagcgc 5160
   gcgccggcgg aggcggcgct gcctgcgcag gagctcgacg tggcgatcat cgggctggcc 5220
   ggccgctatc cgcacgcgcg caccctcgac gagttctggg agaacctgcg cgcggggaag 5280
   gactgcatta cggaggtccc cccggaccgc tggccgatcg agggtttcta tgacggcgcg 5340
   gccgagtcgt cgccggggaa cgggaagagc tacagcaagt ggggcggctt cctcgaggga 5400
   ttcgcggatt tcgatccgct cttcttcggg atctctccgg gcgaagcgga gagcctggat 5460
   ccccaggagc gtagcttcct cgagatctgc tgggaggtca tcgaggacgc cggctatacg 5520
   cggcgcacgc tgcagtcgcg gcacggcggc cgcgtgggcg tgtatgcggg gatcacgaag 5580
   accgggttcg acctctacgg gcctgcgctc tgggcgacgg gaaaggacgc ctatccgcac 5640
   acgtccttca gctccctcgc gaaccgcgtg tcgtacctct tcgatctccg cggcccgagc 5700
   atcccggtcg acaccatgtg ctcggcgtcg ctgacggcga tccacgaggc gtgcgagcac 5760
   ctgaagcgcg gcgagtgcga gctcgcgata gcgggcggcg tcaatctcta cctgcacccg 5820
   tccaactacg tgatcctctg cagtcaccgc atgctgtcgc ctgacggcaa gtgcaggagt 5880
   ttcggcgcgg gggccaacgg gtacgtgccc ggggaggggg tgggcgctgt gctgctcaag 5940
   ctccttcggc gggccgaggc ggacggcgat cacatctacg gtgtcatcaa ggggacagcg 6000
   atcaaccacg gcgggcggac acacgggtac accgtgccca acccgaatgc ccaggcggag 6060
   gtggtcgcga tggcgctggc ccgcgcccgg atcgacgcgc gcgcggtcag ctatatcgag 6120
   gcgcacggga ccggcacgga gctcggcgat cccatcgaga tcacgggcct caccaaggca 6180
   ttccgcgcga gcgccgcggg gccgctcacg cgccggtgcg cgatcggctc gctcaagagc 6240
   aacatcggcc atgccgagag cgccgcgggc atcgccggcg tgacgaaggt cctcttgcag 6300
   atgaagcacg gccagctcgt gccgagcttg cacgcgcgga cgccgaaccc gaacatcgac 6360
   ttcgatcgca cgcccttcgt ggtgcagcag gagctcgaga gctgggggcg tctcacccag 6420
   gtcgtcgacg gccgcaccga ggagctcccg cggatcgcgg ggatctcgtc gttcggcgcg 6480
   ggcggcgcga acgcacacgt cgtgatccag gagtacgccc cggcgcccga agagccgtcc 6540
   gccgatgggg agcagccccc ggcgatcatc gtcctgtcgg cgcgtaccga ggcgcagctc 6600
   gcggagcagg cgcggaacct cctgcgcgcg atccgggttc agcgccgcac cgacggcgat 6660
   ctggcgagga tcgcctggac gctgcagacg gggcgcgagg cgatggagga gcgcctcgcg 6720
   tgcgtggcat cgagcctcga tgagctggtc gcgacgctgg gagcgttcgt cgcgtcgcct 6780
   tcaaccgcgc cggggatcta tcgaggttcg gcgcggaagc gaaaggacgt atcggcgttc 6840
   gcggcgaacg aggagctacg cgaggtcgta tcgaggtgga tcgagcggcg ccgctacgcg 6900
   cagctcctcg agctgtgggt cagcgggctg gaggtggact gggagcagct gtacggcgag 6960
   cgcaagccgc ggcggatcag cctgccgacg tatccgttcg agcgggagcg ttactggctg 7020
   ccgcagggcg agggcgcggt cgttgcggga gctgcgtcgc cggtcgaggc gtcgggagca 7080
   gcggcgggcg gcggacggct ggagcagggg cccggcccgg ccgaggtgtc gcgtgtggag 7140
   gcgggcgagg ctgcggaggt ggtggagtac gaggaggcct gggagcggct ggggtgggaa 7200
   gagcgagagc ggcaccagga aggcgtgcgc tgggtggtgg tcggcggaga cgaggcgtgg 7260
   cgggcgtcgg tggtggaggc gctgaggagg gaggagcccg ggagcgaggc gctcggggtg 7320
   agcgtgggcg gcgcgtacgg ctgggaagga agcgcgcggt gcacggtgcc cgaggcgagc 7380
   gaggtctggt tccggtcggt gttcgagcag ctcggggcgc gggacggggc gcatgggggt 7440
   gggggccggc cgtgcggcgt ggtgtacgcg tggggcaggg ggctggagcg gctgcgcggc 7500
   gtgcacgcgc tggtgcgagg cgcgaagggg agcggagcgt tgctgcgccg tctcgtgctg 7560
   acgggtcgtc aggtccgggg cgaggtgagc gggtgctacg acgcggcgct gatcggggtc 7620
   gagcggtcgc tgggcctcgt tttgccggag ctgagcgtcg gtgtggtgct ggggggcgcg 7680
   gaggggatag aggcgggcgc gctggcgcgg gaggtgtgga gcgggggagt gatccgtcac 7740
   gagggaggag agcgtcagcg gctcggtgtc cgtcgagcgt tgcccagcgg gaggagcgag 7800
   gcgccgctgc gggacggagg cgtgtacgtg atcaccggtg gagccggggg gcttgggcgg 7860
   ctgttcgcgg agcacttggc gcggcgctgc gcgggtcggc ttgcgctggt gggtcggcgt 7920
   ggacgtgacg agcagatcga gggtgtgctg tcgcggcttc ggggcgctgg agcgagggaa 7980
   gcggagtact acgaggcgga cgtttcggac gcggcgcaga tgcggtgcgt ggtggaggcg 8040
   ctggagtcga ggtggggttc aatccacggc gttctgcacg cggcaggagt ggagtcgggt 8100
   ggagcgttgt tggacaagag gtgggaagag gtcgaggaga taacgcggcc gaagatggag 8160
   ggcacgcgtg tggtggatga ggtgacggcg gggcatccgc tggacttcat gtgcctgttc 8220
   tcatcgacgg cggcggtgct gggggacgga gggtcgtgcg actacgcgag cgcgaaccgg 8280
   ttccaggtgg cgtacgcgca gatgcggcaa gagctgagag agagagggga gcggagcgga 8340
   gcgacggtgg cggtgtgctg gccgctgtgg cgtgcgggtg gaatgggcac ggccgagcga 8400
   gaaggcatgg cggtgtactt gcggagcagc ggtcagcgtt acctggagag cgatgagggg 8460
   ctgggggcgt gggagcgcgc gatgggcgcg gccgtcagcc cgcggctcgt gttcgcgggg 8520
   cagccgcgcc gcgtgcacgc cttcctctcg cgcatctacg ccggcgcgcg cgcagacggc 8580
   gcgtccacgg acggcgagct cacccgcggc gcgccgccgc gcagggatct cccggcccgg 8640
   cctgccggcg cgccgtcgac gtccaccgcg gagcgcgtcc tgcgcgatct gaagtcgtgc 8700
   gtcgaggcgg tgttgaaggt ccccgcgtcg cggctgagcg aggacacgag cttcgccgat 8760
   attggcttcg actcgatcgg gctcacgcgc gtcgcgcgcc ttctttcgaa gcatttcggc 8820
   ttcgagatcg tgccgagcgt cttcttcagc tacacgacgc tcgagaagtt ctgtggtcat 8880
   ctgctcgagc agcaccgcga cgcgctggag cgcctctatg aatccgcact cccgcccgac 8940
   ccgggcgccg cccgcgcgcc ggcgcgcgac gcccacggga gctcggacga ttcggcggcc 9000
   gtcgccgccg agcccggccg cgagccagaa gcgcagcgcc tccacggcgc gcgcgcagcc 9060
   tcagccagcc ggccggcgct cgcgccgtcg tcgccggacg agcccgtggc gatcatcggg 9120
   atgtcggcgc gcgccgcggg ggccggcgac gtcgacgagc tgtggcagct cctgatcgag 9180
   ggccgcacgc atatcgaaga catcccgggt gatcgctggg acgctcgccc gtactacgcg 9240
   ggccccggcg cgccgggcaa ccggatcgtc acgaacaagg ggggcttcat ccgggggctc 9300
   gaccgcttcg acccgctctt cttcgagatc tccccgcgcg aggcgcagtc gatggatccg 9360
   cgccagcggc tgatgctcca ggaggcgtgg cgcgcgttcg aggacgcagg ctacgccggc 9420
   gacgcgctgc gcggcacgcg cgccggcgtc ttcgtcggcg tggaggaggg ggtgagcgcg 9480
   caggacggca cggaggggct cgcgacgagc caccacaacg ccatcctcgc cgcgcgcatc 9540
   tcctacgtcc tcgacctgcg cggcccgaac ctggcgatca acacggcttg ctcctccggg 9600
   ctcgtcgcgc tgcacaccgc gtgtcagagc gttcgacgcg gcgagtgctc gatggcgctc 9660
   gtcggcgcgg tgaacgtcct cgcctcgccg ctgatgtacg tcgcgctcag ccaaggcggg 9720
   atgctctccg ccgacggcga gtgctacgcg ttcgaccagc gcgccaacgg gctcgtgccc 9780
   ggcgaggccg ccgttgccgt ggtcgtgaag cggctctctc aggccatcac ggacggcgac 9840
   acggtcctcg gcgtgatcag ggccagcggc gtgaactacg acggcaggac caacggcctc 9900
   accgcgccga gcggcctcgc ccaggcgtcg ctcctcgagg agatgtacca ggaccacgcg 9960
   atcgacgtcg aggacatcac ctacatcatc gcgcacggca cggggaccaa gctcggcgac 10020
   cccgtggagg tgaacagctt gatggatgtc ttcggccggc gcaccagcgc ccggggcttc 10080
   tgcgcggtgg gatcggtcaa gcccaacttc ggccacacgt tcgccgcctc cggcctcgtg 10140
   agcctcgtcg cgatgctcat ggcgatgcgg caccggcaga tcccaccgtc ggccaaccac 10200
   gcgcagaaca acgagttcat cgacttcgcg aagggggcct tctacgtcaa cgaccgtccg 10260
   cgcgcgtggc tggcgcgcgg aggggcgaag cggctcggcg cgatcagcgc cttcgggatg 10320
   agcggcacga acgcccatgt cgtgatcgag gagcacgtgc ccgaggcggc cgccgccgga 10380
   cacgcggccc tgcccgccgg ggccgtcgcg atcgtcccgc tctccgcgcg gagggtcgac 10440
   gacctcgttg cgtacgcgga gagcctgcgg gcgttcctgg ccgacggcag gcgcgccggc 10500
   gcggaggcgc ccgacctcgc gagcgtcgct tacacgctgc aaacagggcg cgaggcgatg 10560
   cggcacaggg cggtcttctg cgtccgcgat ctcgacgagc tcgagagcaa gctccgcgcg 10620
   tatgcggccg cggggcccgg gcgcggcgcg gacgatcgcg acgcggacga gctggccgcc 10680
   cggtgggcgc gcggggaggc ggtcgactgg cgcgcgatgg ttccaggcgc gcggccggtg 10740
   aagacgcggc tcccgaccta tcccttccac gccgcggccc gcctctcgtc cgcgaacggc 10800
   tcgcgcgagc tgcggccgcc tgccgagcgc gccgcgccga cgccacgcgc tctccacccc 10860
   ctggttcacc ggatcgtgtc ggtatga 10887
<400> 7
<210> 8
   <211> 10737
   <212> DNA
   <213> Sorangium cellulosum
<400> 8
<210> 9
   <211> 17922
   <212> DNA
   <213> Sorangium cellulosum
<400> 9
<210> 10
   <211> 1377
   <212> DNA
   <213> Sorangium cellulosum
<400> 10
   atgaaggacc tgacacaggg ctcgatagcc cgtcacatcg tgacgatggc ggtgcccatc 60
   gcggtgggga tgatcttcca gacgctctac tacatgatcg atctgtattt cgtcggggag 120
   ctcggcgatg tgccgctcgc cggcgtcagc gcggcgggta acgcgacgtt cctcgtgctc 180
   gccctgacac aggtcctcgg cgtcggcacg gtggcaatga tatcgcaggc cgtcggtcgg 240
   aaggaccgcg acgaggccaa cgtcgtcttc aatcagtcgc tcaccctggc cgcggtcagc 300
   ggcctggccg tgctcgtcct agggtacctg acgacgacgc cttacatgcg ctcggtcgcc 360
   gccgacgccg cgacggtcga ggccggacgc acgtacctgt tctggttcat gcccggcctt 420
   gcgctccagt tcgcgctcgt cgccatgggt tcagcgctcc gcggcaccgg tctcgtccag 480
   ccgacgctcg tcgttcagct cctcacgatc gtcctcaacg ccatcctcgc gccgatcctc 540
   atcgcggggt gggggaccca ccgcccgatg ggggtcgccg gggcgggtct cgcgagcacg 600
   ctctcgctcg tcgtcgcggt catcgcgctg tgggcctatt tccggcggct cgagcgctac 660
   gtcggcgtgt ccgcgccgct cctccggccg cgcctgccca tctggcggca gctcctcgcg 720
   atcgggctcc cgtcgggagg cgagttcgcg ctcatcttcg tgtactccgc gatcgtctac 780
   tggacgctcc gcgagttcgg ctcggcggcg caagccggct acggagccgg ctcgcgcctc 840
   atccaggggc tcatcttgcc ggcgatggcg gtctccttcg cggcggggcc catcgccggc 900
   cagaacttcg gcgcgaagct ggccgaccgc gtgcggagca cgttcagcat ctcggcggcg 960
   atcatcagcg cgctgatggt ccttctgacg ctgttcgtcg gctggcgcgc cgaggccctg 1020
   atcggcagct tcgccaagaa cgcggactcc atcgccgtgg gcgcgcagtt cctccgcttc 1080
   gcgtgctgga atttcgtcgc gcaggggctc atcttcacgt gttcgagcat cttccagggg 1140
   ttgggcaaca ccacaccgtc gttgctcagc tccggcgcgc gactggtgac gttcgccgtg 1200
   cccgcgatct ggctgtccac gctgcccggc ttccgcatcg agctcgtctg gtacctgtcg 1260
   atcgcgagca cgacgctcca ggcgatcgcc agcgttctct tgctgcgcgc cgagttcaag 1320
   cagcgcttcg tccccgggtt tgtcgccagc ggcgccggtc tcggcgcgaa ttcttga 1377
<210> 11
<211> 2079
   <212> DNA
   <213> Sorangium cellulosum
<400> 11
   atgttctccg gtcagggctc gcattacttt cgaatgggcg aggggctcta cgcccggcac 60
   ccgacgttcc gtcgctggat ggaccgcctg gacgtcctgg tgcgcgatct cgcggggagc 120
   tcggtcgtcg aggcgctcta ccaggagggc cgcggcaagg gggacgtgtt cgatcggacg 180
   ctgctcacgc acccggcgct cctcatgatg gagtacgcgc tcgcggagtc gctgatcgag 240
   gccggcgtcg tgccggacgt cgtgatcggc gccagcctgg ggaccgtcgc cgccgcctgc 300
   gtggccggct gcctcgacgt cgaggcagcc ctggagatga cgatccggca cgcgcggtcc 360
   ctggaggcct gctgcgagcg aggggccatg atcgcggtgc tcgggccgac gagcctgtac 420
   gacgacgccg gcttgagccg tcacggcgag ctggcggcgg tcaatttcga cgcgcatttc 480
   gtcgtggccg ccacgatgca gggagcagcg gagatcgaga agatcctgcg cgtgaaggac 540
   atcgtcttcc agcgtttggc ggtctcgttc gccttccact cgcggtggct cgacgccgcg 600
   gaggcgctct tcaaggagcg atcgagatcg atgcgcgccc gtccggcgcg cctgccgctg 660
   gtctgctgcg cgcgcacgag ggcgatcgac acgttcacgg ccgaccatgc gtggtccgtg 720
   gcccgtgagc cgatccgctt cttcgagacg atgaagcgcc tcgaggagag cggaccctat 780
   cgctacgtgg acgttggtcc gtcgggcacg ctggcgacgt tcatgaagta catcctctcg 840
   aaggggggct cccgatccac gcatcacacg gtgctgtccc cgttcggccg cgatttcgag 900
   cgtttcaccg ctgccgccac cgagctcggc gcgcgccccc cgcgtcccgg cacggacgag 960
   cgcgccgagg cgccgtcgcc ttcgggcgcc gccgcggacg cgcgtgccgc cgggccgtcg 1020
   cctcggggct tcgaggcggc ctcgcgccct gcggcgcctg cgggtgcgag ctcgagcgat 1080
   cgcgccgtgg gttctgagcc atccggcgcg ccggcgcgcg ccccgagcga cgggccgcgg 1140
   ccgctccgcg cggaggcgag cggcgcggcc gccacgaacg gcgtgcacgc tggcccgcgg 1200
   gcgacggtcg ccttcatgtt ccctgggcaa gggtcgcaga agcgcggaat gggcgcgggg 1260
   ctcttcgact cggtgcccga gtacgcggcg gtcgagcggg aggtcgacgc gttgctcggc 1320
   tactccatgc gcgcgctctg ccacgaggat cccgacgggc gcctgaagga gacacagtac 1380
   acgcagcccg cgctctacgt cgtgaacgcg ctccactact acgacgcgat cgcgcgaggg 1440
   cagcggccgg actacgtggc cggccacagc ctcggcgagt acaacgccct gctcgcggcg 1500
   ggcgccttcg acctcttgac cgggctgcgc ctcgtgaaga agcgcggcga gctgatggcg 1560
   gcgtccgtga gcggaggcat ggccgccgtg atcgggatgg acgaaggtcg catcaagcag 1620
   gtcctcacgg agaacgggct gggtacgatc gacgtcgcga acttcaactc tccctcgcag 1680
   atcgtcatct ctggcccagt cgccgacatc gcccgaggcc acacggtgtt cgaggacgct 1740
   ggcgcgcgca cgtacgtgat attgccggtc agcgccgcat ttcactcgcg ctacgtcgag 1800
   gagacggggc gcgccttcgc cgacttcatc gctccgatga ggttcgaggc actccggatc 1860
   ccggtcatca gcaatgtctc cgcgaggccc tacgaggcga aggacccatc agcgaccatc 1920
   aagtcgctgc tcgtccagca gatcacccgt cccgtgcagt gggtgcagag cgtgagcttc 1980
   ctgatggcgc agggggtgaa agagttcagg gagattggcc cggggaacgt gctgacgcgc 2040
   ctcgtccagc agatcgagcg gcagccatcg ggggcgtga 2079
<210> 12
   <211> 1044
   <212> DNA
   <213> Sorangium cellulosum
<400> 12
   gtgagcgggc ggcgacgatc cggagacgcc ctgcccgttc cacctggccg acgggaggac 60
   cgtcgccaga ggcatctacc actgcaagct ggagttcgga gaggatggat acgtcgaagt 120
   cgcggactcc atcacggttc atcgctgctt cgggaaccgc ttcggcaagc tcgacaaggc 180
   ttctcgcact gggaccggtg gctcctgcgg ctcgcgctgg cggagccgcg cggtctcgat 240
   gcgatcgcgt gtgagctccg tgcgcgcgcg gcgagtcgac gcgtcagcgg tgaggccgcc 300
   gaggcgttgc tcgcgcaggt ggtcgacctg cgtggccggc tcgcgcgcct cgcgcgaact 360
   ccggaagagg tcctcgatgc ggcagagcga gcgagcgggt ggctgctgaa gaaagcctgg 420
   aagcgggtct ggcatgacgg tccgaaccgc cgcacggacg cgatgcgcaa cacgcctcgc 480
   cggcggctcg aggcacacgc gctccggggg aactggccgc gcttcccggt gtcgcccgcg 540
   cgcttcgagc ccgagctccg ccgcgtcgtc ggtgaccacg cgtactacga tcagcgcgcc 600
   acggatctgg tcgcgcacct cctcgagagc catgtcgacc tcctcgcagc gatggcagcc 660
   tcggatctgg agcggctggc gctatatcgg gcggcgatga ccgtgatcat tgagatgatg 720
   gaccgggtcg acgactccct cgccgacatg agcgaggtgt tcgcggcgag cgagcgcacg 780
   taccttgcgc tcgcccgcga ccatgccggg ctcgacggga tcgtgcgcga cctcctcgag 840
   ctcgccgtgt gggaggatta cgggttgctc cgtagcgtcg gcgacttcct gggggcgctc 900
   caggaggagc acgcgaacct cgccgtccgc gagctctccg ggatcatcgc ggacctccgg 960
   cgagagcgcc tcgatgacca gctcgcccgt gccctacgcc agaaaagcga gcaccatcgt 1020
   cacgttggcg atcaccggcg ctag 1044
<210> 13
   <211> 1659
   <212> DNA
   <213> Sorangium cellulosum
<400> 13
   atgcgaaacc tccggaggaa tcgagggcga ggtgggcagc ggcgctgccc acccgggcgt      60
   ctggcggacg gcgcccttga cggctgcccg aaccaccatg gcggcaagaa atcgggaatg     120
   gctgcgaagg gcgacccggt cgacgtggtg acgggccggg tgttcacgat tcccgccgtc     180
   gacctcgagc ttccgggacc gctgccgctc tcgatcgtgc ggtcctacac caccgcggcg     240
   ttcgagcgcg acgtcgggct ggggttcggg tggagccact cgctggcgtg ggagatcgag     300
   gtgcggcagc gcatcctccg cgtctggacc gacgatgggc cggtggatct cgacacgatg     360
   cccgtgggcg ccggcgtggc gggcccccac ggatgggtcc tcgcgcggga ggagcgcggc     420
   ttcgtcctcg attccggcga cggccactgg tacgtgttcg ccgaagcggc cgggaagcgg     480
   ttccgactca ccgcgatcaa ggaccgatac aacaacgaga tcaggctctc gtaccgcgag     540
   ggcgtgctca ccgagatcac cgacagcgtc ggccggatcg tccgggtccg gcgggcgccc     600
   gacgggcgca tcggcgcgtt cgagatcaag aacgcgcggg agcgcggtgt ctgggtgccc     660
   ttcgcgcagt acagctacga cgccgccgga gatctcacgt ccgcgaccga cgccgacgga     720
   tacacgacgc gctacacgta ccaggaacac ctcctcacct cccatacgag ccctacaggg     780
   ctcacgttct cattccgcta cgacgatcgg cgccgctgcg tcgagacatg gggcgcttac      840
   ggcgatgggc gtgaccgcag cctggcggag gacgtaccga cgcacctggc cgacgggagg     900
   accgtcgcca gaggcatcta ccactgcaag ctggagttcg gagagggcgg atacgtcgaa     960
   gtcgcggact ccatcacggt tcatcgctac ttcgggaacc gcttcggcaa gctcgacaag    1020
   gccgtgagcg ccggggccgt gttcagccgg acgtacgacg agcacgggga cgcgctcggg    1080
   ggcaagacac agctggccta tgactactgg ggccggtgcc gctcgatccg ggatacgagc    1140
   ggtgcggtgc tgtcgtacac ctatgacaac cgcggcaacc tcaccggggt cctccggccc    1200
   gacggcggga cgacccgcta cgaatacgac gggatcgggg actgcaccgc ggtcctccag    1260
   ccgaaccagc aggtgacccg gttcgtgcga ggcggcttcc acaagctctg cgaggtgcac    1320
   aagccgaatg gagagagcac acggttccgg tacaaccgtg aggggcgcct cgtcgagatc    1380
   aagaacgcgc tcggggagcg ccacaggatc gagctcgacg ccgcggggat ggtgaccagc    1440
   gagcgtgggt tcgacgggcg cgtgtctcga accaagtacg attacaatgg ccggctcatc    1500
   tccttccaga acgccacggg cgagaggacg gagtacacgt acgacctcgc cgggcagctc    1560
   gtcgcgcgca ccttcgatga cggcagtgtc gagaggttca cgtacgacga ccgcggcgac    1620
   gtgatcgccg ccgagagccc cgccggttcg ttcacgtag                           1659
<210> 14
   <211> 534
   <212> DNA
   <213> Sorangium cellulosum
<400> 14
   atggccaagg cctcgagcga gatcgagacc gccggtggca agaggcaccg cggatcgagc      60
   gcgttcgggg ggccgctcga ccccgcgctc cagaccgcgg tcgacaatgc ccacaagaag     120
   ttcgacgagg gccaggtggc ggggccgaag ccgtcgagcg ccgggcagtg cgccgagatc     180
   gaggcgctga acaacctggc gaaagacatc cggcaggacc gccagaagaa cggtaagaca     240
   ttgaaagatc ctgcggccga agacaccgag atcaggaagg agatgcaaaa acaggtgaag     300
   gagggcaaga tgacgaccag gacggacaag gacggtctgc tcatggatcc gtgcaagttc     360
   tgcgcgcagg tcatgcgtga gctcggcctt cacccggcga acaacggggg cactggcaag     420
   aagaacggtg tcatcggctc ggactgcaag gcgtggaacg gcaatgtctg gcacaaggga     480
   cgctccatgc cgttcaggac cgctccctcg agcacgccgc ccttcgcagg caca           534
<210> 15
   <211> 1380
   <212> DNA
   <213> artificial
<220>
   <223> complementary sequence
<400> 15
   atgatcaccg cgaaatccct gggcgatcgc tcgttcaagg cggactatgg cgtggactac      60
   gcctacgtcg cgggggcgat gtacaagggc atcgcgtcca aggagctcgt cgtcgcgatg     120
   ggcaaggcgg gcttcatcgc gtacctcggt acgggcgggc tcgacgagcg cgagatcgag     180
   gcgtccattc gctctatcca gtccgtgatc gcggggagag cgtatggaat gaacctgctc     240
   tcgaacctcg agtcgccgga gctggaagag cgcacgatcg atctctatct gcggcacggg     300
   gtgcggtgtg tcgaggccgc ggcctacatg cgggtcacgc cagcgctcgt gcggtaccgg     360
   ctccaggggc tcgcgagcgg cgcggggcgg accctggtcg cgccccggcg cgtcctcgcc     420
   aaggtgtcgc ggcccgaggt ggcggcggcc ttcatgcagc ccgctccgga ggcggtggtc     480
   cgacagctcg tcgagtcggg gaagctcacc gagcaggacg ccgcgctcgc tcctctcgtg     540
   ccgatggcgg acgacgtctg cgtggaggcg gactcggggg gccacaccga tcagggcgtc     600
   gcgttcgcgc tgctgccggc gatgctcgtg ctccgcgacg agatgatgac gaggtaccgc     660
   tacgagaagc gtatccgcgt cggcgcggcg ggcgggatag gcacgcctca cgcggccgcg     720
   gcggccttcg tcatgggcgc cgacttcatc ctcacgggct cgatcaacca gtgtacgcgc     780
   gaagcgggca cgagcgagcc ggtgaaggcg ctgctccagc atttgaacgt gcaggacacg     840
   acgtacgcgc cggccggcga catgttcgag ctcgggtcga agattcaagt cgtgcggcgc     900
   gggctgttct tcccggcgcg ggcgaacaag ctccacgagc tctacatgcg acacggctcg     960
   ctggaggaga tcgacgcgaa gacccggcag cagatccagg agaagtactt ccggcgatcg    1020
   ttcgacgcgg tctggagcga gacgaggagc tactacgcga ggacctaccc gcacaggctc    1080
   gcggagatcg agcgcagccc gaagcagaag atggccgcgg tgtttcggtg gtatttcgcc    1140
   cacacgacgc gcctcgccct ggagggcgtc gaggaccagc gtctcgatta ccagatccac    1200
   tgcggccccg cgctgggcgc gttcaaccaa tggacgaagg ggaccgcgat cgagccgtgg    1260
   cagaaccgct atgtcgccga catcgctcga cgcatcatgg aagggactgc tgatctgttg    1320
   aacgcacggt tcggcgcgat gaaggaagcc gccgagcccc ctcaatctgt gagctcgtaa    1380
<210> 16
   <211> 249
   <212> DNA
   <213> artificial
<220>
   <223> complementary sequence
<400> 16
   atgaaccggc aagaaatatt cgatttgatc gcccgccatg cgcgcgaggt gctgcccgag      60
   ctggagcagc accggttcca ggacagcgac cggttgcagg atctgggagc caactccgtg     120
   gaccgcgccg agatcgtcat gctgacgctc gagtcgctgt cgctcaagat cccgcgggtc     180
   gagacgttcg gtccaagcaa catcggcgag cttgcggatc tgttgcatgc gaagctccgg     240
   ggagcctga                                                              249
<210> 17
   <211> 1233
   <212> DNA
   <213> artificial
<220>
   <223> complementary sequence
<400> 17
   atgcgaagct ccggggagcc tgaagcggcc atcacgggcg tcggcgtcac ctcggcgatc      60
   gggcagacgc gcgacgcgtt cgcgcgtgcc cttctgcgcg gcgagtcggc cttcggggtg     120
   atgcagcgac ccgggcgcca gaagggcact gctttcctgg gggccgagat cgcctcgctc     180
   caggttccgg agggtatggc gccgcagacg ctgcgcaagg cgtcgttctc cgcccgggtc     240
   gccctcgcga cattgcacga ggcatggcgc gacgcgcgcc tcgacgacgt cgatccgcgg     300
   cgtatcggcc tcatcgtcgg cggctcgaac gtgcagcagc gcgacctcgt cctggcgcac     360
   gaggagtacg cgggccgggc agacttcctc cggccgacct acggcgtgtc gttcatggac     420
   acggatctgt gcggcttctg caccgagcag ttcggtatcc gggggctcgc gtacaccgcg     480
   ggcggcgcgt ccgcgagcgg gcagctcgcg atcatccaag cgatccaggc ggtgcaggcc     540
   ggccacgtcg acgtgtgcat cgccctcggc gcgctgatgg atatctcttt ctgggagtgc     600
   caagggctcg cgagcctcgg cgcgatgggc tccgagcgcc acgccgccgc ccccgagcgg     660
   gcgtgtcgcc cgttcgaccg cgctcgcgac gggttcatct acggcgagtg ctgcggcgcg     720
   gtggtcgtcg aggcgctgcc gcgcgctgcg cgccgcggcg tcagacccta cgcctggctg     780
   tcgggctggg ggatcgcgat ggacgccaac cggaacccgg atccgtcgct cgacggcgag     840
   gtcgaggcga tccgcagggc gctgcgatgc gccgggtgcg cgcccgctga catcgactac     900
   gtgaacccgc acggcacggg ctcggtggtg ggcgacgaga cggagctcga ggcgctccgc     960
   gcctgcgggc tatcccacgc tcgcctgaac gcgaccaaat cgctggtggg ccacgggctg    1020
   agcgccgcgg gcgcggtcga ggtgatcgcg acgctccttc agatgcagga aggtcggctg    1080
   cacccgtcaa gaaatctcga cgacccgatc gacccgtcat tcaactgggt gcgcggagag    1140
   gccgtcgcgc acacgatgaa gaccgcgctc acgctgagca tgggcttcgg cgggatcaac    1200
   acagccttgt gtctgcgcaa gtacagcgga tga                                 1233
<210> 18
   <211> 1260
   <212> DNA
   <213> artificial
<220>
   <223> complementary sequence
<400> 18
   atggcagtag gaatcgaagc gataaacgcg ttctgcggga cggcgtgcct cgacgttgcg      60
   cagctcgcgc gacaccggaa gctggatgcg ggccgcttcc aggggctcct gatgatggag 120
   aagacggtcg ccctgcccta cgaggatcct gtgacgttcg gcgcgaacgc cgcgcggccg 180
   atcgtcgacg cgctctccac cgcggagaag gatcgtatcg agatgctgat cacgtgcacg 240
   gagtcgggca tcgacttcgg caagtcgatc agcacgtacc tgcaccatca cctgaggctg 300
   agtcgcaact gccgtctctt cgagatcaag caggcctgct actccggaac cgctgggctc 360
   caggcggccg tgagcttcgt gctctcgcag gtgtcgcccg gggcgaaggc gctggtcgtg 420
   gcgacggaca tctcgcgcta catggtggcc gagggcgcgg acgcgccggc gccggaggcg 480
   gcgttcgcgg agcctagcgc cggcgccggc gccgtcgcgc tcctcgtcag cgatcgcccg 540
   cgcgtcttcc agcttgacgt tggggccagc ggttactacg gctacgaggt gatggatacg 600
   tgccgtccgg ctcccgacat cgaggctggc gacgccgatc tatcgctgct ctcgtacctc 660
   gactgctgcg agaacgcatt cctcgaatac aagaagaggg tccgcgacgt cgattacgtg 720
   cgctcgttcg gatacctggc gttccacacg ccgttcggcg ggatggtgaa gggcgcgcac 780
   cgcaccatga tgcgcaagat ggcgcgggcg aagccggcgg agatcgagga ggacttcgcg 840
   cggcgcgtga gcccggggct cgtctactgc cagcgcgtcg gcaacatcat gggcgccacg 900
   tcgctgctgt cgctcgtgag cacgatcgcc cacggcgact tccaggcacc ggagcgcatc 960
   gggtatttct cgtacggctc ggggtgctgt tccgagttcc tgagcggcgt cgtacgccgc 1020
   gaggggcagg aagccgtgcg cgagctccgc atcggccggc agctcgacga ccgtcacccg 1080
   ttgtcgatcg cagagtacga ttacctgatc cgcggaaata gccagctccg gttcggcacg 1140
   cgggacgtcg aggtgcgcga ggcaggcctg ccggcggcga agccggtccg cgaaggggag 1200
   cgccgtctga agctccgggc ggtagcgaac ttccaccgcg agtacgagtg gacgccgtga 1260
<210> 19
   <211> 768
   <212> DNA
   <213> artificial
<220>
   <223> complementary sequence
<400> 19
   gtgaccgccg tccctcccta ccagacgctg cgcgtcagga accaggacgg gatctgcttc 60
   atgcagatcc accgacccga ggcgcagaac acgatcgacg atcggctgat cgacgaatgt 120
   tccgacgtgc tcgatcgctg cgaggacgcc gcgaccatcg tggtgatcga ggggctcccg 180
   gagacgttct gcttcggcgc cgacttctcg gcaatacggg cggcgcaggg cgtaatgcgc 240
   ggccccgagc cgctgtacga cctctggatg cggctcacca ccggcccgta cgtcgtgatc 300
   tcgcacgtgc gcgggaaggc gaacgccggc ggcatcggct tcgtcgcggc gagcgacatc 360
   gtggtcgccg acgcgacagc gacgttcagc ctctccgagc tcctcttcgg gttgatcccg 420
   gcctgcgtca tgccgttcct tgtgcgccgc gtgggcttcc agaaggcgca ctatatgacc 480
   ctcatgactc atccgctcga cgtgtcacaa gccgcggcgt acggtctcgt cgacgcgtac 540
   gacgcgaaca gcgacgcgct cctccggcgg cacctctcgc gcctccggag gttgagcaag 600
   accgcggtag cccgctacaa gcggttctcg tcagccctcg gcggcgccgt cgccgcgagc 660
   cggccgctgg cgctcgcgac gaacaaggag gtgttctccg atccgcgcaa catcgagggc 720
   atcatgcgct acgtcgatga ggggatcttc ccctgggagc gcccctaa 768
<210> 20
   <211> 750
   <212> DNA
   <213> artificial
<220>
   <223> complementary sequence
<400> 20
   atggcaacgc ccgtcgttca cctcacgacc gtcgagcctt ccatcgtgca gatcaagatg 60
   gaagatcgca cgtacaagaa cacgttctcc gaggggatct gcgccgggct ggtggacgcg 120
   ttccgtcgcg tggcgtccga tccggattgc aaggtcgtcg tcgtcaccgg gtacgacacc 180
   tacttctgct cgggaggcac gcaggagggg ctcctgagcc tctccggtgg gcaggggaag 240
   ttcaccgaca cgccgatcta ccacctcccg ctcgcctgcg aggtgccggt gatctccgcc 300
   atgcaagggc acgggatcgg cggcggcttc gcgctcggcc tgttcgccga cttcgtcgtc 360
   ctcagccgcg agagcgtcta caccgccaac ttcatgaaat acggattcac gcccggcttc 420
   ggctcgacgc tcatcttgcg cgagaagctg ggcctcccgc tggcgcagga gatgctcatg 480
   acggcgggga gctaccgggg cgccgagctg gcccagcggg gcgtgtcgtt cccggtcgtg 540
   ccgagggcgg aggtgctgcc gcacgcgtac cagctcgcgc gacagctcgc ggagaagccg 600
   cgccgctcgc tcgtcatcct gaagagccac ctggtcgccg atctccgagc acggctcccc 660
   gtggtcgccg acatggaggt cgccatgcat gagcagacct ttcatcaccc cgaggtccgg 720
   gagcggatca agtcccgctt cggagcgtag 750
<210> 21
   <211> 3336
   <212> DNA
   <213> artificial
<220>
   <223> complementary sequence
<400> 21
   atgcacgtga gaatcgtcga caggcccggc gcgggcgagc gggaagacgc gatcgacatc      60
   tcgatcgaac gccccgcggg aaaggagacc ttcgccgcct cggcgaacgc cgcgctcgcc     120
   ggcgccgagc tcgacgcacg gctcgcctgg tacttcgcgg agtatcccca gaccacggcg     180
   ctgccgcccg acgatcgcgg cgtcgcgagc gccatcgccg acgtagggcg ggcgatgggc     240
   gagcgcctcg cgaacggcga ggagcgcccc gccgcgctcg ccacggcgat cggggagcgc     300
   gagccgcgcc gcgtcggcgt cctcatcgag tcgtcgcgcg cctcgttcct cgcggcgccg     360
   tgggagctca tggtgctgcc gggcgccgac cagccgtggt cgacgctggc ggcgcacttc     420
   gtccgccgct ttcatcgcgg agggcgctcg gaggacgatt ctcagatcgc ctacgagctg     480
   ggcgtgacgc cgccggagcc ggaggcggtg gccgcgttga aggcgcagat gcggggcgac    540
   gcgccggcgc cgcaaccccc gcccgacgcg cccttgcgga tcctccacgt cgtgtcgagg    600
   gctctccgcg atctcctgcc gttcgcctcg agccacgcgc tcgagtgggc cgcggagtcg    660
   ctcggcgccg agggggccgt cgactacgag atcttccccg cgggctgggc cgagctccag    720
   gcgcgcctcg tcgatcgccg gcgcccggtg cacgtcctcc actacgatgg gccgatcgtc    780
   ctcgacggcg gcatcgccag cgtgctcctc ggcgggcccg acggcccgtt cgagcgcgtg    840
   gcggtggcgg agctcgcctg ggcgctcgtg cgcagcaagg tggcggcgct cgcggtcgac    900
   acccgcggag ccttcgacgg cgcccacccc gtcgctgtgc ccgcggcggt gagcgccgtc    960
   gcgagcgccg cgcgcgacgc gtccctcggc aacgtcatcg ccgcggcgta cgcagccgat   1020
   ccgtggacga gcggacgatg cttccaggaa gtctacacgc gcctggccgc gggcctcacg   1080
   ctcggcgagg cggtggtcga cgcccggcgc gcgctctgcg agcgcgcgga ggcatcgcgc   1140
   ttcacgtcgg agccgcgccc gttccacgcc tggcccctgc tcgtccacta cggcgggcag   1200
   tccgtctcgt tcttcgcgca ggcgcaggcg cgcgcgagcc tcgaagaatc gcaggcgctc   1260
   gcgcgcgcac ggcgccgtct gctcggcttt cacgcgacgc tcctgccgcc cctcctccgg   1320
   cacgccggcg acggcgtcgc ggtcgccgcg ctcggcgcca tcgcccgggg ccggcgcgcg   1380
   atcgcgctga cgggcgagcc cggcgccggc aagtctcacg ccgcgcaccg cgtcgcgctg   1440
   tacctcgcgc agcgcgacgc ggtcgacttc gccttctact tcgactttgg cgccgaaccg   1500
   tactcggtgg acgacatact cgagatgatc gcgccggtcc tcgggctcgc gccagatcga   1560
   aaggccgaga cgagggtcgc gctcgcgaag ctccgctgct gcttcgcgct cgacgacgtg   1620
   gatcgcgccg cccatcgcgc caacggtgcg ccgggggcag ccgccggccc gttgctcgag   1680
   ttcgtccgcg agctcctcgc ggccggacac gtcgtggtca cgatcggggc gaccaaggcc   1740
   gccgctctcg cgccggactt cgtcgagctc ccggtggctc cgctctccgc cgacgagcag   1800
   caggcgctgg cggccgacga gctccgcgcg cgcgacctcc agggccggga ccgcgacgcg   1860
   gactggcccg cgctcctcgc ggcgacgcga ggccacccgt tcctcctcga gtgcgcggtc   1920
   cgcctcctgc gcgacggctc ggtgaggggc gtcgccgacg gggtacgatc gcgcgtcccg   1980
   acggggcacg ccccggtcga ggcgttctac gggtggcagt gggctgagat gccgcagccc   2040
   tggaggagcc tgctcctcgc gtgcgcgcag gtcgacggcc tgctcctcga gatcctgatg   2100
   gtggccgtcg atcaaaagat gccgttcgcg ccggcgaaga ggctcctggc gcggctgggc   2160
   gacgagtccg ccgtcttctc ccaggggctc gcgctgtggg agcggcgtgg gttcctcgtg   2220
   cgactcccgc acggccggat gatcgacccg cggtgccgcg ccttcctgga gctcgccggc   2280
   cgaagcgagc gctgggacgg cggctcggcc gacgcggagc tcgagctcag ccagacgctc   2340
   tgcgaggggc tgcggatcct gagcgcgcac ctgcagggcc gggccaaccc cgcgctcacg   2400
   cactatctcc tgatgaaccg gcggcactgg gtcgcgcatt tcgagcgcct ctggttcgcg   2460
   ggcgatctcc gcggcttcct cggcgtgatg cacgcgttca accagctctt gcgccaggcc   2520
   cagctcggag cggagggcgc cgcgtggtcg ctccgcctcg tgcagcggtc gcctgtgccc   2580
   gacgtggacg gcgaggcgcc gccggaggct tcgctggcct ggctggtcgt cgcgctacag   2640
   gcgctcggcg cgagcggcgc cgctgccgac cgggcgctgt gcgagggggc cgccgcctgg   2700
   cagcggtggc tcgacgcgct gccggagccc atcgagctgc gccgcgcgcg ccagttccag   2760
   caggtcgcca ccttcctcga cgcgctctac cgcgcccggc aggactgggc gacggccatc   2820
   gccgtcgacg agaaggctca ccgcttctac aagcgcatcg gggcgtggcc ggccgccgtt   2880
   caggcgctca ggtcgctcgc gcgctgtcac gtcgagctcg gcgacgcggg ggcggcgctg   2940
   cgtctcgagg acgagatcct ccacgacatc ccgtacgcgg acgcgccgcc cgggttccag   3000
   gcgcagcagc tcttcgacgt cgccgtggcg cgcgccgcgc ggggcgcctt cgccggcgcg   3060
   cagtcggcgc tggaccgact gaaggcgatg gatgacgcga agcggttcgg cgactcgctc   3120
   gaggcgctgc aggccgacgt gcgctatgag caggggagct acgccgacgc tctgcgcgac   3180
   tactgcgcgc tgtggacgcg cgccctagag acgcagcagg cgccgttcgt cgagcgcctg   3240
   cggacgcggc tcctcgatct gcgcggcaag ctcggcgaaa aggagttcga gcggcgcatc   3300
   gagcgcgagc tgcccgaggg gatcgctcgc ccctag                             3336
<210> 22
   <211> 366
   <212> DNA
   <213> artificial
<220>
   <223> complementary sequence
<400> 22
   atgggaacgg agttgatcga gagaggctcg ctgcggatcg tccatgtcac ctcgacggac     60
   aagcccatcc gcgcacggca ggacgcgctc gacgtgatgg tggggcacgg ctacaccgac    120
   gtggccggca tcgcgatcga cgagacctgc ttcgaccccg acttcttccg cctgaagacc    180
   gggatcgcag gtgagctctt gcagatcttc gtcaactacc gtatccgcgt cgcgatcctc    240
   ggcgatttct cgaaataccc cggcgacacc cttcacgcct tcatcgccga gtgcaatcga    300
   gggcaacaca tcttcttcgt ccatgacgtg gaagaggcga ttcgatattt cagccaccac    360
   gcatga                                                               366
<210> 23
   <211> 1410
   <212> DNA
   <213> artificial
<220>
   <223> complementary sequence
<400> 23
   atgaaagggt ccgggccgat gacgcttgcg gacaatctgg taaattacga cgcctccgcg 60
   ctggcggagg cgatccggca gcggagcctc tccgccgctg atgtcgtcga ggcgtgctat 120
   gcgcgcatcg agcggctgaa cggcgcggtc aacgcgatcg taacgctgga caaggagcgc 180
   gcccgcgcgc gagcacgcgc ggccgacctc gcgctcgcgc gcggcgaggc cgtcggaccg 240
   ctgcacggcg tcccggtgac agtgaaggac gccctgcaca ccgccggcct ccggaccacc 300
   gccggccacg agcggctccg cgatttcgtc ccggagcgcg acgcagctgc catcgaccgg 360
   atccaggagg ccggcgcgat cgtgatcggc aagacgaact gctcgacctt gtgcggcgac 420
   ctgcagacga gcaaccccat cttcgggacc acgaacaatc cgtgggagaa cacgcggacg 480
   tcgggcggca gctccggcgg cgaggccgcg gcggtggcgc tcgggctctc cgcgctcggc 540
   atcgggaccg acacgggcgg gagcatccgc gtgcccgcga gctactgcgg cgtctacggc 600
   ttcaagccgt cgctgcggaa ggtgccgtcg gacgggccgg cgtttccgct cgacgccgcc 660
   cctcgccggg aagatcacct gacggtgatc gggccgatcg cgcgctcggt gcgcgatctc 720
   atgctctgct acgatgtgct ctccggcgag tcatgctcgg ccgcgacgag cgctccaccg 780
   ccccgcgtcg cgtggacgca cgagttcgcc ttccccatgg acgacgcggt aacggcggag 840
   ctcgagaggg cgttcgacgc gctgcgcggg agcgcggcgc aggtcgagaa ggtcgcctct 900
   ccgttcgatc tctggaagac gaacctgagc tatttcatgc tgaacatgta cgaattcaag 960
   acgaaggaga ccgacgcggc gttctatcgc ttgttctgga tgttcgagtg gatgcgctcg 1020
   gcgttccgcg gcgggctcga tggcagctac gccaggctca aggcggagca gcgcgagctg 1080
   tcggggcgct tcgagagctt cattgaagca tacgattgtt gggtggtccc cgcgactccg 1140
   tcggtcgcca tccgccatca aaagactggc cgctccgtcc ccatgacggt gaacgggaaa 1200
   gagaagaagg tcgaatactg gaacgcggtc ggtggattcg ccttcttcgt gaatctgctc 1260
   ggcaacccga gcgtcgtgat ccctgtcggc aggagcaagg acgggttgcc cgtaggcgtg 1320
   caggtcgtcg ggaagcacgg acaggacgcg agcctcctgc gcgccgcgcg gttcatcgcc 1380
   gacacgatcg gccagctccg cacggcgtga 1410
<210> 24
   <211> 789
   <212> DNA
   <213> artificial
<220>
   <223> complementary sequence
<400> 24
   atgatccgga ccgtgcgcac gacccgctat gtagccccgc tccgtgaggg cgggtcggtg 60
   ccggccatcg tcgaggcgga cgacgatggc ctctacgtgg tgaagctccg cggcgccggc 120
   caggggccga aggccctgat cgcggagctc gtcgcggggc agatcgggcg agccctcggg 180
   ctgccagtgc cggagatcgt cctcatcgag ctcgacgtgg agttcgggcg caccgagccg 240
   gaccccgaga tcgctaagat actctcggcg agcgccggcc tgaacgtcgc gctcgactac 300
   ctccccgggt cgctcgcgtt ccagccgttc accggcggaa agctcgatcc ggcgctcgcg 360
   tcggccgtgg tctggttcga cgccttcgtc agcaacatgg accgcactcc ccgcaacccg 420
   aacctgctcc tctggcacag gaagctctgg ctcatcgatc acggcgcggc gctgtacgtc 480
   catcacgcct ggagcgaggg cgtcgatccc ctggccgagg cccacgatcg cttcccgcag 540
   atcgagagcc acatactgct cccctatgcg accgacatcg cggctgcgga cgccgtgcag 600
   agcgccctgc tcacgcccga gaagatcgcg ggcatcgtcg cggagatccc ggatgcgtgg 660
   ctggtcgatc gggcgccctc gacggacccc gaggagagcc gcgcggccta cgccgactat 720
   ctgaaccgcc gcctcgctcc gccgcgggga ttcgtggagg aggcgctcga tgctcgggcc 780
   aggcgctag 789
<210> 25
   <211> 357
   <212> DNA
   <213> artificial
<220>
   <223> complementary sequence
<400> 25
   gtgctccgcg tggtcccgcg catcgagcgc ggcgagcgcg tcaacgtcgg ggtggtgctc 60
   cactgtcgct cccggggctt tctcgacgtg cgctgcgagc ctgacgatcg ccgcctgctc 120
   gccctggcgc cggacctcga tctaccggcg gtgcgccagc acctggagtt tgtgcgcctg 180
   gtgtgcgccg gcgatccggc ctcgggctat gtcgccgggc tccctcagga ggagcggttt 240
   gggtggctgg tctccccgtc gagtaccatc gtgcagccgt cgccctccca caccgggctg 300
   tgcgccgatc ctgccgagac cctggggtgg ctgctcgatc gcctcgtgcg ggtctga 357
<210> 26
   <211> 104
   <212> PRT
   <213> Sorangium cellulosum
<400> 26
   Met Ser Tyr Phe Arg Gly Val Gln Arg Pro Glu Leu Val Ser Met Glu
   1 5 10 15
   Ala Ile Val Lys Arg Tyr Leu Ser Glu Arg Leu Leu Ile Glu Phe Asp
   20 25 30
   Gly Glu Ser Gly Val Asp Ala Asp Ser Asp Leu Phe Gln Leu Gly Leu
   35 40 45
   Met Asp Ser Gln Ala Tyr Val Glu Leu Ile Gln Phe Val Glu Arg Glu
   50 55 60
   Phe Gly Leu Arg Phe Thr Asp Glu Glu Leu Leu Ser Asn Val Val Val
   65 70 75 80
   Ser Leu Ser Gly Ile Val Ser Leu Val Ser Gly Ala Met Asp Glu Arg
   85 90 95
   Ala Arg Gln Asn Ala Leu Arg Gly
   100
<210> 27
   <211> 305
   <212> PRT
   <213> Sorangium cellulosum
<400> 27
   Met Lys Ala Leu Leu Phe Pro Gly Gln Gly Ala Gln Asp Pro Ala Met
   1               5                   10                  15
   Cys Ala Gly Val Val Gly His Pro Arg Phe Ala Glu Arg Tyr Gly Val
               20                  25                  30
   Ile Cys Asp Ser Thr Arg Thr Asp Val Leu Arg Arg Ile Gln Glu Gly
           35                  40                  45
   Asp Ala Gly Ile Leu Asp Gln Asn Arg Phe Ser Ser Leu Met Thr Val
       50                  55                  60
   Leu Val Ser Ala Ile Leu Leu Asp Met Leu Arg Glu Arg Asp Ala Ala
   65                  70                  75                  80
   Thr Pro Glu Tyr Phe Ala Gly Tyr Ser Val Gly Gln Trp Ser Ala Met
                   85                  90                  95
   Tyr Ala Ala Gly Met Leu Thr Tyr Glu Gln Thr Ile Glu Ala Leu Val
               100                 105                 110
   Glu Arg Ala Arg Leu Met Asp Glu Cys Val Asp Arg Arg Pro Gly Ala
           115                 120                 125
   Met Cys Ala Val Ile Gly Leu Arg Glu Ala Val Leu Glu Gln Val Leu
       130                 135                 140
   Ala Asp Leu Arg Ser Arg Gly His Leu Val Phe Ile Ala Asn Tyr Asn
   145                 150                 155                 160
   Cys Val Gly Gln Tyr Ser Ile Ser Gly Ala Val Pro Ala Ile Glu Leu
                   165                 170                 175
   Ala Glu Glu Glu Leu Arg Ala His Arg Pro Lys Lys Ile Val Arg Leu
               180                 185                 190
   Pro Val Ser Gly Ala Trp His Cys Pro Met Leu Glu Asp Ala Gln Val
           195                 200                 205
   Arg Phe Ala Glu Val Leu Ala Gly Val Arg Phe Gly Pro Arg Ala Ala
       210                 215                 220
   Pro Val Leu Asp Asn Val Thr Gly Asp Trp Leu Pro Asp Glu Pro Glu
   225                 230                 235                 240
   Ala Leu Arg Asp Gly Leu Ala Arg His Ile Ser His Pro Val Gln Trp
                   245                 250                 255
   Glu Arg Gly Ile Lys Lys Leu Ala Ala Leu Gly Cys Arg Arg Phe Val
   260 265 270
   Glu Val Gly Tyr Gly Asn Ser Leu Thr Lys Phe Gly Phe Phe Ile Asp
   275 280 285
   Arg Gln Ala Glu Phe Ser Ala Phe His Gly Glu Gly Val Ala Cys Ala
   290 295 300
   Gly
   305
<210> 28
   <211> 633
   <212> PRT
   <213> Sorangium cellulosum
<400> 28
   Val Arg Met Leu Ala Arg Leu Arg His Arg Gly Pro Asp Glu Met Gly
   1 5 10 15
   Tyr Tyr Phe Asp Asp Ser Val Ala Ile Gly Asn Ala Arg Leu Ser Ile
   20 25 30
   Val Asp Leu Ala Gly Gly Gln Gln Pro Ile Gly Asp Pro Thr Gly Arg
   35 40 45
   Tyr Trp Ile Val Phe Asn Gly Glu Ile Tyr Asn Tyr Lys Glu Leu Gly
   50 55 60
   Ala Glu Leu Ala Ala Ala Gly His Arg Phe Ala Ser Ser Ser Asp Thr
   65 70 75 80
   Glu Val Leu Leu His Ala Trp Ile Ala Trp Gly Pro Ala Ala Phe Thr
   85 90 95
   Arg Leu Asn Gly Ala Phe Ala Phe Cys Ile Tyr Asp Arg Arg Asp Lys
   100 105 110
   Thr Leu Val Leu Ala Arg Asp Arg Phe Gly Lys Arg Pro Leu Tyr Tyr
   115 120 125
   Val Arg Asp Ala Glu Gly Val Val Phe Gly Ser Glu Leu Lys Cys Phe
   130 135 140
   Leu Glu Tyr Glu Pro Phe Arg Phe Glu Leu Asp Pro Gly Gln Leu Ala
   145 150 155 160
   Ser Ile Phe Arg Ile Trp Thr Pro Leu Glu Asp Gln Cys Gly Phe Arg
   165 170 175
   Ser Val Arg Gln Val Pro Ala Gly Ala Tyr Ala Gln Val Thr Gly Ser
   180 185 190
   Ser Phe Asp Ile Val His Tyr Ala Pro Leu Arg Ile Asp Arg Pro Pro
   195 200 205
   Pro Asp Leu Ser Glu Gln Glu Ala Ala Ala Gln Val Arg Asp Ala Leu
   210 215 220
   Ser Lys Ser Val Gln Leu Arg Leu Arg Ser Asp Val Glu Val Gly Thr
   225 230 235 240
   Tyr Leu Ser Gly Gly Ile Asp Ser Ala Ile Val Ala Thr Leu Ile Ala
   245 250 255
   Glu Asn Ser Thr Gly Arg Thr Lys Ser Phe Ser Ile Arg Phe Glu Glu
   260 265 270
   Asp Glu Phe Asp Glu Ser Gly Asp Gln Glu Leu Met Ser Ser Phe Leu
   275 280 285
   Gly Thr Glu His Ala Ser Ile Arg Ile Gly Ser Gln Glu Ile Val Asp
   290 295 300
   Ala Phe Pro Glu Ala Leu Trp His Ala Glu Val Pro Val Phe Arg Thr
   305 310 315 320
   Ala Phe Val Pro Met Phe Leu Leu Ser Arg Leu Val Lys Asp His Gly
   325 330 335
   Ile Lys Val Val Leu Thr Gly Glu Gly Ala Asp Glu Ala Phe Leu Gly
   340 345 350
   Tyr Asp Ile Phe Arg Glu Thr Leu Leu Arg Ala Glu Trp Ala Glu Met
   355 360 365
   Glu Pro Ala Ala Arg Gln Arg Lys Leu Ser Arg Leu Tyr Pro Tyr Leu
   370 375 380
   Arg Gln Phe Asp Glu Arg Asn His Ala Ala Leu Ala Ala Leu Phe Glu
   385 390 395 400
   Arg Leu Ser Arg Asp Pro Pro Thr Asp Phe Phe Ser His Glu Leu Arg
   405 410 415
   Phe Leu Asn Ser Gln Leu Ser Ala Arg Leu Leu Ala Gly Arg His Asp
   420 425 430
   Ala Leu Arg Pro Leu Thr Glu His Val Ala Ala Pro His Glu Arg Tyr
   435 440 445
   Ala Pro Leu Ser Arg Val Gln Lys Ala Gln Trp Leu Glu Phe Lys Thr
   450 455 460
   Leu Leu Ala Gly Tyr Leu Leu Ser Thr Gln Gly Asp Arg Met Ser Leu
   465 470 475 480
   Ala His Gly Val Glu Asn Arg Cys Pro Phe Leu Asp Pro Asp Val Val
   485 490 495
   Ala Leu Gly Cys Ala Thr Ser Leu Ala Phe Asp Asp Gly Phe Asp Glu
   500 505 510
   Lys Tyr Leu Leu Lys Lys Ala Phe Ala Ala Lys Leu Pro Glu Arg Ile
   515 520 525
   Leu Lys Lys Pro Lys Gln Pro Tyr Arg Ala Pro Asp Ala Ser Ala Phe
   530 535 540
   Leu Lys Arg Gln Pro Asp Tyr Leu Ser Val Ile Arg Ser Lys Ala Glu
   545 550 555 560
   Leu Glu Lys Ile Gly Val Leu Asp Val Lys Phe Cys Ala Ala Phe Val
   565 570 575
   Glu Arg Met Leu Ser Lys Pro Ala Glu Ser Ile Ser Gln Ala Glu Asn
   580 585 590
   Gln Thr Phe Leu Phe Leu Leu Ser Thr Ala Leu Leu His Arg Arg Phe
   595 600 605
   Val Glu Arg Asp Val Pro Ala Pro Pro Thr Ile Glu Pro Leu Leu Val
   610 615 620
   Arg Arg Phe Asp Gly Arg Leu Ile Ala
   625 630
<210> 29
   <211> 5146
   <212> PRT
   <213> Sorangium cellulosum
<400> 29
   Met Lys Asp Glu Lys Lys Ser Pro Asp Leu Gly Gly Ser Asp Val Ala
   1 5 10 15
   Val Val Gly Val Gly Gly Arg Phe Pro Gln Ala Arg Gly Pro Glu Gln
   20 25 30
   Leu Trp Glu Asn Leu Arg Ser Gly Arg Cys Ala Ile Thr Ala Val Pro
   35 40 45
   Pro Gly Arg Trp Ser Asp Ala Ala Arg Ala Gly Glu Phe Arg Gly Gly
   50 55 60
   Phe Val Glu Asp Ala Asp Cys Phe Asp Ala Ala Phe Phe Lys Ile Ser
   65 70 75 80
   Pro Lys Glu Ala Glu Leu Met Asp Pro Gln Gln Arg Met Leu Leu Glu
   85 90 95
   Val Leu Trp His Thr Leu Glu Asp Ala Arg Val Lys Pro Ser Ser Leu
   100 105 110
   Glu Gly Arg Lys Ile Gly Val Phe Met Gly Val Cys Asn Asp Asp Tyr
   115 120 125
   Ala Glu Leu Leu His Glu His Asp Ala Tyr Asp Ala Ser Leu Tyr Gly
   130 135 140
   Ser Thr Gly Thr Ser Gly Ser Ile Leu Ser Asn Arg Val Ser Phe Phe
   145 150 155 160
   Phe Asp Phe Arg Gly Pro Ser Val Thr Val Asp Thr Ala Cys Ser Ser
   165 170 175
   Ser Leu Val Ala Val His Leu Ala Ala Arg Ala Ile Arg Ala Gly Glu
   180 185 190
   Cys Glu Ala Ala Leu Ala Gly Gly Ala Asn Leu Cys Trp Thr Glu Ser
   195 200 205
   Arg Phe Leu Ala Phe Arg Ser Ala Gly Met Leu Ser Lys Asp Gly Val
   210 215 220
   Cys Arg Thr Phe Asp Ala Arg Ala Asn Gly Tyr Leu Arg Gly Glu Gly
   225 230 235 240
   Ala Gly Ala Val Leu Leu Arg Pro Leu Arg Asp Ala Leu Arg Asp Gly
   245 250 255
   Asp Tyr Ile Tyr Gly Val Ile Lys Gly Gly Ala Ile Asn His Gly Gly
   260 265 270
   Arg Thr Arg Gly Leu Thr Val Thr Ser Pro Ala Gln Gln Arg Gln Leu
   275 280 285
   Leu Ile Asp Ala Tyr Arg Asp Ala Gly Val His Pro Asp Thr Val Gly
   290 295 300
   Tyr Ile Glu Thr His Gly Thr Gly Thr Ser Leu Gly Asp Pro Ile Glu
   305 310 315 320
   Phe Leu Gly Leu Lys Gln Ala Phe Glu Ala Leu Arg Glu Gln Gly Ser
   325 330 335
   Glu Arg Arg Val Arg Pro Gly Thr Cys Ala Leu Gly Ala Leu Lys Thr
   340 345 350
   Asn Ile Gly His Leu Glu Ala Ala Ala Gly Val Ala Gly Leu Ile Lys
   355 360 365
   Val Leu Leu Ser Leu Lys His Gly Ala Ile Pro Gly Asn Leu His Phe
   370 375 380
   Asp Glu Leu Asn Ser Arg Ile Ser Leu Asp Gly Thr Ser Phe Tyr Phe
   385 390 395 400
   Pro Thr Ser Thr Arg Ala Trp Pro Ala Gly Glu Pro Gly Asp Gly Ala
   405 410 415
   Pro Pro Arg Arg Ala Gly Val Ser Ala Phe Gly Tyr Gly Gly Ala Tyr
   420 425 430
   Ala His Val Val Val Glu Glu His Cys Ala Pro Pro Pro Glu Ala Ser
   435 440 445
   Ala Pro Ala Pro Gly Pro His Leu Val Ala Leu Ser Ala Pro Thr Pro
   450 455 460
   Ala Arg Leu Arg Ala Trp Ala Glu Ala Leu Val Glu His Ile Gly Ala
   465 470 475 480
   Arg Ala Val Glu Ala Pro Val Ala Leu Ser Glu Leu Ala Tyr Ser Leu
   485 490 495
   Gln Thr Thr Arg Glu Glu Phe Glu Glu Arg Leu Ala Leu Ile Ala Glu
   500 505 510
   Asp Ser Ala Ala Leu Ser Glu Gln Leu Arg Ala Phe Val Ser Gly Val
   515 520 525
   Asp Pro Ala Gly Cys Phe Arg Gly Asn Val Arg Thr Ala Lys Gly Ala
   530 535 540
   Pro Val Asp Ala Ala Val Val Asp Ala His Ile Gly Gly Arg Ala Leu
   545 550 555 560
   Ser Ser Leu Ala Glu Leu Trp Val Arg Gly Gln Ser Ile Pro Trp Arg
   565 570 575
   Gly Leu Tyr Ala Gly Ala Val Pro Arg Thr Ile Ala Leu Pro Leu Phe
   580 585 590
   Ser Phe Glu Arg Arg Arg Tyr Trp Val Thr Arg Arg Gln Val Ala Pro
   595 600 605
   Ala Pro Glu Val Ser Pro Thr Val Ala Pro Pro Gly Pro Ala Ala Ile
   610 615 620
   Glu Lys Pro Ser Gly Ile Ala Leu Arg Ala Pro Gly Glu Glu Arg Phe
   625 630 635 640
   Ala Ala Pro Ala Val Ser Ser Gly Val Glu His Thr Val Ser Leu Ser
   645 650 655
   Pro Val Gly Ala Ala Val Asn Gln Ala Ala Val Val Ala Gly Ser Ala
   660 665 670
   Met Gly Ala Ala Pro Tyr Gly Gly Gly Ser Pro Ala Gly Gly Ala Ala
   675 680 685
   Ser Ser Ala Ile Ala Gly Val Glu Arg Val Thr Pro Arg Ala Thr Glu
   690 695 700
   Gly Gly Val Gly Gly His Met Pro Gly Thr Thr Ala Thr Ala Arg Asp
   705 710 715 720
   Arg Arg Ala Ser Pro Val Asp Asn Ala Ala Gly Gly Arg Ser Leu Ala
   725 730 735
   Ala Leu Glu Gln Glu Leu Thr Gly Ser Leu Ala Glu Ala Leu Phe Met
   740 745 750
   Asp Arg Ala Asp Val Glu Pro Asp Arg Pro Phe Met Glu Leu Gly Leu
   755 760 765
   Asp Ser Ile Val Gly Val Glu Trp Val Arg Val Ile Asn Thr Thr Tyr
   770 775 780
   Gly Leu Ser Leu Pro Ala Thr Cys Val Tyr Asp His Pro Thr Ile Arg
   785 790 795 800
   Arg Leu Ala Ala His Leu Ser Ser Ile Ala Pro Arg Thr Glu Val Ser
   805 810 815
   Ala Val Ala His Arg Arg Pro Arg Gly Gly Ala Ala Glu Ala Gly Arg
   820 825 830
   Ala Arg Ala His Asp Leu Gly Ala Leu Glu Glu Gly Leu Thr Ser Ser
   835 840 845
   Leu Ala Glu Ala Leu Tyr Thr Asp Arg Ala Glu Ile Asp Pro Asp Arg
   850 855 860
   Pro Phe Thr Glu Leu Gly Leu Asp Ser Ile Val Gly Val Glu Trp Ile
   865 870 875 880
   Arg Ala Ile Asn Lys Ala Tyr Gly Leu Ser Leu Pro Ala Thr Cys Val
   885 890 895
   Tyr Asp His Pro Thr Ile Gly Arg Leu Ala Arg His Leu Ser Pro Met
   900 905 910
   Leu Gly Gly Ala Gly Ala Gly Pro Ser Ala Gln His Gly Gly Glu Asp
   915 920 925
   Ala Leu His Leu Ser Arg Asp Asp Ala Glu Gly Gln Pro Arg Asp Ala
   930 935 940
   Arg Val Gly Ala Ala Arg Ser Ser Glu Leu Asp Ala Leu Gln Val Glu
   945 950 955 960
   Leu Ala Lys Ser Leu Ala Asp Ala Leu Tyr Met Glu Pro Gly Asp Val
   965 970 975
   Asp Val Asp Arg Pro Phe Val Asp Leu Gly Leu Asp Ser Ile Val Gly
   980 985 990
   Val Glu Trp Thr Arg Ala Ile Asn Lys Ala Tyr Gly Val Ser Leu Pro
   995 1000 1005
   Ala Thr Arg Leu Tyr Asp His Ser Thr Val Arg Arg Leu Ala Ala
   1010 1015 1020
   His Leu Ser Gly Glu Leu Asp Val Arg Ala Ala Ala Gly Arg Pro
   1025 1030 1035
   Val Ala Thr Glu Arg Glu Arg Pro Ala Leu Gly Gly Asp Ile Ala
   1040 1045 1050
   Ala Arg Pro Ala Arg Glu Pro Glu Ala Arg Ala Pro Arg Pro Ala
   1055 1060 1065
   Pro Glu His Glu Gly Ile Ala Val Ile Gly Met Ser Gly Ala Phe
   1070 1075 1080
   Pro Lys Ala Arg Asp Leu Asp Glu Phe Trp Glu Asn Ile Ala Arg
   1085 1090 1095
   Gly Val Asp Cys Val Thr Glu Ile Pro Pro Asp Arg Trp Ala Ile
   1100 1105 1110
   Ala Glu His Tyr Asp Pro Arg Pro Asp Ala Pro Arg Lys Thr Tyr
   1115 1120 1125
   Ser Lys Trp Met Gly Val Leu Asp Asp Val Asp Arg Phe Asp Pro
   1130 1135 1140
   Leu Phe Phe Gly Ile Thr Pro Thr Glu Ala Glu Leu Met Asp Pro
   1145 1150 1155
   Gln Gln Arg Leu Phe Leu Glu His Cys Trp Ser Cys Ile Glu Ser
       1160                1165                1170
   Ala Gly Ile Asp Pro Gln Arg Leu Ser Gly Ser Arg Cys Gly Val
       1175                1180                1185
   Tyr Val Gly Cys Met Pro Gly Asp Tyr Val Arg Val Gly Asp Gly
       1190                1195                1200
   Thr Asp Leu Thr Leu His Trp Leu Met Gly Ser Ser Ser Ser Ile
       1205                1210                1215
   Leu Ala Ala Arg Ile Ser Tyr Cys Leu Asn Leu Lys Gly Pro Cys
       1220                1225                1230
   Leu Ala Ile Asp Thr Ala Cys Ser Ser Ser Leu Val Ala Ile Val
       1235                1240                1245
   Glu Ala Cys Asp Ser Leu Leu Leu Gly Arg Ser Asp Leu Ala Leu
       1250                1255                1260
   Ala Gly Gly Val Ser Val Ala Val Arg Pro Glu Thr His Ile Ile
       1265                1270                1275
   Gly Ser Gly Ala Gly Met Leu Ser Pro Arg Gly Arg Cys Phe Thr
       1280                1285                1290
   Phe Asp Ala Arg Ala Asp Gly Phe Val Pro Ala Glu Cys Val Gly
       1295                1300                1305
   Val Val Leu Leu Lys Arg Leu Ala Asp Ala Val Arg Asp Gly Asp
       1310                1315                1320
   Ile Ile Ala Gly Val Leu Arg Gly Trp Ala Leu Asn Gln Asp Gly
       1325                1330                1335
   Arg Thr Asn Gly Ile Thr Ala Pro Ser Val Thr Ser Gln Ala Ala
       1340                1345                1350
   Leu Gln Lys Gln Val Tyr Asp His Phe Gly Ile Asp Pro Glu Thr
       1355                1360                1365
   Ile Ser Leu Val Glu Ala His Gly Thr Gly Thr Lys Leu Gly Asp
       1370                1375                1380
   Pro Ile Glu Val Gln Ala Leu Thr Glu Ser Phe Arg Ala Tyr Thr
       1385                1390                1395
   Asp Glu Arg Glu Tyr Cys Ala Leu Gly Ser Val Lys Ser Asn Ile
       1400                1405                1410
   Gly His Gly Leu Ala Gly Ala Gly Val Ala Gly Val Leu Lys Val
   1415 1420 1425
   Leu Leu Ala Leu Arg His Glu Arg Leu Pro Pro Thr Ile His Tyr
   1430 1435 1440
   Ala Thr Arg Asn Glu His Ile Thr Phe Glu Gly Ser Pro Phe Tyr
   1445 1450 1455
   Val Asn Thr Glu Leu Arg Pro Trp Ala Arg Arg Ala Gly Ser Pro
   1460 1465 1470
   Arg Arg Ala Ala Ile Asn Ser Phe Gly Phe Ser Gly Thr Asn Ala
   1475 1480 1485
   His Val Val Ile Glu Glu Ala Pro Pro Arg Ser Gly Glu Val Thr
   1490 1495 1500
   Phe Ala Glu Ala Gly Leu Pro Val Leu Ile Val Leu Ser Ala Lys
   1505 1510 1515
   Arg Pro Glu Arg Leu Glu Ala Tyr Ala Ala Lys Leu Ala Arg Trp
   1520 1525 1530
   Leu Glu Arg Gln Pro Ala Leu Asp Leu Thr Ala Leu Ala Tyr Thr
   1535 1540 1545
   Leu Gln Ile Gly Arg Glu Ala Met Ala Cys Arg Ala Ala Phe Val
   1550 1555 1560
   Ala Asp Gly Arg Arg Arg Ile Val Asp Val Leu Gly Glu Leu Ala
   1565 1570 1575
   Arg Gly Val Arg Ala Pro Gly Val Phe Met Gly Gln Ala Lys Arg
   1580 1585 1590
   Arg Gly Glu Thr Gly Ala Pro Ser Ala Ser Asp Ala Ala Thr Gly
   1595 1600 1605
   Arg Leu Leu Gly Ala Arg Ala Arg Glu Glu Arg Leu Ser Glu Leu
   1610 1615 1620
   Gly Glu Leu Trp Val Gln Gly Thr Thr Ile Ala Trp Asp Ala Leu
   1625 1630 1635
   Tyr Gln Asp Arg Thr Pro Ala Arg Leu Ser Ile Pro Thr Tyr Pro
   1640 1645 1650
   Phe Ala Arg Glu Arg Tyr Trp Phe Pro Leu Pro Gly Gly Thr Ala
   1655 1660 1665
   Ser Ala Pro Leu Leu Phe Glu Pro Arg Trp Asp Ala Lys Glu Ala
   1670 1675 1680
   Ala Gly Asp Ala Arg Glu Gly Asp Arg Ala Ala Ala Arg Phe Ala
   1685 1690 1695
   Arg Arg Cys Val Leu Val Glu Pro Ala Tyr Glu Asp Ala Ala Arg
   1700 1705 1710
   Ala Leu Ala Ser Ala Cys Pro Gly Thr Glu Ile Glu Ala Leu Arg
   1715 1720 1725
   Val His Gly Gly Thr Leu Ala Asp Arg Phe Ala Gly Arg Val Glu
   1730 1735 1740
   Gln Val Phe Glu Arg Val Arg Glu Leu Leu Arg Asp Arg Leu Asp
   1745 1750 1755
   Gly Arg Ala Leu Leu Gln Val Val Val Ser His Gln Gly Glu Gly
   1760 1765 1770
   Ala Glu Ala Leu Ala Gly Val Ser Gly Leu Leu Lys Thr Ala Ser
   1775 1780 1785
   Arg Glu Asn Pro Asp Leu Val Ala Gln Leu Val Gln Ile Ala Ser
   1790 1795 1800
   Pro Met Arg Ala Asp Glu Leu Ala Ala Arg Leu Arg Ser Asp Ala
   1805 1810 1815
   Ala Ala Ser Asp Asp Arg Val Ile Arg Tyr Arg Glu Gly Gly Arg
   1820 1825 1830
   Tyr Val Ala Thr Val Ala Ala Leu Ala Pro Ala Pro Asp Asp Gly
   1835 1840 1845
   Ala Arg Ala Pro Gly Ala Gln Ala Gly Trp Lys Ser Asp Gly Val
   1850 1855 1860
   Tyr Leu Ile Ala Gly Gly Gly Gly Gly Ile Gly Arg Ile Val Ala
   1865 1870 1875
   Arg Asp Ile Val Glu His Ala Asp Gly Ala Thr Val Leu Leu Ala
   1880 1885 1890
   Gly Arg Ser Pro Leu Thr Gly Glu Leu Arg Ala Ala Leu Asp Ala
   1895 1900 1905
   Leu Arg Ala Glu Gly Glu Arg Arg Arg Ala Arg Val Glu Tyr Leu
   1910 1915 1920
   Thr Met Asp Val Ala Asp Val Ala Ala Val Glu Gly Arg Val Arg
   1925 1930 1935
   Asp Ile Leu Glu Arg Tyr Gly Arg Leu Ser Gly Val Ile His Ala
   1940 1945 1950
   Ala Gly Val Leu Arg Asp Gly Phe Ile Val Arg Lys Gln Thr Ser
   1955 1960 1965
   Asp Leu His Ala Val Leu Ala Pro Lys Val Arg Gly Val Val His
   1970 1975 1980
   Leu Asp Arg Ala Thr Ala Arg Leu Pro Leu Asp Phe Phe Ala Ile
   1985 1990 1995
   Phe Ser Ser Ile Ala Gly Val Phe Gly Asn Val Gly Gln Ala Asp
   2000 2005 2010
   Tyr Ala Ala Ala Asn Gly Phe Leu Asp Gly Tyr Ala Ala Tyr Arg
   2015 2020 2025
   Asp Arg Leu Val Ala Asp Gly Glu Arg Arg Gly Arg Thr Ile Ala
   2030 2035 2040
   Ile Ala Trp Pro Leu Trp Ala Asp Gly Gly Met His Val Asp Pro
   2045 2050 2055
   Val Thr Ala Glu Ala Leu Asp Arg Ala Gly Leu Ala Pro Leu Ser
   2060 2065 2070
   Ala Ala Gln Gly Leu Ala Ala Leu His Glu Ala Leu Ala Ser Ser
   2075 2080 2085
   Arg Ala Arg Val Val Val Leu Ala Gly Gly Ile Glu Arg Leu Gln
   2090 2095 2100
   Ala Met Gly Val Ala Thr Gly Pro Ala Pro Ala Pro Ala Thr Arg
   2105 2110 2115
   Ser Ala Ala Ala Thr Ala Ala Gln Ala Val Arg Glu Ala Val Pro
   2120 2125 2130
   Ile Thr Ala Ala Arg Asp Ala Ala Ala Ile Asp Pro Arg Leu Leu
   2135 2140 2145
   Glu Glu Lys Thr Leu Arg Arg Leu Arg Gly Leu Phe Ala Asp Val
   2150 2155 2160
   Thr Lys Leu Pro Ser Ser Gln Ile Asp Pro Glu Glu Ser Leu Glu
   2165 2170 2175
   Leu Tyr Gly Ile Asp Ser Leu Leu Ile Met Arg Leu Asn Glu Ala
   2180 2185 2190
   Leu Ser Lys Val Phe Gly Asp Ile Ser Lys Thr Leu Trp Phe Glu
   2195 2200 2205
   Tyr Gln Thr Leu Ala Ala Leu Ala Gly His Leu Ser Ala Arg His
   2210 2215 2220
   Ala Ala Ala Cys Ala Arg Trp Thr Gly Met Asp Arg Leu Ala Asp
   2225 2230 2235
   Ala Pro Val His Ala Asn Glu Pro Gly Gly Ala Met Ala Pro Leu
   2240 2245 2250
   Asp Ala Gly Ala Arg Arg Asp Glu Ala Pro Leu Pro Arg Ser Glu
   2255 2260 2265
   Glu Ala Ala Gly Ala Arg Pro Ala Arg Gly Pro Ile Ala Ile Ile
   2270 2275 2280
   Gly Leu Ser Gly Arg Tyr Pro Gln Ala Glu Asp Val Gly Glu Phe
   2285 2290 2295
   Trp Glu His Leu Lys Ala Gly Arg Ser Cys Thr Arg Glu Ile Pro
   2300 2305 2310
   Ala Asp Arg Trp Ser Leu Asp Gly Phe Tyr Glu Pro Asp Pro Glu
   2315 2320 2325
   Gln Ala Val Met Glu Gly Lys Ser Tyr Gly Lys Trp Gly Gly Phe
   2330 2335 2340
   Leu Asp Gly Phe Ala Asp Phe Asp Pro Leu Phe Phe Asn Val Ser
   2345 2350 2355
   Pro Arg Glu Ser Met Asn Ile Asp Pro Gln Glu Arg Leu Phe Ile
   2360 2365 2370
   Glu Cys Cys Trp Ser Val Leu Glu Asp Ala Gly Tyr Thr Arg Asp
   2375 2380 2385
   Leu Ile Ala Ser Gln His Gly Gly Arg Val Gly Val Phe Ala Gly
   2390 2395 2400
   Ile Thr Lys Thr Gly Phe Asp Leu His Gly Pro Glu Met Arg Leu
   2405 2410 2415
   His Asp Arg Gln Ser Phe Pro His Thr Ser Phe Gly Ser Val Ala
   2420 2425 2430
   Asn Arg Val Ser Tyr Leu Leu Asn Leu Arg Gly Pro Ser Met Pro
   2435 2440 2445
   Ile Asp Thr Met Cys Ser Ser Ser Leu Thr Ala Ile His Glu Ala
   2450 2455 2460
   Cys Glu His Leu Leu Arg Asp Glu Cys Glu Ile Ala Ile Ala Gly
   2465 2470 2475
   Gly Val Asn Leu Tyr Val His Pro Ser Thr Tyr Val Gly Leu Ser
   2480 2485 2490
   Ala Gln Arg Met Leu Ser Arg Thr Gly His Cys Lys Ser Phe Gly
   2495 2500 2505
   Glu Gly Gly Asp Gly Phe Val Pro Gly Glu Cys Val Gly Ala Val
   2510 2515 2520
   Leu Leu Lys Pro Leu Gln Arg Ala Ile Asp Asp Gly Asp His Ile
   2525 2530 2535
   Tyr Ala Val Val Lys Gly Thr Ser Ile Asn His Gly Gly Lys Thr
   2540 2545 2550
   Asn Gly Tyr Thr Val Pro Asn Pro Val Ala Gln Arg Glu Leu Ile
   2555 2560 2565
   Leu Ser Ala Leu Ala Arg Ala Gly Val Asp Ala Arg Ala Val Ser
   2570 2575 2580
   Tyr Val Glu Ala His Gly Thr Gly Thr Glu Leu Gly Asp Pro Ile
   2585 2590 2595
   Glu Leu Ala Gly Leu Thr Gln Ala Phe Arg Ala Trp Thr Glu Glu
   2600 2605 2610
   Arg Glu Tyr Cys Ala Ile Gly Ser Val Lys Ser Asn Ile Gly His
   2615 2620 2625
   Cys Glu Ser Ala Ala Gly Ile Ala Gly Val Thr Lys Val Val Leu
   2630 2635 2640
   Gln Met Lys His Gly Val Leu Val Pro Ser Leu His Ser Glu Arg
   2645 2650 2655
   Leu Asn Pro His Ile Asp Phe Glu Asn Ser Pro Phe Phe Val Gln
   2660 2665 2670
   Arg Glu Leu Ser Ala Trp Arg Arg Pro Val Val Lys Val Asp Gly
   2675 2680 2685
   Lys Glu Arg Glu His Pro Arg Ile Ala Gly Val Ser Ser Phe Gly
   2690 2695 2700
   Ala Gly Gly Ala Asn Ala His Val Val Leu Glu Glu Tyr Val Glu
   2705 2710 2715
   Arg Ala Gly Glu Arg Gly Ala Leu Ala Ser Gly Ala Gly Arg Gly
       2720                2725                2730
   Ala Val Ala Val Val Leu Ser Ala Arg Ser Ala Glu Ala Leu Arg
       2735                2740                2745
   Arg Gln Ala Ser Arg Leu Arg Arg Ala Leu Glu Arg Gly Glu Tyr
       2750                2755                2760
   Gly Glu Gly Asp Leu Glu Asp Ile Ala Tyr Thr Leu Gln Thr Gly
       2765                2770                2775
   Arg Glu Ala Met Glu His Arg Leu Gly Phe Ile Ala Ser Asn Leu
       2780                2785                2790
   Asp Glu Val Lys Ser Lys Leu Ser Ala Tyr Glu Arg Gly Ala Gly
       2795                2800                2805
   Glu Leu Glu Glu Phe Trp Gln Gly Glu Val Lys Arg Glu Lys Glu
       2810                2815                2820
   Val Leu Ser Leu Leu Gly Ser Asp Glu Glu Leu Gln Glu Ala Ile
       2825                2830                2835
   Ser Lys Trp Ala Leu Arg Gly Lys Tyr Ala Arg Leu Leu Glu Leu
       2840                2845                2850
   Trp Val Lys Gly Leu Ala Phe Asp Trp Ser Arg Val His Ala Gly
       2855                2860                2865
   Arg Arg Ala Arg Arg Val Ser Leu Pro Thr Tyr Pro Phe Glu Arg
       2870                2875                2880
   Glu Arg Tyr Trp Leu Pro Ser Ser Arg Gly Arg Ser Gly Ser Ala
       2885                2890                2895
   Gly Ala Gly Ala Gly Arg Ile His Pro Leu Val His Ala Asn Thr
       2900                2905                2910
   Ser Ser Leu Ser Glu Glu Arg Phe Thr Ser Glu Phe Thr Gly Gln
       2915                2920                2925
   Glu Pro Phe Phe Ala Asp His Val Val Asp Glu Glu Arg Val Leu
       2930                2935                2940
   Pro Gly Val Ala Tyr Leu Glu Met Ala Arg Arg Ala Val Gln Glu
       2945                2950                2955
   Ala Val Gly Gly Glu Gly Glu Val Gly Glu His Gly Ile Glu Leu
       2960                2965                2970
   His His Val Val Trp Ser Gln Pro Ile Val Gln Arg Gly Ala Glu
   2975 2980 2985
   Ala Val Arg Val His Val Gln Val Glu Ser Ala Glu Gly Ser Glu
   2990 2995 3000
   Pro Ala Glu Gly Trp Thr Leu Arg Tyr Glu Ile Tyr Ser Gly Gly
   3005 3010 3015
   Ala Gly Val Ser Gly Val Ser Gly Glu Arg Val Val Ser Gly Val
   3020 3025 3030
   Ser Ser Glu Arg Val Val His Ser Gln Gly Tyr Ala Arg Leu Val
   3035 3040 3045
   Glu Lys Gln Pro Ala Pro Arg Val Asp Val Ser Glu Leu Arg Ser
   3050 3055 3060
   Arg Cys Glu Gln Glu Arg Ser Gly Ala Ser Cys Tyr Ala Ala Phe
   3065 3070 3075
   Asp Ala Met Gly Leu His Tyr Gly Pro Ser His Arg Gly Leu Val
   3080 3085 3090
   Ser Val Trp Arg Gly Glu Glu Thr Gly Gly Glu Arg Phe Ala Leu
   3095 3100 3105
   Ala Arg Val Gln Leu Pro Ala Ser Ala Met Ser Gly Arg Glu Glu
   3110 3115 3120
   Tyr Val Leu His Pro Ser Val Leu Asp Ser Ala Leu Gln Ala Thr
   3125 3130 3135
   Ile Ala Leu Ser Asp Gly Gln Thr Leu Thr Leu Pro Phe Ala Leu
   3140 3145 3150
   Asp Ala Ala Gln Val Phe Gly Ala Ser Pro Pro Ser Gly Tyr Val
   3155 3160 3165
   Val Ile Arg Glu Glu Arg Gly Gly Glu Lys Leu Thr Leu Glu Leu
   3170 3175 3180
   Cys Asp Asp Gly Gly Val Val Cys Val Arg Leu Ala Gly Phe Ala
   3185 3190 3195
   Ser Arg Ala Gln His Arg Ser Gly Val Glu Leu Leu Arg Arg Arg
   3200 3205 3210
   Trp Arg His Ser Pro Val Glu His Ser Ala Gln Ser Gly His Val
   3215 3220 3225
   Gln Arg Trp Val Leu Leu Asp Ser Ala Tyr Gly Ser Gln Ala Ala
   3230 3235 3240
   Ala Leu Ser Glu Arg Leu Pro Gly Ala Gln Ile Thr Ala Leu Arg
   3245 3250 3255
   Ala Ser Gly Gly Val Ala Glu Glu Phe Glu Gln Val Val Glu Gln
   3260 3265 3270
   Val Met Ala Thr Val Gln Thr Ile Leu Arg Glu Arg Pro Arg Gly
   3275 3280 3285
   Glu Val Leu Leu Gln Ile Ala Leu Ser Arg Gly Glu Arg Gly Glu
   3290 3295 3300
   Arg Leu Gly Ala Val Ser Gly Leu Leu Lys Ser Ala Ser Gln Glu
   3305 3310 3315
   Asn Pro Lys Leu Val Gly Gln Val Leu Glu Leu Glu Asp Ser Ala
   3320 3325 3330
   Thr Leu Leu Asp Lys Leu Glu Glu Asn Ala Ala Ala Arg Gly Asp
   3335 3340 3345
   Val Glu Ile Arg Tyr Gln Gly Gly Glu Arg Gln Thr Pro Ala Leu
   3350 3355 3360
   Glu Pro Val Val Glu Ser Arg Ala Ala Ala Ala Glu Val Pro Trp
   3365 3370 3375
   Lys Gln Asp Gly Val Tyr Leu Ile Thr Gly Gly Ala Gly Gly Leu
   3380 3385 3390
   Gly Leu Leu Phe Ala Lys Glu Ile Ile Asp Arg Ala Pro Gly Ala
   3395 3400 3405
   Thr Leu Ile Leu Thr Gly Arg Ser Arg Leu Ser Gly Thr Lys Gln
   3410 3415 3420
   Ala Ala Val Glu Ser Leu Arg Gly Arg Gly Ser Arg Val Glu Tyr
   3425 3430 3435
   Met Gln Leu Asp Val Ser Asp Gly Ala Ala Val Ser Arg Ala Val
   3440 3445 3450
   Glu Gln Leu Gln Ser Glu His Gly Gly Leu Asn Gly Leu Val His
   3455 3460 3465
   Ala Ala Gly Val Leu Gln Asp Ser Phe Val Val Asn Lys Thr Glu
   3470 3475 3480
   Ala Gln Trp Arg Ser Val Leu Gly Pro Lys Val Arg Gly Leu Val
   3485 3490 3495
   His Leu Asp Glu Ala Thr Lys His Leu Ala Leu Asp His Met Val
   3500 3505 3510
   Val Phe Ser Ser Val Ala Gly Leu Val Gly Asn Val Gly Gln Ala
   3515 3520 3525
   Asp Tyr Ala Ala Ala Asn Ala Phe Met Ala Arg Phe Ala Glu Gln
   3530 3535 3540
   Arg Ser Glu Leu Val Arg Ala Gly Arg Arg Gln Gly Arg Thr Val
   3545 3550 3555
   Ser Ile Asp Trp Pro Leu Trp Glu Ser Gly Gly Met Gln Val Asp
   3560 3565 3570
   Ala Ser Thr Val Arg Arg Leu Arg Gln Thr Tyr Gly Val Thr Pro
   3575 3580 3585
   Leu Ala Thr Glu His Gly Ile Ala Ala Phe Tyr Arg Thr Met Ala
   3590 3595 3600
   Ser Gly Leu Ala His Val Gly Val Val Ala Gly Asp Val Ala Lys
   3605 3610 3615
   Trp Arg Ser Gln Leu Ala Ala Thr His Ala Thr Ala Gly Glu Gly
   3620 3625 3630
   Asp Ala Ser His Asp Ala Gly Ser Thr Ala Val Ala Ser Asp Val
   3635 3640 3645
   Met Glu Ala Lys Ala Ala Gln Leu Leu Arg Lys Val Leu Ser Ser
   3650 3655 3660
   Ser Leu Lys Val Pro Leu Glu Arg Ile Glu Gly Asp Thr Pro Leu
   3665 3670 3675
   Asp Arg Tyr Gly Ile Glu Ser Val Met Val Met Glu Leu Thr Arg
   3680 3685 3690
   Glu Leu Glu Lys Thr Phe Gly Pro Leu Ser Lys Thr Leu Phe Phe
   3695 3700 3705
   Glu His Gln Asp Ile Arg Ser Leu Ser Arg Tyr Phe Ala Glu Ala
   3710 3715 3720
   His Arg Arg Arg Leu Ile Glu Val Leu Gly Gly Asp Asp Glu Pro
   3725 3730 3735
   Ala Lys Ser Ala Ala Arg Arg Pro Gln Glu Pro Ala Ser Ala Ser
   3740 3745 3750
   Ala Pro Arg Arg Asp Arg Leu Val Thr Gln Pro Arg Gly Gly Ala
   3755 3760 3765
   Val Ala Arg Pro Leu Arg Pro His Asp Ile Ala Ile Ile Gly Val
   3770 3775 3780
   Ser Gly Arg Tyr Pro Gly Ala Asp Asp Leu Glu Ala Phe Trp Glu
   3785 3790 3795
   Asn Leu Lys Ser Gly Arg Asp Cys Ile Thr Glu Ile Pro Glu Asp
   3800 3805 3810
   Arg Trp Ser Leu Glu Glu Phe Phe Asp Pro Gln Arg Gly Lys Pro
   3815 3820 3825
   Gly Lys Ile Tyr Ser Arg Trp Gly Gly Phe Ile Asp Gly Val Asp
   3830 3835 3840
   Arg Phe Asp Pro Leu Phe Phe Asn Ile Ser Pro Arg Glu Ala Gln
   3845 3850 3855
   Phe Met Asp Pro Gln Glu Arg Leu Phe Leu Glu Cys Ala Tyr Glu
   3860 3865 3870
   Thr Ile Glu Asp Ala Gly Tyr Thr Arg Asp Ala Leu Lys Lys Pro
   3875 3880 3885
   Ala Ala Ala Gly Gly Pro Pro Gly Thr Val Gly Val Phe Val Gly
   3890 3895 3900
   Val Met Tyr Glu Glu Tyr Gln Leu Phe Gly Ala Gln Ala Gln Val
   3905 3910 3915
   Leu Gly Gln Pro Phe Ala Leu Asn Gly Asn Pro Ser Ser Ile Ala
   3920 3925 3930
   Asn Arg Val Ser Tyr Phe Leu Asp Cys His Gly Pro Ser Met Ala
   3935 3940 3945
   Val Asp Thr Met Cys Ser Ser Ser Leu Thr Ala Ile His Leu Ala
   3950 3955 3960
   Cys Gln Ser Leu Leu His Gly Gly Cys Asp Leu Ala Ile Ala Gly
   3965 3970 3975
   Gly Val Asn Val Ser Ile His Pro Asn Lys Tyr Leu Ala Leu Ser
   3980 3985 3990
   Gln Gly Gln Phe Ala Ser Ser Arg Gly Arg Cys Glu Ser Phe Gly
   3995 4000 4005
   Glu Gly Gly Asp Gly Tyr Val Pro Ala Glu Gly Val Gly Ala Val
   4010 4015 4020
   Leu Leu Lys Pro Leu Gln Arg Ala Ile Asp Asp Gly Asp His Ile
   4025 4030 4035
   Tyr Ala Val Ile Lys Gly Thr Ser Ile Asn His Gly Gly Lys Thr
   4040 4045 4050
   Asn Gly Tyr Thr Val Pro Ser Pro Val Ala Gln Ala Gln Leu Val
   4055 4060 4065
   Ala Gln Ala Leu Thr Glu Ala Lys Ile Glu Pro Arg Ala Val Ser
   4070 4075 4080
   Tyr Val Glu Ala His Gly Thr Gly Thr Glu Leu Gly Asp Pro Ile
   4085 4090 4095
   Glu Leu Ala Gly Leu Thr Gln Ala Phe Arg Arg Arg Thr Glu Glu
   4100 4105 4110
   Arg Gly Tyr Cys Ala Ile Gly Ser Val Lys Ser Asn Ile Gly His
   4115 4120 4125
   Cys Glu Ser Ala Ala Gly Ile Ala Gly Val Thr Lys Val Val Leu
   4130 4135 4140
   Gln Met Lys His Gly Val Leu Val Pro Ser Leu His Ser Glu Arg
   4145 4150 4155
   Leu Asn Pro His Ile Asp Phe Glu Asn Ser Pro Phe Phe Val Gln
   4160 4165 4170
   Arg Glu Leu Ser Ala Trp Arg Arg Pro Val Val Lys Val Asp Gly
   4175 4180 4185
   Lys Glu Arg Glu His Pro Arg Ile Ala Gly Val Ser Ser Phe Gly
   4190 4195 4200
   Ala Gly Gly Ala Asn Ala His Val Val Leu Glu Glu Tyr Val Glu
   4205 4210 4215
   Arg Ala Gly Glu Arg Gly Ala Leu Ala Ser Gly Ala Gly Arg Gly
   4220 4225 4230
   Ala Val Ala Val Val Leu Ser Ala Arg Ser Ala Glu Ala Leu Arg
   4235 4240 4245
   Arg Gln Ala Ser Arg Leu Arg Arg Ala Leu Glu Arg Gly Glu Tyr
   4250 4255 4260
   Gly Glu Gly Asp Leu Glu Asp Ile Ala Tyr Thr Leu Gln Thr Gly
   4265 4270 4275
   Arg Glu Ala Met Glu His Arg Leu Gly Phe Ile Ala Ser Asn Leu
   4280 4285 4290
   Asp Glu Val Lys Ser Lys Leu Ser Ala Tyr Glu Arg Gly Ala Gly
   4295 4300 4305
   Glu Leu Glu Glu Phe Trp Gln Gly Glu Val Lys Arg Glu Arg Glu
   4310 4315 4320
   Met Leu Ser Leu Leu Gly Ser Asp Glu Glu Leu Gln Glu Ala Ile
   4325 4330 4335
   Gly Lys Trp Ala Leu Arg Gly Lys Tyr Ala Arg Leu Leu Glu Leu
   4340 4345 4350
   Trp Val Lys Gly Leu Ala Phe Asp Trp Ser Arg Val His Ala Gly
   4355 4360 4365
   Arg Arg Ala Arg Arg Val Ser Leu Pro Thr Tyr Pro Phe Glu Arg
   4370 4375 4380
   Glu Arg Tyr Trp Leu Pro Ser Ser Arg Gly Arg Ser Gly Ala Ala
   4385 4390 4395
   Gly Ala Gly Ala Gly Ala Gly Ala Gly Arg Ile His Pro Leu Val
   4400 4405 4410
   His Ala Asn Thr Ser Ser Leu Ser Glu Glu Arg Phe Thr Ser Glu
   4415 4420 4425
   Phe Thr Gly Gln Glu Pro Phe Phe Ala Asp His Val Val Asp Glu
   4430 4435 4440
   Glu Arg Val Leu Pro Gly Val Ala Tyr Leu Glu Met Ala Arg Arg
   4445 4450 4455
   Ala Val Gln Glu Ala Val Gly Gly Glu Gly Glu Val Gly Glu His
   4460 4465 4470
   Gly Ile Glu Leu His His Val Val Trp Ser Gln Pro Ile Val Gln
   4475 4480 4485
   Arg Gly Ala Glu Ala Val Arg Val His Val Gln Val Glu Ser Ala
   4490 4495 4500
   Glu Gly Ser Glu Pro Ala Glu Gly Trp Thr Leu Arg Tyr Glu Ile
   4505 4510 4515
   Tyr Ser Gly Gly Ala Gly Val Ser Gly Glu Arg Val Val His Ser
   4520 4525 4530
   Gln Gly Tyr Ala Arg Leu Val Glu Lys Gln Pro Ala Pro Arg Val
   4535 4540 4545
   Asp Val Ser Glu Leu Arg Ser Arg Cys Glu Gln Glu Trp Ser Gly
   4550 4555 4560
   Ala Ser Cys Tyr Ala Ala Phe Asp Ala Met Gly Leu His Tyr Gly
   4565 4570 4575
   Pro Ser His Arg Gly Leu Val Ser Val Trp Arg Gly Glu Glu Thr
   4580 4585 4590
   Gly Gly Glu Arg Phe Ala Leu Ala Arg Val Gln Leu Pro Ala Ser
   4595 4600 4605
   Ala Met Ser Gly Arg Glu Glu Tyr Val Leu His Pro Ser Val Leu
   4610 4615 4620
   Asp Ser Ala Leu Gln Ala Thr Ile Ala Leu Ser Asp Gly Gln Thr
   4625 4630 4635
   Leu Arg Leu Pro Phe Ala Leu Asp Ala Val Glu Val Phe Gly Ala
   4640 4645 4650
   Ser Pro Pro Ser Gly Tyr Val Val Ile Arg Glu Glu Arg Gly Gly
   4655 4660 4665
   Glu Lys Leu Thr Leu Glu Leu Cys Asp Asp Gly Gly Val Val Cys
   4670 4675 4680
   Val Arg Leu Ala Gly Phe Ala Ser Arg Val Gln His Arg Ser Gly
   4685 4690 4695
   Val Glu Leu Leu Gln Arg Arg Trp Arg Arg Ser Pro Val Glu His
   4700 4705 4710
   Ser Ala Gln Ser Gly His Val Gln Arg Trp Val Leu Leu Asp Ser
   4715 4720 4725
   Ala Tyr Gly Ser Gln Ala Ala Ala Leu Ser Glu Arg Leu Pro Gly
   4730 4735 4740
   Ala Gln Ile Thr Ala Leu Arg Ala Ser Gly Gly Val Ala Glu Glu
   4745 4750 4755
   Phe Glu Gln Phe Val Glu Gln Val Met Ala Thr Val Gln Thr Ile
   4760 4765 4770
   Leu Arg Glu Arg Pro Arg Gly Glu Val Leu Leu Gln Ile Ala Leu
   4775 4780 4785
   Ser Arg Gly Glu Arg Gly Glu Arg Leu Gly Ala Val Ser Gly Leu
   4790 4795 4800
   Leu Lys Ser Ala Ser Gln Glu Asn Pro Lys Leu Val Gly Gln Val
   4805 4810 4815
   Leu Glu Leu Glu Asp Ser Ala Thr Leu Leu Asp Lys Leu Glu Glu
   4820 4825 4830
   Asn Ala Ala Ala Arg Gly Asp Val Glu Ile Arg Tyr Gln Gly Gly
   4835 4840 4845
   Glu Arg Gln Thr Pro Ala Leu Glu Pro Val Val Glu Ser Arg Ala
   4850 4855 4860
   Ala Ala Ala Glu Val Pro Trp Lys Gln Asp Gly Val Tyr Leu Ile
   4865 4870 4875
   Thr Gly Gly Ala Gly Gly Leu Gly Leu Leu Phe Ala Lys Glu Ile
   4880 4885 4890
   Ile Asp Arg Ala Pro Gly Ala Thr Leu Ile Leu Thr Gly Arg Ser
   4895 4900 4905
   Arg Leu Ser Gly Thr Lys Gln Ala Ala Val Glu Ser Leu Arg Gly
   4910 4915 4920
   Arg Gly Ser Arg Val Glu Tyr Met Gln Leu Asp Val Ser Asp Gly
   4925 4930 4935
   Ala Ala Val Ser Arg Ala Val Glu Gln Leu Gln Ser Glu His Gly
   4940 4945 4950
   Gly Leu Asn Gly Leu Val His Ala Ala Gly Val Leu Gln Asp Ser
   4955 4960 4965
   Phe Val Val Asn Lys Thr Glu Ala Gln Trp Arg Ser Val Leu Gly
   4970 4975 4980
   Pro Lys Val Arg Gly Leu Val His Leu Asp Glu Ala Thr Lys His
   4985 4990 4995
   Leu Ala Leu Asp His Met Val Val Phe Ser Ser Val Ala Gly Leu
   5000 5005 5010
   Val Gly Asn Val Gly Gln Ala Asp Tyr Ala Ala Ala Asn Ala Phe
   5015 5020 5025
   Met Ala Arg Phe Ala Glu Gln Arg Ser Glu Leu Val Arg Ala Gly
   5030 5035 5040
   Arg Arg Gln Gly Arg Thr Val Ser Ile Asp Trp Pro Leu Trp Glu
   5045 5050 5055
   Ser Gly Gly Met Gln Met Asp Ala Ser Thr Val Gln Arg Leu Arg
   5060 5065 5070
   Gln Thr His Gly Val Ile Pro Leu Ala Thr Glu His Gly Ile Ala
   5075 5080 5085
   Ala Phe Tyr Arg Thr Met Ala Ser Gly Leu Ala His Val Gly Val
   5090 5095 5100
   Phe Ala Thr Glu Ala Ser Arg Glu Ala Leu Thr Asp Ala Asp Asp
   5105 5110 5115
   Asp Ser Thr Leu Tyr Met Glu Leu Phe Glu Thr Met Val Glu Gly
   5120 5125 5130
   Val Leu Ser Glu Asp Glu Phe Arg Glu Trp Leu Val Ala
   5135 5140 5145
<210> 30
   <211> 3828
   <212> PRT
   <213> Sorangium cellulosum
<400> 30
   Met Thr Gln Gly Ser His Asp Ala Asp Ala Arg Asp Glu Ile Ala Ala
   1 5 10 15
   Leu Phe Ser Leu Ile Lys Glu Gln Lys Leu Thr Arg Ala Glu Ala Leu
   20 25 30
   Asp Arg Leu Arg Ala Val Arg Ala Arg Leu Arg Ser Glu Arg Ala Ala
   35 40 45
   Ala Pro Ala Ala Ala Gln Ile Ala Ala Thr Pro Pro Ser Gly Gly Ala
   50 55 60
   Val Glu Leu Val Glu Ala Glu Val Val Asp Tyr Phe Arg Lys Gln Val
   65 70 75 80
   Ala Ser Ala Leu Lys Leu Pro Leu Asp Arg Val Asp Ala Asp Thr Pro
   85 90 95
   Leu Glu Arg Tyr Gly Ile Asp Ser Val Val Val Met His Leu Thr Ser
   100 105 110
   Ala Leu Glu Lys His Phe Gly Pro Leu Ser Lys Thr Leu Phe Phe Glu
   115 120 125
   Phe Asp Thr Ile Asp Ala Val Ala Arg His Phe Leu Glu Asn His Arg
   130 135 140
   Ala Ala Leu Ser Arg Ile Leu Gly Lys Ser Ala Pro Ser Ala Leu Asp
   145 150 155 160
   Arg Pro Val Ser Ala Pro Pro Arg Ser Ala Arg Pro Leu Gly Arg Pro
   165 170 175
   Val Val Ala Val Arg Lys Ala Val Ser Ala Ser Val Asp Ser Pro Val
   180 185 190
   Ala Ala Pro Pro Val Ala Ala Pro Pro Val Ala Ala Pro Pro Val Ala
   195 200 205
   Ala Pro Pro Val Ala Ala Pro Pro Ile Ala Ala Pro Pro Ile Ala Ala
   210 215 220
   Pro Pro Val Ala Ala Pro Pro Gly Val Ser Ala Arg Gly Ala Gly Pro
   225 230 235 240
   Tyr Asp Ile Ala Ile Ile Gly Val Ser Gly Arg Tyr Pro Gln Ala Asn
   245 250 255
   Asp Leu Gly Glu Phe Trp Asp Asn Leu Arg Ser Gly Lys Asp Cys Ile
   260 265 270
   Thr Glu Ile Pro Pro Asp Arg Trp Asp Tyr Arg Ala Tyr Phe Asp Ala
   275 280 285
   Gln Lys Gly Arg Ile Gly Lys Ser Tyr Ser Lys Trp Gly Gly Phe Ile
   290 295 300
   Asp Gly Val Asp Arg Phe Asp Pro Leu Phe Phe His Val Ser Pro Arg
   305 310 315 320
   Glu Ala Gln Leu Ile Asp Pro Gln Glu Arg Leu Phe Leu Gln Cys Ala
   325 330 335
   Tyr Glu Thr Leu Glu Asp Ala Gly Tyr Thr Arg Asp Ser Leu Ser Gly
   340 345 350
   Pro Ala Val Arg Gly Arg Val Gly Val Phe Val Gly Val Met Tyr Glu
   355 360 365
   Glu Tyr Gln Leu Tyr Gly Val Pro Ser Gln Gly Ala Glu Gln Pro Leu
   370 375 380
   Val Pro Ser Gly Arg Pro Ser Ser Ile Ala Asn Arg Val Ser Phe Phe
   385 390 395 400
   Cys Asn Phe His Gly Pro Ser Val Ala Val Asp Thr Met Cys Ser Ser
   405 410 415
   Ala Leu Thr Ala Ile His Phe Ala Cys Arg Ser Ile Leu Glu Gly Glu
   420 425 430
   Cys Asp Leu Ala Ile Ala Gly Gly Val Asn Val Ser Val His Pro Asn
   435 440 445
   Lys Tyr Leu Ala Leu Ser Gln Gly Gln Phe Val Ser Ser Ala Gly Arg
   450 455 460
   Cys Glu Ser Phe Gly Glu Gly Gly Asp Gly Tyr Val Pro Ser Glu Gly
   465 470 475 480
   Val Gly Ala Val Leu Leu Lys Pro Leu Ser Arg Ala Ile Ala Asp Arg
   485 490 495
   Asp His Ile Tyr Gly Val Ile Lys Gly Thr Ser Val Asn His Gly Gly
   500 505 510
   Arg Thr Asn Gly Tyr Ser Val Pro Asn Pro Asn Ala Gln Ser Glu Val
   515 520 525
   Ile Thr Asp Ala Leu Gln Arg Ala Gly Ile Asp Pro Arg Ala Ile Ser
   530 535 540
   Tyr Ile Glu Ala His Gly Thr Gly Thr Ser Leu Gly Asp Pro Ile Glu
   545 550 555 560
   Ile Ala Gly Leu Ser Lys Ala Phe Gln Lys Ala Ser Gly Glu Ala Leu
   565 570 575
   Val Gln Thr Cys Ala Ile Gly Ser Val Lys Ser Asn Ile Gly His Gly
   580 585 590
   Glu Ser Ala Ala Gly Ile Ala Gly Val Thr Lys Val Leu Leu Gln Leu
   595 600 605
   Glu His Arg Gln Leu Ala Pro Ser Leu His Ala Arg Thr Leu Asn Pro
   610 615 620
   His Ile Asp Phe Asp Arg Thr Pro Phe Arg Val Gln Gln Glu Leu Ala
   625 630 635 640
   Ala Trp Pro Arg Pro Arg Leu Arg Ile Asp Gly Val Glu Arg Glu Val
   645 650 655
   Pro Arg Leu Ala Gly Val Ser Ala Phe Gly Ala Gly Gly Ser Asn Ala
   660 665 670
   His Val Ile Ile Glu Glu Tyr Val Ala Arg Gly Glu Ala Pro Ala Pro
   675 680 685
   Pro Ala Glu Leu Pro Val Val Ile Val Leu Ser Ala Lys Ser Glu Ala
       690                 695                 700
   Gln Leu Arg Ala Gln Ala Arg Asn Leu Val Ala Ala Ile Arg Arg Glu
   705                 710                 715                 720
   Gly Tyr Thr Glu Ala Asp Leu Pro Ser Ile Ala Tyr Thr Leu Gln Val
                   725                 730                 735
   Gly Arg Glu Pro Met Glu Glu Arg Ala Ala Thr Thr Val Ala Ser Leu
               740                 745                 750
   Ala Ala Leu Val Glu Arg Leu Glu Met Leu Cys Ala Glu Pro Pro Asp
           755                 760                 765
   Glu Arg Asp Trp Thr Arg Gly Ser Val Glu Thr Ser Lys Ala Ala Ile
       770                 775                 780
   Ala Phe Phe Lys Ser Asp Asp Glu Gly Arg Glu Leu Leu Ala Ser Trp
   785                 790                 795                 800
   Val Phe Arg Arg His Tyr Asp Arg Ile Ala Gly Leu Trp Val Lys Gly
                   805                 810                 815
   Leu Pro Phe Asp Trp Glu Arg Leu Tyr Pro Ala Gly Gly Pro Gly Arg
               820                 825                 830
   Val Arg Leu Pro Lys Tyr Pro Phe Ala Asn Glu Arg Tyr Trp Val Glu
           835                 840                 845
   Thr Arg Pro Ala Ala Arg Pro Ser Gly Ala Ala Arg Leu His Pro Leu
       850                 855                 860
   Leu His Glu Asn Val Ser Thr Ala Phe Glu Ser Lys Phe Val Ser Thr
   865                 870                 875                 880
   Phe Thr Gly Asp Glu Pro Phe Leu Arg Asp His Arg Val Arg Arg Asp
                   885                 890                 895
   Ala Val Leu Pro Gly Ala Cys His Leu Glu Met Ala Leu Ala Ala Ala
               900                 905                 910
   Lys Arg Ser Leu Asp Thr Asp Arg Ala Val Thr Leu Ala Gly Val Ala
           915                 920                 925
   Trp Leu Arg Pro Leu Ala Val Ala Gln Gly Gly Arg Met Val Gln Ile
       930                 935                 940
   Asp Val Ile Ala Ser Ser Glu Thr Glu Ala Glu Phe Glu Ile His Ser
   945                 950                 955                 960
   Gly Pro Gly Asp Asp Ala Asp Val Val Tyr Cys Arg Gly Leu Ala Arg
   965 970 975
   Val Val Ala Ala Ala Arg Pro Pro Ala Leu Asp Leu Ser Arg Leu Arg
   980 985 990
   Glu Ala Cys Ser Arg Ala Glu Leu Ala Pro Glu Arg Ala Tyr Glu Ala
   995 1000 1005
   Leu Gly Ala Ala Gly Leu Glu Tyr Gly Ala Ala His Arg Ser Ile
   1010 1015 1020
   Glu Glu Leu Arg Val Gly Ser Asp Ala Ala Gly Arg Pro Gln Val
   1025 1030 1035
   Leu Ala Arg Leu Ala Ile Pro Glu Cys Val Ala Gly Ala Ala Gly
   1040 1045 1050
   Glu Phe Gln Leu His Pro Ser Ile Val Asp Gly Ala Ile Gln Ala
   1055 1060 1065
   Ala Val Gly Leu Ala Leu Pro Ala Gly Gly Ala Ser Glu Arg Ala
   1070 1075 1080
   Ser Arg Pro Ala Leu Pro Ser Gly Leu Ala Ala Leu Ser Leu His
   1085 1090 1095
   Gly Pro Thr Pro Gln Arg Ala Tyr Ala Trp Leu Arg Phe Arg Asp
   1100 1105 1110
   Gly Ser Ser Pro Asp Asp Asp Val Arg Ala Ile Asp Ile Ser Leu
   1115 1120 1125
   Cys Asp Glu Leu Gly Glu Val Arg Ala Gln Leu Asp Gly Leu Thr
   1130 1135 1140
   Leu Arg Val Met Ser Asp Ala Pro Arg Ala Glu Thr Met Leu Leu
   1145 1150 1155
   Glu Pro Arg Trp Ala Glu Glu Pro Ala Arg Gly Ala Arg Ala His
   1160 1165 1170
   Ala Ala Ile Ala Asp Glu Asp Arg Arg Leu Phe Val Val Gly Ala
   1175 1180 1185
   Leu Gly Ser Glu Gln Arg Ala Ala Leu Glu Arg Gln Leu Arg Gly
   1190 1195 1200
   Gly Glu Leu His Phe Val Ala Pro Gly Thr Gly Ser Leu Ala Glu
   1205 1210 1215
   Arg Tyr Glu Arg Val Ala Ala Ala Leu Leu Gly Val Leu Arg Ala
   1220 1225 1230
   Leu Leu Val Arg Ala Pro Ala Arg Arg Arg Leu Leu Gln Val Val
   1235 1240 1245
   Val Ala Ser Ser Asp Pro Glu Glu Gly Ala Leu Tyr Ala Gly Leu
   1250 1255 1260
   Ser Ala Met Leu Lys Ser Ala Ser Arg Glu Asn Pro His Leu Val
   1265 1270 1275
   Thr Gln His Ile Thr Ile Gly Glu Pro Ala Ser Ala Asn Gly Trp
   1280 1285 1290
   Phe Asp Val Leu Asp Asp Glu Ala Ala Arg Leu Asp Ala Asp Glu
   1295 1300 1305
   Ile Arg Tyr Arg Gly Glu Arg Arg Glu Val Arg Arg Leu Val Glu
   1310 1315 1320
   Leu Ala Arg Leu Asp Ala Ser Asp Gly Ala Ala Pro Pro Trp Arg
   1325 1330 1335
   Asp Gly Gly Val Tyr Trp Ile Thr Gly Gly Leu Gly Gly Leu Gly
   1340 1345 1350
   Leu Val Val Ala Thr Ser Ile Ala Arg Ala Ser Arg Gly Ala Ala
   1355 1360 1365
   Leu Val Val Thr Gly Arg Ser Pro Val Val Gly Ser Asp Gly Gln
   1370 1375 1380
   Ser Lys Leu Ala Glu Leu Arg Ala Leu Gly Ala Arg Val Glu Tyr
   1385 1390 1395
   Arg Ser Val Asp Val Ala Asp Arg Ala Ala Val Glu Asp Leu Val
   1400 1405 1410
   Arg His Val Arg Ala Thr Tyr Gly Gln Leu Asp Gly Val Val His
   1415 1420 1425
   Thr Ala Gly Val Val Glu Asp Ser Phe Leu Val Arg Lys Thr Asp
   1430 1435 1440
   Asp Glu Leu Arg Arg Val Phe Ala Ala Lys Val Ala Gly Ile Ala
   1445 1450 1455
   Asn Leu Asp Glu Ala Thr Arg Gly Glu Pro Leu Asp Cys Phe Val
   1460 1465 1470
   Cys Phe Ser Ser Val Ala Gly Ala Met Gly Asn Val Gly Gln Ser
   1475 1480 1485
   Asp Tyr Ala Ala Ala Asn Ala Phe Met Asp Ala Tyr Ala Arg Arg
   1490 1495 1500
   Arg Ala Glu Gln Val Glu Arg Gly Ala Arg Ser Gly Arg Thr Ile
   1505 1510 1515
   Ser Ile Gly Trp Pro Leu Trp Ala Glu Gly Gly Leu His Val Asp
   1520 1525 1530
   Ser Ala Val Glu Ala Arg Leu Arg Arg Asp Gly Leu Thr Pro Leu
   1535 1540 1545
   Gly Thr Ala Ala Gly Leu Ala Ala Leu Ala Arg Cys Ile Arg Asp
   1550 1555 1560
   Pro Gly Arg Pro Ser His Val Val Val Leu Phe Gly Asp Ala Arg
   1565 1570 1575
   Ala Ile Arg Gly Ala Leu Leu Ala Arg Ser Asn Arg Pro Ser Pro
   1580 1585 1590
   Gly Asp Leu Ala Ala Ala Ser Pro Ala Asn Ala Ser Thr Pro Ala
   1595 1600 1605
   Ile Arg Glu Glu Ala Leu Arg Gln Val Lys Lys Leu Leu Ala Pro
   1610 1615 1620
   Val Ile Lys Leu Pro Val Glu Arg Ile Asp Ala Ala Ala Ser Phe
   1625 1630 1635
   Glu Thr Tyr Gly Ile Glu Ser Val Met Ala Val Glu Leu Thr Asp
   1640 1645 1650
   Arg Leu Glu Ala Val Phe Gly Pro Leu Ser Lys Thr Leu Leu Phe
   1655 1660 1665
   Glu Ala Lys Thr Val Arg Glu Leu Ala Asp Tyr Phe Val Glu His
   1670 1675 1680
   His Ala Ala Thr Leu Gly Ser Leu Leu Gly Gly Ala Thr Ala Pro
   1685 1690 1695
   Ala Ala Pro Ala Thr Ala Val Ser Ala Gly Ala Gly Pro Ala Arg
   1700 1705 1710
   Arg Arg Ala Arg Arg Leu Pro Ser Arg Gly Arg Ala Gly Glu Ala
   1715 1720 1725
   Arg Glu Ala Ala Ile Ala Ile Ile Gly Leu Ser Gly Arg Tyr Pro
   1730 1735 1740
   Gln Ala Asn Asp Leu Ala Glu Phe Trp Glu Asn Leu Arg Ser Gly
   1745 1750 1755
   Lys Asp Cys Ile Thr Glu Ile Pro Arg Asp Arg Trp Asp His Arg
   1760 1765 1770
   Ala Tyr Phe Asp Ala Glu Arg Gly Lys Pro Gly Lys Ser Tyr Ser
   1775 1780 1785
   Lys Trp Gly Gly Phe Leu Glu Gly Ile Asp Gln Phe Asp Pro Leu
   1790 1795 1800
   Phe Phe Asn Ile Ser Pro Arg Glu Ala Gln Leu Met Asp Pro Gln
   1805 1810 1815
   Glu Arg Leu Phe Leu Leu Cys Ala Tyr Gln Thr Leu Glu Asp Ala
   1820 1825 1830
   Gly Tyr Thr Arg Glu Ala Leu Ser Gly Pro Ala Val Arg Gly Arg
   1835 1840 1845
   Val Gly Val Phe Ala Gly Val Met Tyr Glu Glu Tyr His Leu Tyr
   1850 1855 1860
   Ser Thr Pro Ser Ala Glu Val Ala Gln Pro Tyr Ile Pro Gly Gly
   1865 1870 1875
   Thr Leu Ser Ser Ile Ala Asn Arg Val Ser Tyr Phe Cys Asp Phe
   1880 1885 1890
   His Gly Pro Ser Leu Thr Leu Ser Thr Met Cys Ser Ser Ser Leu
   1895 1900 1905
   Thr Ala Ile His Leu Ala Cys Gln Ser Leu Gln Gln Gly Glu Cys
   1910 1915 1920
   Glu Leu Ala Leu Ala Gly Gly Val Asn Ala Ser Ile His Pro Asn
   1925 1930 1935
   Lys Tyr Leu Gly Leu Ser Gln Gly Gln Phe Val Ser Ser Thr Gly
   1940 1945 1950
   Arg Cys Glu Ser Phe Gly Ala Gly Gly Asp Gly Tyr Val Pro Gly
   1955 1960 1965
   Glu Gly Val Gly Ala Val Leu Leu Lys Pro Leu Ser Arg Ala Ile
   1970 1975 1980
   Ala Asp Arg Asp His Ile Tyr Gly Val Ile Lys Gly Thr Ser Ile
   1985 1990 1995
   Asn His Gly Gly Lys Thr Asn Gly Tyr Thr Val Pro Asn Pro Thr
   2000 2005 2010
   Ala Gln Thr Asp Leu Ile Leu Ala Ala Ile Glu Lys Ala Arg Val
   2015 2020 2025
   Asp Pro Arg Ala Ile Ser Tyr Val Glu Ala His Gly Thr Gly Thr
   2030 2035 2040
   Ser Leu Gly Asp Pro Ile Glu Ile Ala Gly Leu Ser Lys Ala Phe
   2045 2050 2055
   Gln Arg Ala Ser Gly Glu Ala Ile Val Gln Thr Cys Ala Ile Gly
   2060 2065 2070
   Ser Val Lys Ser Asn Ile Gly His Cys Glu Ser Ala Ala Gly Ile
   2075 2080 2085
   Ala Gly Val Thr Lys Val Leu Leu Gln Leu Lys His Arg Gln Leu
   2090 2095 2100
   Val Pro Ser Leu His Ser Arg Thr Leu Asn Pro Tyr Ile Asp Phe
   2105 2110 2115
   Gly Ser Thr Pro Phe Arg Val Gln Gln Glu Leu Glu Ala Trp Arg
   2120 2125 2130
   Arg Pro Arg Leu Arg Val Asp Gly Val Glu Arg Glu Ala Pro Arg
   2135 2140 2145
   Ile Ala Ala Ile Ser Ala Phe Gly Ala Gly Gly Ser Asn Ala His
   2150 2155 2160
   Leu Ile Ile Glu Glu His Val Glu Leu Asp Asp Ala Pro Thr Ser
   2165 2170 2175
   Val Ser Asp Ala Pro Val Val Ile Val Leu Ser Ala Ala Thr Glu
   2180 2185 2190
   Ala Gln Leu Arg Ala Gln Ala Arg Asn Leu Leu Ala Ala Ile Arg
   2195 2200 2205
   Arg Glu Gly Tyr Thr Gln Arg Asp Leu Pro Ser Ile Ala Tyr Thr
   2210 2215 2220
   Leu Gln Val Gly Arg Glu Pro Met Gln Glu Arg Met Ala Thr Thr
   2225 2230 2235
   Val Thr Thr Met Ser Ser Leu Leu Glu Arg Leu Glu Arg Ala Cys
   2240 2245 2250
   Ser Glu Pro Pro Asp Leu Gly Asp Trp Val Arg Gly Val Ala Glu
   2255 2260 2265
   Gly Arg Gly Glu Leu Val Ser Val Phe Ala Gln Asp Asp Asp Phe
   2270 2275 2280
   Arg Gly Leu Val Arg Ala Trp Leu Glu Arg Gly Lys Leu Ser Arg
   2285 2290 2295
   Leu Val Glu Leu Trp Val Lys Gly Leu Thr Phe Asp Trp Asp Gln
   2300 2305 2310
   Val Pro Pro Ala Gly Val Arg Pro Arg Arg Val Ser Leu Pro Pro
   2315 2320 2325
   Tyr Pro Phe Ala Met Lys Arg Tyr Trp Gly Pro Trp Pro Ala Pro
   2330 2335 2340
   Ser Ala Ala Pro Pro Ala Thr Ala Pro Ser Arg Pro Ala Ser Pro
   2345 2350 2355
   Pro Thr Ser Ile Ala Leu Ala Gly Ala Asp Ala Ala Val Arg Ser
   2360 2365 2370
   Asp Ala Ala Ser Pro Pro Ile Ala Arg Ser Ala Pro Lys Glu Arg
   2375 2380 2385
   Ala Ala Glu Ile Arg Ala Asp Ala Ala Pro Ile Ser Leu Val Ala
   2390 2395 2400
   Leu Val Pro Pro Ala Ala Met Asp Thr Ala Ala Ser Ser Ala Ala
   2405 2410 2415
   Arg Ser Ala Pro Val Ala Ile Ser Leu Ser Pro Leu Arg Ala Glu
   2420 2425 2430
   Pro Ala Ser Ala Ala Val Ala Arg Arg Ala Ser Asp Glu Pro Pro
   2435 2440 2445
   Ala Ser Gly Gly Glu Pro Leu Thr Gly Arg Leu Arg Ala Ser Leu
   2450 2455 2460
   Ala Arg Ala Leu Cys Thr Asp Glu Ala Asp Leu Asp Gln Asp Ala
   2465 2470 2475
   Val Phe Asn Asp Leu Gly Leu Asp Ser Val Ile Gly Val Glu Trp
   2480 2485 2490
   Ala Arg Ser Ile Glu Arg Glu Leu Asp Val Pro Leu Thr Ala Ser
   2495 2500 2505
   Thr Leu Tyr Asp His Pro Thr Leu Thr Arg Leu Ser Ala His Val
   2510 2515 2520
   Ser Gly Leu Met Thr Ser Ala Arg Ala Asp Leu Arg Ala Ala Pro
   2525 2530 2535
   Gly Pro Ala Pro Glu Val Pro Glu Ala Pro Ala Ser Pro Ala Leu
   2540 2545 2550
   Pro Ala Arg Gly Ala Gln His Glu Ile Ser Ile Pro Glu Val Ala
   2555 2560 2565
   Pro Arg Pro Val Glu Glu Leu Val Ala Gly Leu Thr Gln Thr Leu
   2570 2575 2580
   Ala Ala Ala Leu Tyr Ala Asp Ala Ser Asp Ile Asp Pro Asp Arg
   2585 2590 2595
   Leu Phe Val Asp His Gly Val Asp Ser Val Ile Ser Val Glu Trp
   2600 2605 2610
   Val Arg Arg Ile Asn Ala Thr Phe His Leu Asp Leu Lys Ala Thr
   2615 2620 2625
   Val Leu Phe Asp His Pro Thr Ile His Gln Leu Ala Glu His Leu
   2630 2635 2640
   Ala Thr Leu Asp Ala Thr Ala Val Arg Ala Ala Val Asp Gly Ile
   2645 2650 2655
   Ser Ala Ala Glu Arg Ala Gly Ala His Glu Ser Thr Ala Arg Arg
   2660 2665 2670
   Glu Arg Glu Gly Ala Ala Glu Arg Glu Ala Ala Ala Arg Arg Ala
   2675 2680 2685
   Arg Glu Asp Ala Ala Glu Arg Gln Val Ala Ala Arg His Glu Pro
   2690 2695 2700
   Glu Ala Ser Phe Glu Val Glu Ala Arg Arg Ala Ser Asp Ala Val
   2705 2710 2715
   Ala Cys Val Ala Val Arg Glu Arg Val Ile Ala Ala Gly Pro Gly
   2720 2725 2730
   Glu Ala Ala Cys Arg Ile Ala Val Ile Gly Ala Ser Gly Arg Tyr
   2735 2740 2745
   Pro Arg Ala Gly Asp Leu Asp Glu Tyr Trp Ser Asn Leu Ala Arg
   2750 2755 2760
   Gly Val Asp Ala Val Ala Glu Val Pro Ala Ser Arg Trp Ser Val
   2765 2770 2775
   Glu Arg Tyr Tyr Asp Pro Ala Val Pro Ser Thr Gly Lys Val Tyr
   2780 2785 2790
   Cys Lys Trp Leu Ala Ala Leu Glu Asp Val Asp Arg Phe Asp Pro
   2795 2800 2805
   Leu Phe Phe Asn Ile Ser Pro Ala Glu Ala Asp Leu Met Asp Pro
   2810 2815 2820
   Gln His Arg Leu Phe Leu Glu Glu Ser Tyr Lys Ala Phe Glu Asp
   2825 2830 2835
   Ala Gly Tyr Ser Pro Ser Arg Leu Asp Gly Ala Arg Cys Gly Val
   2840 2845 2850
   Tyr Leu Gly Ile Met Gly Ser Glu Tyr Thr Gln Ile Val Ala Gln
   2855 2860 2865
   Ser Gly Arg Arg Ser Ala Thr Ala Asn Ser Ala Ala Ile Gly Ala
   2870 2875 2880
   Gly Arg Val Ala Tyr His Leu Asn Leu Lys Gly Pro Ala Ile Pro
   2885 2890 2895
   Val Asp Thr Ala Cys Ser Ser Ser Leu Val Ala Ile His Leu Ala
   2900 2905 2910
   Val Gln Ala Leu Arg Ala Gly Glu Ile Glu Met Ala Leu Ala Gly
   2915 2920 2925
   Gly Val Thr Leu Tyr Leu Thr Pro Asp Ala Tyr Ile Ala Met Cys
   2930 2935 2940
   Glu Ala Gly Met Leu Ser Pro Asp Gly Arg Cys Lys Thr Phe Asp
   2945 2950 2955
   Glu Ala Ala Asn Gly Phe Val Pro Gly Glu Gly Val Gly Val Val
   2960 2965 2970
   Val Leu Lys Arg Leu Pro Asp Ala Glu Arg Asp Gly Asp Phe Ile
   2975 2980 2985
   His Gly Val Ile Ile Gly Ser Gly Val Asn Gln Asp Gly Lys Thr
   2990 2995 3000
   Asn Gly Ile Thr Ala Pro Ser Val Gln Ser Gln Thr Ala Leu Leu
   3005 3010 3015
   Lys Asp Val Tyr Ala Arg Phe Gly Ile Asp Pro Arg Thr Ile Ser
   3020 3025 3030
   Tyr Val Glu Thr His Gly Thr Gly Thr Lys Leu Gly Asp Pro Ile
       3035                3040                3045
   Glu Leu Ala Ala Leu Ser Ala Ala Phe Gly Ala Arg Asp Gly Arg
       3050                3055                3060
   Arg Cys Ala Leu Gly Ser Val Lys Ser Asn Val Gly His Thr Thr
       3065                3070                3075
   Ala Ala Ala Gly Val Ala Gly Val His Lys Val Leu Ala Cys Met
       3080                3085                3090
   Gln His Gly Arg Leu Val Pro Thr Leu His Phe Thr Arg Pro Asn
       3095                3100                3105
   Ser His Phe Ser Phe Glu Asp Ser Pro Phe Tyr Val Asn Thr Glu
       3110                3115                3120
   Val Arg Pro Trp Glu Arg Asp Gly Ala Ser Pro Arg Arg Ala Ala
       3125                3130                3135
   Val Ser Ser Phe Gly Phe Ser Gly Thr Asn Ala His Leu Val Ile
       3140                3145                3150
   Glu Glu Tyr Val Ala Pro Thr Arg Arg Pro Gly Arg Arg Val Thr
       3155                3160                3165
   Pro Ser Asn Pro Ala Leu Val Val Leu Ser Ala Lys Thr Glu Ala
       3170                3175                3180
   Gln Leu Gln Thr Leu Ala Gly Arg Leu Arg Asp Ala Leu Ser Ser
       3185                3190                3195
   Pro Arg Trp Thr Asp Asp Asp Leu Leu Asp Ile Ala Tyr Thr Leu
       3200                3205                3210
   Arg Val Gly Arg Ala His Leu Glu Glu Arg Leu Gly Ile Ala Ala
       3215                3220                3225
   Ser Ser Leu Asp Ala Leu Arg Ser Gly Leu Arg Ala Phe Leu Asp
       3230                3235                3240
   Gly Gly Pro Leu Ser Pro Asn Leu Ala Arg Gly Arg Ala Lys Asn
       3245                3250                3255
   Ala Arg Pro Val Gly Ala Ala Arg Arg Glu Thr Ala Gly Gly Ala
       3260                3265                3270
   Thr Pro Thr Glu Leu Leu Ala Arg Trp Val Asp Gly Gly Ala Cys
       3275                3280                3285
   Ala Trp Ser Glu Pro Tyr Gly Asp Gly Ala Thr Pro Gln Arg Val
   3290 3295 3300
   Arg Leu Pro Thr Tyr Pro Leu Ser Arg Gln Arg Cys Trp Val Asp
   3305 3310 3315
   Ser Gly Ala Ala Pro Glu Val Ala Pro Pro Val Pro Gly Arg Val
   3320 3325 3330
   Thr Pro Leu Glu Met Leu Ala Pro Arg Trp Glu Ile Val Glu Gln
   3335 3340 3345
   Ala Pro Ala Arg Gly Ala Gly Leu Gly Pro Glu Arg Gly Ile Ala
   3350 3355 3360
   Val Leu Gly Ala Arg Pro Gly Pro Leu Arg Asp Ala Leu Ala Ala
   3365 3370 3375
   Arg Ala Pro Arg Ala Arg Phe Val Asp Ala Pro Cys Asp Ala Ser
   3380 3385 3390
   Ile Asp Glu Leu Val Glu Ala Leu Arg Ala Ala Pro Ile Asp Thr
   3395 3400 3405
   Val Ile Trp Leu Leu Pro Glu Glu Ser Ala Ser Ser Val Ala Ala
   3410 3415 3420
   Glu Glu His Ile Ala Ala Gln Ser Ala Gly Ala Leu Phe Gly Phe
   3425 3430 3435
   Arg Trp Ile Lys Ala Leu Leu Ala Leu Gly Tyr Gly Gly Arg Pro
   3440 3445 3450
   Leu Arg Ile Asp Val Val Thr Trp Gln Thr Gln Gly Val His Gly
   3455 3460 3465
   Glu Leu Val Tyr Pro Thr His Ala Ser Val His Gly Leu Ile Gly
   3470 3475 3480
   Ser Leu Ala Lys Glu Leu Arg Ser Trp Ser Val Arg Leu Val Asp
   3485 3490 3495
   Leu Pro Leu Ala Val Asp Arg Ala Ala Arg Ile Leu Ile Asp Ser
   3500 3505 3510
   Leu His Leu Glu Ala Asp Pro Asp Gly Asp Ala Ser Ala Trp Arg
   3515 3520 3525
   Asp Gly Arg Trp Tyr Arg Gln Arg Leu Leu Pro Phe Glu Val Pro
   3530 3535 3540
   Ala Glu Gln Ala Ser Ala Ala Phe Arg Arg Gly Gly Val Tyr Val
   3545 3550 3555
   Val Ile Gly Gly Ala Gly Gly Leu Gly Glu Ala Leu Thr Glu His
   3560 3565 3570
   Leu Val Arg Glu Tyr His Ala Asn Val Val Trp Leu Gly Arg Arg
   3575 3580 3585
   Ala Leu Asp Ala Thr Ile Asp Ala Lys Ala Asp Arg Ile Ser Arg
   3590 3595 3600
   Leu Gly Pro Arg Pro Val Tyr Ile Gln Ala Asp Ala Ala Asp Ala
   3605 3610 3615
   Ser Ala Leu Ala Arg Ala Arg Arg Asp Val Leu Ser Arg Phe Asp
   3620 3625 3630
   Arg Ile His Gly Val Val His Gly Pro Leu Val Leu Ser Asp Arg
   3635 3640 3645
   Ser Leu Ala Arg Met Glu Glu Arg Gln Phe Glu Ala Gly Leu Arg
   3650 3655 3660
   Ala Lys Val Asp Thr Ala Val Arg Leu Ala Gln Thr Phe Gly Glu
   3665 3670 3675
   Asp Asp Leu Asp Phe Val Ala Phe Phe Ser Ser Met Gln Thr Phe
   3680 3685 3690
   Val Lys Ala Pro Gly Gln Ser Asn Tyr Ala Ala Gly Cys Thr Phe
   3695 3700 3705
   Ser Asp Ala Phe Ala Gln Arg Leu Gly Gln Ala Trp Lys Cys Pro
   3710 3715 3720
   Val Lys Val Val Asn Trp Gly Tyr Trp Gly Ser Thr Gly Ser Val
   3725 3730 3735
   Ala Asp Glu Gly His Arg Arg Arg Met Ala Gly Ala Gly Ile Gly
   3740 3745 3750
   Ser Ile Glu Pro Asp Glu Gly Met Asp Ala Val Glu Thr Leu Leu
   3755 3760 3765
   Arg Leu Pro Val Arg Gln Leu Ala Val Val Lys Trp Ile Gly Ala
   3770 3775 3780
   Thr Glu Arg Ser Ala Ala Ala Ala Val Glu Thr Thr Pro Ala Pro
   3785 3790 3795
   Gln Glu Gly Pro Thr Met Arg Ala Met Gly Val Val Glu Thr Thr
   3800 3805 3810
   Leu Ala Ala Gln Thr Ala Pro Ala Met Glu Ala Met Trp Ala Glu
   3815 3820 3825
<210> 31
   <211> 3628
   <212> PRT
   <213> Sorangium cellulosum
<400> 31
   Val Leu Ala Pro Pro Leu Pro Ser Ser Glu Gln Asp Ala Ala Tyr Glu
   1               5                   10                  15
   Glu Ile Thr Thr Leu Leu Leu Ala Ile Val Trp Arg Gln Leu Asp Arg
               20                  25                  30
   Thr Gly Leu Arg Leu Thr Gly Asp Gly Trp Arg Glu Ser Ala Arg Ala
           35                  40                  45
   Arg Pro Leu Tyr Ala Arg Trp Leu Asp Glu Thr Glu Arg Leu Leu Arg
       50                  55                  60
   Arg His Gly Tyr Pro Ala Ala Gly Ala Ala Val Gly Asp Leu Asp Ala
   65                  70                  75                  80
   Leu Trp Ala Arg Trp Glu Arg Tyr Arg Thr Glu Gly Arg Val Glu Gln
                   85                  90                  95
   Ala Gly Leu Val Lys Gln Ala Asp Leu Val Gln Ala Thr Leu Glu Ala
               100                 105                 110
   Leu Pro Asp Ile Leu Thr Gly Arg Arg Arg Ala Thr Asp Val Leu Phe
           115                 120                 125
   Pro Gly Ala Ser Met Ser Arg Val Glu Ala Val Tyr Lys Asn Asn Pro
       130                 135                 140
   Gln Ala Asp Tyr Phe Asn Gly Val Leu Ala Gln Arg Val Ala Ser Leu
   145                 150                 155                 160
   Ile Glu Ala Arg Arg Ala Tyr Gly His Thr Glu Pro Leu His Ile Leu
                   165                 170                 175
   Glu Ile Gly Ala Gly Thr Gly Gly Thr Ser Glu Val Val Phe Glu Ala
               180                 185                 190
   Leu Arg Pro Leu Gln Asp Leu Val Gly Thr Tyr Thr Tyr Thr Asp Val
           195                 200                 205
   Ser Lys Ala Phe Leu Leu His Ala Glu Asp Ala Tyr Gly Ala Gly Arg
       210                 215                 220
   Pro Tyr Leu Arg Tyr Arg Leu Leu Asp Ile Gln Gln Pro Leu Glu Ala
   225 230 235 240
   Gln Gly Leu Ser Leu Gly Thr Tyr Asp Ile Val Ile Ala Ala Asn Val
   245 250 255
   Leu His Ala Thr Thr Asp Ile Arg Thr Thr Leu Arg His Thr Lys Ala
   260 265 270
   Ala Leu Lys Arg Asn Gly Ala Leu Leu Ile Asn Glu Ile Val Ala Lys
   275 280 285
   Ser Leu Phe Ala His Leu Thr Phe Gly Leu Thr Glu Gly Trp Trp Leu
   290 295 300
   Val Glu Asp Pro Glu Leu Arg Ile Glu Gly Cys Pro Ala Leu Ser Ile
   305 310 315 320
   Asp Gly Trp Asn Ala Leu Leu Thr Glu Glu Gly Phe Asp Arg Val Arg
   325 330 335
   Trp Pro Ala Ala Glu Ala Ser Ala Leu Gly Gln Gln Ile Ile Met Ala
   340 345 350
   Arg Ser Asp Gly Val Ile Arg Pro Ala Ser Ala His Ala Lys Lys Ile
   355 360 365
   Ala Pro Ser Thr Glu Ala Arg Arg Pro Glu Ala Gln Ala Pro Val Pro
   370 375 380
   Arg Glu Pro Val Asn Val Asp Asp Asp Ala Leu Ala Asp Glu Val Arg
   385 390 395 400
   Arg Ala Ile Ala Ala Gln Leu Ser Ile Asn Leu Arg Val Pro Ile Ser
   405 410 415
   Gln Ile Gly Tyr His Asp Ala Phe Gly Asp Tyr Gly Val Asp Ser Ile
   420 425 430
   Ser Gly Val Arg Leu Ile Gln Gln Leu Asn Gly Ala Phe Gly Ile Arg
   435 440 445
   Leu Glu Thr Thr His Leu Tyr Asp Phe Ser Ser Ile Gln Arg Leu Ala
   450 455 460
   Glu His Met Leu Ser Ala Trp Gly Pro Glu Leu Arg Ala Arg Arg Ala
   465 470 475 480
   Thr Asn Asp Gly Gly Arg Ala Ala Ala Ser Glu Pro Arg Arg Ala Ser
   485 490 495
   Ser Met Asn Gly Gly Ala Ala Ile Ala Ala Phe Ala Ser Ala Pro Asp
   500 505 510
   Ala Ser Gly Ser Ala Ala Ser His Ala Arg Glu Pro Ala Ala Ala Ser
   515 520 525
   Thr Val Ser Ser Gln Gly His Ala Pro Ser Pro Glu Pro Ala Tyr Arg
   530 535 540
   Arg Glu Pro Ile Ala Val Ile Gly Met Ser Gly Arg Phe Ala Glu Ala
   545 550 555 560
   Asp Asp Leu Asp Ala Leu Trp Arg His Leu Ala Ala Gly Ala Asp Leu
   565 570 575
   Val Arg Pro Val Thr Arg Trp Arg Leu Pro Glu Gln Thr Ala Thr Gly
   580 585 590
   Glu Pro Phe Cys Arg His Gly Gly Leu Leu Asn Gly Ile Asp Arg Phe
   595 600 605
   Asp Pro Leu Phe Phe Asn Ile Ser Val Thr Glu Ala Thr Thr Met Asp
   610 615 620
   Pro Gln Gln Arg Phe Phe Leu Glu Glu Ala Trp Lys Ala Leu Glu Asp
   625 630 635 640
   Gly Gly Tyr Ala Gly Asp Gly Leu Ser Gly Ser Arg Cys Gly Val Tyr
   645 650 655
   Val Gly Tyr Asn Val Thr Asp Tyr Gln Thr Leu Leu Gly Glu Asp Ala
   660 665 670
   Pro Pro Gln Ala Met Trp Gly Asn Ala Gly Ser Ile Leu Ser Ala Arg
   675 680 685
   Ile Ser Tyr Tyr Leu Asn Leu Gln Gly Pro Ala Ile Thr Val Asp Thr
   690 695 700
   Ala Cys Ser Ser Ala Leu Val Ala Thr His Leu Ala Cys Gln Gly Leu
   705 710 715 720
   Trp Asn His Glu Thr Glu Met Ala Leu Ala Gly Gly Ala Phe Leu Gln
   725 730 735
   Thr Thr Pro Gly Phe Tyr Leu Leu Ala Gly Arg Ala Gly Met Leu Ser
   740 745 750
   Pro Ser Gly Arg Cys Tyr Thr Phe Asp Ala Arg Ala Asp Gly Phe Val
   755 760 765
   Pro Gly Glu Gly Val Gly Val Val Leu Leu Lys Arg Leu Cys Asp Ala
   770 775 780
   Ile Ala Asp Gly Asp His Ile His Gly Val Ile Cys Gly Ser Gly Val
   785 790 795 800
   Asn Gln Asp Gly Asn Thr Asn Gly Ile Thr Ala Pro Ser Ala Leu Ser
   805 810 815
   Gln Glu Arg Leu Glu Arg Gln Val Tyr Asp Thr Phe Gly Ile His Ala
   820 825 830
   Glu Ser Ile Gln Leu Val Glu Ala His Gly Thr Gly Thr Lys Leu Gly
   835 840 845
   Asp Pro Val Glu Phe Arg Ala Leu Thr Ser Ala Phe Arg Lys Asp Thr
   850 855 860
   Ser Arg Thr Arg Phe Cys Ala Val Asp Ser Ile Lys Thr Asn Ile Gly
   865 870 875 880
   His Thr Ala Ala Ala Ala Gly Ile Ala Gly Leu Leu Lys Val Leu Leu
   885 890 895
   Ala Leu Arg His Arg Gln Leu Pro Pro Ala Val His Tyr Ala Gln Pro
   900 905 910
   Asn Pro Ala Ile Asp Phe Glu Ala Ser Pro Phe Tyr Val Asn Thr Thr
   915 920 925
   Leu Thr Asp Trp Gly Cys Asp Ala Gly Lys Lys Arg Arg Ala Ala Val
   930 935 940
   Ser Ser Phe Gly Phe Ser Gly Thr Asn Ala His Leu Val Val Glu Glu
   945 950 955 960
   Ala Asp Pro Val Arg Arg Pro Glu Arg Val Glu Arg Pro Gly His Leu
   965 970 975
   Leu Val Leu Ser Ala Arg Thr Gln Glu Gln Leu Arg Thr Gln Val Arg
   980 985 990
   Asn Leu Leu Asp His Val Ala Ala Arg Pro His Ala Asp Leu Gly Asp
   995 1000 1005
   Val Cys Phe Thr Leu Met Val Gly Arg Lys Arg Leu Ala His Arg
   1010 1015 1020
   Leu Ala Cys Val Ser Arg Ser Leu Asp Glu Leu Val Gly Ala Leu
   1025 1030 1035
   Gly Gln Trp Ile Glu Ser Glu Ser Ala Pro Gly Val Leu Ala Gly
   1040 1045 1050
   His Phe Lys Asp Asn Glu Ser Arg Glu Arg Pro Ser Leu Arg Arg
   1055 1060 1065
   Tyr Gly Asn Gln Cys Ile Leu Glu Cys Arg Arg Gly Glu Val Ala
   1070 1075 1080
   Gly Arg Asp Tyr Ile Glu Arg Leu Glu Val Val Gly Asp Leu Phe
   1085 1090 1095
   Val Gln Gly Tyr Ala Leu Asp Tyr Ala Asp Leu Phe Ala Pro Gly
   1100 1105 1110
   Ala Tyr Arg Arg Val Pro Leu Pro Thr Tyr Pro Phe Ala Arg Glu
   1115 1120 1125
   Ala Tyr Trp Val Lys Ala Arg Gly Arg Ala Gly Ala Ala Ser Ala
   1130 1135 1140
   Leu Glu Gly Ala Thr Gln Ala Glu Arg Ala Thr Thr Leu Leu Ala
   1145 1150 1155
   Leu Pro Ala Trp Arg Ala Gln Pro Ala Ala Gly Asp Gly Ala Arg
   1160 1165 1170
   Met Arg Asp Val Asp Phe Ala Ala His Arg Val Leu Ile Ala Gly
   1175 1180 1185
   Glu Leu Pro Glu Ala Ala Leu Arg Ala Leu Arg Glu Gln Leu Pro
   1190 1195 1200
   Arg Ala Arg Cys Glu Val Val Ala Pro Arg Glu Gly Ser Leu Ala
   1205 1210 1215
   Gln Arg Tyr Glu Arg Ala Ala Val Ala Ala Leu Arg Ser Val Arg
   1220 1225 1230
   Leu Ala Leu Glu Glu Gly Gly Gly Arg Ser Ser Leu Val Gln Leu
   1235 1240 1245
   Val Val Ala Thr Gly Gly Asp Arg Arg Glu Ala Cys Phe Ala Gly
   1250 1255 1260
   Leu Ser Gly Leu Leu Lys Thr Ala Ser Arg Glu Asn Pro Arg Leu
   1265 1270 1275
   Ala Thr Gln Thr Leu Glu Leu Gly Asp Ala Gly Ala Val Asp Val
   1280 1285 1290
   Leu Pro Arg Ala Leu Leu Glu Asp Ala Arg Asp Val Asp Glu Pro
   1295 1300 1305
   Glu Leu Leu Tyr Arg Asp Gly Arg Arg Trp Val Arg Arg Leu Glu
   1310 1315 1320
   Arg Ala Ala Arg Leu Asp Asp Pro Arg Thr Ile Gln Ala Pro Trp
   1325 1330 1335
   Arg Asp Gly Gly Val Tyr Leu Ile Thr Gly Gly Leu Gly Gly Leu
   1340 1345 1350
   Gly Tyr Leu Phe Ala Thr Ala Ile Ala Glu Ser Thr Arg Gly Ser
   1355 1360 1365
   Thr Leu Val Leu Val His Arg Ser Ala Pro Ser Gly Glu Gln Arg
   1370 1375 1380
   Ala Arg Leu Asp Ala Leu Arg Ser Leu Gly Ala Arg Val Asp Asp
   1385 1390 1395
   Gln Arg Arg Asp Val Gly Asp Ala Arg Asp Val Asp Gly Leu Ile
   1400 1405 1410
   Glu His Val Arg Ala Ser His Gly Ala Leu Thr Gly Val Leu His
   1415 1420 1425
   Ala Ala Gly Thr Thr Arg Asp Ala Leu Leu Pro Arg Lys Thr Pro
   1430 1435 1440
   Glu Glu Val Asn Asp Val Leu Arg Pro Lys Val Ala Gly Leu Val
   1445 1450 1455
   His Leu Asp Glu Ala Thr Arg Asp Leu Pro Leu Asp His Phe Val
   1460 1465 1470
   Cys Phe Ser Ala Phe Ala Gly Val Tyr Gly Asn Pro Gly Gln Ala
   1475 1480 1485
   Asp Tyr Ala Ala Ala Asn Ala Phe Met Asp Ala Tyr Val Glu Tyr
   1490 1495 1500
   Arg Arg Gly Leu Val Glu Glu Gly Arg Arg His Gly Thr Ser Leu
   1505 1510 1515
   Ala Ile Ala Trp Pro Leu Trp Ala Asp Gly Gly Met Lys Met Thr
   1520 1525 1530
   Asp Ala Gly Lys Ala Arg Met His Ala Ala Gly Leu Arg Glu Leu
   1535 1540 1545
   Asp Thr Arg Asp Gly Leu Ala Ala Cys Ser Ala Ala Val Ala Arg
   1550 1555 1560
   Lys Ser Pro Arg Val Val Val Leu Thr Gly Asp Glu Gly Lys Leu
   1565 1570 1575
   Glu Ala Phe Phe Glu Pro Ser Ser Pro Arg Ala Pro Arg Arg Pro
   1580 1585 1590
   Glu Pro Thr Gly Gly Val Ala Arg Glu Gly Ala Ser Leu Leu His
   1595 1600 1605
   Ala Thr Leu Ala Arg Leu Lys Glu Leu Leu Ala Val Val Thr Thr
   1610 1615 1620
   Ile Glu Ala Glu Arg Ile Asp Gly Ala Glu Pro Phe Glu Ala Tyr
   1625 1630 1635
   Gly Leu Asp Ser Ile Met Ile Thr Gln Leu Asn Gln Arg Leu Glu
   1640 1645 1650
   Ala Ile Phe Gly Ala Ile Ser Lys Thr Leu Phe Phe Glu His Arg
   1655 1660 1665
   Thr Leu Glu Gly Leu Ala Gln His Leu Ala Glu Glu His Arg Glu
   1670 1675 1680
   Ala Cys Gln Arg Trp Ala Gly Gly Pro Gly Asp Gly Ala Pro Asp
   1685 1690 1695
   Asp Val Ala Arg Ala Glu Pro Arg Gly Pro Ser Ser Ala Ala Gln
   1700 1705 1710
   Glu Ala Arg Ser Gly Glu Arg Ala Pro Ala Glu Ala Ala Leu Pro
   1715 1720 1725
   Ala Gln Glu Leu Asp Val Ala Ile Ile Gly Leu Ala Gly Arg Tyr
   1730 1735 1740
   Pro His Ala Arg Thr Leu Asp Glu Phe Trp Glu Asn Leu Arg Ala
   1745 1750 1755
   Gly Lys Asp Cys Ile Thr Glu Val Pro Pro Asp Arg Trp Pro Ile
   1760 1765 1770
   Glu Gly Phe Tyr Asp Gly Ala Ala Glu Ser Ser Pro Gly Asn Gly
   1775 1780 1785
   Lys Ser Tyr Ser Lys Trp Gly Gly Phe Leu Glu Gly Phe Ala Asp
   1790 1795 1800
   Phe Asp Pro Leu Phe Phe Gly Ile Ser Pro Gly Glu Ala Glu Ser
   1805 1810 1815
   Leu Asp Pro Gln Glu Arg Ser Phe Leu Glu Ile Cys Trp Glu Val
   1820 1825 1830
   Ile Glu  Asp Ala Gly Tyr Thr  Arg Arg Thr Leu Gln  Ser Arg His
       1835                 1840                 1845
   Gly Gly  Arg Val Gly Val Tyr  Ala Gly Ile Thr Lys  Thr Gly Phe
       1850                 1855                 1860
   Asp Leu  Tyr Gly Pro Ala Leu  Trp Ala Thr Gly Lys  Asp Ala Tyr
       1865                 1870                 1875
   Pro His  Thr Ser Phe Ser Ser  Leu Ala Asn Arg Val  Ser Tyr Leu
       1880                 1885                 1890
   Phe Asp  Leu Arg Gly Pro Ser  Ile Pro Val Asp Thr  Met Cys Ser
       1895                 1900                 1905
   Ala Ser  Leu Thr Ala Ile His  Glu Ala Cys Glu His  Leu Lys Arg
       1910                 1915                 1920
   Gly Glu  Cys Glu Leu Ala Ile  Ala Gly Gly Val Asn  Leu Tyr Leu
       1925                 1930                 1935
   His Pro  Ser Asn Tyr Val Ile  Leu Cys Ser His Arg  Met Leu Ser
       1940                 1945                 1950
   Pro Asp  Gly Lys Cys Arg Ser  Phe Gly Ala Gly Ala  Asn Gly Tyr
       1955                 1960                 1965
   Val Pro  Gly Glu Gly Val Gly  Ala Val Leu Leu Lys  Leu Leu Arg
       1970                 1975                 1980
   Arg Ala  Glu Ala Asp Gly Asp  His Ile Tyr Gly Val  Ile Lys Gly
       1985                 1990                 1995
   Thr Ala  Ile Asn His Gly Gly  Arg Thr His Gly Tyr  Thr Val Pro
       2000                 2005                 2010
   Asn Pro  Asn Ala Gln Ala Glu  Val Val Ala Met Ala  Leu Ala Arg
       2015                 2020                 2025
   Ala Arg  Ile Asp Ala Arg Ala  Val Ser Tyr Ile Glu  Ala His Gly
       2030                 2035                 2040
   Thr Gly  Thr Glu Leu Gly Asp  Pro Ile Glu Ile Thr  Gly Leu Thr
       2045                 2050                 2055
   Lys Ala  Phe Arg Ala Ser Ala  Ala Gly Pro Leu Thr  Arg Arg Cys
       2060                 2065                 2070
   Ala Ile  Gly Ser Leu Lys Ser  Asn Ile Gly His Ala  Glu Ser Ala
       2075                 2080                 2085
   Ala Gly Ile Ala Gly Val Thr Lys Val Leu Leu Gln Met Lys His
   2090 2095 2100
   Gly Gln Leu Val Pro Ser Leu His Ala Arg Thr Pro Asn Pro Asn
   2105 2110 2115
   Ile Asp Phe Asp Arg Thr Pro Phe Val Val Gln Gln Glu Leu Glu
   2120 2125 2130
   Ser Trp Gly Arg Leu Thr Gln Val Val Asp Gly Arg Thr Glu Glu
   2135 2140 2145
   Leu Pro Arg Ile Ala Gly Ile Ser Ser Phe Gly Ala Gly Gly Ala
   2150 2155 2160
   Asn Ala His Val Val Ile Gln Glu Tyr Ala Pro Ala Pro Glu Glu
   2165 2170 2175
   Pro Ser Ala Asp Gly Glu Gln Pro Pro Ala Ile Ile Val Leu Ser
   2180 2185 2190
   Ala Arg Thr Glu Ala Gln Leu Ala Glu Gln Ala Arg Asn Leu Leu
   2195 2200 2205
   Arg Ala Ile Arg Val Gln Arg Arg Thr Asp Gly Asp Leu Ala Arg
   2210 2215 2220
   Ile Ala Trp Thr Leu Gln Thr Gly Arg Glu Ala Met Glu Glu Arg
   2225 2230 2235
   Leu Ala Cys Val Ala Ser Ser Leu Asp Glu Leu Val Ala Thr Leu
   2240 2245 2250
   Gly Ala Phe Val Ala Ser Pro Ser Thr Ala Pro Gly Ile Tyr Arg
   2255 2260 2265
   Gly Ser Ala Arg Lys Arg Lys Asp Val Ser Ala Phe Ala Ala Asn
   2270 2275 2280
   Glu Glu Leu Arg Glu Val Val Ser Arg Trp Ile Glu Arg Arg Arg
   2285 2290 2295
   Tyr Ala Gln Leu Leu Glu Leu Trp Val Ser Gly Leu Glu Val Asp
   2300 2305 2310
   Trp Glu Gln Leu Tyr Gly Glu Arg Lys Pro Arg Arg Ile Ser Leu
   2315 2320 2325
   Pro Thr Tyr Pro Phe Glu Arg Glu Arg Tyr Trp Leu Pro Gln Gly
   2330 2335 2340
   Glu Gly Ala Val Val Ala Gly Ala Ala Ser Pro Val Glu Ala Ser
   2345 2350 2355
   Gly Ala Ala Ala Gly Gly Gly Arg Leu Glu Gln Gly Pro Gly Pro
   2360 2365 2370
   Ala Glu Val Ser Arg Val Glu Ala Gly Glu Ala Ala Glu Val Val
   2375 2380 2385
   Glu Tyr Glu Glu Ala Trp Glu Arg Leu Gly Trp Glu Glu Arg Glu
   2390 2395 2400
   Arg His Gln Glu Gly Val Arg Trp Val Val Val Gly Gly Asp Glu
   2405 2410 2415
   Ala Trp Arg Ala Ser Val Val Glu Ala Leu Arg Arg Glu Glu Pro
   2420 2425 2430
   Gly Ser Glu Ala Leu Gly Val Ser Val Gly Gly Ala Tyr Gly Trp
   2435 2440 2445
   Glu Gly Ser Ala Arg Cys Thr Val Pro Glu Ala Ser Glu Val Trp
   2450 2455 2460
   Phe Arg Ser Val Phe Glu Gln Leu Gly Ala Arg Asp Gly Ala His
   2465 2470 2475
   Gly Gly Gly Gly Arg Pro Cys Gly Val Val Tyr Ala Trp Gly Arg
   2480 2485 2490
   Gly Leu Glu Arg Leu Arg Gly Val His Ala Leu Val Arg Gly Ala
   2495 2500 2505
   Lys Gly Ser Gly Ala Leu Leu Arg Arg Leu Val Leu Thr Gly Arg
   2510 2515 2520
   Gln Val Arg Gly Glu Val Ser Gly Cys Tyr Asp Ala Ala Leu Ile
   2525 2530 2535
   Gly Val Glu Arg Ser Leu Gly Leu Val Leu Pro Glu Leu Ser Val
   2540 2545 2550
   Gly Val Val Leu Gly Gly Ala Glu Gly Ile Glu Ala Gly Ala Leu
   2555 2560 2565
   Ala Arg Glu Val Trp Ser Gly Gly Val Ile Arg His Glu Gly Gly
   2570 2575 2580
   Glu Arg Gln Arg Leu Gly Val Arg Arg Ala Leu Pro Ser Gly Arg
   2585 2590 2595
   Ser Glu Ala Pro Leu Arg Asp Gly Gly Val Tyr Val Ile Thr Gly
   2600 2605 2610
   Gly Ala Gly Gly Leu Gly Arg Leu Phe Ala Glu His Leu Ala Arg
   2615 2620 2625
   Arg Cys Ala Gly Arg Leu Ala Leu Val Gly Arg Arg Gly Arg Asp
   2630 2635 2640
   Glu Gln Ile Glu Gly Val Leu Ser Arg Leu Arg Gly Ala Gly Ala
   2645 2650 2655
   Arg Glu Ala Glu Tyr Tyr Glu Ala Asp Val Ser Asp Ala Ala Gln
   2660 2665 2670
   Met Arg Cys Val Val Glu Ala Leu Glu Ser Arg Trp Gly Ser Ile
   2675 2680 2685
   His Gly Val Leu His Ala Ala Gly Val Glu Ser Gly Gly Ala Leu
   2690 2695 2700
   Leu Asp Lys Arg Trp Glu Glu Val Glu Glu Ile Thr Arg Pro Lys
   2705 2710 2715
   Met Glu Gly Thr Arg Val Val Asp Glu Val Thr Ala Gly His Pro
   2720 2725 2730
   Leu Asp Phe Met Cys Leu Phe Ser Ser Thr Ala Ala Val Leu Gly
   2735 2740 2745
   Asp Gly Gly Ser Cys Asp Tyr Ala Ser Ala Asn Arg Phe Gln Val
   2750 2755 2760
   Ala Tyr Ala Gln Met Arg Gln Glu Leu Arg Glu Arg Gly Glu Arg
   2765 2770 2775
   Ser Gly Ala Thr Val Ala Val Cys Trp Pro Leu Trp Arg Ala Gly
   2780 2785 2790
   Gly Met Gly Thr Ala Glu Arg Glu Gly Met Ala Val Tyr Leu Arg
   2795 2800 2805
   Ser Ser Gly Gln Arg Tyr Leu Glu Ser Asp Glu Gly Leu Gly Ala
   2810 2815 2820
   Trp Glu Arg Ala Met Gly Ala Ala Val Ser Pro Arg Leu Val Phe
   2825 2830 2835
   Ala Gly Gln Pro Arg Arg Val His Ala Phe Leu Ser Arg Ile Tyr
   2840 2845 2850
   Ala Gly Ala Arg Ala Asp Gly Ala Ser Thr Asp Gly Glu Leu Thr
   2855 2860 2865
   Arg Gly Ala Pro Pro Arg Arg Asp Leu Pro Ala Arg Pro Ala Gly
   2870 2875 2880
   Ala Pro Ser Thr Ser Thr Ala Glu Arg Val Leu Arg Asp Leu Lys
   2885 2890 2895
   Ser Cys Val Glu Ala Val Leu Lys Val Pro Ala Ser Arg Leu Ser
   2900 2905 2910
   Glu Asp Thr Ser Phe Ala Asp Ile Gly Phe Asp Ser Ile Gly Leu
   2915 2920 2925
   Thr Arg Val Ala Arg Leu Leu Ser Lys His Phe Gly Phe Glu Ile
   2930 2935 2940
   Val Pro Ser Val Phe Phe Ser Tyr Thr Thr Leu Glu Lys Phe Cys
   2945 2950 2955
   Gly His Leu Leu Glu Gln His Arg Asp Ala Leu Glu Arg Leu Tyr
   2960 2965 2970
   Glu Ser Ala Leu Pro Pro Asp Pro Gly Ala Ala Arg Ala Pro Ala
   2975 2980 2985
   Arg Asp Ala His Gly Ser Ser Asp Asp Ser Ala Ala Val Ala Ala
   2990 2995 3000
   Glu Pro Gly Arg Glu Pro Glu Ala Gln Arg Leu His Gly Ala Arg
   3005 3010 3015
   Ala Ala Ser Ala Ser Arg Pro Ala Leu Ala Pro Ser Ser Pro Asp
   3020 3025 3030
   Glu Pro Val Ala Ile Ile Gly Met Ser Ala Arg Ala Ala Gly Ala
   3035 3040 3045
   Gly Asp Val Asp Glu Leu Trp Gln Leu Leu Ile Glu Gly Arg Thr
   3050 3055 3060
   His Ile Glu Asp Ile Pro Gly Asp Arg Trp Asp Ala Arg Pro Tyr
   3065 3070 3075
   Tyr Ala Gly Pro Gly Ala Pro Gly Asn Arg Ile Val Thr Asn Lys
   3080 3085 3090
   Gly Gly Phe Ile Arg Gly Leu Asp Arg Phe Asp Pro Leu Phe Phe
   3095 3100 3105
   Glu Ile Ser Pro Arg Glu Ala Gln Ser Met Asp Pro Arg Gln Arg
   3110 3115 3120
   Leu Met Leu Gln Glu Ala Trp Arg Ala Phe Glu Asp Ala Gly Tyr
   3125 3130 3135
   Ala Gly Asp Ala Leu Arg Gly Thr Arg Ala Gly Val Phe Val Gly
   3140 3145 3150
   Val Glu Glu Gly Val Ser Ala Gln Asp Gly Thr Glu Gly Leu Ala
   3155 3160 3165
   Thr Ser His His Asn Ala Ile Leu Ala Ala Arg Ile Ser Tyr Val
   3170 3175 3180
   Leu Asp Leu Arg Gly Pro Asn Leu Ala Ile Asn Thr Ala Cys Ser
   3185 3190 3195
   Ser Gly Leu Val Ala Leu His Thr Ala Cys Gln Ser Val Arg Arg
   3200 3205 3210
   Gly Glu Cys Ser Met Ala Leu Val Gly Ala Val Asn Val Leu Ala
   3215 3220 3225
   Ser Pro Leu Met Tyr Val Ala Leu Ser Gln Gly Gly Met Leu Ser
   3230 3235 3240
   Ala Asp Gly Glu Cys Tyr Ala Phe Asp Gln Arg Ala Asn Gly Leu
   3245 3250 3255
   Val Pro Gly Glu Ala Ala Val Ala Val Val Val Lys Arg Leu Ser
   3260 3265 3270
   Gln Ala Ile Thr Asp Gly Asp Thr Val Leu Gly Val Ile Arg Ala
   3275 3280 3285
   Ser Gly Val Asn Tyr Asp Gly Arg Thr Asn Gly Leu Thr Ala Pro
   3290 3295 3300
   Ser Gly Leu Ala Gln Ala Ser Leu Leu Glu Glu Met Tyr Gln Asp
   3305 3310 3315
   His Ala Ile Asp Val Glu Asp Ile Thr Tyr Ile Ile Ala His Gly
   3320 3325 3330
   Thr Gly Thr Lys Leu Gly Asp Pro Val Glu Val Asn Ser Leu Met
   3335 3340 3345
   Asp Val Phe Gly Arg Arg Thr Ser Ala Arg Gly Phe Cys Ala Val
   3350 3355 3360
   Gly Ser Val Lys Pro Asn Phe Gly His Thr Phe Ala Ala Ser Gly
   3365 3370 3375
   Leu Val Ser Leu Val Ala Met Leu Met Ala Met Arg His Arg Gln
   3380 3385 3390
   Ile Pro Pro Ser Ala Asn His Ala Gln Asn Asn Glu Phe Ile Asp
   3395 3400 3405
   Phe Ala Lys Gly Ala Phe Tyr Val Asn Asp Arg Pro Arg Ala Trp
   3410 3415 3420
   Leu Ala Arg Gly Gly Ala Lys Arg Leu Gly Ala Ile Ser Ala Phe
   3425 3430 3435
   Gly Met Ser Gly Thr Asn Ala His Val Val Ile Glu Glu His Val
   3440 3445 3450
   Pro Glu Ala Ala Ala Ala Gly His Ala Ala Leu Pro Ala Gly Ala
   3455 3460 3465
   Val Ala Ile Val Pro Leu Ser Ala Arg Arg Val Asp Asp Leu Val
   3470 3475 3480
   Ala Tyr Ala Glu Ser Leu Arg Ala Phe Leu Ala Asp Gly Arg Arg
   3485 3490 3495
   Ala Gly Ala Glu Ala Pro Asp Leu Ala Ser Val Ala Tyr Thr Leu
   3500 3505 3510
   Gln Thr Gly Arg Glu Ala Met Arg His Arg Ala Val Phe Cys Val
   3515 3520 3525
   Arg Asp Leu Asp Glu Leu Glu Ser Lys Leu Arg Ala Tyr Ala Ala
   3530 3535 3540
   Ala Gly Pro Gly Arg Gly Ala Asp Asp Arg Asp Ala Asp Glu Leu
   3545 3550 3555
   Ala Ala Arg Trp Ala Arg Gly Glu Ala Val Asp Trp Arg Ala Met
   3560 3565 3570
   Val Pro Gly Ala Arg Pro Val Lys Thr Arg Leu Pro Thr Tyr Pro
   3575 3580 3585
   Phe His Ala Ala Ala Arg Leu Ser Ser Ala Asn Gly Ser Arg Glu
   3590 3595 3600
   Leu Arg Pro Pro Ala Glu Arg Ala Ala Pro Thr Pro Arg Ala Leu
   3605 3610 3615
   His Pro Leu Val His Arg Ile Val Ser Val
   3620 3625
<210> 32
   <211> 5070
   <212> PRT
   <213> Sorangium cellulosum
<400> 32
   Met Thr Ser Gly Leu Met Glu Ser Ser Gln Thr Phe Glu Thr Leu Leu
   1               5                   10                  15
   Asp Gly Arg Glu Phe Phe Leu Asp Asp His Arg Val Leu Gly Glu Ala
               20                  25                  30
   Val Leu Pro Gly Ala Ala Tyr Leu Glu Met Ala Arg Ala Ala Thr Val
           35                  40                  45
   His Ser Gly Arg Ala Arg Val Leu Gly Leu Arg Asn Val Val Trp Leu
       50                  55                  60
   Ala Pro Val Val Val Asn Gly Thr Pro Arg Ala Val Arg Ile Glu Ile
   65                  70                  75                  80
   Ala Ala Lys Ala Gly Lys Thr Arg Phe Ser Val Arg Ser Ala Arg Gly
                   85                  90                  95
   Gly Val Ser Glu Pro Glu Val Leu His Gly Gln Gly Glu Val Val Thr
               100                 105                 110
   Ala Glu Ser Gly Gly Ala Thr Ala Pro Ala Ser Arg Val Glu Leu Asp
           115                 120                 125
   Glu Ile Arg Ala Arg Cys Ala Ser Thr Leu Ser Gly Ala Asp Leu Tyr
       130                 135                 140
   Ala Leu Phe Glu Arg Gln Gly Leu Thr Tyr Gly Glu Thr Phe Arg Cys
   145                 150                 155                 160
   Ile Glu Glu Leu Arg Arg Ser Asp Ala Glu Val Leu Ala Arg Phe Arg
                   165                 170                 175
   Leu Ala Pro Pro Ala Ile Gln Val Gly Ala Pro Phe Glu Leu His Pro
               180                 185                 190
   Gly Val Thr Asp Ala Ala Leu Gln Ser Thr Val Gly Leu Leu Val Gly
           195                 200                 205
   Asp Gly Arg Leu Pro Ala Gly Ser Val His Val Pro Phe Ser Leu Lys
       210                 215                 220
   Glu Leu Val Leu His Thr Gln Thr Pro Ala Ala Gly Tyr Val Val Ala
   225                 230                 235                 240
   Arg Trp Ser Pro Gly Ala Ser Pro Arg Ser Asn Val Leu Lys Phe Asp
                   245                 250                 255
   Ile Asp Ile Val Asp Glu Ala Gly Val Val Cys Val Ser Met Arg Glu
   260 265 270
   Phe Ala Ser Arg Arg Tyr Asp Lys Ala Pro Ala Ala Gly Val Leu Phe
   275 280 285
   Gly Thr Thr Thr Trp Leu Ala Glu Pro Leu Ala Pro Pro Arg Pro Ala
   290 295 300
   Glu Arg Gly Ala Pro Ala Arg Arg Asp Ala Leu Val Leu Leu Ala Gly
   305 310 315 320
   Glu Ser Asp Glu Leu Ala Ala Leu Val Ser Glu Arg Ala Gln Thr Val
   325 330 335
   Arg Leu Pro Arg Val Leu Asp Ala Gly Gly Ala Val Glu His Ala Arg
   340 345 350
   Val Val Ala Ala Ala Arg Gln Ala Leu Glu Ala Ile Gln Arg Val Val
   355 360 365
   Ser Gly Arg Pro Ser Gly Pro Arg Glu Val Val Ala Leu Ala Ser Val
   370 375 380
   Arg Asp Glu Ala Phe Ala His Ala Pro Leu Ala Gly Leu Leu Lys Thr
   385 390 395 400
   Met Arg Arg Glu His Arg Asp Leu Arg Gly Lys Leu Val Leu Val Pro
   405 410 415
   Ala Glu Leu Gly Pro Glu Ala Arg Ala Arg Leu Leu Ser Leu Glu Leu
   420 425 430
   Asp Ala Ala Tyr Asp Asp Val Glu Val Arg Tyr Asp Arg Ser Glu Arg
   435 440 445
   Arg Glu Val Arg Arg Phe Ile Glu Ile Glu Leu Ala Gly Gly Ala Ala
   450 455 460
   Leu Arg Pro Thr Phe Glu Ala Gly Gly Val Tyr Trp Leu Thr Gly Gly
   465 470 475 480
   Leu Gly Gly Leu Gly Arg Ile Phe Ala Arg His Ile Ala Gly Thr Ala
   485 490 495
   Gly Ala Thr Val Val Leu Thr Gly Arg Ser Pro Leu Ser Glu Glu Ser
   500 505 510
   Arg Ala Trp Leu Asp Ala Leu Arg Ala Thr Thr Gly Ala Thr Ile Asp
   515 520 525
   Tyr Leu Pro Ala Asp Val Ala Asp Glu Arg Arg Leu Arg Thr Val Leu
   530 535 540
   Ala Asp Ile Lys Glu Arg His Gly Arg Leu Thr Gly Ile Leu His Gly
   545 550 555 560
   Ala Gly Val Leu Arg Asp Ser Thr Ile Ala Lys Lys Thr Ala Ala Glu
   565 570 575
   Leu Glu Ala Val Phe Ala Ala Lys Ala Leu Gly Leu Leu Ala Ile Asp
   580 585 590
   Glu Ala Thr Arg His Glu Asp Leu Asp Phe Leu Val Leu Phe Ser Ser
   595 600 605
   Ala Ser Ala Val Trp Gly Asn Ala Gly Gln Ala Asp Tyr Val Ala Ala
   610 615 620
   Asn Ala Leu Leu Asp Glu Phe Ala Val His Arg Asn Glu Leu Val Lys
   625 630 635 640
   Ala Gly Arg Arg Ser Gly Arg Thr Leu Ser Ile Asp Trp Pro Leu Trp
   645 650 655
   Arg Ser Gly Gly Met Lys Val Asp Asp Ala Asn Glu Arg Leu Val Glu
   660 665 670
   Arg Ala Tyr Gly Leu Val Pro Leu Asp Glu Ala Ala Gly Leu Trp Ala
   675 680 685
   Phe Glu Val Ala Met Arg Ser Ser Leu Ala Gln Val Val Val Leu Ser
   690 695 700
   Gly Gln Ala Glu Lys Leu Arg Ser Lys Val Ile Gly Ala Glu Arg Glu
   705 710 715 720
   Ala Ala Asp Ala Ala Ala Arg Val Ala Ala Glu Pro Val Gly Ala Thr
   725 730 735
   Pro Ala Ser Glu Gln Pro Ala Pro Leu Ala Asp Gly Ala Gly Glu Ala
   740 745 750
   Ser Gly Leu Arg Asp Leu Val Glu Arg Thr Leu Val Arg Ala Val Ser
   755 760 765
   Arg Val Leu Lys Ile His Glu Ala Asp Ile Glu Leu Asp Ala Glu Leu
   770 775 780
   Ser Ala Phe Gly Phe Asp Ser Leu Ser Leu Thr Glu Leu Ser Asn Arg
   785 790 795 800
   Leu Ser Ala Glu Leu Gly Val Glu Leu Leu Pro Thr Val Phe Phe Glu
   805 810 815
   His Pro Ser Leu Ala Ala Leu Glu Thr Phe Leu Leu Ala Thr His Leu
   820 825 830
   Gly Ala Leu Ala Arg Thr Phe Arg Ala Glu Val Ala Ala Arg Ala Pro
   835 840 845
   Arg Arg Glu Pro Ala Arg Pro Pro Ile Glu Arg Pro Ala Ala Ile Ile
   850 855 860
   Asp Gln Ala Gly Ala His Asp Ala Pro Ala Ala Ala Val Val Asp Arg
   865 870 875 880
   Ala Asp Ala Arg Asp Ala Arg Pro Ala Gly Thr Ala Ile Ile Gly Ile
   885 890 895
   Ser Gly Arg Phe Pro Arg Ala Ser Asp Val Asp Ala Phe Trp Ser Asn
   900 905 910
   Leu Ile Glu Gly Arg Asp Cys Ile Glu Val Val Pro Glu Asp Arg Trp
   915 920 925
   Asp Trp Arg Ala Ile Tyr Gly Asp Pro His Ala Glu Ala Asn Lys Thr
   930 935 940
   Asn Val Lys Trp Gly Gly Phe Ile Asp Glu Leu Ala Ser Phe Asp Pro
   945 950 955 960
   Gln Phe Phe Gly Ile Ser Pro Lys Glu Ala Glu Leu Met Asp Pro Gln
   965 970 975
   His Arg Leu Leu Met Glu His Val Trp Ser Ala Met Glu Asp Ala Gly
   980 985 990
   Tyr Ser Ala Lys Ala Leu Ser Gly Thr Arg Thr Gly Val Phe Leu Ala
   995 1000 1005
   Ile Gly Pro Gly Ser Tyr Arg Gln Pro Ala Ser Gln Pro Ile Glu
   1010 1015 1020
   Ala Tyr Ser Ala Thr Gly Ala Val Pro Ser Met Ala Pro Asn Arg
   1025 1030 1035
   Ile Ser Phe Phe Leu Asn Leu Arg Gly Pro Ser Glu Pro Val Glu
   1040 1045 1050
   Thr Ala Cys Ser Ser Ala Leu Ile Ala Val His Arg Ala Val Gln
   1055 1060 1065
   Ala Ile Asp Ala Gly Val Cys Asp Gln Ala Phe Val Gly Ala Val
   1070 1075 1080
   Asn Val Met Tyr Ser Pro Glu Leu His Ile Cys Phe Ser Lys Ala
   1085 1090 1095
   Gly Met Leu Ser Val Asp Gly Arg Cys Lys Thr Phe Ser Arg Arg
   1100 1105 1110
   Ala Asp Gly Tyr Val Arg Gly Glu Gly Val Gly Val Met Leu Leu
   1115 1120 1125
   Lys Arg Leu Arg Gln Ala Gln Ala Asp Arg Asp Ala Ile Tyr Gly
   1130 1135 1140
   Val Ile Arg Gly Thr Asn Val Asn His Gly Gly Arg Ala Ala Ser
   1145 1150 1155
   Leu Thr Ala Pro Asn Ala Ser Ala Gln Ala Glu Leu Leu Phe Asp
   1160 1165 1170
   Ala Tyr Ser Arg Ala Gly Val Asp Val Arg Ala Val Thr Tyr Val
   1175 1180 1185
   Glu Thr His Gly Thr Gly Thr Pro Leu Gly Asp Pro Val Glu Ile
   1190 1195 1200
   Asn Ala Leu Lys Lys Ala Phe Gly Ala Leu Tyr Glu Arg Gln Gly
   1205 1210 1215
   Gly Ala Ile Glu Ala Gln Arg Cys Ala Leu Gly Ser Val Lys Thr
   1220 1225 1230
   Asn Val Gly His Leu Glu Val Ala Ala Gly Ile Ala Gly Ile Phe
   1235 1240 1245
   Lys Val Leu Met Ala Met His His Gly Glu Leu Pro Lys Thr Leu
   1250 1255 1260
   His Cys Glu Glu Thr Asn Pro Tyr Ile Glu Leu Glu Gly Ser Pro
   1265 1270 1275
   Phe Phe Leu Leu Lys Glu Arg Arg Pro Trp Leu Arg Pro Ala Met
   1280 1285 1290
   Pro Asp Gly Gly Val Ala Pro Arg Leu Ala Gly Ile Ser Gly Phe
   1295 1300 1305
   Gly Phe Gly Gly Ala Asn Ala His Val Ile Val Glu Glu His Ala
   1310 1315 1320
   Glu Gly Gly Gly Gly Ala Leu Asp Glu Ile Ala Arg Glu Arg Pro
   1325 1330 1335
   Leu Gln Ile Val Pro Leu Ser Ala Arg Ser Glu Ala Arg Leu Arg
   1340 1345 1350
   Ala His Ala Gly Gln Leu Leu Ala Phe Leu Arg Glu Val Thr Ala
   1355 1360 1365
   Ser Ala Gly Ala Gln Pro Gly Ser Val Pro Asp Glu Val Ala Glu
   1370 1375 1380
   Gln Leu Arg Arg Asp Leu Val Gly Val Val Ala Ala Thr Met Ala
   1385 1390 1395
   Ile Gln Ala Ser Asp Val Ser Ala Asp Glu Arg Phe Ala Asp Tyr
   1400 1405 1410
   Gly Ala Asp Leu Gly Leu Leu Ala Asn Glu Ala His Arg Ala Leu
   1415 1420 1425
   Gly Ile His Ile Thr Ala Gln Ala Ile Gly Glu Ala Gly Ser Ile
   1430 1435 1440
   Asp Ala Leu Val Pro Tyr Leu Phe Glu Arg His Arg Glu Ser Leu
   1445 1450 1455
   Cys Ala Arg Tyr Pro Val Ala Ser Arg Ala Glu Ser Ala Glu Thr
   1460 1465 1470
   Arg Arg Ala Gly Ala Pro Lys Arg Val Arg Pro Gly Pro Ala Leu
   1475 1480 1485
   Leu Val Asp Val Ala Tyr Thr Leu Gln Val Gly Arg Asp Ala Met
   1490 1495 1500
   Asp His Arg Val Ala Phe Leu Ala Arg Asp Val Glu Glu Leu Glu
   1505 1510 1515
   Arg Lys Leu Glu Arg Phe Leu Ala Gly Ala Glu Glu Thr Asp Gly
   1520 1525 1530
   Ala Phe Thr Gly Gln Val Arg Arg Glu Arg Asp Ser Met Lys Val
   1535 1540 1545
   Leu Asn Ala Asp Ala Ser Ala Asn Glu Met Val Ala Thr Trp Met
   1550 1555 1560
   Arg Gln Gly Lys Leu Ala Ser Leu Cys Glu Leu Trp Val Arg Gly
   1565 1570 1575
   Leu Glu Val Asp Trp Glu Arg Leu Tyr Gly Glu Arg Lys Pro Arg
   1580 1585 1590
   Arg Ile His Leu Pro Thr Tyr Pro Phe Glu Arg Glu Arg Tyr Trp
       1595                1600                1605
   Leu Pro Gln Gly Glu Gly Ala Val Val Ala Gly Ala Ala Pro Pro
       1610                1615                1620
   Val Glu Ala Ser Cys Ala Ala Ala Cys Gly Gly Arg Leu Glu Gln
       1625                1630                1635
   Gly Ser Gly Thr Ala Glu Val Ser Arg Val Glu Ala Gly Glu Ala
       1640                1645                1650
   Ala Glu Val Val Glu Tyr Glu Glu Val Trp Glu Arg Leu Gly Trp
       1655                1660                1665
   Glu Glu Arg Glu Arg Arg Gln Glu Gly Val Arg Trp Leu Val Val
       1670                1675                1680
   Gly Gly Asp Glu Ala Trp Arg Ala Ser Val Val Glu Ala Leu Arg
       1685                1690                1695
   Arg Glu Glu Pro Gly Ser Glu Ala Leu Gly Val Ser Val Gly Gly
       1700                1705                1710
   Ala Tyr Gly Trp Glu Gly Ser Ala Arg Cys Thr Val Pro Glu Ala
       1715                1720                1725
   Ser Glu Val Trp Phe Arg Ser Val Phe Glu Gln Leu Gly Ala Arg
       1730                1735                1740
   Asp Gly Ala His Gly Gly Gly Gly Arg Pro Cys Gly Val Val Tyr
       1745                1750                1755
   Ala Trp Gly Arg Gly Leu Glu Arg Leu Arg Gly Val His Ala Leu
       1760                1765                1770
   Val Arg Gly Ala Lys Gly Ser Gly Ala Leu Leu Arg Arg Leu Val
       1775                1780                1785
   Leu Thr Gly Arg Gln Val Arg Gly Glu Val Ser Gly Cys Tyr Asp
       1790                1795                1800
   Ala Ala Leu Ile Gly Val Glu Arg Ser Leu Gly Leu Val Leu Pro
       1805                1810                1815
   Glu Leu Ser Val Gly Val Val Leu Gly Gly Ala Glu Gly Ile Glu
       1820                1825                1830
   Ala Gly Ala Leu Ala Arg Glu Val Trp Ser Gly Gly Val Ile Arg
       1835                1840                1845
   His Glu Gly Gly Glu Arg Gln Arg Leu Gly Val Arg Arg Ala Leu
       1850                1855                1860
   Pro Ser Gly Arg Ser Glu Ala Pro Leu Arg Asp Gly Gly Val Tyr
   1865 1870 1875
   Val Ile Thr Gly Gly Ala Gly Gly Leu Gly Arg Leu Phe Ala Glu
   1880 1885 1890
   His Leu Ala Arg Arg Cys Ala Gly Arg Leu Ala Leu Val Gly Arg
   1895 1900 1905
   Arg Gly Arg Asp Glu Gln Ile Glu Gly Val Leu Ser Arg Leu Arg
   1910 1915 1920
   Gly Ala Gly Ala Arg Glu Ala Glu Tyr Tyr Glu Ala Asp Val Ser
   1925 1930 1935
   Asp Ala Ala Gln Met Arg Cys Val Val Glu Ala Leu Glu Ser Arg
   1940 1945 1950
   Trp Gly Ser Ile His Gly Val Leu His Ala Ala Gly Val Glu Ser
   1955 1960 1965
   Gly Gly Ala Leu Leu Asp Lys Arg Trp Glu Glu Val Glu Glu Ile
   1970 1975 1980
   Thr Arg Pro Lys Met Glu Gly Thr Arg Val Val Asp Glu Val Thr
   1985 1990 1995
   Ala Gly His Pro Leu Asp Phe Met Cys Leu Phe Ser Ser Thr Ala
   2000 2005 2010
   Ala Val Leu Gly Asp Gly Gly Ser Cys Asp Tyr Ala Ser Ala Asn
   2015 2020 2025
   Arg Phe Gln Val Ala Tyr Ala Gln Met Arg Gln Glu Leu Arg Glu
   2030 2035 2040
   Arg Gly Glu Arg Ser Gly Ala Thr Val Ala Val Cys Trp Pro Leu
   2045 2050 2055
   Trp Arg Ala Gly Gly Met Gly Thr Ala Glu Arg Glu Gly Met Ala
   2060 2065 2070
   Val Tyr Leu Arg Ser Ser Gly Gln Arg Tyr Leu Glu Ser Asp Glu
   2075 2080 2085
   Gly Leu Gly Ala Trp Glu Arg Ala Met Gly Ala Ala Val Ser Pro
   2090 2095 2100
   Arg Leu Val Phe Ala Gly Gln Pro Arg Arg Val His Ala Phe Leu
   2105 2110 2115
   Ser Arg Ile Tyr Ala Gly Ala Arg Ala Ser Ala Val Asn Gly Ala
   2120 2125 2130
   Pro Ser Gly Ser Arg Ser Ala Val Ala Pro Ser Ile Ser Thr Ala
   2135 2140 2145
   Ala Ala Pro Gly Leu Pro Ser Gly Gly Ala Pro Ala Arg His Glu
   2150 2155 2160
   Arg Gly Ser Ala Ser Leu Pro Glu Arg Val Val His Asp Leu Lys
   2165 2170 2175
   Val His Ala Glu Ala Val Leu Lys Ile Pro Ala Gly Arg Ile Asp
   2180 2185 2190
   Glu Glu Thr Ser Phe Ala Asp Ile Gly Phe Asp Ser Ile Gly Leu
   2195 2200 2205
   Ala Gln Phe Ala Arg Leu Leu Thr Thr Gln Leu Gly Phe Asp Val
   2210 2215 2220
   Leu Pro Asn Ala Leu Leu Asn His Ser Thr Ile Arg Lys Leu Ala
   2225 2230 2235
   Gly His Leu Val Arg Glu His Arg Glu Ala Leu Glu His Leu Tyr
   2240 2245 2250
   Arg Ala Asn Gly Glu Pro Ala Arg Ala Leu Pro Ala His Pro Ala
   2255 2260 2265
   Leu Asp Asp Ala Pro Pro Ser Pro Leu Glu Ala Asp Arg Ala Ala
   2270 2275 2280
   Pro Arg Ala Ala Ala Pro Ser Pro Ala Arg Asp Glu Val Ala Ile
   2285 2290 2295
   Ile Gly Met Ser Ala Arg Val Ala Gly Ala Asp Asp Ala Asp Ala
   2300 2305 2310
   Leu Trp Ala Leu Leu Leu Glu Gly Arg Lys Gln Ile Gln Glu Ile
   2315 2320 2325
   Pro Ser Glu Arg Trp Asp Phe His Ala Tyr Tyr Glu Gly Pro Gly
   2330 2335 2340
   Ala Pro Gly Asn Arg Ile Thr Thr Asn Arg Gly Ala Phe Leu Arg
   2345 2350 2355
   Gly Leu Asp Glu Phe Asp Pro Leu Phe Phe Glu Ile Ser Pro Arg
   2360 2365 2370
   Glu Ala Gln Trp Met Asp Pro Arg Gln Arg Leu Met Leu Glu Ala
   2375 2380 2385
   Ala Trp Ala Ala Phe Glu Asp Ala Gly His Ala Gly Ala Arg Leu
   2390 2395 2400
   Arg Gly Thr Asn Cys Gly Val Phe Ile Gly Val Glu Glu Met Ala
   2405 2410 2415
   His Thr Gly Asp Gly Ala Pro Gly Leu Ala Thr Ser His His Asn
   2420 2425 2430
   Gly Ile Leu Ala Ala Arg Ile Ser Tyr Val Leu Asp Leu His Gly
   2435 2440 2445
   Pro Asn Leu Ala Leu Asn Thr Ala Cys Ser Ser Gly Leu Val Ala
   2450 2455 2460
   Val His Ser Ala Cys Gln Ser Leu Leu Arg Gly Glu Cys Ser Met
   2465 2470 2475
   Ala Leu Ala Gly Gly Val Asn Val Leu Thr Ser Pro Leu Thr Tyr
   2480 2485 2490
   Val Ala Leu Ser Gln Ser Gly Met Leu Ser Ala Glu Gly Glu Cys
   2495 2500 2505
   Tyr Ala Phe Asp Glu Arg Ala Ser Gly Met Val Pro Gly Glu Ala
   2510 2515 2520
   Val Ala Ala Val Val Leu Lys Arg Leu Ser Gln Ala Ile Ala Asp
   2525 2530 2535
   Gly Asp Pro Ile His Ala Val Ile Lys Ala Ser Gly Val Asn Tyr
   2540 2545 2550
   Asp Gly Lys Thr Asn Gly Ile Thr Ala Pro Ser Ser Val Ser Gln
   2555 2560 2565
   Gln His Leu Val Glu Ser Val Leu Arg Gln Ala Glu Leu Arg Pro
   2570 2575 2580
   Ala Asp Val Gln Tyr Val Leu Gly His Ser Val Gly Ser Gln Leu
   2585 2590 2595
   Gly Asp Pro Val Glu Val Gln Ala Leu Arg Asp Ala Phe Arg Ala
   2600 2605 2610
   Glu Pro Gly Glu Arg Gln Trp Cys Ala Leu Gly Ser Ile Lys Pro
   2615 2620 2625
   Leu Ile Gly His Thr Phe Ala Ala Ser Gly Val Val Ser Leu Ile
   2630 2635 2640
   Ala Met Cys Leu Ala Met Lys His Gly Thr Ile Pro Ala Thr Arg
   2645 2650 2655
   Asn Tyr Arg Arg Ala Asn Glu Tyr Ile Ile Asp Ile Ala Arg Ser
   2660 2665 2670
   Pro Phe Phe Val Ser Asp Val Glu Arg Pro Trp Ile Gly Ser Gly
   2675 2680 2685
   Pro Glu Arg Arg Arg Arg Gly Leu Val Gly Ala Thr Gly Met Ser
   2690 2695 2700
   Gly Thr Asn Ala Phe Ala Leu Ile Glu Glu His Ala Pro Ser Arg
   2705 2710 2715
   Ala Thr Pro Leu Pro Arg Arg Ala Ala Gly Ala Ala Asp Arg Ala
   2720 2725 2730
   Leu Ile Ala Leu Ser Ala Lys Ser Asp Gly Ala Leu Arg Gln Tyr
   2735 2740 2745
   Ala Gln Arg Leu Arg Ala His Leu Val Lys His Pro Ala Val Asp
   2750 2755 2760
   Leu His Gly Val Ala Tyr Thr Leu Gln Thr Gly Arg Glu Ala Met
   2765 2770 2775
   Pro Cys Arg Leu Ala Ile Val Ala Pro Ser Val Gly Ala Leu Ile
   2780 2785 2790
   Glu Gln Leu Asp Val Val Leu Asp Ala Ala Asp Gly Arg Ala Leu
   2795 2800 2805
   Ser Asp Arg Ala Ile Trp Thr Ser Ala Ser Ser Ala Val Pro Ala
   2810 2815 2820
   Pro Ser Ala Asp Gly Asp Pro Leu His Asp Ala Ala Arg Ala Trp
   2825 2830 2835
   Ala Ala Gly Gly Val Leu Asp Pro Ala Ala Leu Tyr Pro Ala Ala
   2840 2845 2850
   Lys Pro Thr Arg Leu Ala Gly Leu Pro Thr Tyr Pro Phe Ala Arg
   2855 2860 2865
   Arg Ser Val Ser Gln Gly Glu Arg Gly Gly Ala Pro Ala Ala Pro
   2870 2875 2880
   Ala Pro Pro Ala Arg Pro Gln Asp Ala Ala Pro Gly Asp Lys Ala
   2885 2890 2895
   Ala Glu Tyr Tyr Thr Phe Asp Ala Gln Arg Arg Ser Asp Glu Tyr
   2900 2905 2910
   Arg Glu Glu Tyr Leu Thr Phe Cys Pro Phe Glu Arg Glu Val Pro
   2915 2920 2925
   Gly Phe Ser Met Thr Arg Val Leu Ser Ala Pro Glu Ala Phe Pro
   2930 2935 2940
   Asp Glu Tyr Arg Leu Val Ile Glu Lys Gln Leu Glu Met Arg Arg
   2945 2950 2955
   Val Ile Phe Arg His Glu Arg Leu Glu Ser Val Arg Lys Val Leu
   2960 2965 2970
   Asp Ile Gly Cys Gly Cys Gly Thr Asp Leu Val Glu Val Ala Lys
   2975 2980 2985
   Arg Cys Pro His Ala Lys Ala His Gly Phe Thr Ile Thr Arg Ala
   2990 2995 3000
   Gln Ala Asp Leu Ala Asn Arg Arg Ile Gly Arg Leu Gly Leu Ala
   3005 3010 3015
   Glu Arg Val Arg Val Phe His Ala Asp Ser Ser Lys Asp Arg Phe
   3020 3025 3030
   Pro Asp Thr Tyr Asp Leu Ile Leu Gly Phe Glu Val Thr Cys His
   3035 3040 3045
   Ile Ala Asp Lys Thr Gly Val Phe Gly Asn Ile Ser Ser Ala Leu
   3050 3055 3060
   Ser Pro His Gly Ser Val Leu Leu Met Asp Phe Val Ser Asn Leu
   3065 3070 3075
   Arg Gly Ala Ile Val Asp Gly Asn Val Asp Ile Ala Ile Ser Thr
   3080 3085 3090
   Gln Asp Glu Trp Ile Asn Val Leu Thr Ala Ser Ser Leu Val Val
   3095 3100 3105
   Asp Asp Ile Val Asp Leu Ser Pro Gln Ile Ala Asn Tyr Val Phe
   3110 3115 3120
   Asp Pro Asp Val Glu Lys Asn Ile Ala His Phe Pro Glu Ala Ala
   3125 3130 3135
   Arg Arg Ser Ser Arg Asn Phe Ala Asn Met Ala Val Ser Leu Arg
   3140 3145 3150
   Glu Glu Trp Ile Ser Tyr Cys Leu Leu Arg Leu Thr Lys Ala Gly
   3155 3160 3165
   Pro Gly Trp Thr Ala Asp Arg Leu Arg Ala His Asn Ala Ser Lys
   3170 3175 3180
   Ile Ala Lys Lys Thr Pro Tyr Ala Ala Ala Leu Ala Glu Leu Gln
   3185 3190 3195
   Glu Ala Pro Gly Ser Pro Arg Pro Glu Ala Ala Ala Glu Asp Glu
   3200 3205 3210
   Arg Ala Pro Arg Gln Gly Ala Ser Val Asp His Val Arg Arg Arg
   3215 3220 3225
   Leu Glu Gln Ile Phe Glu Glu Ala Leu Gln Leu Thr Arg Asp Glu
   3230 3235 3240
   Leu Ala Ala Ala Thr Thr Phe Ser Ala Met Gly Val Gly Ser Leu
   3245 3250 3255
   Ile Ala Val Arg Leu Leu Gln Thr Ile Asn Thr Ala Phe Asp Leu
   3260 3265 3270
   Arg Glu Pro Thr Ser Val Met Phe Gln Phe His Asp Leu Gln Ser
   3275 3280 3285
   Leu Ala Glu His Leu Ala Ser Lys Ile Val Val Thr Ala Pro Pro
   3290 3295 3300
   Ala Arg Ala Thr Ala Ala Ala Leu Ala Pro Pro Ser Ala Arg Val
   3305 3310 3315
   Gln Glu Ala Ala Thr Thr Arg Gln Pro Ala Ala Pro Ala Glu Gly
   3320 3325 3330
   Ile Ala Ile Val Gly Met Ser Cys Arg Cys Ala Gly Ala Arg Asp
   3335 3340 3345
   Lys Arg Glu Leu Trp Asp Val Ile Val Glu Gly Arg Gln His Val
   3350 3355 3360
   Glu Arg Val Glu Glu Arg Tyr Pro Glu Trp Arg Glu Ala Cys Val
   3365 3370 3375
   Glu Gln Gly Val Asp Pro Ser Ser Leu His Ala Gly Phe Ile Asp
   3380 3385 3390
   Asp Ala Asp Cys Phe Asp Ser Leu Phe Phe Asp Ile Ser Pro Val
   3395 3400 3405
   Glu Ala Asp Gln Met Asp Ala Ser Gln Arg Leu Val Leu Glu Gly
   3410 3415 3420
   Ile His Ala Ala Leu Glu Asp Ala Gly Tyr Asp Pro Arg Ser Leu
   3425 3430 3435
   Ser Gly Arg Arg Val Gly Thr Phe Ile Gly Ser Met Gly Thr Gly
   3440 3445 3450
   Ala Pro Arg Gly Thr Ser His Leu Ala Met Leu Gly Ser Asp Gly
   3455 3460 3465
   Ala Ile Leu Ser Ser Arg Ile Ala Tyr His Leu Asn Phe Ala Gly
   3470 3475 3480
   Pro Ala Ile Thr Val Asn Ser Ala Cys Ser Ser Ser Leu Val Ala
   3485 3490 3495
   Val Glu Leu Ala Cys Gln Lys Leu Arg Ser Gly Asp Ile Asp Met
   3500 3505 3510
   Ala Val Ala Gly Gly Val Thr Leu Tyr Ser Gln Pro Ala Ser Phe
   3515 3520 3525
   Val Met Met His Asn Ala Gly Met Leu Ser Pro Ser Gly Arg Cys
   3530 3535 3540
   Arg Pro Phe Asp Asp Gly Ala Asp Gly Ile Val Val Gly Asp Gly
   3545 3550 3555
   Val Gly Val Val Val Leu Met Pro Leu Ser Arg Ala Ile Gly Glu
   3560 3565 3570
   Gly Ala His Val Tyr Gly Val Ile Arg Ala Ile Gly Thr Asn Gln
   3575 3580 3585
   Asp Gly Lys Thr Ser Gly Ile Thr Ala Pro Ser Phe Leu Ala Gln
   3590 3595 3600
   Ser Arg Leu Glu Arg Glu Val Tyr Glu Lys Ala Gly Ile Ser Pro
   3605 3610 3615
   Glu Gln Leu Gln Val Val Glu Ala His Gly Thr Gly Thr Lys Leu
   3620 3625 3630
   Gly Asp Pro Val Glu Ile His Ala Leu Thr Glu Ala Phe Arg Ala
   3635 3640 3645
   Ser Thr Gln Ala Arg Gln Phe Cys Ala Ile Gly Ser Leu Lys Ala
   3650 3655 3660
   Asn Ile Gly His Thr Thr Ala Ala Ser Gly Val Leu Gly Leu Ile
   3665 3670 3675
   Lys Val Leu Leu Ala Met Glu His Gly Cys Ile Pro Pro Ala Ile
   3680 3685 3690
   Asn Phe Thr Arg Pro Asn Arg His Ile Asp Leu Ala Asp Ser Pro
   3695 3700 3705
   Phe Tyr Val Ser Thr Arg Pro Ala Pro Trp Pro Glu Asn Pro Gly
   3710 3715 3720
   Gly Ser Arg Leu Ala Ala Val Ser Ala Phe Gly Phe Ser Gly Thr
   3725 3730 3735
   Asn Ala His Val Val Ile Glu Gly Leu Ala Arg Arg Glu Ala Ser
   3740 3745 3750
   Ala Pro Arg Ala Ser Ala Pro Pro Glu Glu Gln Val Val Val Leu
   3755 3760 3765
   Ser Ala Lys Arg Pro Glu Leu Leu His Glu Tyr Ala Arg Arg Leu
   3770 3775 3780
   Leu Ala Phe Thr Pro Ala Ala Gly Thr Ser Leu Ala Asp Val Ala
   3785 3790 3795
   Trp Thr Ser Gln Ile Gly Arg Ala Ala Met Asp Tyr Arg Leu Ala
   3800 3805 3810
   Ile Val Ala Ala Thr Phe Glu Glu Leu Arg Gly Arg Leu Ser Ala
   3815 3820 3825
   Tyr Leu Ala Gly Ala Glu Ser Ser Gln Gly Cys Phe Ser Gly His
   3830 3835 3840
   Ala Thr Gly Arg Arg Glu Gly Arg Pro Ala Gly Ser Thr Pro Ala
   3845 3850 3855
   Gln Leu Ala Ala Phe Trp Ala Ser Gly Gly Ala Val Asp Trp Glu
   3860 3865 3870
   Ala Glu Leu His Arg Pro Ala Gly Ala Arg Arg Arg Val Pro Leu
   3875 3880 3885
   Pro Thr Tyr Pro Phe Glu Lys Val Arg Phe Ala Arg Ala Gly Thr
   3890 3895 3900
   Glu Gly Arg Arg Asp Ala Ala Pro Arg Pro Thr Cys Leu Val Glu
   3905 3910 3915
   Lys Thr Trp Leu Pro Ala Arg Ala Gln Val Ala Thr Ala Glu Val
   3920 3925 3930
   Asp Leu Ala Gly Arg Cys Phe Ala Ile Leu Ala Thr Pro Gly Thr
   3935 3940 3945
   Arg Ala Pro Ala Asp Glu Leu Leu Arg Arg Leu Pro Arg Ser Glu
   3950 3955 3960
   Ile Leu Met Pro Glu Asp Val His Ala Ala Val Arg Gly Gly Ala
   3965 3970 3975
   Ala Pro Trp Asp Ala Tyr Ala Gly Cys Val Asp Leu Thr Gly Leu
   3980 3985 3990
   Asp Pro Gly His Arg Asp Glu Asp Leu Gly Trp Ile Glu Trp Leu
   3995 4000 4005
   Gln Glu Leu Val Gly Arg Ala Asp Ala Arg Arg Ala Ala Pro Leu
   4010 4015 4020
   Val Val Leu Gln Val Thr Ser Gly Leu Glu Arg Phe Arg Gly Glu
   4025 4030 4035
   Arg Ala Arg Leu Ala Gly Ala Lys Arg Ala Gly Leu Tyr Arg Met
   4040 4045 4050
   Leu Gln Ser Glu Tyr Ser Lys Val Leu Ser Arg His Val Asp Thr
   4055 4060 4065
   Asp Ala Thr Leu Asp Gly Glu Arg Arg Asp Leu Val Ala Gln Val
   4070 4075 4080
   Val Ala Glu Leu Gly Ala Ala Ser Glu Ala Gly Glu Val Cys Tyr
   4085 4090 4095
   Arg Asp Gly Val Arg Tyr Glu Ala Ala Leu Glu Arg Leu Asp Glu
   4100 4105 4110
   Ala Ala Arg Ala Arg Glu Pro Gly Ala Gln Arg Pro Ala Gln Tyr
   4115 4120 4125
   Pro Pro Ala Glu Val Leu Trp Val Thr Gly Gly Thr Arg Gly Ile
   4130 4135 4140
   Gly Met Leu Cys Ala Lys His Phe Val Asp Ala His Gly Val Arg
   4145 4150 4155
   Lys Leu Val Leu Met Gly Arg Glu Ala Leu Pro Glu Arg Asp Ala
   4160 4165 4170
   Trp Asp Ala Tyr Leu Thr Ser Gly Ser Asp Glu Ile Cys Lys Arg
   4175 4180 4185
   Lys Ile Glu Asp Ile Leu Tyr Leu Glu Ala Arg Gly Ala Gln Val
   4190 4195 4200
   Arg Val Ser Thr Thr Pro Leu Thr Asp Glu Ala Arg Met Ala Ser
   4205 4210 4215
   Glu Val Ala Arg Val Arg Arg Glu Met Gly Glu Ala Leu Gly Val
   4220 4225 4230
   Val His Cys Ala Gly Asn Thr Asp Phe Thr Asn Pro Ala Phe Val
   4235 4240 4245
   Arg Lys Thr Arg Ala Gly Ile Glu Gly Val Ser Ala Pro Lys Ile
   4250 4255 4260
   Arg Gly Val Asp Val Leu Arg Gln Val Phe Ala Asp Val Pro Leu
   4265 4270 4275
   Arg Phe Val Leu Leu Phe Ser Ser Val Ala Ala Ala Val Pro Arg
   4280 4285 4290
   Leu Ala Val Gly Gln Ser Glu Tyr Ala Met Ala Asn Ala Tyr Leu
   4295 4300 4305
   Asp Tyr Val Ala Ala Ala Gly Phe Gly Pro Lys Val Ala His Cys
   4310 4315 4320
   Ala Ser Ile Gln Trp Pro Ser Trp Lys Asp Thr Gly Met Gly Ala
   4325 4330 4335
   Ala Ser Ser Ala Ala Tyr Arg Ser Thr Gly Leu Gln Thr Cys Ala
   4340 4345 4350
   Asp Asp Glu Gly Leu Arg Phe Leu Asp Glu Ile Leu Ala Arg Arg
   4355 4360 4365
   Leu Gly Pro Val Val Met Pro Leu Val Ile Asp Glu Ala Arg Phe
   4370 4375 4380
   Asp Thr Arg Ala Leu Leu Arg Arg Ala Thr Pro Ala Lys Gly Gln
   4385 4390 4395
   Ala Gly Pro Ser Pro Glu Arg Ser Ala Asp Pro Val Gly His Ala
   4400 4405 4410
   Ala Glu Ala Ser Arg Arg Ala Pro Ser Ala Asp Val Ala Arg Asp
   4415 4420 4425
   Met Asn Ala His His Ala Gly Ser Ser Thr Asp Thr Ala Ser Ala
   4430 4435 4440
   Val Asn Glu Trp Leu Arg Arg Leu Val Ser Gln Glu Leu Lys Val
   4445 4450 4455
   Lys Tyr Ala Asp Val Asp Ala Ala Arg Ser Phe Asp Gln Tyr Gly
   4460 4465 4470
   Val Asp Ser Ile Met Leu Ala Gln Met Ile Arg Gln Ile Asp Ala
   4475 4480 4485
   Gly Leu Pro Gly Ile Lys Val Asp Pro Thr Val Val Ile Glu Asn
   4490 4495 4500
   Pro Ser Ile Glu Arg Leu Gly Ala Tyr Leu Val Arg Thr Tyr Arg
   4505 4510 4515
   Asp Gln Leu Asp Arg Gln Phe Ala Phe Ala Lys Gly Ala Ala Gly
   4520 4525 4530
   Arg Val Thr Glu Arg Gln Leu Asp Arg Arg Ser Thr Glu Ala Gln
   4535 4540 4545
   Gly Ala Ala Gly Arg Val Thr Glu Gly Arg Ala Asp Lys Val Ala
   4550 4555 4560
   Ile Val Gly Met Ala Cys His Phe Pro Gly Ala Pro Asp Ile Gln
   4565 4570 4575
   Gln Phe Trp Lys Asn Leu Cys Glu Gly Lys Asp Ala Ile Gly Pro
   4580 4585 4590
   Val Pro Ala Ser Arg Trp Asp Ala Glu Arg Tyr His Gly Asp Pro
   4595 4600 4605
   Glu Arg Glu Pro Gly Arg Ser Val Ser Lys Trp Gly Gly Phe Leu
   4610 4615 4620
   Glu Asp Ile Glu Ser Phe Asp Pro Ser Tyr Phe Gly Ile Gln Glu
   4625 4630 4635
   Ser Ile Ala Ile Gly Val Asp Pro Leu Ala Arg Gln Trp Leu Glu
   4640 4645 4650
   Val Cys Ala Glu Ala Leu Ala Asp Ala Gly Tyr Gly Lys Thr Asp
   4655 4660 4665
   Leu Trp Asp Gln Arg Val Gly Val Phe Cys Gly Thr Arg Ser Ser
   4670 4675 4680
   Asn Tyr Ala Ser Lys His Gly Gln Leu Asp Ser Lys Ala Val Ile
   4685 4690 4695
   Gly Val Gly Gln Asn Phe Ile Ser Ala His Leu Ser His Ser Tyr
   4700 4705 4710
   Asn Phe Ala Gly Pro Asn Val Ile Val Asp Thr Ala Cys Ala Ser
   4715 4720 4725
   Ala Leu Thr Ala Val His Met Ala Val Gln Ser Ile Glu Arg Gly
   4730 4735 4740
   Glu Ser Ala Leu Ala Ile Ala Gly Gly Val Asp Ile Leu Leu Asp
   4745 4750 4755
   Glu Gly Pro Phe Val Leu Leu Ser Ser Ala Lys Ile Leu Ser Ser
   4760 4765 4770
   Glu Gly Arg Cys Lys Ala Phe Asp Glu Thr Ala Asp Gly Ile Gly
   4775 4780 4785
   Ile Gly Glu Gly Cys Gly Val Val Ile Leu Lys Pro Leu Ser Ala
   4790 4795 4800
   Ala Leu Arg Asp Gly Asp Lys Val Tyr Gly Val Ile Asp Gly Ser
   4805 4810 4815
   Ala Ile Asn Cys Asp Gly His Thr Met Gly Ile Thr Thr Pro Asn
   4820 4825 4830
   Pro Arg Arg Gln Gln Glu Leu Ile Glu Arg Ala Ile Ala Ala Ala
   4835 4840 4845
   Gly Val Asp Ala Arg Thr Ile Ser Tyr Val Glu Ala His Gly Thr
   4850 4855 4860
   Gly Thr Leu Ile Gly Asp Pro Ile Glu Leu Arg Ser Leu Thr Asn
   4865 4870 4875
   Val Leu Gly Arg Glu Ser Arg His Val Cys Gly Val Gly Ser Val
   4880 4885 4890
   Lys Ser Asn Leu Gly His Leu Leu Ser Ala Ala Gly Ala Ala Gly
   4895 4900 4905
   Leu Val Lys Val Leu Leu Ser Ile Val His Arg Arg Leu Pro Pro
   4910 4915 4920
   Thr Leu His Cys Ser Arg Pro Asn Pro Arg Phe Asp Phe Glu Gln
   4925 4930 4935
   Ser Pro Leu Tyr Pro Val Leu Arg Asn Glu Glu Trp Ala Gly Val
   4940 4945 4950
   Asp Gly Val His Arg Ala Gly Val Ser Ala Phe Gly Leu Gly Gly
   4955 4960 4965
   His Asn Ala His Val Ile Val Ser Asp Glu Gly Ile Pro Glu Glu
   4970 4975 4980
   Arg Arg Ala Thr Met Asp Pro Arg Gly Ala Pro Val Val Phe Glu
   4985 4990 4995
   Arg Lys Arg Tyr Trp Pro Asp Ala Ala Ala Pro Arg Pro Ser Pro
   5000 5005 5010
   Gly Val Ser Gly Gly Ala Asp Ser Ala Phe Ala Ala Arg Val Thr
   5015 5020 5025
   Ala Asp Ala Pro Gly Ser Val Thr Ala Gln Arg Ala Met Pro Lys
   5030 5035 5040
   Tyr Phe Ala Val Lys Arg Ile Glu Arg Asp Val Ala Thr Arg Ala
   5045 5050 5055
   Pro Ser Leu Glu Ala Ser Gln Ser Lys Ser Arg Pro
   5060 5065 5070
<210> 33
   <211> 3578
   <212> PRT
   <213> Sorangium cellulosum
<400> 33
   Met Thr Val Arg Ala Asn Glu Thr Phe Glu Ile Glu Phe Asp Asn Asp
   1 5 10 15
   Asn Pro Ile Val Arg Asp His Arg Val Tyr Gly Val His Ile Val Pro
   20 25 30
   Gly Val Thr Leu Ile Asp Val Val Tyr Arg Leu Gly Ala His Val Val
   35 40 45
   Gly His Asp Arg Phe Glu Leu Val Asn Val Leu Phe Lys Val Pro Ile
   50 55 60
   Ala Thr Thr Arg Glu Phe Ala Arg Arg Ile His Ala Ser Phe Thr Pro
   65 70 75 80
   Leu Gln Gln Gly Glu Trp Arg Val Glu Ile Ser Ser Val Arg Val Ser
   85 90 95
   Ser Ala Gly Gly Val Ser Asp Arg Val Asp Glu Asn Ala Glu Cys Leu
   100 105 110
   Leu Arg Cys Thr Asp Glu Glu Pro Arg Ala Ala Arg Gly Phe Asp Cys
   115 120 125
   Ala Ala Phe Ile Arg Thr Ala Glu Arg Arg Trp Ser Val Asp Glu Ile
   130 135 140
   Tyr Arg Gly Val Arg Glu Val Gly Val Val His Gly Pro Phe Met Gln
   145 150 155 160
   Thr Leu Gly Asp Val Tyr Gln Arg Gly Asp Glu Glu Leu Met Arg Leu
   165 170 175
   Ser Leu Gly Asp Glu Ala Glu Ala Leu Arg Glu Met Phe His Ala His
   180 185 190
   Pro Ala Phe Leu Asp Gly Ala Thr Phe Ala Gly Ser Ser Tyr Lys Leu
   195 200 205
   Ala Gly Gly Ser Ala Ala Asp Tyr Arg Asp Val Arg Pro Tyr Ile Pro
   210 215 220
   Phe Ala Ile Arg Arg Val Arg Leu His Arg Pro Phe Ser Ser Gln Ile
   225 230 235 240
   Phe Val Tyr Ser Arg His Ala Asp Lys Leu Asp Val Lys Arg Gly Ala
   245 250 255
   Ala Pro Arg Glu Val Thr Ala Ser Asp Leu Thr Ile Tyr Gly Glu Asp
   260 265 270
   Gly Ala Val Leu Ala Glu Leu Glu Arg Leu Ser Tyr Lys Arg Val Arg
   275 280 285
   Gln Glu Ile Asp Val Glu Lys Leu Ile Ala Thr Glu Pro Val Ala Ala
   290 295 300
   Gly Ala Glu Ala Glu Pro Ala Gly Glu Ala Gly Asp Asp Ala Lys Leu
   305 310 315 320
   Phe Ala Ser Ser Val Ala Arg Phe Val Glu Glu Gln Leu Val Gly Leu
   325 330 335
   Lys Gly Pro Gly Thr Pro Pro Leu Asp Pro Asp Ala Leu Phe Phe Asp
   340 345 350
   Ala Gly Leu Asp Ser Thr His Leu Leu Arg Leu Val Arg Ala Leu Glu
   355 360 365
   Lys Arg Cys Gly His Asp Leu Tyr Pro Thr Leu Leu Phe Glu Tyr Gln
   370 375 380
   Thr Leu Asn Ala Leu Ile Gly Tyr Leu Tyr Glu His Glu Arg Ala His
   385 390 395 400
   Phe Ala Pro Ser Arg Ala Leu Val Ala Arg Pro Ser Gly Ala Gly Ala
   405 410 415
   Pro Ala Asp Pro Ile Val Ala Arg Gly Ala Gly Ala Ala Arg Val Asn
   420 425 430
   Gly Val Ala Arg Ser Ala Pro Val Glu Val Thr Gly Ala Gly Arg Pro
   435 440 445
   Gly Ala Ala Leu Gly Asp Ala Arg Arg Ala Ala Arg Asp Ile Ala Val
   450 455 460
   Val Gly Leu Ala Gly Arg Tyr Pro Gly Ala Ser Thr Leu Glu Asp Phe
   465 470 475 480
   Trp Asp Val Val Val Glu Gly Arg Ile Cys Thr Arg Glu Leu Pro Ser
   485 490 495
   Glu His Trp Gly Arg Phe Ala Gly Pro Asp Ala Ala Pro Ala Arg Ala
   500 505 510
   Ala Gly Ala Pro Ser Pro Pro Cys Phe Gly Gly Val Leu Asp Asp Val
   515 520 525
   Asp Ala Phe Asp Ser Leu Phe Phe Gln Val Thr Pro Val Glu Ala Asp
   530 535 540
   Ala Leu Asp Pro Gln Leu Arg Leu Val Leu Glu Thr Ala Trp His Ala
   545 550 555 560
   Met Glu Asp Ala Gly Val Thr Val Asp Ser Leu Gln Arg Ser Arg Thr
   565 570 575
   Gly Val Tyr Val Gly Val Met Asn Asp Asp Tyr Thr Trp Ile Ala Ala
   580 585 590
   Glu Arg Leu Ala Arg Thr Gly Glu Tyr Ala Ser Ala Gly Ser Tyr Ala
   595 600 605
   His Glu Leu Ala Asn Arg Ile Ser Tyr His Leu Asn Leu Arg Gly Pro
   610 615 620
   Ser Met Thr Val Glu Ala Ala Cys Ala Ser Ser Met Val Ala Leu His
   625 630 635 640
   Leu Ala Arg Val Ala Leu Ala His Gly Glu Cys Asp Ala Ala Phe Ala
   645 650 655
   Gly Gly Val Asn Leu Ser Leu His Arg Ser Lys Tyr Leu Leu Leu Ala
   660 665 670
   Gly Leu Gly Val Met Ser Pro Asp Gly Ser Glu Lys Thr Phe Asp Ala
   675 680 685
   Ala Ala Asn Gly Tyr Val Pro Gly Glu Gly Val Gly Val Ala Leu Leu
   690 695 700
   Lys Pro Leu Glu Arg Ala Val Ala Asp Gly Asp Arg Ile Tyr Gly Val
   705 710 715 720
   Ile Thr Gly Ser Ala Val Asn His Ser Gly Arg Gly Gly Gly Arg Tyr
   725 730 735
   Ser Pro Asn Leu Asn Ala Leu Thr Asp Val Ile Gly Arg Gly Leu Ala
   740 745 750
   Asp Ala Gly Val Asp Ala Glu Ala Leu Thr Tyr Val Glu Ala His Gly
   755 760 765
   Thr Gly Ser Pro Val Gly Asp Pro Ile Glu Val Gln Ala Leu Ala Lys
   770 775 780
   Ala Leu Gly Arg Thr Asp Ala Ala Ala Pro Arg Cys Ala Leu Gly Ser
   785 790 795 800
   Lys Ala Asn Leu Gly His Leu Glu Ser Ala Ser Gly Ile Cys Ser Leu
   805 810 815
   Thr Lys Val Leu Leu Ala Ile Arg His Gly Arg Ile Pro Pro Cys Ala
   820 825 830
   Asn Val Gln Thr Ile His Pro Gly Leu Arg Leu Glu Ser Thr Pro Phe
   835 840 845
   Val Ile Pro Arg Glu Pro Val Gly Trp Pro Ala Pro Glu Gly Arg Arg
   850 855 860
   Val Ala Gly Ile His Ser Phe Gly Ile Gly Gly Thr Asn Ala Phe Met
   865 870 875 880
   Val Val Arg Gly His Asp Ala Gly Ala Asp Ala Arg Ala Ala Ala Glu
   885 890 895
   Ser Ala Pro Arg Ser Glu Ile Val Pro Leu Ser Ala Lys Thr Lys Ser
   900 905 910
   Ala Leu Glu Arg Tyr Cys Ala Asp Leu Leu Ala His Leu Glu Arg Gly
   915 920 925
   Gly Asp Glu His Ser Ala Ala Leu Ala Asp Leu Ala Tyr Thr Leu Gln
   930 935 940
   Thr Gly Arg Thr Ala Met Gln His Arg Val Ala Phe Val Ala Ala Ser
   945 950 955 960
   Ala Ala Glu Leu Arg Asp Ala Leu Arg Asp Leu Cys Ala Gly Arg Gly
   965 970 975
   Ala His Glu Gly Val Phe Ser Gly Ser Ala Gly Pro Gly Ala Val Ala
   980 985 990
   Ala Val Ala Pro Arg Ala Glu Asp Val Gly Ser Ala Arg Ala Leu Gly
   995 1000 1005
   Arg Ile Ala Glu Ala Trp Val Arg Gly Ala Asp Val Asp Trp Thr
   1010 1015 1020
   Arg Leu Gln Glu Gly Arg Ala Arg Arg Arg Val Ala Ala Pro Met
   1025 1030 1035
   Tyr Pro Phe Glu Arg Ser Arg His Ala Ile Thr Thr Ser Gly Glu
   1040 1045 1050
   Ala Arg Glu Leu Thr Ala Pro Arg Ala Ala Val Val His Gly Thr
   1055 1060 1065
   Ala Ala Trp Arg Glu Lys Ala Ala Ala Ser Val Ala Pro Glu Arg
   1070 1075 1080
   Ala Glu Ser Ala Ala His Gly Ser Ser Val Leu Leu Leu Ala Gly
   1085 1090 1095
   Gly Leu Ser Asp Glu Val Val Ala Ala Val Glu Arg Ser Ile Pro
   1100 1105 1110
   Leu Ala Ser Arg Glu Arg Leu Pro Arg Ser Glu Gly Gly Ser Leu
   1115 1120 1125
   Ala Glu Ser Ala Thr Arg Ala Phe Leu His Ala Leu Gly Ile Val
   1130 1135 1140
   Lys Asp Ala Leu Ala Arg Arg Ala Pro Glu Val Arg Gln Leu Ala
   1145 1150 1155
   Val Ile Val Asp Glu Arg Ala Pro Glu Val Val Tyr Ala Pro Leu
   1160 1165 1170
   Ser Ala Leu Leu Lys Ser Ala Arg Arg Glu Ser Arg Asp Leu Arg
   1175 1180 1185
   Val Lys Val Ile Thr Ile Ala Ser Ala Leu Asp Pro Ser Arg Ala
   1190 1195 1200
   Ala Arg Ala Val Arg Asp Glu Leu Ala Ser Ser Asp Asp Asp Val
   1205 1210 1215
   Asp Val Arg Tyr Thr Ser Glu Gly Arg Arg Glu Val Arg Val His
   1220 1225 1230
   Glu Val Val Glu Pro Gly Ala Val Glu Gly Thr Val Phe Arg Pro
   1235 1240 1245
   Gly Gly Val Tyr Trp Ile Thr Gly Gly Leu Gly Gly Leu Gly Arg
   1250 1255 1260
   Leu Phe Ala Arg Tyr Ile Ala Ser Val Glu Gly Thr Thr Val Ile
   1265 1270 1275
   Leu Ser Gly Arg Thr Ala Pro Ser Glu Ala Gln Arg Arg Glu Leu
   1280 1285 1290
   Glu Ala Leu Gly Ala Pro Ala Ser Arg Val Glu Leu Val Pro Cys
   1295 1300 1305
   Asp Val Ser Ala Arg Asp Asp Val Glu Arg Ala Ala Arg Ala Ile
   1310 1315 1320
   Glu Glu Arg Phe Gly Pro Leu Thr Gly Val Leu His Cys Ala Gly
   1325 1330 1335
   Val Thr Arg Asp Ala Leu Leu Leu His Lys Thr Val Arg Gln Ala
   1340 1345 1350
   Glu Glu Val Leu Ala Ala Lys Val Leu Gly Ala Glu Ala Ile Asp
   1355 1360 1365
   Glu Ala Thr Arg Arg Ser Pro Leu Glu Phe Val Ala Phe Phe Ser
   1370 1375 1380
   Ser Val Ala Ala Ile Trp Gly Asn Val Gly Gln Ala Asp Tyr Ala
   1385 1390 1395
   Ala Ala Asn Gly Phe Leu Asp Ala Tyr Ala Ala Leu Arg Asp Ala
   1400 1405 1410
   Arg Val Ala Arg Gly Glu Arg Arg Gly Lys Thr Val Ser Ile Ala
   1415 1420 1425
   Trp Pro Leu Trp Arg Asp Gly Gly Met Arg Ile Asp Ala Arg Gly
   1430 1435 1440
   Glu Leu Val Leu Lys Glu Lys Thr Gly Met Ile Pro Leu Ser Arg
   1445 1450 1455
   Gly Asp Gly Leu Gln Ala Phe Glu Gln Ala Ile Arg Gly Ala Glu
   1460 1465 1470
   Ser Gln Arg Ile Val Cys Ala Ile Asp Gly Gly Arg Gln Ile Phe
   1475 1480 1485
   Ala Pro Arg Pro Ala Val Arg Ala Glu Asn Ala Ser Ala Pro Ala
   1490 1495 1500
   Ala Val Arg Ala Ala Pro Asp Arg Gly Pro Ala Glu Ala Thr Arg
   1505 1510 1515
   Ala Gly Ala Glu Pro Leu Arg Arg His Ala Val Arg Tyr Leu Ser
   1520 1525 1530
   Asp Ala Leu Ser Arg Val Phe Arg Leu Pro Pro Glu Arg Leu Asp
   1535 1540 1545
   Pro Arg Ala Pro Leu Asp Ala Phe Gly Ile Asp Ser Ala Asn Val
   1550 1555 1560
   Leu Ala Leu Thr Ala Ala Leu Glu Val Asp Leu Gly Pro Leu Pro
   1565 1570 1575
   Lys Thr Leu Phe Tyr Glu Tyr Arg Thr Leu Asp Ala Leu Ala Glu
   1580 1585 1590
   Tyr Leu Val Ala Glu His Gly Ala Ser Leu Glu Arg Ala Leu Arg
   1595 1600 1605
   Val Pro Gly Ala Ala Ala Ser Ala Gly Ala Pro Pro Ser Ala Arg
   1610 1615 1620
   Glu Pro Ala Ala Gly Asp Ala Gly Ala Ala Ala Ser Ala Arg Ser
   1625 1630 1635
   Ala Pro Pro Pro Val Arg Ala Ala Ala Glu Arg Arg Gly Gly Ser
   1640 1645 1650
   Gly Pro Leu Asp Ile Ala Ile Ile Gly Met Ser Gly Arg Phe Pro
   1655 1660 1665
   Arg Ala Arg Ser Val Asp Glu Phe Trp Asp Asn Leu Arg Ala Gly
   1670 1675 1680
   Arg Asp Cys Ile Thr Glu Val Pro Ala Ser Arg Trp Asp Ala Asp
   1685 1690 1695
   Arg Tyr Tyr Asp Pro Asp Thr Ser Ala Ala Gly Lys Val His Cys
   1700 1705 1710
   Lys Trp Gly Gly Phe Ile Asp Gly Val Asp Glu Phe Asp Pro Leu
   1715 1720 1725
   Phe Phe Lys Ile Ser Pro Leu Glu Ala Gly Met Met Asp Pro Gln
   1730 1735 1740
   Glu Arg Leu Phe Leu Gln Ala Ala Trp Glu Thr Leu Glu Asp Gly
   1745 1750 1755
   Gly Tyr Thr Arg Ala Ala Leu Gly Ala Gly Arg Ala Glu Ser Leu
   1760 1765 1770
   Arg Pro Glu Glu Arg Val Gly Val Tyr Val Gly Val Met Tyr Ser
   1775 1780 1785
   Glu Tyr Gln Leu Tyr Gly Phe Glu Val Glu Glu Asp Arg Arg Ile
   1790 1795 1800
   Phe Gly Val Ala Gly Asn Ile Ala Gly Val Ala Asn Arg Val Ser
   1805 1810 1815
   Tyr Val Leu Asp Leu His Gly Pro Ser Ile Ala Leu Asp Thr Met
   1820 1825 1830
   Cys Ser Ser Ser Leu Thr Ala Ile His Leu Ala Cys Arg Ala Leu
   1835 1840 1845
   Arg Ser Gly Glu Cys Gly Leu Ala Leu Ala Gly Gly Val Asn Val
   1850 1855 1860
   Ser Ile His Pro Asn Lys Tyr Ala Val Leu Ser Gln Ala Arg Ile
   1865 1870 1875
   Ile Ser Thr Arg Gly Arg Cys Glu Ser Phe Gly Lys Gly Gly Glu
   1880 1885 1890
   Gly Tyr Val Pro Gly Glu Gly Val Gly Cys Val Leu Leu Lys Pro
   1895 1900 1905
   Leu Glu Arg Ala Arg Ala Asp Arg Asp His Ile Tyr Gly Val Ile
   1910 1915 1920
   Lys Gly Ser Ala Leu Ser His Gly Gly Lys Ala Ser Gly Tyr Thr
   1925 1930 1935
   Val Pro Ser Pro Val Ala Gln Ala Ser Thr Ile Ala Leu Ala Ile
   1940 1945 1950
   Arg Glu Ala Gly Val Asp Pro Arg Glu Ile Ser Tyr Ile Glu Ala
   1955 1960 1965
   His Gly Thr Gly Thr Ser Leu Gly Asp Pro Ile Glu Ile Ala Gly
   1970 1975 1980
   Leu Val Arg Ala Phe Gly Arg Ala Ser Asp Asp Lys Gln Tyr Cys
   1985 1990 1995
   Ala Ile Gly Ser Ala Lys Ser Asn Ile Gly His Cys Glu Ser Ala
   2000 2005 2010
   Ala Gly Ile Ala Gly Val Cys Lys Val Leu Leu Gln Met Lys His
   2015 2020 2025
   Arg Thr Leu Ala Pro Ser Leu His Ala Ala Glu Leu Asn Pro Tyr
   2030 2035 2040
   Ile Asp Phe Ala Ser Thr Pro Phe Val Val Gln Arg Glu Leu Ala
   2045 2050 2055
   Pro Trp Arg Ala Pro Thr Ile His Gly Glu Pro Arg Pro Arg Val
   2060 2065 2070
   Ala Gly Val Ser Ser Phe Gly Ala Gly Gly Ala Asn Ala His Val
   2075 2080 2085
   Ile Ile Glu Glu Trp Thr Glu Asp Asp Ala Pro Leu Ala Asp Ala
   2090 2095 2100
   Arg Ser Ala Arg Gly Ala Ala Val Ile Val Pro Met Ser Ala Arg
   2105 2110 2115
   Thr Glu Glu Arg Leu His Ala Val Val Gly Ala Met Leu Asp Val
   2120 2125 2130
   Ile Ala Arg Ala Ala Ala Gly Glu Gly Ser Ala Ile Glu Leu Ala
   2135 2140 2145
   Asp Val Ala Phe Thr Leu Gln Thr Gly Arg Glu Pro Met Pro Tyr
   2150 2155 2160
   Arg Val Ala Phe Val Ala Ala Asp Leu Gly Gly Leu Ala Ala Glu
   2165 2170 2175
   Met Arg Arg Phe Leu Ser Arg Glu Gln Gly Gly Tyr Arg Ala Gly
   2180 2185 2190
   His Ala Ser Glu Asp Gly Leu Asp Leu Leu Ala Asp Glu Asp Val
   2195 2200 2205
   Lys Gly Ala Val Val Ala Lys Trp Ile Gly Ala Gly Arg Leu Asp
   2210 2215 2220
   Lys Leu Ala Ala Phe Trp Ala Lys Gly Leu Asp Leu Asp Trp Asp
   2225 2230 2235
   Leu Leu Ser Gly Ser Gly Ala Pro Arg Gly Arg Arg Pro Arg Arg
   2240 2245 2250
   Ile Ser Leu Pro Thr Tyr Ser Phe Glu Arg Lys Arg Cys Gly Leu
   2255 2260 2265
   Pro Val Gln Arg Pro Ala Arg Arg Ala Ala Ser Arg Glu Val Ala
   2270 2275 2280
   Gly Gly Leu Leu Gln Arg Met Leu Pro Ala Ala Ser Leu Gly Glu
   2285 2290 2295
   Ala Pro Arg Val Val Phe Glu Ser Glu Ile Glu Ala Ser Asp Pro
   2300 2305 2310
   Leu Val Ala His His Arg Val Gln Gly Arg Arg Val Leu Pro Gly
   2315 2320 2325
   Val Ala Met Ile Glu Met Ala Met Glu Ala Ala Gln Arg Val His
   2330 2335 2340
   Ala Glu Arg Arg Ala Val Leu Lys Asp Val Val Trp Met Lys Pro
   2345 2350 2355
   Val Asp Val Pro Asp Gly Gly Arg Thr Leu Leu Ala Gln Leu Glu
   2360 2365 2370
   Pro His Gly Gly Gln Leu Arg Phe Glu Ile Arg Ala Val Val Glu
   2375 2380 2385
   Gly Ala Gln Gln Val His Ala Gln Gly Leu Val Ala Phe Asp Glu
   2390 2395 2400
   Val Asp Thr Asp Thr Pro Ala Ile Asp Val Gln Ala Leu Met Arg
   2405 2410 2415
   Thr Leu Thr Pro Arg Trp Gly Asp Asp Ala Gly His Asp Gly Phe
   2420 2425 2430
   Tyr Glu Lys His Arg Ala Asn Gly Val Asp Tyr Gly Pro Tyr Phe
   2435 2440 2445
   Arg Gly Leu Arg Arg Leu Trp Gly Ser Thr Ser Glu Ser Leu Gly
   2450 2455 2460
   Leu Val Glu Pro Pro Arg Glu Ala Arg Asp Asp Pro Arg Arg Tyr
   2465 2470 2475
   Ala Trp Thr Pro Cys Val Ala Asp Ala Ala Leu Gln Ser Val Ala
   2480 2485 2490
   Gly Ile Ala Ala Ser Asp Val Ala Pro Gln Gly Asp Gly Glu Val
   2495 2500 2505
   Tyr Leu Pro Tyr Ser Ile Gly Arg Val Glu Leu Leu Gly Pro Ile
   2510 2515 2520
   Glu Gly Arg Ala Phe Val His Ala Arg Arg Leu Ala Pro Gly Arg
   2525 2530 2535
   Phe Asp Ile Val Val Val Asp Gly Ala Gly Ala Pro Leu Ala Ala
   2540 2545 2550
   Leu Arg Asp Phe Val Val Arg Pro Val Lys Ala Ala Pro Pro Arg
   2555 2560 2565
   Gln Leu Thr Tyr Val Gly Arg Trp Arg Arg Arg Gly Leu Ala Pro
   2570 2575 2580
   Ala Ala Pro Ala Arg Glu Gly Ala Arg Ala Leu Ile Ile His Arg
   2585 2590 2595
   Gln Asn Gly Phe Arg Leu Glu Asp Ala Leu Ser Glu Leu Ala Pro
   2600 2605 2610
   Gly Ser Val Asn Val Cys Leu Gly Asp Arg Thr Glu Arg Thr Gly
   2615 2620 2625
   Glu Gln Gly Trp Ile Ile Asp Val Arg Asp Pro Gly Ala Ile Ala
   2630 2635 2640
   Arg Val Leu Arg Asp Val Gly Arg Val Gly Arg Val Tyr Phe Leu
   2645 2650 2655
   Ala Ala Leu Leu Gly Asp Arg Asp Tyr Arg Leu Asp Thr Ala Tyr
   2660 2665 2670
   Val Glu Glu Glu Gln Glu Arg Thr Val Ile Pro Trp Leu Arg Thr
   2675 2680 2685
   Ile Lys Ala Leu Ile Glu Gln Gly Tyr Asp Gln Ala Ser Leu Asp
   2690 2695 2700
   Val Thr Val Leu Thr Gln Asp Gly Glu Ser Leu Pro Leu Gln Ala
   2705 2710 2715
   Glu Leu Asn Ala Gly Gly Gly Ala Leu Pro Gly Leu Thr Gln Ser
   2720 2725 2730
   Leu Ala Arg Glu Tyr Pro His Trp Ser Val Arg His Val Asp Ile
   2735 2740 2745
   Gly Lys Ala Asp Met Ser Gly Pro Gly Ala Ala Arg Gln Ile Ala
   2750 2755 2760
   Glu Leu Leu Gln Arg Glu Ala Ala Gly Ser Pro Leu Pro Val Ala
   2765 2770 2775
   Tyr Arg Ala Gly Ala Arg Tyr Glu Arg Glu Ile Ala Pro Leu Val
   2780 2785 2790
   Leu Pro Arg Asp Glu Arg Pro Ala Phe Thr Glu Gly Gly Val Tyr
   2795 2800 2805
   Val Ile Ala Gly Gly Ala Gly Gly Leu Gly Arg Val Met Thr Glu
   2810 2815 2820
   His Leu Val Arg Arg Tyr Gly Ala His Val Val Trp Trp Gly Arg
   2825 2830 2835
   Arg Pro Met Asp Glu Ala Ile Arg Asp Gln Leu Ser Arg Leu Ala
   2840 2845 2850
   Asp Gly Pro Gly Thr Val Ala Tyr Ala Thr Val Asp Leu Thr Ser
   2855 2860 2865
   Leu Ser Asp Val Ala Arg Ala Arg Asp Val Ile Lys Glu Lys His
   2870 2875 2880
   Gly Arg Ile His Gly Val Ile His Ser Ala Ile Val Leu Arg Asp
   2885 2890 2895
   Arg Ser Leu Arg Glu Met Asp Glu Pro Arg Phe Arg Glu Ala Leu
   2900 2905 2910
   Asp Pro Lys Val Lys Gly Ser Ala Val Leu Gly Glu Ala Phe Gly
   2915 2920 2925
   Arg Glu Asp Leu Asp Trp Ile Cys Phe Tyr Ser Ser Met Gln Ser
   2930 2935 2940
   Val Tyr Ala Gly Ala Gly Gln Ser Asn Tyr Ala Ala Gly Cys Thr
   2945 2950 2955
   Phe Lys Asp Ala Tyr Ala Arg Ala Leu Ser Met Ala Cys Ser Phe
   2960 2965 2970
   Pro Val His Thr Ile Asp Trp Gly Tyr Trp Gly Thr Val Gly Val
   2975 2980 2985
   Val Ala Asn Glu Ala Tyr Arg Ser Arg Met Ala Ser Leu Gly Ile
   2990 2995 3000
   Gly Ser Leu Glu Pro Arg Asp Gly Ile Arg Val Gln Glu Ala Val
   3005 3010 3015
   Ile Ala His Arg Val Ser His Val Leu Ala Val Asp Leu Ala Pro
   3020 3025 3030
   Ala Thr Arg Asp Ala Leu Gly Ile Arg Pro Asp Gly Glu Glu Leu
   3035 3040 3045
   Tyr Ala Ala Thr Gln Pro Ser Cys Phe Glu Arg Val Gln Ala Ala
   3050 3055 3060
   Val Gly Ala Gly Arg Ala Leu Arg Asp Val Asp Ala Ala Gln Leu
   3065 3070 3075
   Arg Glu Leu Asp Ala Ala Asp Ala Cys Leu Asn Glu Tyr Val Val
   3080 3085 3090
   Asp Arg Leu Arg Ala Val Met Gly Ala Ala Gly Val Gly Asp Pro
   3095 3100 3105
   Arg Ala Glu Ala Ala Pro Val Glu Ala Leu Ala Ala Arg Ala Ala
   3110 3115 3120
   Val Val Asp Lys Gln Arg Pro Leu Phe Glu Glu Ile Val Arg Ile
   3125 3130 3135
   Met Arg Ser Asp Ala Pro Ser Pro Gly Ala Pro Arg Arg Ala Glu
   3140 3145 3150
   Asp Val Thr Ser Arg Tyr Pro Glu Leu Ala Pro His Val Ala Leu
   3155 3160 3165
   Ala Asp Ala Cys Leu Gly Ser Leu Pro Gln Val Leu Arg Gly Glu
   3170 3175 3180
   Val Leu Ala Thr Glu Val Leu Phe Pro Gly Ser Ser Thr Ser Leu
   3185 3190 3195
   Val Glu Gly Ile Tyr Lys Lys Asn Ala Tyr Ala Asp Phe Leu Asn
   3200 3205 3210
   Gly Leu Val Ala Asp Ala Val Glu Ala Ala Val Thr Ala Arg Ser
   3215 3220 3225
   Glu Ala Pro Ser Gly Pro Ala Lys Val Arg Ile Leu Glu Ile Gly
   3230 3235 3240
   Ala Gly Thr Gly Gly Thr Ser Gln Gly Ile Phe Glu Arg Leu Ala
   3245 3250 3255
   Ala His Arg Asp Arg Val Glu Tyr Val Tyr Thr Asp Val Ser Arg
   3260 3265 3270
   Ser Phe Leu Gly Leu Ala Gln Ala Lys Tyr Ser Glu Ile Ala Pro
   3275 3280 3285
   Tyr Met Arg Val Arg Pro Phe Asp Val Glu Arg Ala Pro Val Glu
   3290 3295 3300
   Gln Gly Ile Glu Glu Gly Gly Phe Asp Val Val Val Ala Ala Asn
   3305 3310 3315
   Val Leu His Ala Thr Lys Val Ile Ala Ser Thr Leu Arg Asn Val
   3320 3325 3330
   Lys Arg Cys Met Lys Arg Asn Gly Leu Leu Val Leu Asn Glu Ile
   3335 3340 3345
   Gly Gly Lys Arg Pro Phe Thr Thr Leu Thr Phe Gly Leu Leu Asp
   3350 3355 3360
   Gly Trp Trp Ala Ala Glu Asp Arg Glu Val Arg Leu Glu Gly Ser
   3365 3370 3375
   Pro Ala Leu Ser Arg Asp Gln Trp Thr Arg Cys Leu Ala Glu Glu
   3380 3385 3390
   Gly Phe Gly Arg Ala Trp Ala Ser Ala Pro Ala Ser Pro Ala Ala
   3395 3400 3405
   Gln Glu Phe Val Ile Leu Ala Glu Ser Asp Gly Arg Val Arg Arg
   3410 3415 3420
   Ala Asp Pro Gly Pro Arg Ala Ala Pro Ala Ala Pro Ala Arg Pro
   3425 3430 3435
   Pro Thr Arg Ala Ser Gly Ala Arg Lys Ala Arg Asp Thr Gln Gly
   3440 3445 3450
   Asp Leu Pro Ala Tyr Val Glu Ala Gln Val Arg Arg Ala Val Gly
   3455 3460 3465
   Asp Val Leu Lys Leu Ser Asp Asp Asp Leu Asp Pro Gly Leu Ala
   3470 3475 3480
   Leu Ser Asp Leu Gly Val Asp Ser Ile Val Ala Val Asp Leu Val
   3485 3490 3495
   Arg Arg Ile Asn Ala Ser Leu Gly Leu Ser Leu Lys Thr Thr Val
   3500 3505 3510
   Ile Phe Asp Phe Pro Asn Ile Arg Arg Leu Ala Ala His Leu Cys
   3515 3520 3525
   Ala Glu His Pro Asp Asp Leu Ala Arg Arg Gln Ala Gly Thr Gln
   3530 3535 3540
   Ala Asn Gly Gly Ala Val Leu Glu Arg Arg Ala Val Glu Ala Leu
   3545 3550 3555
   Ala Glu Glu Leu Ala Leu Gly Arg Ile Ser Leu Asp Gln Thr Leu
   3560 3565 3570
   Asp Arg Leu Arg Ala
   3575
<210> 34
   <211> 5973
   <212> PRT
   <213> Sorangium cellulosum
<400> 34
   Met Pro Glu His Ser Tyr Glu Pro Arg Gly Glu Ser Thr Val Lys Ser
   1 5 10 15
   Gln Ile Glu Ala Leu Leu Arg Glu Tyr Gln Ser Gly Leu Leu Ser Glu
   20 25 30
   Ala Glu Leu Arg Ala Arg Tyr Ala Gln Leu Val Ala Ser Gly Ser Asp
   35 40 45
   Gly Ala Pro Ala Arg Pro Ala Asp Arg Ala Gly Ala Thr Gln Thr Val
   50 55 60
   Arg Arg Arg Arg Val Val Leu Asp Gly Pro Gly Ser Ile Glu Asp Ile
   65 70 75 80
   Arg Val Val Glu Asp Glu Val Gly Ala Pro Glu Glu His Ala Ile His
   85 90 95
   Val Asp Thr Val Ala Ile Ser Leu Asn Phe Gly Asp Leu Leu Cys Val
   100 105 110
   Gln Gly Leu Tyr Pro Thr Met Pro Ala Tyr Pro Phe Thr Pro Gly Phe
   115 120 125
   Glu Ala Ser Gly Ile Val Arg Ala Val Gly Ser Lys Val Thr Arg Phe
   130 135 140
   Ala Ala Gly Asp Glu Val Ile Val Ser Ala Ser Val Arg Leu Gly Ile
   145 150 155 160
   Gln Ser Thr Leu Ile Val Ala Asp Glu Ser Gln Leu Val Ala Lys Pro
   165 170 175
   Ala Arg Leu Ser Phe Glu Glu Ala Ala Ser Phe Leu Thr Val Gly Leu
   180 185 190
   Thr Ile Val Glu Ala Phe Arg Arg Leu Arg Leu Ala Lys Gly Glu Thr
   195 200 205
   Ile Leu Val Gln Ser Ala Thr Gly Gly Thr Gly Leu Val Ala Val Gln
   210 215 220
   Leu Ala Gln Arg Ala Gly Ala Thr Ile Ile Ala Thr Ala Ser Ser Asp
   225 230 235 240
   Glu Lys Leu Arg Tyr Leu Thr Ala Met Gly Val Pro His Val Ile Asp
   245 250 255
   Tyr Val Arg Arg Asp Phe Glu Ala Glu Val Met Ala Ile Thr Lys Gly
   260 265 270
   Lys Gly Val Asp Ala Val Ile Asn Thr Leu Ala Gly Glu Ala Ile Gln
   275 280 285
   Lys Gly Ile Asn Cys Leu Ser Pro Arg Gly Arg Tyr Val Glu Ile Ala
   290 295 300
   Met Thr Gly Leu Lys Ser Ala Arg Ser Ile Asp Leu Ser Arg Met Ser
   305 310 315 320
   Asn Asn Gln Ser Phe His Ser Leu Asp Leu Arg Lys Leu Leu Leu Asp
   325 330 335
   Val Pro Glu His Ala Ala Asp Leu Gly Glu Glu Leu Phe Ser Leu Val
   340 345 350
   Glu Arg Gly Glu Leu Arg Ile Val Val Gly Lys Thr Phe Asp Phe Asp
   355 360 365
   Ala Ile Gly Asp Ala Tyr Arg Tyr Leu Ala Asp Arg Arg Asn Ile Gly
   370 375 380
   Lys Val Val Val Thr Val Pro Gly Pro Lys Thr Ala Ser Val Arg Arg
   385 390 395 400
   Glu Ala Pro Met Arg Ala Arg Pro Ala Ala Arg Pro Ala Asp Arg Ala
   405 410 415
   Gly Ala Arg Gly Glu Gln Pro Ile Ala Ile Val Gly Met Ser Gly Arg
   420 425 430
   Phe Gly Ala Ala Lys Asp Ile Asp Glu Leu Trp Arg Cys Val Val Glu
   435 440 445
   Met Arg Gly Leu Ile Thr Gln Leu Pro Glu Gly Arg Lys Arg Leu Phe
   450 455 460
   Gln Ser Leu Glu Leu Pro Ala Ala Gln Arg Gly Val Glu Met Asp Pro
   465 470 475 480
   Trp Gly Ser Tyr Leu Asp Asp Ile Glu Cys Phe Asp Pro Leu Phe Phe
   485 490 495
   Ser Ile Ser Gly Gln Glu Ala Gln His Met Asp Pro Gln Gln Arg Ile
   500 505 510
   Phe Leu Gln Glu Ser Trp Lys Ala Leu Glu Asp Ala Gly Leu Thr Leu
   515 520 525
   Ala Arg Leu Asp Gly Ser Arg Thr Gly Val Val Ala Gly Val Val Asp
   530 535 540
   Gly Lys Tyr Ala Ser Tyr Ile Glu Glu Asp Lys Val Gly His Ser Phe
   545 550 555 560
   Trp Gly Thr Ala Gly Ser Ile Leu Pro Ala Arg Ile Ala Tyr Phe Leu
   565 570 575
   Asn Leu Lys Gly Pro Ala Ile Ala Ile Asp Thr Ala Cys Ser Ser Ser
   580 585 590
   Leu Val Ala Val His Ile Gly Cys Gln Ser Leu Trp Ser Arg Glu Thr
   595 600 605
   Asp Val Ile Leu Ala Gly Gly Ala Phe Ile Gln Ala Thr Pro His Phe
   610 615 620
   Gly Val Ala Ala Gly Arg Ala Gln Met Leu Ser Ala Asp Gly Lys Cys
   625 630 635 640
   Phe Thr Phe Asp Asp Arg Ala Asn Gly Met Val Ser Gly Glu Ala Val
   645 650 655
   Ala Val Leu Val Leu Glu Arg Leu Asp Asp Ala Ile Ala Asn Gly Asp
   660 665 670
   Arg Ile His Gly Val Ile Val Gly Ser Gly Ile Asn Gln Asp Gly Ala
   675 680 685
   Thr Asn Gly Ile Thr Ala Pro Ser Ala Ala Ser Gln Arg Gln Leu Glu
   690 695 700
   Leu Asp Val Tyr Asn Arg Phe Gly Ile Asp Pro Gly Val Ile Gln Tyr
   705 710 715 720
   Val Glu Ala His Gly Thr Gly Thr Lys Leu Gly Asp Pro Ile Glu Val
   725 730 735
   Arg Ala Leu Thr Glu Ala Phe Gln Arg Phe Thr Asp Arg Arg Arg Phe
   740 745 750
   Cys Ala Ile Gly Ser Val Lys Pro Asn Val Gly His Thr Val Thr Val
   755 760 765
   Ala Gly Val Thr Gly Val Ile Asn Val Leu Leu Ala Leu Arg His Arg
   770 775 780
   Leu Leu Pro Gly Leu Leu Asn Tyr Gly Ile Pro Asn Arg His Ile Asp
   785 790 795 800
   Phe Glu Asn Ser Pro Phe Tyr Val Asn Thr Ser Ala Val Pro Trp Val
   805 810 815
   Val Glu Gly Gly Lys Arg Arg Arg Ala Ala Val Ser Ser Phe Gly Phe
   820 825 830
   Ser Gly Thr Asn Ala His Val Val Ile Glu Glu Tyr Val Pro Pro Ser
   835 840 845
   Arg Asp Gly His Gly Thr Ala Asp Asp Gly Pro Ser Leu Leu Pro Ile
   850 855 860
   Ser Ala Arg Thr Gly Asp Gln Leu Leu Gln Tyr Val Ser Asp Val Leu
   865 870 875 880
   Ala His Val Asn Ala Leu Ser Ala Ala Glu Ala Asp Arg Thr Thr Arg
   885 890 895
   His Glu Arg Leu Ala Asp Leu Ala Tyr Thr Phe Gln Val Gly Arg Glu
   900 905 910
   Ala Phe Glu His Arg Leu Ala Val Ile Ala Asp Asp Leu Glu Ser Leu
   915 920 925
   Ala Ser Arg Leu Ser Asp Val Val Ala Gly Lys Glu Arg Val Glu Gly
   930 935 940
   Cys Phe Arg Gly Lys Val Lys Lys Ala Lys Ala Pro Glu Arg Ala Ser
   945 950 955 960
   Gly Ser Asp Gly Gly Asp Gly Ala Val Gly Ala Pro Gly Asp Ala Leu
   965 970 975
   Arg Arg Leu Ala Glu Arg Trp Val Glu Gly Tyr Ala Val Asp Trp Ser
   980 985 990
   Ser Leu His Ala Gly Ala Arg Arg Ser Lys Val Ser Ala Pro Thr Tyr
   995 1000 1005
   Pro Phe Ala Arg Asp Arg Tyr Trp Ser Glu Ala Thr Pro Arg Ala
   1010 1015 1020
   Ala Ser Arg Glu Glu Ala Gly Asp Arg Arg Ala Arg Gly Ala Gly
   1025 1030 1035
   Pro Gln Arg His Ala Gly Glu Arg Ala Arg Val Glu Met Ala Pro
   1040 1045 1050
   Ala Glu Ala Ala Thr Ala Arg Arg Ala Ser Val Glu Ala Ala Val
   1055 1060 1065
   Glu Gly Ser Gly Gly Gly Gln Ala Leu Arg Leu Val Pro Val Trp
   1070 1075 1080
   Glu Pro Val Glu Ala Arg Ala Val Gly Arg Val Glu Ala Pro Arg
   1085 1090 1095
   Gly Ala Val Trp Val Val Gly Gly Asn Glu Glu Gln Arg Ala Leu
   1100 1105 1110
   Leu Gly Ala Ser Trp Leu Pro Val Ala Gly Gly His Glu Ala Ser
   1115 1120 1125
   Val Glu Gln Leu Val Glu Gln Leu Gly Ala Gly Gly Glu Val Gly
   1130 1135 1140
   Glu Val Val Trp Ile Ala Pro Arg Ser Arg Tyr Thr Ala Ile Glu
   1145 1150 1155
   Gly Glu Ser Val Leu Glu Glu Gln Arg Ala Gly Val Glu Ser Cys
   1160 1165 1170
   Phe Arg Leu Val Lys Ala Leu Leu Ala Arg Gly Tyr Gly Ser Arg
   1175 1180 1185
   Glu Leu Ala Leu Thr Ala Val Thr Val Arg Ala Gln Ala Val Arg
   1190 1195 1200
   Lys Gly Glu Glu Ile Asp Pro Thr His Ala Ser Val His Gly Leu
   1205 1210 1215
   Trp Gly Thr Val Ala Lys Glu Tyr Pro Arg Trp Lys Val Arg Val
   1220 1225 1230
   Ala Asp Leu Glu Gly Glu Arg Gly Ala Gly Trp Ser Glu Val Leu
   1235 1240 1245
   Thr Leu Ala Pro Ala Gly Glu Gly Asp Ala Trp Ala Asn Arg Gly
   1250 1255 1260
   Gly Glu Trp Phe Arg Gln Thr Leu Leu Pro Cys Glu Val Ser Pro
   1265 1270 1275
   Ser Ser Gly Gly Gly Gly Tyr Arg Glu Gly Gly Val Tyr Val Val
   1280 1285 1290
   Ile Gly Gly Gly Gly Gly Ile Gly Glu Val Leu Ser Glu His Leu
   1295 1300 1305
   Ile Arg Arg Tyr Arg Ala Gln Val Val Trp Ile Gly Arg Arg Gly
   1310 1315 1320
   Ala Thr Glu Glu Leu Glu Ser Lys Arg Lys Arg Leu Gly Glu Leu
   1325 1330 1335
   Gly Pro Arg Pro Glu Tyr Glu Gln Ala Asp Ala Ala Asp Arg Glu
   1340 1345 1350
   Ala Leu Glu Gln Ala Tyr Ala Arg Ile Lys Ala Arg His Gly Ala
   1355 1360 1365
   Ile His Gly Val Val His Ser Ala Val Gly Thr Phe Asp Lys Ser
   1370 1375 1380
   Val Ala Asn Thr Thr Pro Glu Gln Phe Ala Glu Val Leu Arg Ala
   1385 1390 1395
   Lys Val Asp Val Ser Val Arg Leu Ala Gln Val Phe Gly Asn Glu
   1400 1405 1410
   Pro Leu Asp Phe Ala Met Phe Phe Ser Ser Leu Asn Ala Phe Ala
   1415 1420 1425
   Lys Gly Ala Gly Lys Cys Gly Tyr Ser Ala Gly Ser Thr Phe Glu
   1430 1435 1440
   Asp Ala Tyr Ala His Gln Leu Gly Lys Ser Arg Pro Phe Ala Val
   1445 1450 1455
   Lys Thr Met Asn Trp Gly Trp Trp Gly Glu Val Gly Ser Ala Gly
   1460 1465 1470
   Val Val Pro Gln Ser Tyr Lys Asn Arg Ile Ala Leu Ala Gly Ile
   1475 1480 1485
   Gly Val Ile Asp Ala Glu Asp Ala Met Ser Ala Leu Glu Ala Leu
   1490 1495 1500
   Leu Ala Gly Pro Phe Asp Gln Leu Gly Leu Thr Lys Thr Thr Gly
   1505 1510 1515
   Ala Gln Ala Leu Gly Pro Ser Ala Pro Ala Thr Asp Pro Ala Arg
   1520 1525 1530
   Pro Glu Asp Arg Leu Pro Pro Ala Ile Ala Ala Arg Gly Glu Pro
   1535 1540 1545
   Glu Arg Leu Pro Ala Glu Arg Ala Arg His Gly Val Glu Ala Glu
   1550 1555 1560
   Ala Ala Ala Pro Asp Glu Gln Leu Asp Glu Ser Leu Leu Arg Ala
   1565 1570 1575
   Lys Ser Val Ala Tyr Leu Arg Ala Leu Val Ser Arg Thr Leu Arg
   1580 1585 1590
   Val Asn Ala Ala Leu Ile Asp Val Thr Glu Pro Leu Glu Arg Tyr
   1595 1600 1605
   Gly Ile Asp Ser Ile Leu Val Met Gln Leu Ile Asp Ala Leu Lys
   1610 1615 1620
   Glu Ala Ile Pro Asp Val Gly Ser Thr Leu Phe Phe Glu His Gln
   1625 1630 1635
   Thr Ile Asp Ala Leu Ala Asp His Leu Val Ser Thr His Arg Glu
   1640 1645 1650
   Ala Met Arg Gln Leu Thr Gly Leu Thr Ser Ser Ala Ala Ala Ser
   1655 1660 1665
   Ala Ala Glu Gly Pro Arg Ala Ala His Gly Ala Glu Gly Pro Ala
   1670 1675 1680
   Pro Pro Ser Leu Ala Ser Ala Arg Ser Ala Pro Arg Ala Asn Gly
   1685 1690 1695
   Ser Pro Ala Ala Gly Pro Ala Arg Asp Ala Leu Asp Asp Arg Arg
   1700 1705 1710
   Ile Ala Ile Ile Gly Leu Ser Gly Arg Tyr Pro Gln Ala Arg Thr
   1715 1720 1725
   Val Asp Glu Tyr Trp Glu Asn Leu Lys Ala Gly Lys Asp Ser Val
   1730 1735 1740
   Val Glu Ile Pro Pro Glu Arg Trp Ser Leu Asp Gly Phe Tyr Cys
   1745 1750 1755
   Asp Asp Val Glu Glu Ala Val Thr Arg Gly Lys Ser Tyr Cys Arg
   1760 1765 1770
   Trp Gly Gly Phe Leu Asp Gly Phe Thr Glu Phe Asp Pro Leu Phe
   1775 1780 1785
   Phe His Ile Ser Pro Leu Glu Ala Glu Gly Ile Asp Pro Gln Glu
   1790 1795 1800
   Arg Leu Phe Met Gln Ala Ser Trp Asp Val Leu Glu Asp Ala Gly
   1805 1810 1815
   Tyr Thr Arg Glu Ser Leu Ala Arg Arg His Gly Gly Arg Val Gly
   1820 1825 1830
   Val Phe Ala Gly Ile Thr Arg Gly Gly Phe Glu Leu His Gly Pro
   1835 1840 1845
   Gly Leu Trp Gln Arg Gly Ile Ala Ala Phe Pro Arg Thr Ser Phe
   1850 1855 1860
   Ser Ser Val Ala Asn Arg Val Ser Tyr Phe Leu Asn Leu His Gly
   1865 1870 1875
   Pro Ser Met Pro Val Asp Thr Met Cys Ser Ser Ser Leu Thr Ala
   1880 1885 1890
   Ile His Glu Ala Cys Glu His Leu Leu Arg Asp Glu Cys Glu Leu
   1895 1900 1905
   Ala Ile Ala Gly Gly Val Asn Leu Tyr Val His Pro Ser Ser Tyr
   1910 1915 1920
   Val Met Leu Cys Leu Gly Arg Met Leu Ser Pro Arg Gly Arg Cys
   1925 1930 1935
   Arg Ser Phe Gly Val Asp Gly Asp Gly Met Val Pro Gly Glu Gly
   1940 1945 1950
   Val Gly Thr Val Leu Leu Lys Arg Leu Ser Arg Ala Glu Ala Asp
   1955 1960 1965
   Gly Asp His Ile Tyr Gly Val Ile Leu Gly Thr Ser Ile Asn His
   1970 1975 1980
   Gly Gly Lys Thr Asn Gly Tyr Thr Val Pro Asn Pro Ala Ala His
   1985 1990 1995
   Lys Ala Leu Ile Leu Ser Ala Leu Gly Lys Ala Gly Val Asp Ala
   2000 2005 2010
   Arg Ala Val Ser Tyr Val Glu Ala His Gly Thr Gly Thr Glu Leu
   2015 2020 2025
   Gly Asp Pro Ile Glu Ile Ala Gly Leu Thr Gln Ala Phe Arg Ala
   2030 2035 2040
   Ser Thr Pro Asp Val Gln Phe Cys Ala Ile Ala Ser Ala Lys Ser
   2045 2050 2055
   Asn Ile Gly His Cys Glu Ala Ala Ala Gly Ile Ala Gly Leu Thr
   2060 2065 2070
   Lys Val Leu Leu Gln Met Lys His Gly Gln Ile Ala Pro Ser Leu
   2075 2080 2085
   His Val Glu Glu Leu Asn Pro His Ile Ala Phe Glu Lys Thr Pro
   2090 2095 2100
   Phe Val Val Gln Arg Arg Leu Gly Glu Trp Lys Arg Pro Thr Leu
   2105 2110 2115
   Ser Val Gly Gly Arg Ser Val Glu Ala Pro Arg Ile Ala Gly Ile
   2120 2125 2130
   Ser Ser Phe Gly Ala Gly Gly Ala Asn Ala His Val Ile Val Ala
   2135 2140 2145
   Glu Tyr Val Asp Glu Arg Ala Ala Leu Gly Arg Ala Ser Ala Ala
   2150 2155 2160
   Ser Pro Ala Pro Val Ala Val Val Leu Ser Ala Arg Thr Glu Ala
   2165 2170 2175
   Gln Leu Glu Ala Arg Ala Ala Gln Leu Leu Ala Ala Val Arg Ser
   2180 2185 2190
   Ser Glu Tyr Gly Asp Ala Glu Leu Pro Asp Ile Ala Tyr Thr Leu
   2195 2200 2205
   Gln Val Gly Arg Glu Ala Met Asp His Arg Leu Ala Phe Thr Ala
   2210 2215 2220
   Ala Ser Val Arg Glu Leu Cys Gly Lys Leu Ala Arg Phe Val Ala
   2225 2230 2235
   Gly Gly Glu Pro Ser Glu Asp Ile His Arg Gly Glu Ile Lys Arg
   2240 2245 2250
   Ala Lys Asp Gly Ile Ser Leu Phe Ala Glu Asp Glu Asp Leu Arg
   2255 2260 2265
   Arg Ala Val Asp Ala Trp Ile Gly Lys Lys Lys Tyr Ala Arg Leu
   2270 2275 2280
   Leu Glu Leu Trp Val Arg Gly Leu Ser Ile Asp Trp Thr Arg Leu
   2285 2290 2295
   His Glu Gly Gly Ala Pro Arg Arg Val Pro Leu Pro Thr Tyr Pro
   2300 2305 2310
   Phe Ala Arg Asp Arg Tyr Trp Pro Ala Gly Gly Ala Met Gly Ala
   2315 2320 2325
   Ala Ser Ala Leu Gln Ala Ser Asp Gly Ala Val Ala Ala Leu His
   2330 2335 2340
   Pro Leu Leu His Arg Asn Thr Ser Asp Phe Ala Gly Gln Arg Phe
   2345 2350 2355
   Thr Ser Glu Leu Thr Gly Ser Glu Phe Phe Leu Lys Glu His Val
   2360 2365 2370
   Val Gln Gly Arg Gly Val Leu Pro Gly Ala Ala Tyr Leu Glu Met
   2375 2380 2385
   Val Arg Ala Ala Leu Glu Leu Ser Ser Gly Asp Gly Ala Gly Asp
   2390 2395 2400
   Val Ala Leu His Asp Val Ala Trp Leu Gln Pro Phe Thr Val Val
   2405 2410 2415
   Asp Gly Arg Ser Ala Leu His Val Ser Leu Asp Val Pro Asp Glu
   2420 2425 2430
   His Gly Ala Ser Arg Phe Glu Val Tyr Ser Glu Gly Glu Gly Gly
   2435 2440 2445
   Ser Glu Ile Val His Cys Arg Gly Glu Ala Ala Leu Val Glu Lys
   2450 2455 2460
   Ser Ala Pro Gln Arg Leu Asp Leu Gly Glu Leu Arg Ala Arg Cys
   2465 2470 2475
   Ala Ala Gly Glu Val Asp Gly Ala Gly Cys Tyr Glu Ala Phe Asp
   2480 2485 2490
   Ala Ala Gly Ile Ala Tyr Gly Ala Ala Phe Arg Val Leu Arg Arg
   2495 2500 2505
   Ala His Leu Gly Ala Arg Glu Ala Leu Val Glu Leu Glu Leu Pro
   2510 2515 2520
   Glu Glu Gln Arg Ala Ser Ser Ala Phe Val Ile His Pro Ser Val
   2525 2530 2535
   Leu Asp Gly Ala Leu Gln Gly Thr Ala Cys Val Ser Met Gly Glu
   2540 2545 2550
   Pro Glu Gly Pro Gly Ala Ala Gly Ser Ala Pro Lys Ala Ala Leu
   2555 2560 2565
   Leu Phe Ser Val Arg Asp Val Asp Val Val Arg Ala Ser Ala Pro
   2570 2575 2580
   Arg Met Trp Ala Trp Ile Arg Val Ser Ala Ala Ser Thr Glu Arg
   2585 2590 2595
   Val Arg Gln Phe Asp Ile Asp Val Cys Asp Glu Glu Gly Val Val
   2600 2605 2610
   Cys Ala Arg Leu Ser Gly Val Ser Ala Arg Val His Asp Gly Ala
   2615 2620 2625
   Pro Ala Arg Arg Ala Ser Val Glu Ala Ala Ala Glu Gly Ser Gly
   2630 2635 2640
   Gly Gly Gln Ala Leu Arg Leu Val Pro Val Trp Glu Pro Val Glu
   2645 2650 2655
   Ala Arg Ala Val Gly Arg Val Glu Ala Pro Arg Gly Ala Val Trp
   2660 2665 2670
   Val Val Gly Gly Asn Glu Glu Gln Arg Ala Gln Leu Gly Ala Ser
   2675 2680 2685
   Trp Leu Pro Val Ala Gly Gly His Glu Ala Ser Val Glu Gln Leu
   2690 2695 2700
   Val Glu Gln Leu Gly Ala Gly Gly Glu Val Gly Glu Val Val Trp
   2705 2710 2715
   Ile Ala Pro Arg Ser Arg Tyr Thr Ala Ile Glu Gly Glu Ser Val
   2720 2725 2730
   Leu Glu Glu Gln Arg Val Gly Val Glu Ser Cys Phe Arg Leu Val
   2735 2740 2745
   Lys Ala Leu Leu Ala Arg Gly Tyr Gly Ser Arg Glu Leu Ala Leu
   2750 2755 2760
   Thr Ala Val Thr Val Arg Ala Gln Ala Val Arg Lys Gly Glu Glu
   2765 2770 2775
   Ile Asp Pro Thr His Ala Ser Val His Gly Leu Trp Gly Thr Val
   2780 2785 2790
   Ala Lys Glu Tyr Pro Arg Trp Lys Val Arg Val Ala Asp Leu Glu
   2795 2800 2805
   Gly Glu Gln Gly Ala Gly Trp Ser Glu Val Leu Thr Leu Ala Pro
   2810 2815 2820
   Ala Gly Glu Gly Asp Ala Trp Ala Asn Arg Gly Gly Glu Trp Phe
   2825 2830 2835
   Arg Gln Thr Leu Leu Pro Cys Glu Val Ser Pro Ser Ser Gly Gly
   2840 2845 2850
   Arg Gly Tyr Arg Glu Gly Gly Val Tyr Val Val Ile Gly Gly Gly
   2855 2860 2865
   Gly Gly Ile Gly Glu Val Leu Ser Glu His Leu Ile Arg Arg Tyr
   2870 2875 2880
   Arg Ala Gln Val Val Trp Ile Gly Arg Arg Gly Ala Thr Glu Glu
   2885 2890 2895
   Leu Glu Ser Lys Arg Lys Arg Leu Gly Glu Leu Gly Pro Arg Pro
   2900 2905 2910
   Glu Tyr Glu Gln Ala Asp Ala Ala Asp Arg Glu Ala Leu Glu Gln
   2915 2920 2925
   Ala Tyr Ala Arg Ile Lys Ala Arg His Gly Ala Ile His Gly Val
   2930 2935 2940
   Val His Ser Ala Val Gly Thr Phe Asp Lys Ser Val Ala Asn Thr
   2945 2950 2955
   Thr Pro Glu Gln Phe Ala Glu Val Leu Arg Ala Lys Val Asp Val
   2960 2965 2970
   Ser Val Arg Leu Ala Gln Val Phe Gly Asn Glu Pro Leu Asp Phe
   2975 2980 2985
   Ala Met Phe Phe Ser Gly Met Asn Ser Phe Ser Lys Asp Ala Gly
   2990 2995 3000
   Lys Cys Gly Tyr Ser Ala Gly Gly Thr Phe Glu Asp Ala Tyr Ala
   3005 3010 3015
   His Gln Leu Gly Lys Ser Arg Pro Phe Ala Val Lys Thr Met Asn
   3020 3025 3030
   Trp Gly Trp Trp Gly Glu Val Gly Ala Ala Gly Val Val Pro Gln
   3035 3040 3045
   Ser Tyr Lys Asn Arg Met Ala Leu Ala Gly Ile Ser Ala Ile Gly
   3050 3055 3060
   Ala Glu Asp Ala Ala Ser Ala Leu Glu Ala Leu Leu Ala Gly Pro
   3065 3070 3075
   Phe Asp Gln Leu Gly Leu Met Lys Phe Glu Lys Leu Gly Leu Asn
   3080 3085 3090
   Lys Val Ala Arg Ala Gly Thr Ser Gly Ser Val Glu Ala Glu Thr
   3095 3100 3105
   Pro Trp Leu Val Ala Pro Pro Pro Arg Asp Pro Ser Val Ile Gly
   3110 3115 3120
   Ala Met Ser Leu Arg Glu Ala Pro Pro Pro Ala Leu Asp Ala Asp
   3125 3130 3135
   Arg Ala Ser Arg Arg Met Phe Glu Gln Leu Asp Glu His Leu Arg
   3140 3145 3150
   Glu Ile Leu Ser Ile His Leu Arg Asp Leu Gly Leu Leu Asp Glu
   3155 3160 3165
   Gly Gly Ala Ser Leu Val Glu Lys Ser Arg Ala Ile Gly Met Pro
   3170 3175 3180
   Pro Leu Tyr Asp Arg Trp Leu Arg Glu Ser Leu Arg Leu Leu Arg
   3185 3190 3195
   Ala Arg Gly Ala Ala Arg Ser Asp Val Asp Ala Ala Gln Ala Trp
   3200 3205 3210
   Arg Arg Trp Glu Gln Glu Lys Val Ala Trp Ser Ala Glu Pro Asn
   3215 3220 3225
   Leu Arg Ala Gln Val Arg Leu Val Glu Ala Thr Leu Arg Ala Leu
   3230 3235 3240
   Pro Arg Ile Leu Thr Gly Ala Ile Pro Ala Thr Glu Val Ile Phe
   3245 3250 3255
   Pro Arg Ser Ser Met Ala Leu Val Glu Asp Val Tyr Lys Asn Asn
   3260 3265 3270
   Gly Pro Ala Asp Tyr Phe Asn Ala Ala Leu Ala Asp Gln Val Leu
   3275 3280 3285
   Ala Phe Val Asp Ala Arg Ile Ala Arg Asp Pro Ala Ala Arg Ile
   3290 3295 3300
   Arg Ile Val Glu Ile Gly Ala Gly Thr Gly Ser Ala Ser Ala His
   3305 3310 3315
   Val Phe Glu Arg Leu Trp Ser Arg Arg Glu His Val Glu Val Tyr
   3320 3325 3330
   Arg Tyr Thr Asp Leu Ser Lys Ala Phe Leu Phe His Ala Gln Glu
   3335 3340 3345
   His Tyr Ala Thr Arg Ala Pro Tyr Leu Lys Cys Gln Ile Phe Asp
   3350 3355 3360
   Val Glu Arg Asp Pro Ser Glu Gln Ser Ile Glu Ile Gly Thr Tyr
   3365 3370 3375
   Asp Val Ala Ile Ala Ala Asn Val Leu His Ala Thr Arg Asn Val
   3380 3385 3390
   Arg Arg Thr Leu Arg Asn Val Lys Ala Met Leu Lys Pro Asn Gly
   3395 3400 3405
   Val Leu Val Leu Asn Glu Ile Thr Ser His Ala Leu Leu Val His
   3410 3415 3420
   Leu Thr Phe Gly Leu Leu Glu Gly Trp Trp Arg Tyr Glu Asp Asp
   3425 3430 3435
   Ala Leu Arg Met Pro Gly Thr Pro Gly Leu Ser Ala Gln Thr Trp
   3440 3445 3450
   Arg Arg Val Leu Glu Asp Glu Gly Phe Arg Ser Val Leu Phe Pro
   3455 3460 3465
   Thr Glu Arg Ala Ala Glu Ile Gly Gln Gln Val Ile Ala Ala Glu
   3470 3475 3480
   Ser Asp Gly Ile Val Arg Arg Pro Arg Pro Pro Glu Pro Met Pro
   3485 3490 3495
   Ala Ala Gln Pro Leu Pro Ala Ala Gln Pro Pro Arg Ser Pro Ala
   3500 3505 3510
   Arg Ser Gly Ala Glu Ile Ala Glu Ser Arg Arg Ala Arg Pro Thr
   3515 3520 3525
   Glu Arg Ala Ala Thr Pro Ala Val Asp Ala Thr Pro Pro Ala Ala
   3530 3535 3540
   Gly Gly Arg Glu Gln Leu Leu Arg Glu Arg Thr Val Ala His Leu
   3545 3550 3555
   Leu Gly Leu Val Gly Lys Ala Leu Lys Ile Asp Val Ala Gln Leu
   3560 3565 3570
   Asp Pro Ala Glu Ser Leu Glu Ser Tyr Gly Ile Asp Ser Ile Leu
   3575 3580 3585
   Val Val Gln Leu Thr Asp Ala Leu Lys Glu Ala Ile Pro Asp Ile
   3590 3595 3600
   Ser Ser Thr Leu Phe Phe Glu His Gln Thr Ile Asn Ala Leu Val
   3605 3610 3615
   Asp His Leu Leu Arg Thr Lys Arg Asp Val Leu Arg Arg Leu Thr
   3620 3625 3630
   Gly Val Gly Asp Ala Ala Ala Ala Ala Pro Ser Thr Thr Ala Pro
   3635 3640 3645
   Ala Ser Ser Val Ala Ala Pro Pro Val Arg Ala Gly Arg Arg Ala
   3650 3655 3660
   Gly Ala Arg Val Arg Pro Ser Val Ser Arg Gly Gly Arg Val Glu
   3665 3670 3675
   Thr Ala Ala Ala Glu Arg Ala Ala Asp Asp Arg Arg Ile Ala Ile
   3680 3685 3690
   Ile Gly Leu Ser Gly Arg Tyr Pro Gln Ala Arg Thr Ile Asp Glu
   3695 3700 3705
   Tyr Trp Glu Asn Leu Lys Ala Gly Lys Asp Ser Val Val Glu Ile
   3710 3715 3720
   Pro Pro Glu Arg Trp Ala Ile Glu Gly Phe Tyr Cys Glu Asp Pro
   3725 3730 3735
   Arg Ala Ala Val Glu Ala Gly Lys Ser Tyr Cys Arg Trp Gly Gly
   3740 3745 3750
   Phe Leu Asp Gly Phe Ala Glu Phe Asp Pro Met Phe Phe Asn Ile
   3755 3760 3765
   Ser Pro Leu Glu Ala Glu Gly Ile Asp Pro Gln Glu Arg Leu Phe
   3770 3775 3780
   Met Gln Ala Ser Trp Asp Val Leu Glu Asp Ala Gly Tyr Thr Arg
   3785 3790 3795
   Glu Ser Leu Ala Arg Arg His Gly Gly Arg Val Gly Val Phe Ala
   3800 3805 3810
   Gly Ile Thr Lys Thr Gly Phe Asp Leu Tyr Gly Pro Glu Leu Trp
   3815 3820 3825
   Arg Arg Gly Ala Ala Ala Phe Pro His Thr Ser Phe Ser Ser Val
   3830 3835 3840
   Ala Asn Arg Val Ser Tyr Phe Leu Asn Ile Arg Gly Pro Ser Met
   3845 3850 3855
   Pro Ile Asp Thr Met Cys Ser Ser Ser Leu Thr Ala Ile His Glu
   3860 3865 3870
   Ala Cys Glu His Leu Leu Arg Gly Glu Cys Glu Leu Ala Ile Ala
   3875 3880 3885
   Gly Gly Val Asn Leu Tyr Leu His Pro Ser Ser Tyr Val Leu Leu
   3890 3895 3900
   Cys Met Ala Gly Met Leu Ser Lys Gln Gly Arg Ser Arg Ser Phe
   3905 3910 3915
   Gly Arg Gly Ala Asp Gly Met Val Pro Gly Glu Ala Val Gly Ala
   3920 3925 3930
   Val Leu Leu Lys Pro Leu Ser Arg Ala Glu Ala Asp Gly Asp Pro
   3935 3940 3945
   Ile Tyr Gly Val Ile Leu Gly Thr Ser Ile Asn His Gly Gly Lys
   3950 3955 3960
   Thr Asn Gly Tyr Leu Val Pro Asn Pro Ala Ala His Arg Glu Leu
   3965 3970 3975
   Ile Leu Ser Ala Leu Ala Arg Ala Gly Val Asp Ala Arg Ala Val
   3980 3985 3990
   Ser Tyr Val Glu Ala His Gly Thr Gly Thr Glu Leu Gly Asp Pro
   3995 4000 4005
   Ile Glu Ile Ala Gly Leu Thr Gln Ala Phe Arg Ala Ser Thr Pro
   4010 4015 4020
   Asp Val Gln Phe Cys Ala Ile Ala Ser Ala Lys Ser Asn Ile Gly
   4025 4030 4035
   His Cys Glu Ala Ala Ala Gly Ile Ala Gly Leu Thr Lys Val Leu
   4040 4045 4050
   Leu Gln Met Lys His Gly Gln Ile Ala Pro Ser Leu His Val Asp
   4055 4060 4065
   Glu Leu Asn Pro His Ile Ala Phe Glu Glu Thr Pro Phe Val Val
   4070 4075 4080
   Gln Arg Arg Leu Gly Glu Trp Lys Arg Pro Thr Leu Thr Val Gly
   4085 4090 4095
   Gly Arg Ser Val Glu Ala Pro Arg Ile Ala Gly Ile Ser Ser Phe
   4100 4105 4110
   Gly Ala Gly Gly Ala Asn Ala His Val Ile Val Ala Glu Tyr Ala
   4115 4120 4125
   Gln Ala Glu Arg Arg Asp Arg Asp Ala Asn Ala Pro Ala Ala Ala
   4130 4135 4140
   Pro Val Leu Ile Ala Leu Ser Ala Lys Ser Ser Asp Ala Val Val
   4145 4150 4155
   Glu Arg Ala Arg Asp Leu Val Ala Ala Ile Arg Ser Arg Gly Leu
   4160 4165 4170
   Thr Asp Ala Asp Leu Arg Asp Val Ala Tyr Thr Leu Gln Val Gly
   4175 4180 4185
   Arg Glu Ala Met Asp Glu Arg Leu Ala Val Ile Val Gly Ser Val
   4190 4195 4200
   Ala Asp Leu Glu Glu Lys Leu Arg Ala Phe Val Ala Gly Ser Ala
   4205 4210 4215
   Ala Pro Gly Glu Val Ile Arg Gly Arg Ala Arg Leu Glu Lys Asp
   4220 4225 4230
   Ala Leu Gly Val Val Ala His Asp Asp Asp Phe Glu Glu Thr Ile
   4235 4240 4245
   Gly Lys Trp Ile Gln Arg Gly Lys Leu Arg Lys Leu Ala Glu Leu
   4250 4255 4260
   Trp Val Arg Gly Leu Asp Leu Asp Trp Glu Arg Leu Tyr Gly Glu
   4265 4270 4275
   Arg Lys Pro Arg Arg Ile Ser Leu Pro Ala Tyr Pro Phe Ala Arg
   4280 4285 4290
   Glu Arg His Trp Leu Pro Leu Gly Glu Gly Ala Val Thr Ala Ala
   4295 4300 4305
   Ala Pro Ala Val Glu Ala Ser Pro Glu Ala Ala Ala Val Val Glu
   4310 4315 4320
   Tyr Glu Glu Val Trp Glu Arg Leu Gly Arg Glu Glu Arg Ala Ala
   4325 4330 4335
   Gly Glu Pro Gly Leu Arg Trp Leu Val Val Gly Gly Asp Glu Pro
   4340 4345 4350
   Trp Arg Ala Ser Val Val Glu Ala Leu Gly Arg His Glu Pro Gly
   4355 4360 4365
   Cys Glu Ala Ile Gly Val Ser Val Gly Gly Ala Phe Gly Trp Asp
   4370 4375 4380
   Gly Gly Ala Arg Ser Ala Val Pro Glu Ala Ser Glu Ala Trp Phe
   4385 4390 4395
   Leu Ser Leu Phe Glu Glu Met Glu Arg Arg Asp Ala Ala Arg Gly
   4400 4405 4410
   Ser Ala Gly Arg Ala Leu Gly Val Val Tyr Ala Trp Gly Arg Glu
   4415 4420 4425
   Leu Glu Arg Leu Gln Gly Val His Gly Leu Leu Arg Gly Ala Arg
   4430 4435 4440
   Gly Ser Gly Ala Pro Leu Arg Arg Leu Val Leu Thr Gly Arg Val
   4445 4450 4455
   Ala Asp Gly Glu Val Ser Gly Cys Tyr Asp Ala Ser Leu Ile Gly
   4460 4465 4470
   Val Glu Arg Ser Ala Ala Phe Ala Leu Pro Glu Leu Ser Val Gly
   4475 4480 4485
   Val Val Leu Gly Gly Ala Glu Gly Val Glu Ala Asp Ala Val Ala
   4490 4495 4500
   Arg Glu Ala Trp Met Ala Gly Val Ile Arg Cys Glu Ala Ala Glu
   4505 4510 4515
   Arg Tyr Arg Leu Gly Val Arg Arg Ala Ser Ser Ser Gly Ala Ala
   4520 4525 4530
   Gln Ala Gln Leu Arg Asp Gly Gly Val Tyr Val Ile Thr Gly Gly
   4535 4540 4545
   Ala Gly Gly Leu Gly Gln Val Phe Gly Glu His Leu Ala Arg Arg
   4550 4555 4560
   Cys Ala Gly Arg Ile Ala Leu Val Gly Arg Arg Arg Arg Asp Glu
   4565 4570 4575
   Arg Ile Glu Ala Gln Leu Gly Arg Leu Arg Glu Ala Gly Ala Thr
   4580 4585 4590
   Glu Ala Glu Tyr Phe Glu Ala Asp Val Ala Asp Ala Ala Gln Met
   4595 4600 4605
   Ser Gly Ala Val Ala Ala Ile Glu Ala Arg Trp Gly Ala Ile His
   4610 4615 4620
   Gly Val Leu His Ala Ala Gly Val Glu Ser Ser Gly Ala Val Val
   4625 4630 4635
   Asp Arg Ser Trp Glu Gln Val Ala Ala Val Ala Arg Pro Lys Val
   4640 4645 4650
   Glu Gly Thr Arg Val Ile Asp Glu Val Thr Ala Arg Gln Pro Leu
   4655 4660 4665
   Asp Phe Met Cys Leu Phe Ser Ser Thr Ala Ala Val Leu Gly Asp
   4670 4675 4680
   Gly Gly Ser Cys Asp Tyr Ala Thr Ala Asn Arg Phe Gln Val Ala
   4685 4690 4695
   Tyr Ala Gln His Arg Glu Gln Leu Arg Arg Arg Gly Glu Arg Ser
   4700 4705 4710
   Gly Ala Thr Val Ala Val Cys Trp Pro Leu Trp Arg Asp Gly Gly
   4715 4720 4725
   Met Gly Ala Val Gly Ser Glu Gly Ile Glu Met Tyr Leu Arg Thr
   4730 4735 4740
   Ser Gly Gln Arg Tyr Leu Glu Arg Ala Glu Gly Leu Ala Ala Trp
   4745 4750 4755
   Glu Arg Ala Met Gly Ala Gly Val Ser Pro Arg Leu Val Leu Ala
   4760 4765 4770
   Gly Gln Pro Arg Arg Val His Gly Phe Leu Ser Arg Ile Tyr Ala
   4775 4780 4785
   Gly Thr Ser Ala Ala Leu Arg Ser Pro Pro Ser Ala Glu Gly Gly
   4790 4795 4800
   Val Gly Ala Ser Ile Ala Arg Arg Glu Gln Arg Gly Ala Ser Ala
   4805 4810 4815
   Ala Ala Ala Pro Leu Ala Ala Ala Ala Pro Gly Ser Ala Ala Arg
   4820 4825 4830
   Ser Pro Ala Thr Leu Arg Glu Arg Val Ala Ala Glu Val Lys Ala
   4835 4840 4845
   Arg Val Glu Gly Ala Leu Lys Leu Pro Ala Gly Arg Leu Asp Glu
   4850 4855 4860
   Asp Ala Ser Phe Ala Asp Ile Gly Leu Asp Ser Ile Gly Leu Ala
   4865 4870 4875
   Gln Leu Ala Arg Leu Leu Thr Arg His Phe Gly Phe Glu Val Met
   4880 4885 4890
   Pro Asn Val Phe Phe Ser Tyr Ser Thr Ile Glu Lys Leu Ala Ala
   4895 4900 4905
   His Leu Val Glu Gln His Arg Asp Ala Ile Glu Ala Ala Tyr Gln
   4910 4915 4920
   Arg Ala Asp Ala Pro Leu Ala Pro Ala Glu Pro Arg Gln Ala Asp
   4925 4930 4935
   Asp Arg Arg Gly Ala Ala Ile Ala Arg Ala Pro Leu Val Asn Gly
   4940 4945 4950
   Ala His His Ala Ala Arg Pro Asp Val Pro Arg Glu Gly Pro Arg
   4955 4960 4965
   Arg Leu Gly Ala Arg Val Ser Pro Ser Val Gln Glu Pro Val Ala
   4970 4975 4980
   Ile Ile Gly Met Ser Val Arg Ala Ala Gly Ala Asp Asp Val Asp
   4985 4990 4995
   Asp Leu Trp Glu Leu Leu Leu Lys Gly His Lys Gln Ile Gln Glu
   5000 5005 5010
   Val Pro Gln Glu Arg Trp Asp Trp Arg Pro Tyr Tyr Ser Gly Pro
   5015 5020 5025
   Gly Ala Thr Asp Asn Arg Ile Ala Ser Asn Arg Gly Ala Phe Leu
   5030 5035 5040
   Arg Gly Leu Asp His Phe Asp Pro Leu Phe Phe Glu Ile Ser Pro
   5045 5050 5055
   Arg Glu Ala Glu Trp Met Glu Pro Arg Gln Arg Leu Met Leu Gln
   5060 5065 5070
   Glu Ala Trp Arg Ala Phe Glu Asp Ala Gly Tyr Ala Gly Glu Arg
   5075 5080 5085
   Leu Arg Gly Thr Asp Cys Gly Val Phe Ile Gly Val Glu Glu Ala
   5090 5095 5100
   Leu Pro Ala Pro Asp Ala Ala Pro Gly Pro Ala Thr Ser Ala His
   5105 5110 5115
   Asn Gly Ile Leu Ala Ala Arg Ile Ser Tyr Val Leu Asp Leu Arg
   5120 5125 5130
   Gly Pro Asn Leu Ala Leu Asn Thr Ala Cys Ser Ser Gly Leu Val
   5135 5140 5145
   Ala Val His Thr Ala Cys Gln Ser Leu Leu His Gly Glu Cys Ser
   5150 5155 5160
   Met Ala Leu Ala Gly Gly Val Asn Val Leu Thr Ser Pro Ala Thr
   5165 5170 5175
   Tyr Val Ala Leu Thr Arg Ser Gly Met Leu Ser Ala Glu Gly Glu
   5180 5185 5190
   Cys Tyr Ala Phe Asp Glu Arg Ala Ser Gly Met Val Pro Gly Glu
   5195 5200 5205
   Ala Val Ala Ala Val Val Leu Lys Arg Leu Ser Gln Ala Val Ala
   5210 5215 5220
   Asp Gly Asp Pro Ile His Ala Val Ile Arg Ala Thr Gly Val Asn
   5225 5230 5235
   Tyr Asp Gly Arg Thr Asn Gly Ile Thr Ala Pro Ser Gly Leu Ser
   5240 5245 5250
   Gln Gln Arg Leu Ile Glu Ser Val Leu Arg Arg Ala Glu Ile Gly
   5255 5260 5265
   Pro Glu Arg Ile Gln Tyr Val Leu Gly His Ser Val Gly Ser Ala
   5270 5275 5280
   Leu Gly Asp Pro Ile Glu Ala Gln Ala Leu Arg Asp Ala Leu Asp
   5285 5290 5295
   Gln Arg Thr Glu Gly Arg Arg Pro Phe Ala Leu Gly Ser Ile Lys
   5300 5305 5310
   Pro Val Ile Gly His Thr Phe Ala Ala Ser Gly Val Val Ser Leu
   5315 5320 5325
   Ile Ala Ile Cys Leu Ala Met Arg His Arg Gln Ile Pro Gly Thr
   5330 5335 5340
   Ser Asn Tyr Arg Arg Pro Ser Glu Tyr Ile Asp Leu Ala Thr Cys
   5345 5350 5355
   Pro Phe Ile Ile Ser Asp Val Ala Arg Pro Trp Thr Val Ser Ala
   5360 5365 5370
   Ala Gly Glu Arg Arg Cys Gly Met Val Gly Ala Thr Gly Met Ser
   5375 5380 5385
   Gly Thr Asn Val Phe Ala Val Val Glu Glu His Glu Pro Glu Arg
   5390 5395 5400
   Ala Ser Ala His Ala Pro Gly Ala Leu Gly Ala Ala Ala Ser Gly
   5405 5410 5415
   Arg Ala Val Ile Val Leu Ser Ala Arg Ser Asp Ala Ala Leu Arg
   5420 5425 5430
   Ser Tyr Ala Arg Thr Leu Arg Ala Arg Leu Ala Ser Thr Pro Leu
   5435 5440 5445
   Ser Asp Leu His Asp Val Ala Phe Thr Leu Gln Thr Gly Arg Glu
   5450 5455 5460
   Ala Met Pro Cys Arg Leu Ala Leu Val Ala Ser Ser Val Ser Ala
   5465 5470 5475
   Ala Ile Glu Lys Leu Asp Val Tyr Ala Ser Ala Ser Gly Asp Glu
   5480 5485 5490
   Gly Leu Gln Ser Arg Ser Ile Phe Thr Thr Gly Gln Glu Arg Thr
   5495 5500 5505
   Ile Ala Thr Ala Asp Pro Ser Arg Ala Ala Glu Leu Ala Glu Ala
   5510 5515 5520
   Trp Val Arg Gly Ala Ala Val Asp Trp Ser Gln Val His Glu Arg
   5525 5530 5535
   Ala Gln Ser Arg Arg Val Ser Leu Pro Ala Tyr Pro Phe Thr Ala
   5540 5545 5550
   Gly Gly Arg Ser Arg Glu Ala Ala Ser Gly Ala Leu Asp Pro Ile
   5555 5560 5565
   Leu Glu Leu Ile Ala Gly Tyr Ser Lys Arg Pro Ile Asp Ala Ile
   5570 5575 5580
   Asp Thr Ala Arg Pro Leu Ala Asp Leu Gly Ile Asp Ser Leu Leu
   5585 5590 5595
   Leu Met Asn Met Ile Gly Ala Leu Glu Asp Arg Tyr Ser Ile His
   5600 5605 5610
   Val Ser Leu Glu Asp Val Thr Ser Cys Ala Ser Ile Ala Ala Leu
   5615 5620 5625
   Cys Ala Met Leu Arg Gly Lys Ala Gly Ala Arg Ala Gly Ala Gly
   5630 5635 5640
   Thr Leu Pro Arg Arg Tyr Lys Glu Leu Glu Val Leu Gln Ser Lys
   5645 5650 5655
   Gly Asp Leu Pro Pro Val Phe Trp Phe His Gly Ser Leu Gly Thr
   5660 5665 5670
   Val Gln Thr Tyr Leu Gly Leu Ser Arg Gly Leu Gly Pro Thr Leu
   5675 5680 5685
   Pro Phe Tyr Ala Val Gln Ser Arg Gly Val Lys Ala Asn Glu Glu
   5690 5695 5700
   Pro Ile Asp Asp Leu Ser Glu Met Ala Lys Tyr Tyr Ala Glu Ile
   5705 5710 5715
   Leu Leu Ala Ala Arg Gly Gly Ala Gly Ala Ala Phe Gln Leu Gly
   5720 5725 5730
   Gly Tyr Ser Glu Gly Gly Leu Ile Ala Tyr Glu Val Thr Arg His
   5735 5740 5745
   Leu Gln Arg Met Gly His Asp Val Arg Ser Leu Val Met Ile Asp
   5750 5755 5760
   Thr Pro Tyr Leu His Gly Gly Val His Leu Leu Lys Asp Glu Asp
   5765 5770 5775
   Thr Arg Lys Leu Arg Tyr Ala Met Val Tyr Ala Asn Ile Leu Met
   5780 5785 5790
   Ile Asn Lys Leu Gly Gly Phe Thr Asp Ile Gly Ala Leu Pro Leu
   5795 5800 5805
   Gln Gly Ala Ser Ala Glu Asp Val Leu Pro Leu Leu Val Arg His
   5810 5815 5820
   Gly Leu Glu Arg Gly Leu Pro Tyr Ser Pro Asp Glu Leu Arg Glu
   5825 5830 5835
   Thr Ile Glu Arg Phe His Arg Ile Ala Glu Ala Asn Glu Arg Ala
   5840 5845 5850
   Ala Glu Ser Tyr Glu Ile Ala Asp Leu Pro Arg Pro Gly Ala Val
   5855 5860 5865
   Glu Cys His Tyr Phe Gln Arg Arg Thr Ala Gly Val Tyr Phe Asp
   5870 5875 5880
   Pro Ser Arg Phe Ala Gly Ala Met Val Glu Arg Thr Asn Asp Tyr
   5885 5890 5895
   Phe Arg Asp Lys Gly Cys Ala Glu Ala Trp Met Arg Arg Leu Pro
   5900 5905 5910
   Arg Phe Ser Leu His Leu Thr Ser Ala Thr Asp His Phe Ser Leu
   5915 5920 5925
   Leu Ser Glu Ala Asp Pro Phe Glu Arg Ile Arg Asp Thr Cys Arg
   5930 5935 5940
   Arg Leu Tyr Gly Val Ser Ala Glu Gly Thr Lys Pro Phe Ala Gly
   5945 5950 5955
   Ala Ala Ala Gly Arg Ala Ser Thr Ser Leu Ser Glu Glu Ile Ser
   5960 5965 5970
<210> 35
   <211> 458
   <212> PRT
   <213> Sorangium cellulosum
<400> 35
   Met Lys Asp Leu Thr Gln Gly Ser Ile Ala Arg His Ile Val Thr Met
   1 5 10 15
   Ala Val Pro Ile Ala Val Gly Met Ile Phe Gln Thr Leu Tyr Tyr Met
   20 25 30
   Ile Asp Leu Tyr Phe Val Gly Glu Leu Gly Asp Val Pro Leu Ala Gly
   35 40 45
   Val Ser Ala Ala Gly Asn Ala Thr Phe Leu Val Leu Ala Leu Thr Gln
   50 55 60
   Val Leu Gly Val Gly Thr Val Ala Met Ile Ser Gln Ala Val Gly Arg
   65 70 75 80
   Lys Asp Arg Asp Glu Ala Asn Val Val Phe Asn Gln Ser Leu Thr Leu
   85 90 95
   Ala Ala Val Ser Gly Leu Ala Val Leu Val Leu Gly Tyr Leu Thr Thr
   100 105 110
   Thr Pro Tyr Met Arg Ser Val Ala Ala Asp Ala Ala Thr Val Glu Ala
   115 120 125
   Gly Arg Thr Tyr Leu Phe Trp Phe Met Pro Gly Leu Ala Leu Gln Phe
   130 135 140
   Ala Leu Val Ala Met Gly Ser Ala Leu Arg Gly Thr Gly Leu Val Gln
   145 150 155 160
   Pro Thr Leu Val Val Gln Leu Leu Thr Ile Val Leu Asn Ala Ile Leu
   165 170 175
   Ala Pro Ile Leu Ile Ala Gly Trp Gly Thr His Arg Pro Met Gly Val
   180 185 190
   Ala Gly Ala Gly Leu Ala Ser Thr Leu Ser Leu Val Val Ala Val Ile
   195 200 205
   Ala Leu Trp Ala Tyr Phe Arg Arg Leu Glu Arg Tyr Val Gly Val Ser
   210 215 220
   Ala Pro Leu Leu Arg Pro Arg Leu Pro Ile Trp Arg Gln Leu Leu Ala
   225 230 235 240
   Ile Gly Leu Pro Ser Gly Gly Glu Phe Ala Leu Ile Phe Val Tyr Ser
   245 250 255
   Ala Ile Val Tyr Trp Thr Leu Arg Glu Phe Gly Ser Ala Ala Gln Ala
   260 265 270
   Gly Tyr Gly Ala Gly Ser Arg Leu Ile Gln Gly Leu Ile Leu Pro Ala
   275 280 285
   Met Ala Val Ser Phe Ala Ala Gly Pro Ile Ala Gly Gln Asn Phe Gly
   290 295 300
   Ala Lys Leu Ala Asp Arg Val Arg Ser Thr Phe Ser Ile Ser Ala Ala
   305 310 315 320
   Ile Ile Ser Ala Leu Met Val Leu Leu Thr Leu Phe Val Gly Trp Arg
   325 330 335
   Ala Glu Ala Leu Ile Gly Ser Phe Ala Lys Asn Ala Asp Ser Ile Ala
   340 345 350
   Val Gly Ala Gln Phe Leu Arg Phe Ala Cys Trp Asn Phe Val Ala Gln
   355 360 365
   Gly Leu Ile Phe Thr Cys Ser Ser Ile Phe Gln Gly Leu Gly Asn Thr
   370 375 380
   Thr Pro Ser Leu Leu Ser Ser Gly Ala Arg Leu Val Thr Phe Ala Val
   385 390 395 400
   Pro Ala Ile Trp Leu Ser Thr Leu Pro Gly Phe Arg Ile Glu Leu Val
   405 410 415
   Trp Tyr Leu Ser Ile Ala Ser Thr Thr Leu Gln Ala Ile Ala Ser Val
   420 425 430
   Leu Leu Leu Arg Ala Glu Phe Lys Gln Arg Phe Val Pro Gly Phe Val
   435 440 445
   Ala Ser Gly Ala Gly Leu Gly Ala Asn Ser
   450 455
<210> 36
   <211> 692
   <212> PRT
   <213> Sorangium cellulosum
<400> 36
   Met Phe Ser Gly Gln Gly Ser His Tyr Phe Arg Met Gly Glu Gly Leu
   1 5 10 15
   Tyr Ala Arg His Pro Thr Phe Arg Arg Trp Met Asp Arg Leu Asp Val
   20 25 30
   Leu Val Arg Asp Leu Ala Gly Ser Ser Val Val Glu Ala Leu Tyr Gln
   35 40 45
   Glu Gly Arg Gly Lys Gly Asp Val Phe Asp Arg Thr Leu Leu Thr His
   50 55 60
   Pro Ala Leu Leu Met Met Glu Tyr Ala Leu Ala Glu Ser Leu Ile Glu
   65 70 75 80
   Ala Gly Val Val Pro Asp Val Val Ile Gly Ala Ser Leu Gly Thr Val
   85 90 95
   Ala Ala Ala Cys Val Ala Gly Cys Leu Asp Val Glu Ala Ala Leu Glu
   100 105 110
   Met Thr Ile Arg His Ala Arg Ser Leu Glu Ala Cys Cys Glu Arg Gly
   115 120 125
   Ala Met Ile Ala Val Leu Gly Pro Thr Ser Leu Tyr Asp Asp Ala Gly
   130 135 140
   Leu Ser Arg His Gly Glu Leu Ala Ala Val Asn Phe Asp Ala His Phe
   145 150 155 160
   Val Val Ala Ala Thr Met Gln Gly Ala Ala Glu Ile Glu Lys Ile Leu
   165 170 175
   Arg Val Lys Asp Ile Val Phe Gln Arg Leu Ala Val Ser Phe Ala Phe
   180 185 190
   His Ser Arg Trp Leu Asp Ala Ala Glu Ala Leu Phe Lys Glu Arg Ser
   195 200 205
   Arg Ser Met Arg Ala Arg Pro Ala Arg Leu Pro Leu Val Cys Cys Ala
   210 215 220
   Arg Thr Arg Ala Ile Asp Thr Phe Thr Ala Asp His Ala Trp Ser Val
   225 230 235 240
   Ala Arg Glu Pro Ile Arg Phe Phe Glu Thr Met Lys Arg Leu Glu Glu
   245 250 255
   Ser Gly Pro Tyr Arg Tyr Val Asp Val Gly Pro Ser Gly Thr Leu Ala
   260 265 270
   Thr Phe Met Lys Tyr Ile Leu Ser Lys Gly Gly Ser Arg Ser Thr His
   275 280 285
   His Thr Val Leu Ser Pro Phe Gly Arg Asp Phe Glu Arg Phe Thr Ala
   290 295 300
   Ala Ala Thr Glu Leu Gly Ala Arg Pro Pro Arg Pro Gly Thr Asp Glu
   305 310 315 320
   Arg Ala Glu Ala Pro Ser Pro Ser Gly Ala Ala Ala Asp Ala Arg Ala
   325 330 335
   Ala Gly Pro Ser Pro Arg Gly Phe Glu Ala Ala Ser Arg Pro Ala Ala
   340 345 350
   Pro Ala Gly Ala Ser Ser Ser Asp Arg Ala Val Gly Ser Glu Pro Ser
   355 360 365
   Gly Ala Pro Ala Arg Ala Pro Ser Asp Gly Pro Arg Pro Leu Arg Ala
   370 375 380
   Glu Ala Ser Gly Ala Ala Ala Thr Asn Gly Val His Ala Gly Pro Arg
   385 390 395 400
   Ala Thr Val Ala Phe Met Phe Pro Gly Gln Gly Ser Gln Lys Arg Gly
   405 410 415
   Met Gly Ala Gly Leu Phe Asp Ser Val Pro Glu Tyr Ala Ala Val Glu
   420 425 430
   Arg Glu Val Asp Ala Leu Leu Gly Tyr Ser Met Arg Ala Leu Cys His
   435 440 445
   Glu Asp Pro Asp Gly Arg Leu Lys Glu Thr Gln Tyr Thr Gln Pro Ala
   450 455 460
   Leu Tyr Val Val Asn Ala Leu His Tyr Tyr Asp Ala Ile Ala Arg Gly
   465 470 475 480
   Gln Arg Pro Asp Tyr Val Ala Gly His Ser Leu Gly Glu Tyr Asn Ala
   485 490 495
   Leu Leu Ala Ala Gly Ala Phe Asp Leu Leu Thr Gly Leu Arg Leu Val
   500 505 510
   Lys Lys Arg Gly Glu Leu Met Ala Ala Ser Val Ser Gly Gly Met Ala
   515 520 525
   Ala Val Ile Gly Met Asp Glu Gly Arg Ile Lys Gln Val Leu Thr Glu
   530 535 540
   Asn Gly Leu Gly Thr Ile Asp Val Ala Asn Phe Asn Ser Pro Ser Gln
   545 550 555 560
   Ile Val Ile Ser Gly Pro Val Ala Asp Ile Ala Arg Gly His Thr Val
   565 570 575
   Phe Glu Asp Ala Gly Ala Arg Thr Tyr Val Ile Leu Pro Val Ser Ala
   580 585 590
   Ala Phe His Ser Arg Tyr Val Glu Glu Thr Gly Arg Ala Phe Ala Asp
   595 600 605
   Phe Ile Ala Pro Met Arg Phe Glu Ala Leu Arg Ile Pro Val Ile Ser
   610 615 620
   Asn Val Ser Ala Arg Pro Tyr Glu Ala Lys Asp Pro Ser Ala Thr Ile
   625 630 635 640
   Lys Ser Leu Leu Val Gln Gln Ile Thr Arg Pro Val Gln Trp Val Gln
   645 650 655
   Ser Val Ser Phe Leu Met Ala Gln Gly Val Lys Glu Phe Arg Glu Ile
   660 665 670
   Gly Pro Gly Asn Val Leu Thr Arg Leu Val Gln Gln Ile Glu Arg Gln
   675 680 685
   Pro Ser Gly Ala
   690
<210> 37
   <211> 347
   <212> PRT
   <213> Sorangium cellulosum
<400> 37
   Val Ser Gly Arg Arg Arg Ser Gly Asp Ala Leu Pro Val Pro Pro Gly
   1 5 10 15
   Arg Arg Glu Asp Arg Arg Gln Arg His Leu Pro Leu Gln Ala Gly Val
   20 25 30
   Arg Arg Gly Trp Ile Arg Arg Ser Arg Gly Leu His His Gly Ser Ser
   35 40 45
   Leu Leu Arg Glu Pro Leu Arg Gln Ala Arg Gln Gly Phe Ser His Trp
   50 55 60
   Asp Arg Trp Leu Leu Arg Leu Ala Leu Ala Glu Pro Arg Gly Leu Asp
   65 70 75 80
   Ala Ile Ala Cys Glu Leu Arg Ala Arg Ala Ala Ser Arg Arg Val Ser
   85 90 95
   Gly Glu Ala Ala Glu Ala Leu Leu Ala Gln Val Val Asp Leu Arg Gly
   100 105 110
   Arg Leu Ala Arg Leu Ala Arg Thr Pro Glu Glu Val Leu Asp Ala Ala
   115 120 125
   Glu Arg Ala Ser Gly Trp Leu Leu Lys Lys Ala Trp Lys Arg Val Trp
   130 135 140
   His Asp Gly Pro Asn Arg Arg Thr Asp Ala Met Arg Asn Thr Pro Arg
   145 150 155 160
   Arg Arg Leu Glu Ala His Ala Leu Arg Gly Asn Trp Pro Arg Phe Pro
   165 170 175
   Val Ser Pro Ala Arg Phe Glu Pro Glu Leu Arg Arg Val Val Gly Asp
   180 185 190
   His Ala Tyr Tyr Asp Gln Arg Ala Thr Asp Leu Val Ala His Leu Leu
   195 200 205
   Glu Ser His Val Asp Leu Leu Ala Ala Met Ala Ala Ser Asp Leu Glu
   210 215 220
   Arg Leu Ala Leu Tyr Arg Ala Ala Met Thr Val Ile Ile Glu Met Met
   225 230 235 240
   Asp Arg Val Asp Asp Ser Leu Ala Asp Met Ser Glu Val Phe Ala Ala
   245 250 255
   Ser Glu Arg Thr Tyr Leu Ala Leu Ala Arg Asp His Ala Gly Leu Asp
   260 265 270
   Gly Ile Val Arg Asp Leu Leu Glu Leu Ala Val Trp Glu Asp Tyr Gly
   275 280 285
   Leu Leu Arg Ser Val Gly Asp Phe Leu Gly Ala Leu Gln Glu Glu His
   290 295 300
   Ala Asn Leu Ala Val Arg Glu Leu Ser Gly Ile Ile Ala Asp Leu Arg
   305 310 315 320
   Arg Glu Arg Leu Asp Asp Gln Leu Ala Arg Ala Leu Arg Gln Lys Ser
   325 330 335
   Glu His His Arg His Val Gly Asp His Arg Arg
   340 345
<210> 38
   <211> 552
   <212> PRT
   <213> Sorangium cellulosum
<400> 38
   Met Arg Asn Leu Arg Arg Asn Arg Gly Arg Gly Gly Gln Arg Arg Cys
   1 5 10 15
   Pro Pro Gly Arg Leu Ala Asp Gly Ala Leu Asp Gly Cys Pro Asn His
   20 25 30
   His Gly Gly Lys Lys Ser Gly Met Ala Ala Lys Gly Asp Pro Val Asp
   35 40 45
   Val Val Thr Gly Arg Val Phe Thr Ile Pro Ala Val Asp Leu Glu Leu
   50 55 60
   Pro Gly Pro Leu Pro Leu Ser Ile Val Arg Ser Tyr Thr Thr Ala Ala
   65 70 75 80
   Phe Glu Arg Asp Val Gly Leu Gly Phe Gly Trp Ser His Ser Leu Ala
   85 90 95
   Trp Glu Ile Glu Val Arg Gln Arg Ile Leu Arg Val Trp Thr Asp Asp
   100 105 110
   Gly Pro Val Asp Leu Asp Thr Met Pro Val Gly Ala Gly Val Ala Gly
   115 120 125
   Pro His Gly Trp Val Leu Ala Arg Glu Glu Arg Gly Phe Val Leu Asp
   130 135 140
   Ser Gly Asp Gly His Trp Tyr Val Phe Ala Glu Ala Ala Gly Lys Arg
   145 150 155 160
   Phe Arg Leu Thr Ala Ile Lys Asp Arg Tyr Asn Asn Glu Ile Arg Leu
   165 170 175
   Ser Tyr Arg Glu Gly Val Leu Thr Glu Ile Thr Asp Ser Val Gly Arg
   180 185 190
   Ile Val Arg Val Arg Arg Ala Pro Asp Gly Arg Ile Gly Ala Phe Glu
   195 200 205
   Ile Lys Asn Ala Arg Glu Arg Gly Val Trp Val Pro Phe Ala Gln Tyr
   210 215 220
   Ser Tyr Asp Ala Ala Gly Asp Leu Thr Ser Ala Thr Asp Ala Asp Gly
   225 230 235 240
   Tyr Thr Thr Arg Tyr Thr Tyr Gln Glu His Leu Leu Thr Ser His Thr
   245 250 255
   Ser Pro Thr Gly Leu Thr Phe Ser Phe Arg Tyr Asp Asp Arg Arg Arg
   260 265 270
   Cys Val Glu Thr Trp Gly Ala Tyr Gly Asp Gly Arg Asp Arg Ser Leu
   275 280 285
   Ala Glu Asp Val Pro Thr His Leu Ala Asp Gly Arg Thr Val Ala Arg
   290 295 300
   Gly Ile Tyr His Cys Lys Leu Glu Phe Gly Glu Gly Gly Tyr Val Glu
   305 310 315 320
   Val Ala Asp Ser Ile Thr Val His Arg Tyr Phe Gly Asn Arg Phe Gly
   325 330 335
   Lys Leu Asp Lys Ala Val Ser Ala Gly Ala Val Phe Ser Arg Thr Tyr
   340 345 350
   Asp Glu His Gly Asp Ala Leu Gly Gly Lys Thr Gln Leu Ala Tyr Asp
   355 360 365
   Tyr Trp Gly Arg Cys Arg Ser Ile Arg Asp Thr Ser Gly Ala Val Leu
   370 375 380
   Ser Tyr Thr Tyr Asp Asn Arg Gly Asn Leu Thr Gly Val Leu Arg Pro
   385 390 395 400
   Asp Gly Gly Thr Thr Arg Tyr Glu Tyr Asp Gly Ile Gly Asp Cys Thr
   405 410 415
   Ala Val Leu Gln Pro Asn Gln Gln Val Thr Arg Phe Val Arg Gly Gly
   420 425 430
   Phe His Lys Leu Cys Glu Val His Lys Pro Asn Gly Glu Ser Thr Arg
   435 440 445
   Phe Arg Tyr Asn Arg Glu Gly Arg Leu Val Glu Ile Lys Asn Ala Leu
   450 455 460
   Gly Glu Arg His Arg Ile Glu Leu Asp Ala Ala Gly Met Val Thr Ser
   465 470 475 480
   Glu Arg Gly Phe Asp Gly Arg Val Ser Arg Thr Lys Tyr Asp Tyr Asn
   485 490 495
   Gly Arg Leu Ile Ser Phe Gln Asn Ala Thr Gly Glu Arg Thr Glu Tyr
   500 505 510
   Thr Tyr Asp Leu Ala Gly Gln Leu Val Ala Arg Thr Phe Asp Asp Gly
   515 520 525
   Ser Val Glu Arg Phe Thr Tyr Asp Asp Arg Gly Asp Val Ile Ala Ala
   530 535 540
   Glu Ser Pro Ala Gly Ser Phe Thr
   545 550
<210> 39
   <211> 178
   <212> PRT
   <213> Sorangium cellulosum
<400> 39
   Met Ala Lys Ala Ser Ser Glu Ile Glu Thr Ala Gly Gly Lys Arg His
   1 5 10 15
   Arg Gly Ser Ser Ala Phe Gly Gly Pro Leu Asp Pro Ala Leu Gln Thr
   20 25 30
   Ala Val Asp Asn Ala His Lys Lys Phe Asp Glu Gly Gln Val Ala Gly
   35 40 45
   Pro Lys Pro Ser Ser Ala Gly Gln Cys Ala Glu Ile Glu Ala Leu Asn
   50 55 60
   Asn Leu Ala Lys Asp Ile Arg Gln Asp Arg Gln Lys Asn Gly Lys Thr
   65 70 75 80
   Leu Lys Asp Pro Ala Ala Glu Asp Thr Glu Ile Arg Lys Glu Met Gln
   85 90 95
   Lys Gln Val Lys Glu Gly Lys Met Thr Thr Arg Thr Asp Lys Asp Gly
   100 105 110
   Leu Leu Met Asp Pro Cys Lys Phe Cys Ala Gln Val Met Arg Glu Leu
   115 120 125
   Gly Leu His Pro Ala Asn Asn Gly Gly Thr Gly Lys Lys Asn Gly Val
   130 135 140
   Ile Gly Ser Asp Cys Lys Ala Trp Asn Gly Asn Val Trp His Lys Gly
   145 150 155 160
   Arg Ser Met Pro Phe Arg Thr Ala Pro Ser Ser Thr Pro Pro Phe Ala
   165 170 175
   Gly Thr
<210> 40
   <211> 459
   <212> PRT
   <213> Sorangium cellulosum
<400> 40
   Met Ile Thr Ala Lys Ser Leu Gly Asp Arg Ser Phe Lys Ala Asp Tyr
   1 5 10 15
   Gly Val Asp Tyr Ala Tyr Val Ala Gly Ala Met Tyr Lys Gly Ile Ala
   20 25 30
   Ser Lys Glu Leu Val Val Ala Met Gly Lys Ala Gly Phe Ile Ala Tyr
   35 40 45
   Leu Gly Thr Gly Gly Leu Asp Glu Arg Glu Ile Glu Ala Ser Ile Arg
   50 55 60
   Ser Ile Gln Ser Val Ile Ala Gly Arg Ala Tyr Gly Met Asn Leu Leu
   65 70 75 80
   Ser Asn Leu Glu Ser Pro Glu Leu Glu Glu Arg Thr Ile Asp Leu Tyr
   85 90 95
   Leu Arg His Gly Val Arg Cys Val Glu Ala Ala Ala Tyr Met Arg Val
   100 105 110
   Thr Pro Ala Leu Val Arg Tyr Arg Leu Gln Gly Leu Ala Ser Gly Ala
   115 120 125
   Gly Arg Thr Leu Val Ala Pro Arg Arg Val Leu Ala Lys Val Ser Arg
   130 135 140
   Pro Glu Val Ala Ala Ala Phe Met Gln Pro Ala Pro Glu Ala Val Val
   145 150 155 160
   Arg Gln Leu Val Glu Ser Gly Lys Leu Thr Glu Gln Asp Ala Ala Leu
   165 170 175
   Ala Pro Leu Val Pro Met Ala Asp Asp Val Cys Val Glu Ala Asp Ser
   180 185 190
   Gly Gly His Thr Asp Gln Gly Val Ala Phe Ala Leu Leu Pro Ala Met
   195 200 205
   Leu Val Leu Arg Asp Glu Met Met Thr Arg Tyr Arg Tyr Glu Lys Arg
   210 215 220
   Ile Arg Val Gly Ala Ala Gly Gly Ile Gly Thr Pro His Ala Ala Ala
   225 230 235 240
   Ala Ala Phe Val Met Gly Ala Asp Phe Ile Leu Thr Gly Ser Ile Asn
   245 250 255
   Gln Cys Thr Arg Glu Ala Gly Thr Ser Glu Pro Val Lys Ala Leu Leu
   260 265 270
   Gln His Leu Asn Val Gln Asp Thr Thr Tyr Ala Pro Ala Gly Asp Met
   275 280 285
   Phe Glu Leu Gly Ser Lys Ile Gln Val Val Arg Arg Gly Leu Phe Phe
   290 295 300
   Pro Ala Arg Ala Asn Lys Leu His Glu Leu Tyr Met Arg His Gly Ser
   305 310 315 320
   Leu Glu Glu Ile Asp Ala Lys Thr Arg Gln Gln Ile Gln Glu Lys Tyr
   325 330 335
   Phe Arg Arg Ser Phe Asp Ala Val Trp Ser Glu Thr Arg Ser Tyr Tyr
   340 345 350
   Ala Arg Thr Tyr Pro His Arg Leu Ala Glu Ile Glu Arg Ser Pro Lys
   355 360 365
   Gln Lys Met Ala Ala Val Phe Arg Trp Tyr Phe Ala His Thr Thr Arg
   370 375 380
   Leu Ala Leu Glu Gly Val Glu Asp Gln Arg Leu Asp Tyr Gln Ile His
   385 390 395 400
   Cys Gly Pro Ala Leu Gly Ala Phe Asn Gln Trp Thr Lys Gly Thr Ala
   405 410 415
   Ile Glu Pro Trp Gln Asn Arg Tyr Val Ala Asp Ile Ala Arg Arg Ile
   420 425 430
   Met Glu Gly Thr Ala Asp Leu Leu Asn Ala Arg Phe Gly Ala Met Lys
   435 440 445
   Glu Ala Ala Glu Pro Pro Gln Ser Val Ser Ser
   450 455
<210> 41
   <211> 82
   <212> PRT
   <213> Sorangium cellulosum
<400> 41
   Met Asn Arg Gln Glu Ile Phe Asp Leu Ile Ala Arg His Ala Arg Glu
   1 5 10 15
   Val Leu Pro Glu Leu Glu Gln His Arg Phe Gln Asp Ser Asp Arg Leu
   20 25 30
   Gln Asp Leu Gly Ala Asn Ser Val Asp Arg Ala Glu Ile Val Met Leu
   35 40 45
   Thr Leu Glu Ser Leu Ser Leu Lys Ile Pro Arg Val Glu Thr Phe Gly
   50 55 60
   Pro Ser Asn Ile Gly Glu Leu Ala Asp Leu Leu His Ala Lys Leu Arg
   65 70 75 80
   Gly Ala
<210> 42
   <211> 410
   <212> PRT
   <213> Sorangium cellulosum
<400> 42
   Met Arg Ser Ser Gly Glu Pro Glu Ala Ala Ile Thr Gly Val Gly Val
   1 5 10 15
   Thr Ser Ala Ile Gly Gln Thr Arg Asp Ala Phe Ala Arg Ala Leu Leu
   20 25 30
   Arg Gly Glu Ser Ala Phe Gly Val Met Gln Arg Pro Gly Arg Gln Lys
   35 40 45
   Gly Thr Ala Phe Leu Gly Ala Glu Ile Ala Ser Leu Gln Val Pro Glu
   50 55 60
   Gly Met Ala Pro Gln Thr Leu Arg Lys Ala Ser Phe Ser Ala Arg Val
   65 70 75 80
   Ala Leu Ala Thr Leu His Glu Ala Trp Arg Asp Ala Arg Leu Asp Asp
   85 90 95
   Val Asp Pro Arg Arg Ile Gly Leu Ile Val Gly Gly Ser Asn Val Gln
   100 105 110
   Gln Arg Asp Leu Val Leu Ala His Glu Glu Tyr Ala Gly Arg Ala Asp
   115 120 125
   Phe Leu Arg Pro Thr Tyr Gly Val Ser Phe Met Asp Thr Asp Leu Cys
   130 135 140
   Gly Phe Cys Thr Glu Gln Phe Gly Ile Arg Gly Leu Ala Tyr Thr Ala
   145 150 155 160
   Gly Gly Ala Ser Ala Ser Gly Gln Leu Ala Ile Ile Gln Ala Ile Gln
   165 170 175
   Ala Val Gln Ala Gly His Val Asp Val Cys Ile Ala Leu Gly Ala Leu
   180 185 190
   Met Asp Ile Ser Phe Trp Glu Cys Gln Gly Leu Ala Ser Leu Gly Ala
   195 200 205
   Met Gly Ser Glu Arg His Ala Ala Ala Pro Glu Arg Ala Cys Arg Pro
   210 215 220
   Phe Asp Arg Ala Arg Asp Gly Phe Ile Tyr Gly Glu Cys Cys Gly Ala
   225 230 235 240
   Val Val Val Glu Ala Leu Pro Arg Ala Ala Arg Arg Gly Val Arg Pro
   245 250 255
   Tyr Ala Trp Leu Ser Gly Trp Gly Ile Ala Met Asp Ala Asn Arg Asn
   260 265 270
   Pro Asp Pro Ser Leu Asp Gly Glu Val Glu Ala Ile Arg Arg Ala Leu
   275 280 285
   Arg Cys Ala Gly Cys Ala Pro Ala Asp Ile Asp Tyr Val Asn Pro His
   290 295 300
   Gly Thr Gly Ser Val Val Gly Asp Glu Thr Glu Leu Glu Ala Leu Arg
   305 310 315 320
   Ala Cys Gly Leu Ser His Ala Arg Leu Asn Ala Thr Lys Ser Leu Val
   325 330 335
   Gly His Gly Leu Ser Ala Ala Gly Ala Val Glu Val Ile Ala Thr Leu
   340 345 350
   Leu Gln Met Gln Glu Gly Arg Leu His Pro Ser Arg Asn Leu Asp Asp
   355 360 365
   Pro Ile Asp Pro Ser Phe Asn Trp Val Arg Gly Glu Ala Val Ala His
   370 375 380
   Thr Met Lys Thr Ala Leu Thr Leu Ser Met Gly Phe Gly Gly Ile Asn
   385 390 395 400
   Thr Ala Leu Cys Leu Arg Lys Tyr Ser Gly
   405 410
<210> 43
   <211> 419
   <212> PRT
   <213> Sorangium cellulosum
<400> 43
   Met Ala Val Gly Ile Glu Ala Ile Asn Ala Phe Cys Gly Thr Ala Cys
   1 5 10 15
   Leu Asp Val Ala Gln Leu Ala Arg His Arg Lys Leu Asp Ala Gly Arg
   20 25 30
   Phe Gln Gly Leu Leu Met Met Glu Lys Thr Val Ala Leu Pro Tyr Glu
   35 40 45
   Asp Pro Val Thr Phe Gly Ala Asn Ala Ala Arg Pro Ile Val Asp Ala
   50 55 60
   Leu Ser Thr Ala Glu Lys Asp Arg Ile Glu Met Leu Ile Thr Cys Thr
   65 70 75 80
   Glu Ser Gly Ile Asp Phe Gly Lys Ser Ile Ser Thr Tyr Leu His His
   85 90 95
   His Leu Arg Leu Ser Arg Asn Cys Arg Leu Phe Glu Ile Lys Gln Ala
   100 105 110
   Cys Tyr Ser Gly Thr Ala Gly Leu Gln Ala Ala Val Ser Phe Val Leu
   115 120 125
   Ser Gln Val Ser Pro Gly Ala Lys Ala Leu Val Val Ala Thr Asp Ile
   130 135 140
   Ser Arg Tyr Met Val Ala Glu Gly Ala Asp Ala Pro Ala Pro Glu Ala
   145 150 155 160
   Ala Phe Ala Glu Pro Ser Ala Gly Ala Gly Ala Val Ala Leu Leu Val
   165 170 175
   Ser Asp Arg Pro Arg Val Phe Gln Leu Asp Val Gly Ala Ser Gly Tyr
   180 185 190
   Tyr Gly Tyr Glu Val Met Asp Thr Cys Arg Pro Ala Pro Asp Ile Glu
   195 200 205
   Ala Gly Asp Ala Asp Leu Ser Leu Leu Ser Tyr Leu Asp Cys Cys Glu
   210 215 220
   Asn Ala Phe Leu Glu Tyr Lys Lys Arg Val Arg Asp Val Asp Tyr Val
   225 230 235 240
   Arg Ser Phe Gly Tyr Leu Ala Phe His Thr Pro Phe Gly Gly Met Val
   245 250 255
   Lys Gly Ala His Arg Thr Met Met Arg Lys Met Ala Arg Ala Lys Pro
   260 265 270
   Ala Glu Ile Glu Glu Asp Phe Ala Arg Arg Val Ser Pro Gly Leu Val
   275 280 285
   Tyr Cys Gln Arg Val Gly Asn Ile Met Gly Ala Thr Ser Leu Leu Ser
   290 295 300
   Leu Val Ser Thr Ile Ala His Gly Asp Phe Gln Ala Pro Glu Arg Ile
   305 310 315 320
   Gly Tyr Phe Ser Tyr Gly Ser Gly Cys Cys Ser Glu Phe Leu Ser Gly
   325 330 335
   Val Val Arg Arg Glu Gly Gln Glu Ala Val Arg Glu Leu Arg Ile Gly
   340 345 350
   Arg Gln Leu Asp Asp Arg His Pro Leu Ser Ile Ala Glu Tyr Asp Tyr
   355 360 365
   Leu Ile Arg Gly Asn Ser Gln Leu Arg Phe Gly Thr Arg Asp Val Glu
   370 375 380
   Val Arg Glu Ala Gly Leu Pro Ala Ala Lys Pro Val Arg Glu Gly Glu
   385 390 395 400
   Arg Arg Leu Lys Leu Arg Ala Val Ala Asn Phe His Arg Glu Tyr Glu
   405 410 415
   Trp Thr Pro
<210> 44
   <211> 255
   <212> PRT
   <213> Sorangium cellulosum
<400> 44
   Val Thr Ala Val Pro Pro Tyr Gln Thr Leu Arg Val Arg Asn Gln Asp
   1 5 10 15
   Gly Ile Cys Phe Met Gln Ile His Arg Pro Glu Ala Gln Asn Thr Ile
   20 25 30
   Asp Asp Arg Leu Ile Asp Glu Cys Ser Asp Val Leu Asp Arg Cys Glu
   35 40 45
   Asp Ala Ala Thr Ile Val Val Ile Glu Gly Leu Pro Glu Thr Phe Cys
   50 55 60
   Phe Gly Ala Asp Phe Ser Ala Ile Arg Ala Ala Gln Gly Val Met Arg
   65 70 75 80
   Gly Pro Glu Pro Leu Tyr Asp Leu Trp Met Arg Leu Thr Thr Gly Pro
   85 90 95
   Tyr Val Val Ile Ser His Val Arg Gly Lys Ala Asn Ala Gly Gly Ile
   100 105 110
   Gly Phe Val Ala Ala Ser Asp Ile Val Val Ala Asp Ala Thr Ala Thr
   115 120 125
   Phe Ser Leu Ser Glu Leu Leu Phe Gly Leu Ile Pro Ala Cys Val Met
   130 135 140
   Pro Phe Leu Val Arg Arg Val Gly Phe Gln Lys Ala His Tyr Met Thr
   145 150 155 160
   Leu Met Thr His Pro Leu Asp Val Ser Gln Ala Ala Ala Tyr Gly Leu
   165 170 175
   Val Asp Ala Tyr Asp Ala Asn Ser Asp Ala Leu Leu Arg Arg His Leu
   180 185 190
   Ser Arg Leu Arg Arg Leu Ser Lys Thr Ala Val Ala Arg Tyr Lys Arg
   195 200 205
   Phe Ser Ser Ala Leu Gly Gly Ala Val Ala Ala Ser Arg Pro Leu Ala
   210 215 220
   Leu Ala Thr Asn Lys Glu Val Phe Ser Asp Pro Arg Asn Ile Glu Gly
   225 230 235 240
   Ile Met Arg Tyr Val Asp Glu Gly Ile Phe Pro Trp Glu Arg Pro
   245 250 255
<210> 45
   <211> 249
   <212> PRT
   <213> Sorangium cellulosum
<400> 45
   Met Ala Thr Pro Val Val His Leu Thr Thr Val Glu Pro Ser Ile Val
   1 5 10 15
   Gln Ile Lys Met Glu Asp Arg Thr Tyr Lys Asn Thr Phe Ser Glu Gly
   20 25 30
   Ile Cys Ala Gly Leu Val Asp Ala Phe Arg Arg Val Ala Ser Asp Pro
   35 40 45
   Asp Cys Lys Val Val Val Val Thr Gly Tyr Asp Thr Tyr Phe Cys Ser
   50 55 60
   Gly Gly Thr Gln Glu Gly Leu Leu Ser Leu Ser Gly Gly Gln Gly Lys
   65 70 75 80
   Phe Thr Asp Thr Pro Ile Tyr His Leu Pro Leu Ala Cys Glu Val Pro
   85 90 95
   Val Ile Ser Ala Met Gln Gly His Gly Ile Gly Gly Gly Phe Ala Leu
   100 105 110
   Gly Leu Phe Ala Asp Phe Val Val Leu Ser Arg Glu Ser Val Tyr Thr
   115 120 125
   Ala Asn Phe Met Lys Tyr Gly Phe Thr Pro Gly Phe Gly Ser Thr Leu
   130 135 140
   Ile Leu Arg Glu Lys Leu Gly Leu Pro Leu Ala Gln Glu Met Leu Met
   145 150 155 160
   Thr Ala Gly Ser Tyr Arg Gly Ala Glu Leu Ala Gln Arg Gly Val Ser
   165 170 175
   Phe Pro Val Val Pro Arg Ala Glu Val Leu Pro His Ala Tyr Gln Leu
   180 185 190
   Ala Arg Gln Leu Ala Glu Lys Pro Arg Arg Ser Leu Val Ile Leu Lys
   195 200 205
   Ser His Leu Val Ala Asp Leu Arg Ala Arg Leu Pro Val Val Ala Asp
   210 215 220
   Met Glu Val Ala Met His Glu Gln Thr Phe His His Pro Glu Val Arg
   225 230 235 240
   Glu Arg Ile Lys Ser Arg Phe Gly Ala
   245
<210> 46
   <211> 1111
   <212> PRT
   <213> Sorangium cellulosum
<400> 46
   Met His Val Arg Ile Val Asp Arg Pro Gly Ala Gly Glu Arg Glu Asp
   1 5 10 15
   Ala Ile Asp Ile Ser Ile Glu Arg Pro Ala Gly Lys Glu Thr Phe Ala
   20 25 30
   Ala Ser Ala Asn Ala Ala Leu Ala Gly Ala Glu Leu Asp Ala Arg Leu
   35 40 45
   Ala Trp Tyr Phe Ala Glu Tyr Pro Gln Thr Thr Ala Leu Pro Pro Asp
   50 55 60
   Asp Arg Gly Val Ala Ser Ala Ile Ala Asp Val Gly Arg Ala Met Gly
   65 70 75 80
   Glu Arg Leu Ala Asn Gly Glu Glu Arg Pro Ala Ala Leu Ala Thr Ala
   85 90 95
   Ile Gly Glu Arg Glu Pro Arg Arg Val Gly Val Leu Ile Glu Ser Ser
   100 105 110
   Arg Ala Ser Phe Leu Ala Ala Pro Trp Glu Leu Met Val Leu Pro Gly
   115 120 125
   Ala Asp Gln Pro Trp Ser Thr Leu Ala Ala His Phe Val Arg Arg Phe
   130 135 140
   His Arg Gly Gly Arg Ser Glu Asp Asp Ser Gln Ile Ala Tyr Glu Leu
   145 150 155 160
   Gly Val Thr Pro Pro Glu Pro Glu Ala Val Ala Ala Leu Lys Ala Gln
   165 170 175
   Met Arg Gly Asp Ala Pro Ala Pro Gln Pro Pro Pro Asp Ala Pro Leu
   180 185 190
   Arg Ile Leu His Val Val Ser Arg Ala Leu Arg Asp Leu Leu Pro Phe
   195 200 205
   Ala Ser Ser His Ala Leu Glu Trp Ala Ala Glu Ser Leu Gly Ala Glu
   210 215 220
   Gly Ala Val Asp Tyr Glu Ile Phe Pro Ala Gly Trp Ala Glu Leu Gln
   225 230 235 240
   Ala Arg Leu Val Asp Arg Arg Arg Pro Val His Val Leu His Tyr Asp
   245 250 255
   Gly Pro Ile Val Leu Asp Gly Gly Ile Ala Ser Val Leu Leu Gly Gly
   260 265 270
   Pro Asp Gly Pro Phe Glu Arg Val Ala Val Ala Glu Leu Ala Trp Ala
   275 280 285
   Leu Val Arg Ser Lys Val Ala Ala Leu Ala Val Asp Thr Arg Gly Ala
   290 295 300
   Phe Asp Gly Ala His Pro Val Ala Val Pro Ala Ala Val Ser Ala Val
   305 310 315 320
   Ala Ser Ala Ala Arg Asp Ala Ser Leu Gly Asn Val Ile Ala Ala Ala
   325 330 335
   Tyr Ala Ala Asp Pro Trp Thr Ser Gly Arg Cys Phe Gln Glu Val Tyr
   340 345 350
   Thr Arg Leu Ala Ala Gly Leu Thr Leu Gly Glu Ala Val Val Asp Ala
   355 360 365
   Arg Arg Ala Leu Cys Glu Arg Ala Glu Ala Ser Arg Phe Thr Ser Glu
   370 375 380
   Pro Arg Pro Phe His Ala Trp Pro Leu Leu Val His Tyr Gly Gly Gln
   385 390 395 400
   Ser Val Ser Phe Phe Ala Gln Ala Gln Ala Arg Ala Ser Leu Glu Glu
   405 410 415
   Ser Gln Ala Leu Ala Arg Ala Arg Arg Arg Leu Leu Gly Phe His Ala
   420 425 430
   Thr Leu Leu Pro Pro Leu Leu Arg His Ala Gly Asp Gly Val Ala Val
   435 440 445
   Ala Ala Leu Gly Ala Ile Ala Arg Gly Arg Arg Ala Ile Ala Leu Thr
   450 455 460
   Gly Glu Pro Gly Ala Gly Lys Ser His Ala Ala His Arg Val Ala Leu
   465 470 475 480
   Tyr Leu Ala Gln Arg Asp Ala Val Asp Phe Ala Phe Tyr Phe Asp Phe
   485 490 495
   Gly Ala Glu Pro Tyr Ser Val Asp Asp Ile Leu Glu Met Ile Ala Pro
   500 505 510
   Val Leu Gly Leu Ala Pro Asp Arg Lys Ala Glu Thr Arg Val Ala Leu
   515 520 525
   Ala Lys Leu Arg Cys Cys Phe Ala Leu Asp Asp Val Asp Arg Ala Ala
   530 535 540
   His Arg Ala Asn Gly Ala Pro Gly Ala Ala Ala Gly Pro Leu Leu Glu
   545 550 555 560
   Phe Val Arg Glu Leu Leu Ala Ala Gly His Val Val Val Thr Ile Gly
   565 570 575
   Ala Thr Lys Ala Ala Ala Leu Ala Pro Asp Phe Val Glu Leu Pro Val
   580 585 590
   Ala Pro Leu Ser Ala Asp Glu Gln Gln Ala Leu Ala Ala Asp Glu Leu
   595 600 605
   Arg Ala Arg Asp Leu Gln Gly Arg Asp Arg Asp Ala Asp Trp Pro Ala
   610 615 620
   Leu Leu Ala Ala Thr Arg Gly His Pro Phe Leu Leu Glu Cys Ala Val
   625 630 635 640
   Arg Leu Leu Arg Asp Gly Ser Val Arg Gly Val Ala Asp Gly Val Arg
   645 650 655
   Ser Arg Val Pro Thr Gly His Ala Pro Val Glu Ala Phe Tyr Gly Trp
   660 665 670
   Gln Trp Ala Glu Met Pro Gln Pro Trp Arg Ser Leu Leu Leu Ala Cys
   675 680 685
   Ala Gln Val Asp Gly Leu Leu Leu Glu Ile Leu Met Val Ala Val Asp
   690 695 700
   Gln Lys Met Pro Phe Ala Pro Ala Lys Arg Leu Leu Ala Arg Leu Gly
   705 710 715 720
   Asp Glu Ser Ala Val Phe Ser Gln Gly Leu Ala Leu Trp Glu Arg Arg
   725 730 735
   Gly Phe Leu Val Arg Leu Pro His Gly Arg Met Ile Asp Pro Arg Cys
   740 745 750
   Arg Ala Phe Leu Glu Leu Ala Gly Arg Ser Glu Arg Trp Asp Gly Gly
   755 760 765
   Ser Ala Asp Ala Glu Leu Glu Leu Ser Gln Thr Leu Cys Glu Gly Leu
   770 775 780
   Arg Ile Leu Ser Ala His Leu Gln Gly Arg Ala Asn Pro Ala Leu Thr
   785 790 795 800
   His Tyr Leu Leu Met Asn Arg Arg His Trp Val Ala His Phe Glu Arg
   805 810 815
   Leu Trp Phe Ala Gly Asp Leu Arg Gly Phe Leu Gly Val Met His Ala
   820 825 830
   Phe Asn Gln Leu Leu Arg Gln Ala Gln Leu Gly Ala Glu Gly Ala Ala
   835 840 845
   Trp Ser Leu Arg Leu Val Gln Arg Ser Pro Val Pro Asp Val Asp Gly
   850 855 860
   Glu Ala Pro Pro Glu Ala Ser Leu Ala Trp Leu Val Val Ala Leu Gln
   865 870 875 880
   Ala Leu Gly Ala Ser Gly Ala Ala Ala Asp Arg Ala Leu Cys Glu Gly
   885 890 895
   Ala Ala Ala Trp Gln Arg Trp Leu Asp Ala Leu Pro Glu Pro Ile Glu
   900 905 910
   Leu Arg Arg Ala Arg Gln Phe Gln Gln Val Ala Thr Phe Leu Asp Ala
   915 920 925
   Leu Tyr Arg Ala Arg Gln Asp Trp Ala Thr Ala Ile Ala Val Asp Glu
   930 935 940
   Lys Ala His Arg Phe Tyr Lys Arg Ile Gly Ala Trp Pro Ala Ala Val
   945 950 955 960
   Gln Ala Leu Arg Ser Leu Ala Arg Cys His Val Glu Leu Gly Asp Ala
   965 970 975
   Gly Ala Ala Leu Arg Leu Glu Asp Glu Ile Leu His Asp Ile Pro Tyr
   980 985 990
   Ala Asp Ala Pro Pro Gly Phe Gln Ala Gln Gln Leu Phe Asp Val Ala
   995 1000 1005
   Val Ala Arg Ala Ala Arg Gly Ala Phe Ala Gly Ala Gln Ser Ala
   1010 1015 1020
   Leu Asp Arg Leu Lys Ala Met Asp Asp Ala Lys Arg Phe Gly Asp
   1025 1030 1035
   Ser Leu Glu Ala Leu Gln Ala Asp Val Arg Tyr Glu Gln Gly Ser
   1040 1045 1050
   Tyr Ala Asp Ala Leu Arg Asp Tyr Cys Ala Leu Trp Thr Arg Ala
   1055 1060 1065
   Leu Glu Thr Gln Gln Ala Pro Phe Val Glu Arg Leu Arg Thr Arg
   1070 1075 1080
   Leu Leu Asp Leu Arg Gly Lys Leu Gly Glu Lys Glu Phe Glu Arg
   1085 1090 1095
   Arg Ile Glu Arg Glu Leu Pro Glu Gly Ile Ala Arg Pro
   1100 1105 1110
<210> 47
   <211> 121
   <212> PRT
   <213> Sorangium cellulosum
<400> 47
   Met Gly Thr Glu Leu Ile Glu Arg Gly Ser Leu Arg Ile Val His Val
   1 5 10 15
   Thr Ser Thr Asp Lys Pro Ile Arg Ala Arg Gln Asp Ala Leu Asp Val
   20 25 30
   Met Val Gly His Gly Tyr Thr Asp Val Ala Gly Ile Ala Ile Asp Glu
   35 40 45
   Thr Cys Phe Asp Pro Asp Phe Phe Arg Leu Lys Thr Gly Ile Ala Gly
   50 55 60
   Glu Leu Leu Gln Ile Phe Val Asn Tyr Arg Ile Arg Val Ala Ile Leu
   65 70 75 80
   Gly Asp Phe Ser Lys Tyr Pro Gly Asp Thr Leu His Ala Phe Ile Ala
   85 90 95
   Glu Cys Asn Arg Gly Gln His Ile Phe Phe Val His Asp Val Glu Glu
   100 105 110
   Ala Ile Arg Tyr Phe Ser His His Ala
   115 120
<210> 48
   <211> 469
   <212> PRT
   <213> Sorangium cellulosum
<400> 48
   Met Lys Gly Ser Gly Pro Met Thr Leu Ala Asp Asn Leu Val Asn Tyr
   1 5 10 15
   Asp Ala Ser Ala Leu Ala Glu Ala Ile Arg Gln Arg Ser Leu Ser Ala
   20 25 30
   Ala Asp Val Val Glu Ala Cys Tyr Ala Arg Ile Glu Arg Leu Asn Gly
   35 40 45
   Ala Val Asn Ala Ile Val Thr Leu Asp Lys Glu Arg Ala Arg Ala Arg
   50 55 60
   Ala Arg Ala Ala Asp Leu Ala Leu Ala Arg Gly Glu Ala Val Gly Pro
   65 70 75 80
   Leu His Gly Val Pro Val Thr Val Lys Asp Ala Leu His Thr Ala Gly
   85 90 95
   Leu Arg Thr Thr Ala Gly His Glu Arg Leu Arg Asp Phe Val Pro Glu
   100 105 110
   Arg Asp Ala Ala Ala Ile Asp Arg Ile Gln Glu Ala Gly Ala Ile Val
   115 120 125
   Ile Gly Lys Thr Asn Cys Ser Thr Leu Cys Gly Asp Leu Gln Thr Ser
   130 135 140
   Asn Pro Ile Phe Gly Thr Thr Asn Asn Pro Trp Glu Asn Thr Arg Thr
   145 150 155 160
   Ser Gly Gly Ser Ser Gly Gly Glu Ala Ala Ala Val Ala Leu Gly Leu
   165 170 175
   Ser Ala Leu Gly Ile Gly Thr Asp Thr Gly Gly Ser Ile Arg Val Pro
   180 185 190
   Ala Ser Tyr Cys Gly Val Tyr Gly Phe Lys Pro Ser Leu Arg Lys Val
   195 200 205
   Pro Ser Asp Gly Pro Ala Phe Pro Leu Asp Ala Ala Pro Arg Arg Glu
   210 215 220
   Asp His Leu Thr Val Ile Gly Pro Ile Ala Arg Ser Val Arg Asp Leu
   225 230 235 240
   Met Leu Cys Tyr Asp Val Leu Ser Gly Glu Ser Cys Ser Ala Ala Thr
   245 250 255
   Ser Ala Pro Pro Pro Arg Val Ala Trp Thr His Glu Phe Ala Phe Pro
   260 265 270
   Met Asp Asp Ala Val Thr Ala Glu Leu Glu Arg Ala Phe Asp Ala Leu
   275 280 285
   Arg Gly Ser Ala Ala Gln Val Glu Lys Val Ala Ser Pro Phe Asp Leu
   290 295 300
   Trp Lys Thr Asn Leu Ser Tyr Phe Met Leu Asn Met Tyr Glu Phe Lys
   305 310 315 320
   Thr Lys Glu Thr Asp Ala Ala Phe Tyr Arg Leu Phe Trp Met Phe Glu
   325 330 335
   Trp Met Arg Ser Ala Phe Arg Gly Gly Leu Asp Gly Ser Tyr Ala Arg
   340 345 350
   Leu Lys Ala Glu Gln Arg Glu Leu Ser Gly Arg Phe Glu Ser Phe Ile
   355 360 365
   Glu Ala Tyr Asp Cys Trp Val Val Pro Ala Thr Pro Ser Val Ala Ile
   370 375 380
   Arg His Gln Lys Thr Gly Arg Ser Val Pro Met Thr Val Asn Gly Lys
   385 390 395 400
   Glu Lys Lys Val Glu Tyr Trp Asn Ala Val Gly Gly Phe Ala Phe Phe
   405 410 415
   Val Asn Leu Leu Gly Asn Pro Ser Val Val Ile Pro Val Gly Arg Ser
   420 425 430
   Lys Asp Gly Leu Pro Val Gly Val Gln Val Val Gly Lys His Gly Gln
   435 440 445
   Asp Ala Ser Leu Leu Arg Ala Ala Arg Phe Ile Ala Asp Thr Ile Gly
   450 455 460
   Gln Leu Arg Thr Ala
   465
<210> 49
   <211> 262
   <212> PRT
   <213> Sorangium cellulosum
<400> 49
   Met Ile Arg Thr Val Arg Thr Thr Arg Tyr Val Ala Pro Leu Arg Glu
   1               5                   10                  15
   Gly Gly Ser Val Pro Ala Ile Val Glu Ala Asp Asp Asp Gly Leu Tyr
               20                  25                  30
   Val Val Lys Leu Arg Gly Ala Gly Gln Gly Pro Lys Ala Leu Ile Ala
           35                  40                  45
   Glu Leu Val Ala Gly Gln Ile Gly Arg Ala Leu Gly Leu Pro Val Pro
       50                  55                  60
   Glu Ile Val Leu Ile Glu Leu Asp Val Glu Phe Gly Arg Thr Glu Pro
   65                  70                  75                  80
   Asp Pro Glu Ile Ala Lys Ile Leu Ser Ala Ser Ala Gly Leu Asn Val
                   85                  90                  95
   Ala Leu Asp Tyr Leu Pro Gly Ser Leu Ala Phe Gln Pro Phe Thr Gly
               100                 105                 110
   Gly Lys Leu Asp Pro Ala Leu Ala Ser Ala Val Val Trp Phe Asp Ala
           115                 120                 125
   Phe Val Ser Asn Met Asp Arg Thr Pro Arg Asn Pro Asn Leu Leu Leu
       130                 135                 140
   Trp His Arg Lys Leu Trp Leu Ile Asp His Gly Ala Ala Leu Tyr Val
   145                 150                 155                 160
   His His Ala Trp Ser Glu Gly Val Asp Pro Leu Ala Glu Ala His Asp
                   165                 170                 175
   Arg Phe Pro Gln Ile Glu Ser His Ile Leu Leu Pro Tyr Ala Thr Asp
               180                 185                 190
   Ile Ala Ala Ala Asp Ala Val Gln Ser Ala Leu Leu Thr Pro Glu Lys
           195                 200                 205
   Ile Ala Gly Ile Val Ala Glu Ile Pro Asp Ala Trp Leu Val Asp Arg
       210                 215                 220
   Ala Pro Ser Thr Asp Pro Glu Glu Ser Arg Ala Ala Tyr Ala Asp Tyr
   225                 230                 235                 240
   Leu Asn Arg Arg Leu Ala Pro Pro Arg Gly Phe Val Glu Glu Ala Leu
                   245                 250                 255
   Asp Ala Arg Ala Arg Arg
               260
<210> 50
   <211> 118
   <212> PRT
   <213> Sorangium cellulosum
<400> 50
   Val Leu Arg Val Val Pro Arg Ile Glu Arg Gly Glu Arg Val Asn Val
   1               5                   10                  15
   Gly Val Val Leu His Cys Arg Ser Arg Gly Phe Leu Asp Val Arg Cys
               20                  25                  30
   Glu Pro Asp Asp Arg Arg Leu Leu Ala Leu Ala Pro Asp Leu Asp Leu
           35                  40                  45
   Pro Ala Val Arg Gln His Leu Glu Phe Val Arg Leu Val Cys Ala Gly
       50                  55                  60
   Asp Pro Ala Ser Gly Tyr Val Ala Gly Leu Pro Gln Glu Glu Arg Phe
   65                  70                  75                  80
   Gly Trp Leu Val Ser Pro Ser Ser Thr Ile Val Gln Pro Ser Pro Ser
                   85                  90                  95
   His Thr Gly Leu Cys Ala Asp Pro Ala Glu Thr Leu Gly Trp Leu Leu
               100                 105                 110
   Asp Arg Leu Val Arg Val
           115
<210> 51
   <211> 13
   <212> PRT
   <213> artificial
<220>
   <223> sequence motif
<400> 51
   Gln Gln Gly His Ser Leu Gly Arg Phe His Asn Gln Val
   1               5                   10
<210> 52
   <211> 26
   <212> DNA
   <213> artificial
<220>
   <223> upstream primer
<400> 52
   aagcttgatc ccgaagcgat tgtaga 26
<210> 53
   <211> 25
   <212> DNA
   <213> artificial
<220>
   <223> downstream primer
<400> 53
   ggatcctcag caggatgtcg aacaa 25
<210> 54
   <211> 19
   <212> DNA
   <213> artificial
<220>
   <223> primer
<400> 54
   gagaagctcc gctacctga 19
<210> 55
   <211> 20
   <212> DNA
   <213> artificial
<220>
   <223> primer
<400> 55
   tgaagtcagc cccatacgat 20
<210> 56
   <211> 20
   <212> DNA
   <213> artificial
<220>
   <223> primer
<400> 56
   ggatgccgta gttgagaagg 20
<210> 57
   <211> 20
   <212> DNA
   <213> artificial
<220>
   <223> primer
<400> 57
   ggtgatgtcg gcgatatagg 20
<210> 58
   <211> 20
   <212> DNA
   <213> artificial
<220>
   <223> primer
<400> 58
   cggagatcta tcgtatcgag 20
<210> 59
   <211> 26
   <212> DNA
   <213> artificial
<220>
   <223> primer
<400> 59
   aagcttcgag ccgtacgaga aatacc 26
<210> 60
   <211> 20
   <212> DNA
   <213> artificial
<220>
   <223> primer
<400> 60
   acgaggatcc tgtgacgttc 20
<210> 61
   <211> 20
   <212> DNA
   <213> artificial
<220>
   <223> primer
<400> 61
   cgtactctgc gatcgacaac 20

## Description

The present invention relates to biosynthetic pathway coding sequences, the translation products of which are part of or constitute the biosynthetic pathway metabolism of etnangien, a polyketide compound having antibiotic activity.

In accordance with the provision of the biosynthetic pathway coding sequences or genes encoding the enzymes constituting the biosynthetic pathway involved in the production of etnangien, the present invention provides a process for the production of etnangien, making use of the biosynthetic pathway genes and their translation products, as well as microorganisms, especially bacteria, which are genetically manipulated to contain at least one of the biosynthetic pathway genes, for the production of etnangien.

Further, the present invention provides a production process including an isolation method for separating the novel compound from an aqueous mixture, e.g. from a fermentation broth.

DE 19630980 A1 describes an antibiotic compound termed etnangien, which is obtained by fermentation of *Sorangium cellulosum* (DSM 11028) without genetic manipulation, adsorbed to Amberlite XAD adsorber resin and eluted from the adsorber resin using methanol. The methanol eluate can further be purified by extraction with acetic acid ester, dissolving in diethylether, extracting with aqueous sodium hydrogencarbonate, acidifying, and extracting with dichloromethane. After removal of dichloromethane, the residue can be purified by reverse phase chromatography using methanol/water as the eluent, extracting the aqueous phase with acetic acid ester, and drying.

The structure given for etnangien is:

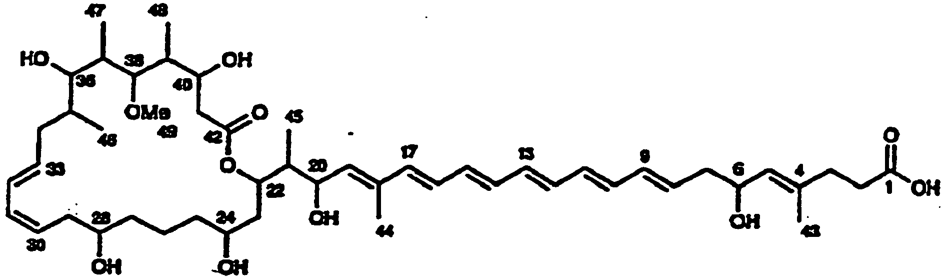

The antibacterial activity that has been found for etnangien is indicated to be based on the inhibition of bacterial DNA- dependent RNA polymerase. However, during the development of the present invention, a compound having the known structure could not be found.

Achupp et al in J. of Bacteriology 177, 13, 3673-3679 (1995) describe the gene cluster for the biosynthesis of the macrolide Soraphen A from Sorangium cellulosum.

Perlova et al in J. of Biotechnoloy, 121, 2 174-191 (2006) describe the identification of the gene cluster for the biosynthesis of the macrolide Chivosazol from Sorangium cellulosum.

### Objects of the invention

In view of the fermentation process for etnangien using a wild-type isolate bacterium as described in the above prior art, the present invention seeks to provide the coding sequences, the translation products of which constitute the biosynthetic pathway enzymes for the production of etnangien, e.g. for use in the genetic manipulation of bacteria.

Further, the present invention seeks to provide microorganisms, e.g. bacteria that are genetically manipulated to contain at least one of the biosynthetic pathway genes, and the use of the said genetically manipulated bacteria for the production of etnangien by a fermentation process involving the genetically manipulated micro-organism.

### General description of the invention

The present invention attains the above-mentioned objects by providing the genes encoding the biosynthetic pathway enzymes and the biosynthetic pathway enzymes for producing in a host micro-organism the compound etnangien according to formula I having antibiotic activity: 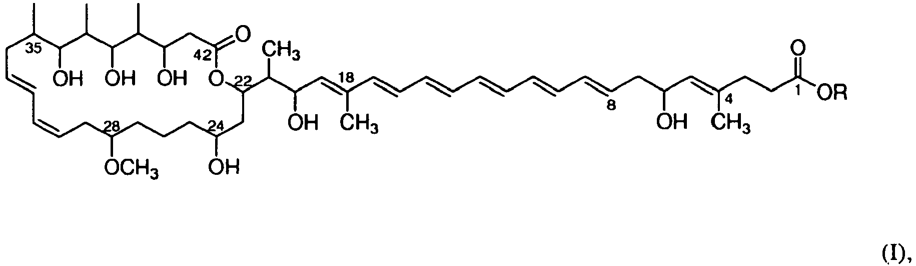 wherein R is hydrogen. Although the core structure of the compounds according to the present invention differs in a hydroxyl group at C-38 instead of the methoxy group at C-38 in the compound according to DE 19630980 A1, the compound produced according to the present invention is presently termed etnangien.

In a first embodiment, the present invention provides the coding sequences and the respective translation products obtainable therefrom, which constitute the coding sequences and the catalytically active translation products obtainable therefrom, necessary for the biosynthetic pathway for the production of etnangien in micro-organisms.

In a second embodiment, the present invention provides genetically modified microorganisms, preferably bacteria, which have been provided with at least one of the coding sequences encoding the biosynthetic pathway enzymes for the production of etnangien, with the coding sequences introduced by genetic manipulation being contained in expression cassettes. The expression cassettes containing the coding sequences for the biosynthetic pathway of etnangien can contain a single coding sequence each, also referred to as open reading frame (orf), or multi-cistronic expression cassettes containing two or more of the coding sequences of the invention.

Since it has been found that biosynthesis of etnangien in bacteria requires presence of the translation products of each orf 1 to orf 25 as identified in the present invention, the coding sequences of the invention in a first aspect can be used for genetic manipulation of bacteria that naturally contain all of orf 1 to orf 25, e.g. for duplication or further increase of copy number of certain orfs by introducing the respective coding sequences in addition to the natural genome.

In a second aspect, the coding sequences of the invention can be used for complementation of an incomplete natural set of the biosynthetic pathway genes, namely by introducing the coding sequence missing in the original bacterial strain. Accordingly, the resultant micro-organism contains a complete set of coding sequences for the biosynthetic pathway enzymes for etnangien synthesis, containing both original coding sequences and coding sequences selected from orf 1 to orf 25 of the invention introduced by genetic manipulation.

In a third aspect, the complete set of coding sequences of the invention, namely orf 1 to orf 25, can be used for introduction into a micro-organism originally devoid of these coding sequences in order to establish the biosynthetic pathway for etnangien for the first time in the respective micro-organism. Accordingly, the resultant micro-organism is provided with the entire set of orf 1 to orf 25 of the invention as additional orfs to the natural genome.

Preferably, the genetically manipulated micro-organism of the invention contains one or more copies of the coding sequences encoding the biosynthetic pathway enzymes for etnangien in order to enhance production rates of etnangien. Micro-organisms can be selected from the group comprising myxobacteria, lysobacteria, streptomyces spp. and pseudomonas spp. In the case of bacteria of the genus Sorangium, also referred to as Polyangium, genetically manipulated bacteria of the invention contain at least one copy of the least one coding sequence of the pathway enzymes for the production of etnangien in addition to naturally occurring copies of genes encoding biosynthetic pathway enzymes, e.g. as described for the first aspect.

It has been found that in the Sorangium strain analysed, designated So ce56, the biosynthetic pathway genes are arranged as consecutive orfs in multicistronic expression cassettes. Accordingly, it is preferred that the microorganisms genetically manipulated according to the invention contain at least two of orf 1 to orf 25 in a multi-cistronic expression cassette that is introduced by genetic manipulation. Especially preferred expression cassettes are a first multi-cistronic expression cassette containing a promoter sequence and, arranged downstream thereof, orf 1 (Seq.-ID No. 1), orf 2 (Seq.-ID No. 2), orf 3 (Seq.-ID No. 3), orf 4 (Seq.-ID No. 4), orf 5 (Seq.-ID No. 5), orf 6 (Seq.-ID No. 6), orf 7 (Seq.-ID No. 7), orf 8 (Seq.-ID No. 8), orf 9 (Seq.-ID No. 9), orf 10 (Seq.-ID No. 10), orf 11 (Seq.-ID No. 11), orf 12 (Seq.-ID No. 12), orf 13 (Seq.-ID No. 13), and a terminator sequence; and/or, a second multi-cistronic expression cassette, containing a promoter sequence, and arranged downstream thereof, orf 15 (Seq.-ID No. 15), orf 16 (Seq.-ID No. 16), orf 17 (Seq.-ID No. 17), orf 18 (Seq.-ID No. 18), orf 19 (Seq.-ID No. 19), orf 20 (Seq.-ID No. 20), orf 21 (Seq.-ID No. 21), followed by a terminator sequence., and/or a third multi-cistronic expression cassette containing a promoter sequence, and arranged downstream thereof, orf 22 (Seq.-ID No. 22), orf 23 (Seq.-ID No. 23), followed by terminator, and/or a fourth multi-cistronic expression cassette, containing a promoter sequence, and arranged downstream thereof, orf 24 (Seq.-ID No. 24, orf 25 (Seq.-ID No. 25), and/or a fifth monocistronic expression cassette, containing a promoter sequence, and arranged downstream thereof, orf 14 (Seq.-ID No. 14), followed by a terminator sequence.

In a preferred embodiment, the host micro-organism harbouring by genetic manipulation at least one copy of an orf encoding a biosynthetic pathway enzyme for the production of etnangien, in addition contains a mutation in the *rpsL* gene coding for the S12 ribosomal protein, causing a lysine to arginine mutation at amino acid No. 88, which can e.g. be caused by an A to G transition at nucleotide 263 in the S12 protein encoding exon.

The biosynthetic pathway genes encoding the biosynthetic pathway enzymes involved in the production of etnangien comprise the coding sequences listed in following table 1, designated orf 1 to orf 25. For each orf, the DNA sequence is given.

Accordingly, the present invention provides the biosynthetic pathway genes encoded by coding sequences for the etnangien biosynthetic pathway enzymes selected from the group comprising Seq.-ID No 1 to Seq.-ID No 14 and complementary sequences Seq.-ID No 15 to Seq.-ID No 25. Preferably, the biosynthetic pathway enzymes are encoded by Seq.-ID No 1 to Seq.-ID No 14 and complementary sequences Seq.-ID No 15 to Seq.-ID No 25, i.e. the biosynthetic pathway enzymes comprise the amino acid sequences of Seq.-ID No 26 to Seq.-ID No. 50.

**Table 1: Coding sequences and their translation products constituting the biosynthetic pathway enzymes for etnangien biosynthesis**

| nucleotide sequence | amino acid sequence | protein/ gene | length, bp/aa | Description |
|---|---|---|---|---|
| **upstream region** | | | | |
| Seq.-ID No 1 | Seq.-ID No 26 | orf 1 | 315/104 | hypothetical protein |
| Seq.-ID No 2 | Seq.-ID No 27 | orf 2 | 918/305 | malonyl CoA-acyl carrier protein transacylase |
| Seq.-ID No 3 | Seq.-ID No 28 | orf 3 | 1902/633 | asparagine synthetase |
| Seq.-ID No 15 (complementary) | Seq.-ID No 40 | orf 15 | 1380/459 | putative FMN-dependent oxidoreductase |
| Seq.-ID No 16 (complementary) | Seq.-ID No 41 | orf 16 | 251/82 | acyl carrier protein |
| Seq.-ID No 17 (complementary) | Seq.-ID No 42 | orf 17 | 1233/410 | ketosynthase-like decarboxylase |
| Seq.-ID No 18 (complementary) | Seq.-ID No 43 | orf 18 | 1260/419 | HMG-CoA synthase |
| Seq.-ID No 19 (complementary) | Seq.-ID No 44 | orf 19 | 768/255 | Enoyl-CoA hydratase |
| Seq.-ID No 20 (complementary) | Seq.-ID No 45 | orf 20 | 750/249 | Enoyl-CoA hydratase |
| Seq.-ID No 21 (complementary) | Seq.-ID No 46 | orf 21 | 3336/1111 | hypothetical protein |
| Seq.-ID No 22 (complementary) | Seq.-ID No 47 | orf 22 | 366/121 | hypothetical protein |
| Seq.-ID No 23 (complementary) | Seq.-ID No 48 | orf 23 | 1410/469 | putative amidase |
| Seq.-ID No 24 (complementary) | Seq.-ID No 49 | orf 24 | 789/262 | conserved hypothetical protein |
| Seq.-ID No 25 (complementary) | Seq.-ID No 50 | orf 25 | 3577118 | conserved hypothetical protein |

| **PKS region** | | | | |
|---|---|---|---|---|
| Seq.-ID No 4 | Seq.-ID No 29 | orf 4 | 15438/5146 | PKS |
| Seq.-ID No 5 | Seq.-ID No 30 | orf 5 | 11484/3828 | PKS |
| Seq.-ID No 6 | Seq.-ID No 31 | orf 6 | 10884/3628 | PKS |
| Seq.-ID No 7 | Seq.-ID No 32 | orf 7 | 15210/5070 | PKS |
| Seq.-ID No 8 | Seq.-ID No 33 | orf 8 | 10734/3578 | PKS |
| Seq.-ID No 9 | Seq.-ID No 34 | orf 9 | 17919/5973 | PKS |

| **downstream region** | | | | |
|---|---|---|---|---|
| Seq.-ID No 10 | Seq.-ID No 35 | orf 10 | 1374/458 | MATE efflux family protein |
| Seq.-ID No 11 | Seq.-ID No 36 | orf 11 | 2076/692 | acyltransferase/ oxidoreductase |
| Seq.-ID No 12 | Seq.-ID No 37 | orf 12 | 1041/347 | - |
| Seq.-ID No 13 | Seq.-ID No 38 | orf 13 | 1656/552 | Rhs family protein |
| Seq.-ID No 14 | Seq.-ID No 39 | orf 14 | 534/178 | - |

The descriptions for enzymatic activities given in table 1 are inferred from computer-assisted analysis of the sequences. Therefore, the coding sequences and their respective translation products define the biosynthetic pathway enzymes of the invention, which may have different activities than indicated in table 1.

It has been found during the preparation of this invention that etnangien produced by fermentation is preferably isolated in the presence of a stabiliser, e.g. 4-tert.-butyl-o-dihydroxybenzene to avoid degradation.

Optionally, the fermentation process is performed in the presence of an adsorber resin, followed by the isolation of cell mass and of the optional adsorber resin, extraction of etnangien from cell mass and/or of the adsorber resin.

Preferably, further extractive and/or chromatographic processing steps follow for purification of etnangien, optionally followed by derivatisation reactions to generate derivatives of etnangien.

Preferably, an adsorber resin having aromatic side chains, e.g. a divinylbenzene copolymer, is present during the fermentation or contacted with the fermentation broth following cultivation. The adsorber resin is preferably isolated from the fermentation broth and transferred to a chromatography column, e.g. an open or expanded bed column. The resin is preferably washed with water to remove adherent cells. For extraction of etnangien, the resin is preferably extracted with an alcohol, e.g. methanol. The alcoholic extract is buffered with aqueous ammonium acetate and concentrated, e.g. by removal of the alcohol. The residue is extracted with an organic solvent, e.g. ethyl acetate (EtOAc), which solution is preferably concentrated by removal of the organic solvent, preferably by evaporation in the presence of toluene, allowing mild evaporation conditions. This first residue can be re-dissolved in the organic solvent and extracted with an aqueous carbonate solution, e.g. sodium hydrogen carbonate. The aqueous phase is acidified, e.g. to pH 5, and extracted with an organic solvent, e.g. EtOAc. The organic phase is preferably partitioned with aqueous buffer solution, e.g. ammonium acetate to shift the pH to a range of 6.5. to 7.3, preferably to pH 6.8 before removal of the organic solvent, e.g. by evaporation to obtain a second residue. Preferably, evaporation is performed in the presence of toluene. The second residue contains etnangien and can be purified further, e.g. by reverse phase chromatography.

Generally, it is preferred to isolate etnangien at temperatures below 10 °C, more preferred below 5 °C, most preferred at about 0 °C. Further, it is generally preferred to add 1,2-dihydroxy-4-tert.-butylbenzene to etnangien containing preparations for enhanced stability. For storage, an alcoholic solution of etnangien, e.g. in methanol, is preferred.

For production of etnangien, a production process is preferred comprising the cultivation of a producer micro-organism, e.g. *Sorangium cellulosum,* genetically manipulated to contain at least one additional copy of at least one of the genes encoding the biosynthetic pathway enzymes for the production of etnangien.

### Detailed description of the invention

The present invention is now described by way of examples with reference to the figures, wherein
- Figure 1 schematically shows the natural arrangement of coding sequences orf 1 to orf 25,
- Figure 2 schematically shows catalytic activities and the respective etnangien biosynthesis steps,
- Figure 3 A schematically shows the integration of a DNA fragment resulting in a knock-out of etnangien production, Figure 3 B shows the analysis of PCR products of the resultant knock-out mutants in comparison to the wild-type genome,
- Figure 4 shows an HPLC chromatogramme of a knock-out mutant and a wild-type etnangien producer, and
- Figure 5 A schematically shows the integration of a DNA fragment resulting in a knock-out of etnangien production and Figure 5 B shows a PCR analysis of respective knock-out mutants.

### Example 1: Identification of coding sequences participating in the biosynthesis of etnangien

Using knock-out mutagenesis by conjugational transfer according to Kopp et al. (J. Biotechnology 29-40 (2004)) of a natural etnangien producer *Sorangium cellulosum* strain, e.g. strain So ce56, subcloning and sequencing resulted in the identification of coding sequences of the biosynthetic pathway enzymes for etnangien.

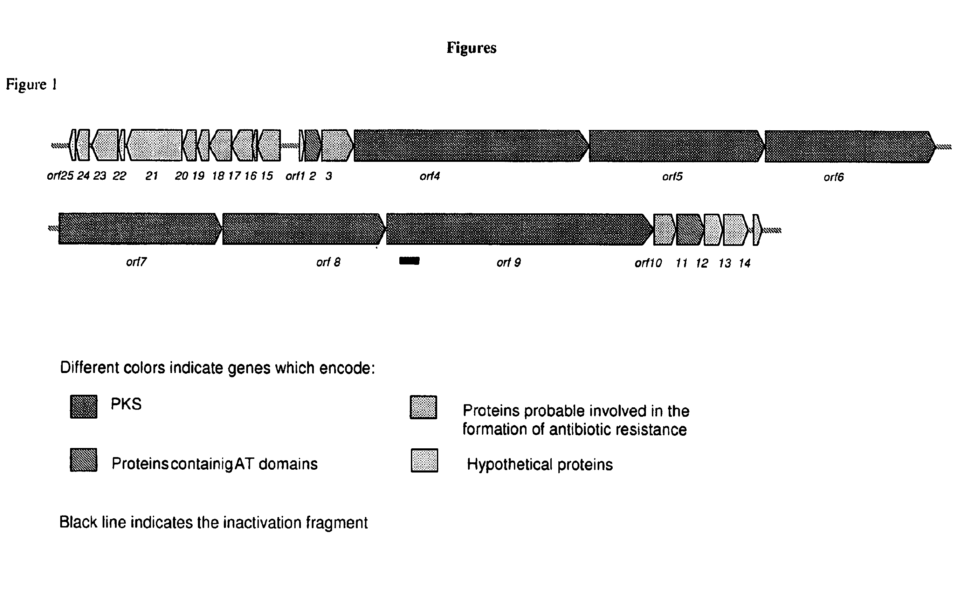

A schematic overview of the natural arrangement of coding sequences is given in Figure 1. In So ce56 the biosynthetic pathway enzymes are encoded in a first cluster comprising orf 1 to orf 13 and it is presently assumed that orf 1 to orf 13 are contained in one common operon, i.e. in one expression cassette functionally arranged downstream of one promoter and arranged upstream of one terminator. Using computer-assisted analysis of orf 1 to orf 13, it was concluded that these have polyketide synthase activity and, accordingly, this arrangement can be referred to as a polyketide synthase expression cassette or cluster. Orf 1 to orf 13 have been found to be arranged on one DNA strand. In orf 11, computer-assisted analysis led to the identification of QQGHSLGRFHNQV (Seq.-ID No. 51) as the active site motif.

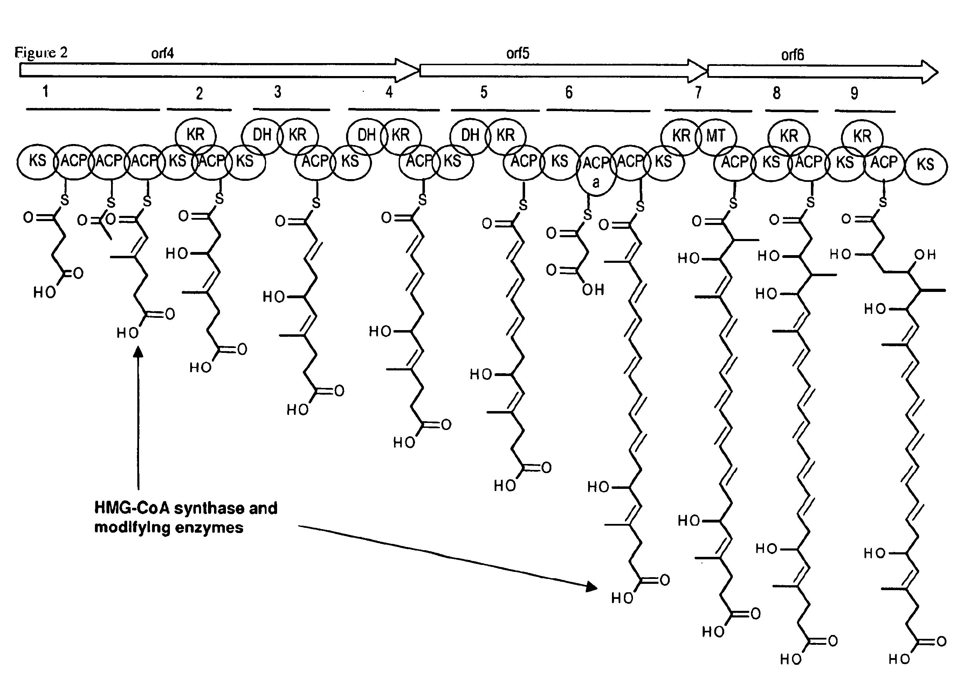

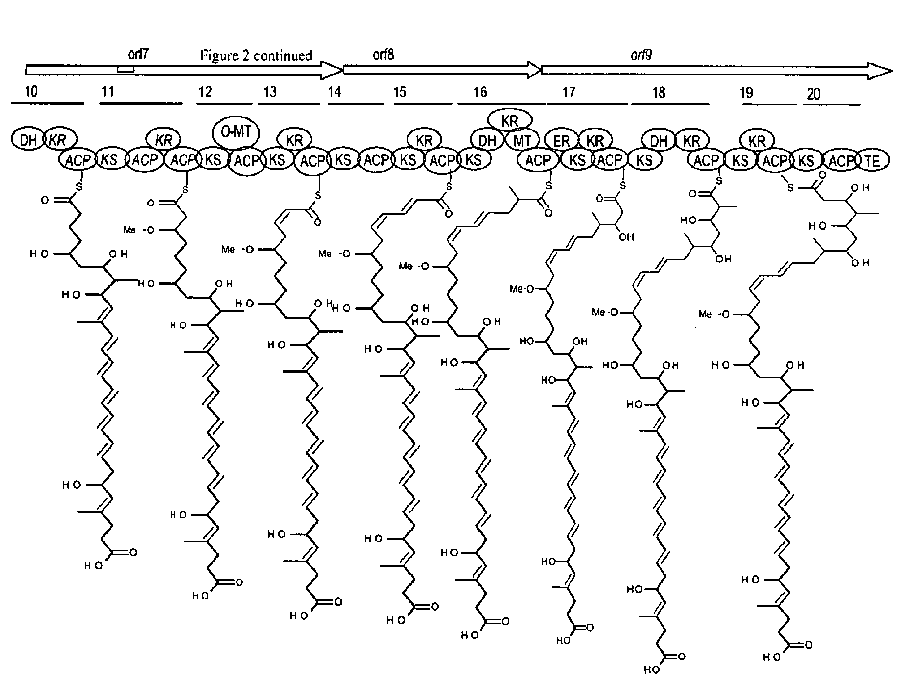

The catalytic activities of the orfs as derived from computer-assisted analysis and the respective deduced etnangien biosynthesis steps are schematically shown in Figure 2. In the figures, KS indicates ketosynthase, PKS indicates polyketide synthase, DH indicates β-hydroxydehydratase, KR indicates β-ketoacyl reductase, ACP indicates acyl carrier protein, O-MT indicates O-methyltransferase and MT indicates methyltransferase activity, respectively.

Figure 2 shows that in module 1 biosynthesis of etnangien starts with succinate being loaded to one of the ACPs, possibly assisted by one of the AT domains. Incorporation of the methylgroup at C4 could be carried out by the HMG-CoA synthase encoded by orf 18 and modifying enzymes ACP encoded by orf 16, a KS-like (KS^{s}) decarboxylase encoded by orf 17 and two ECHs encoded by orfs 19 and 20, respectively.

The intermediary product is transferred to modules 2 to 5 having KS-DH-KR-ACP activity to catalyze the incorporation of acetate units from malonyl-CoA and the formation of double bonds. Module 6 could cooperate with HMG-CoA synthase and modifying enzymes as well as being involved in methylation at C18.

However, as the intermediate product predicted from the PKS modules and loaded onto the ACP of module 6 is shorter than the one expected for subsequent methylation, modules 2 to 5 could be active iteratively to incorporate additional malonyl-CoA units before methylation by HMG-CoA. Modules 7 to 10 catalyze the substitution with additional four malonyl-CoA units. The ER-activity participating in the catalytic steps associated with module 10 could be provided by the ER of module 16, probably involving the enoyl reduction step.

Presumably, biosynthesis continues on module 12, containing an *O*-methyltransferase domain which could catalyse the O-methylation at C28. Incorporation of further malonyl-CoA units is likely carried out by modules 13 and 15 to 19. From this it would follow that DH domains are missing in modules 13 and 15, whereas DH domains were identified in modules 16 and 18. As DH activity is not required in modules 16 and 18, it could be concluded that these DH domains participate in synthesis steps catalysed by modules 13 and 15.

In close spatial relationship and arranged on the opposite strand to orfs 1 to 13, orf 15 to orf 23 have been identified in a polycistronic arrangement, presumably as one common operon.

From computer-assisted analysis, it is at present concluded that orfs 15 to 20 belong to the so-called 3-hydroxymethyl-3-glutaryl (HMG)-CoA synthase cassette protein encoding sequences, including two enoyl-CoA hydratases (ECH) by orf 19 and orf 20, which are probably involved in incorporation of the methyl groups at C4 and C18 of etnangien. From computer-assisted analysis, orf 15 is assumed to contain an 2-nitropropane dioxygenase (NPD) domain associated with S-malonyltransferase with a flavin/adenin binding domain (FAbD), an acyl-carrier protein.

Due to the close arrangement of orf 20 and orf 21, which are separated by 41 bp only, it is at present assumed that orf 21, orf 22 and orf 23 are contained in one expression cassette, e.g. are arranged in one operon.

In detail, the gene cluster comprising the biosynthetic pathway genes for etnangien were identified using an initial knock-out mutant that was generated by plasmid integration containing a PCR amplificate that was generated using primers OP73: 5' - aagcttGATC CCGAAGCGATTGTAGA (Seq.-ID No. 52) and OP74: 5' - ggatcCTCAGCAGGA TGTCGAACAA (Seq.-ID No. 53) on chromosomal So ce56 DNA, which amplificate was introduced by conjugational transfer according to Kopp et al. (J. Biotechnology 29-40 (2004)) of plasmid termed pSUPHyg containing the amplificate, now termed pOPB45, into the wild-type producer.

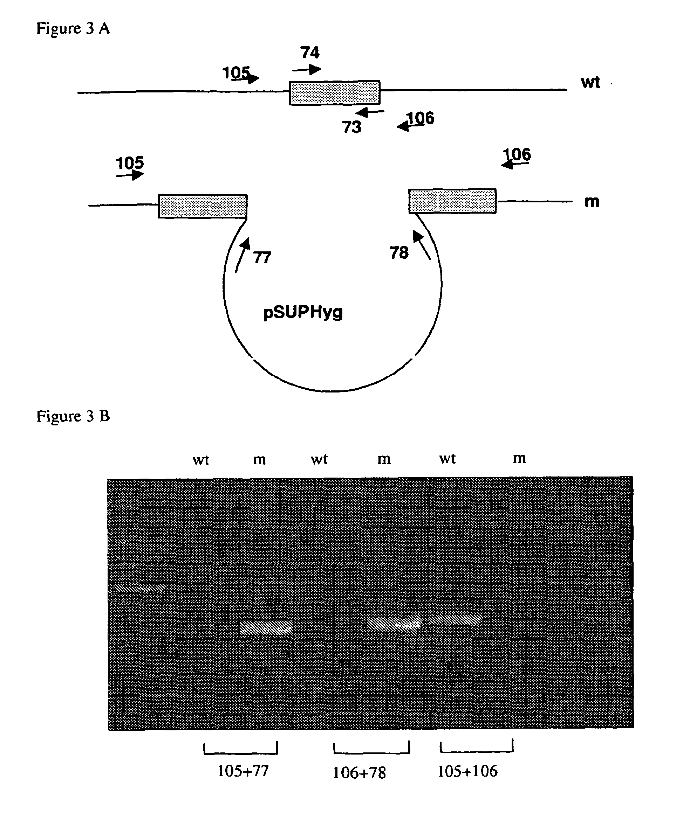

The resultant knock-out mutant is termed So ce56::pOPB45. A schematic representation of the arrangement of the integrating plasmid pOPB45 within the chromosomal biosynthetic gene cluster is shown in Figure 3A. Arrows with numbers indicate the position of the primers used in PCR. For verification of the integration, DNA of the knock-out mutant was analysed by PCR using primers OP105: 5' -GAGAAGCTCCGCTACCTGA (Seq.-ID No. 54) and OP77: 5' - TGAAGTCAGCCCCATACGAT (Seq.-ID No. 55), OP106: 5' - GGATGCCG TAGTTG AGAAGG (Seq.-ID No. 56), and OP78: 5' - GGTGATGTCGGCGATATAGG (Seq.-ID No. 57) as indicated in Figure 3B showing the resultant amplificates on mutant (m) DNA and wild-type (wt) DNA. From the position of primers OP77 and OP78 within the integrated DNA fragment pSUPHyg and the position of primers OP105 and OP106 within adjacent chromosomal DNA, integration of the fragment into the chromosomal etnangien gene cluster is verified.

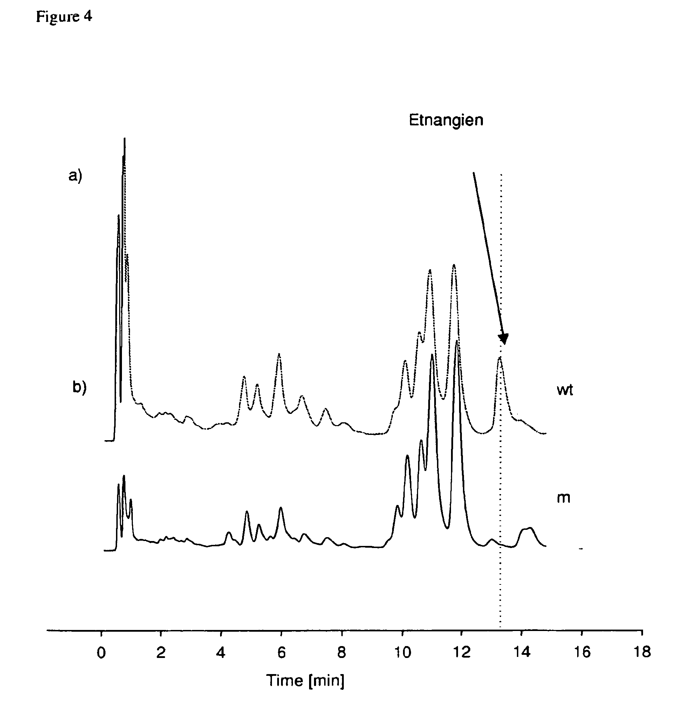

Figure 4 shows an HPLC chromatogramme of culture extracts obtained from the wild-type (wt) *Sorangium cellulosum* in comparison to the knock-out mutant (m). The peak indicating etnangien missing in the mutant extract demonstrates that the integration interrupted the etnangien biosynthetic pathway, and therefore shows that the gene cluster of the present invention is coding for the biosynthetic pathway enzymes of etnangien production in *Sorangium cellulosum.*

The function of HMG-CoA synthase was verified using gene inactivation by insertion of a PCR amplificate of a fragment of orf 18 using primers OP158: 5' - CGGAGA**TCT**ATCGTA TCGAG (Seq.-ID No. 58) with introduced *Bg*/II (underlined) restriction site and OP159: 5'- aagcttCGAGCCGTACGAG AAATACC (Seq.-ID No. 59) with a *Hind*III (underlined) restriction site, which 779 bp amplificate was cloned into a conjugational transfer vector pSUPHyg. The integration of the vector containing the amplificate was confirmed by PCR using primer pair OP188: 5' - acgaggatcctgtgacgttc (Seq.-ID No. 60) and OP77 , and primer pair OP189: 5' - cgtactctgcgatcgacaac (Seq.-ID No. 61) and OP78.

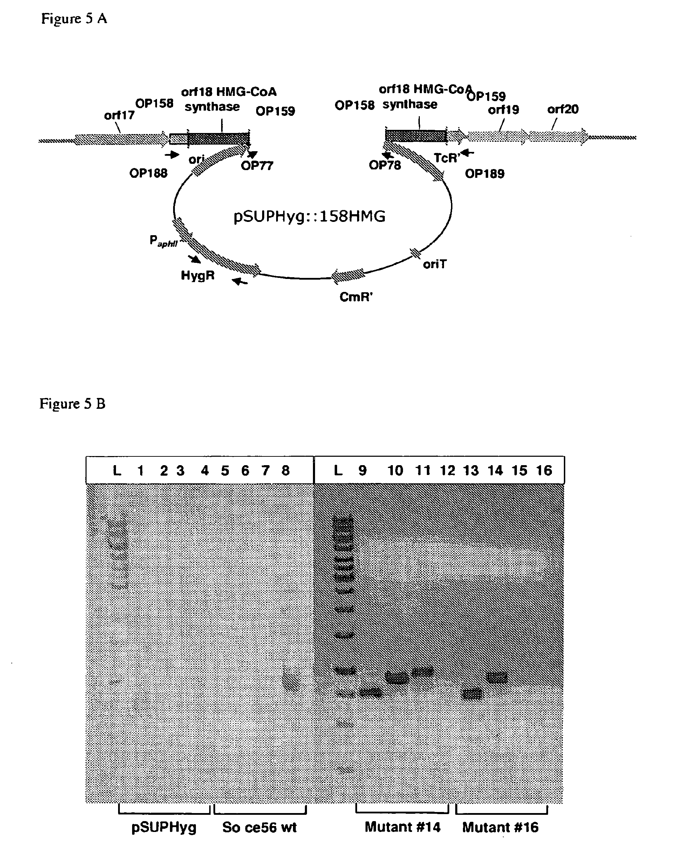

The positions of the primers are shown in Figure 5 A; PCR amplificates are analysed by gel electrophoresis shown in Figure 5 B, wherein L designates a 1 kb ladder, lanes 1, 5, 9, and 13 are amplificates for the hygromycin gene of pSUPHyg, lanes 2, 6, 10, and 14 are from primer pair OP188-OP77, lanes 3, 7, 11, 15 are from primer pair OP189-OP78, and lanes 4, 8, 12, and 16 are from primer pair OP188-OP189, for pSUPHyg vector DNA, wild-type (So ce56 wt), and knock-out mutants (Mutants #14 and #16), respectively.

### Example 2: Production of etnangien in an original non-producer strain after introduction of biosynthetic pathway coding sequences

As an example for an originally non-producer of etnangien, a *Sorangium cellulosum* was provided with expression cassettes containing orf I to orf 25 in the arrangement that had been identified in So ce56. Transformation was done by conjugational transfer according to Kopp et al. (J. Biotechnology 29-40 (2004)) using transposons containing the expression cassettes, or by electroporation according to EP 1 619 241 using linear nucleic acids containing the expression cassettes.

The identity of etnangien produced by transformants was confirmed by HPLC, MS and H-NMR analyses.

### Example 3: Enhanced production of etnangien from coding sequences orf I to orf 25 in heterologous bacteria having a mutation in S12 ribosomal protein

One of the transformants obtained in Example 2 was subjected to random mutagenesis using UV irradiation. Among the mutants, screening identified a strain showing increased etnangien production rates.

Genetic analysis of the mutated transformant revealed that it carried an A to G transition mutation in the *rpsL*gene encoding the S12 ribosomal protein, resulting in a lysine to arginine exchange at amino acid No. 88.

## Claims

1. Synthetic pathway enzymes for the biosynthesis of etnangien having catalytic activities of translation products obtainable from coding sequences selected from the group containing Seq.-ID No 1 to Seq.-ID No 14, sequences complementary to Seq.-ID No 15 to Seq.-ID No 25.

2. Synthetic pathway enzymes according to claim 1, comprising each translation product obtainable from Seq.-ID No 1 to Seq.-ID No 14 and sequences complementary to Seq.-ID No 15 to Seq.-ID No 25.

3. Micro-organism containing the pathway enzymes for the biosynthesis of etnangien, **characterized by** containing at least one artificially introduced DNA sequence encoding at least one translation product having enzymatic activity obtainable from coding sequences selected from the group containing Seq.-ID No 1 to Seq.-ID No 14 and complementary sequences Seq.-ID No 15 to Seq.-ID No 25.

4. Micro-organism according to claim 3, **characterized in that** it originally is devoid of at least one coding sequence homologous to one of the sequences contained in the group comprising Seq.-ID No 1 to Seq.-ID No 14 and complementary sequences Seq.-ID No 15 to Seq.-ID No 25.

5. Micro-organism according to one of claims 3 and 4, **characterized in that** at least two coding sequences artificially introduced are arranged between a promoter and a terminator in one expression cassette.

6. Micro-organism according to one of claims 3 to 5, **characterized in that** it is selected from the group comprising myxobacteria, lysobacteria, streptomyces spp., pseudomonas spp., and *Sorangium,* including natural etnangien producer strains and etnangien non-producer strains.

7. Micro-organism according to one of claims 3 to 5, **characterized in that** it carries a mutation in the gene encoding the S12 ribosomal protein causing an amino acid exchange from lysine to arginine at amino acid number 88.

8. Process for producing etnangien, **characterized by** the fermentation of a micro-organism obtained by genetic manipulation introducing at least one coding sequence selected from the group containing Seq.-ID No 1 to Seq.-ID No 14, complementary sequences Seq.-ID No 15 to Seq.-ID No 25.

9. Process according to claim 8, **characterized in that** the micro-organism is obtained by genetically manipulating a micro-organism originally lacking at least one coding sequence encoding a catalytic activity of the pathway biosynthesis of etnangien by introducing a coding sequence selected from the group containing Seq.-ID No 1 to Seq.-ID No 14, complementary sequences Seq.-ID No 15 to Seq.-ID No 25, which coding sequence encodes a translation product complementing the originally lacking catalytic activity.

10. Process according to claim 8 or 9, **characterized in that** the micro-organism carries a mutation in the gene encoding the S12 ribosomal protein causing an amino acid exchange from lysine to arginine at amino acid number 88.

## Patentansprüche

1. Synthetische Stoffwechselenzyme für die Biosynthese von Etnangien mit katalytischen Aktivitäten von Translationsprodukten, die aus kodierenden Sequenzen erhältlich sind, die aus der Gruppe ausgewählt sind, die Seq.-ID Nr. 1 bis Seq.-ID Nr. 14 und zu Seq.-ID Nr. 15 bis Seq.-ID Nr. 25 komplementäre Sequenzen enthält.

2. Synthetische Stoffwechselenzyme nach Anspruch 1, die jedes Translationsprodukt, das aus Seq.-ID Nr. 1 bis Seq.-ID Nr. 14 und zu Seq.-ID Nr. 15 bis Seq.-ID Nr. 25 komplementären Sequenzen erhältlich ist, umfassen.

3. Mikroorganismus, der die Stoffwechselenzyme für die Biosynthese von Etnangien enthält, **dadurch gekennzeichnet, dass** er zumindest eine künstlich eingeführte DNA-Sequenz enthält, die zumindest ein Translationsprodukt mit enzymatischer Aktivität kodiert, das aus kodierenden Sequenzen erhältlich ist, die aus der Gruppe ausgewählt sind, die Seq.-ID Nr. 1 bis Seq.-ID Nr. 14 und komplementäre Sequenzen Seq.-ID Nr. 15 bis Seq.-ID Nr. 25 enthält.

4. Mikroorganismus nach Anspruch 3, **dadurch gekennzeichnet, dass** er ursprünglich frei von zumindest einer kodierenden Sequenz ist, die homolog ist zu einer der Sequenzen, die in der Gruppe enthalten ist, die Seq.-ID Nr. 1 bis Seq.-ID Nr. 14 und komplementäre Sequenzen Seq.-ID Nr. 15 bis Seq.-ID Nr. 25 umfasst.

5. Mikroorganismus nach einem der Ansprüche 3 und 4, **dadurch gekennzeichnet, dass** zumindest zwei künstlich eingeführte kodierende Sequenzen zwischen einem Promotor und einen Terminator in einer Expressionskassette angeordnet sind.

6. Mikroorganismus nach einem der Ansprüche 3 bis 5, **dadurch gekennzeichnet, dass** er aus der Gruppe ausgewählt ist, die Myxobakterien, Lysobakterien, Streptomyces spp., Pseudomonas spp. und Sorangium, einschließlich natürlicher Produktionsstämme für Etnangien und Nicht-Produktionsstämme für Etnangien umfasst.

7. Mikroorganismus nach einem der Ansprüche 3 bis 5, **dadurch gekennzeichnet, dass** er eine Mutation in dem Gen trägt, das das ribosomale Protein S 12 kodiert, die einen Aminosäureaustausch von Lysin zu Arginin in Aminosäure Nr. 88 verursacht.

8. Verfahren zur Herstellung von Etnangien, **gekennzeichnet durch** die Fermentation eines Mikroorganismus, der **durch** genetische Manipulation **durch** Einführen von zumindest einer kodierenden Sequenz, ausgewählt aus der Gruppe, die Seq.-ID Nr. 1 bis Seq.-ID Nr. 14 und komplementäre Sequenzen Seq.-ID Nr. 15 bis Seq. -ID Nr. 25 enthält, erhalten wurde.

9. Verfahren nach Anspruch 8, **dadurch gekennzeichnet, dass** der Mikroorganismus durch genetisches Manipulieren eines Mikroorganismus erhalten wird, dem ursprünglich zumindest eine kodierende Sequenz fehlt, die die katalytische Aktivität der Stoffwechselbiosynthese von Etnangien kodiert, durch Einführen einer kodierenden Sequenz, die aus der Gruppe ausgewählt ist, die Seq.-ID Nr. 1 bis Seq.-ID Nr. 14 und komplementäre Sequenzen Seq.-ID Nr. 15 bis Seq.-ID Nr. 25 enthält, wobei die kodierende Sequenz ein Translationsprodukt kodiert, das die ursprünglich fehlende katalytische Aktivität komplementiert.

10. Verfahren nach Anspruch 8 oder 9, **dadurch gekennzeichnet, dass** der Mikroorganismus eine Mutation in dem Gen trägt, das das ribosomale Protein S 12 kodiert, die einen Aminosäureaustausch von Lysin nach Arginin in Aminosäure Nr. 88 verursacht.

## Revendications

1. Enzymes de voies de synthèse pour la biosynthèse d'etnangien ayant des activités catalytiques de produits de traduction pouvant être obtenues à partir des séquences de codage choisies parmi le groupe contenant les Seq.-ID No 1 à Seq.-ID No 14, séquences complémentaires à Seq.-ID No 15 à Seq.-ID No 25.

2. Enzymes de voies de synthèse selon la revendication 1, comprenant chaque produit de traduction pouvant être obtenu à partir de la Seq.-ID No 1 à la Seq.-ID No 14 et des séquences complémentaires à Seq.-ID No 15 à la Seq.-ID No 25.

3. Micro-organisme contenant les enzymes de voies pour la biosynthèse d'etnangien, **caractérisé par le fait qu'**il contient au moins une séquence ADN introduite artificiellement codant au moins un produit de traduction ayant une activité enzymatique pouvant être obtenue à partir des séquences de codage choisies parmi le groupe contenant les Seq.-ID No 1 à Seq.-ID No 14 et des séquences complémentaires Seq.-ID No 15 à Seq.-ID No 25.

4. Micro-organisme selon la revendication 3, **caractérisé par le fait qu'**il est originalement privé d'au moins une séquence de codage homologue à une des séquences contenues dans le groupe comprenant les Seq.-ID No 1 à Seq.-ID No 14 et des séquences complémentaires Seq.-ID No 15 à Seq.-ID No 25.

5. Micro-organisme selon l'une des revendications 3 et 4, **caractérisé par le fait qu'**au moins deux séquences codantes introduites artificiellement sont disposées entre un promoteur et un terminateur dans une cassette d'expression.

6. Micro-organisme selon l'une des revendications 3 à 5, **caractérisé par le fait qu'**il est choisi parmi le groupe comprenant des myxobactéries, lysobactéries, streptomyces spp., pseudomonas spp., et du *Sorangium,* comprenant des souches productrices d'etnangien naturelles et des souches non productrices d'etnangien.

7. Micro-organisme selon l'une des revendications 3 à 5, **caractérisé par le fait qu'**il porte une mutation dans le gène codant la protéine ribosomique S 12 entraînant un échange d'acides aminés de la lysine à l'arginine au niveau du numéro d'acides aminés 88.

8. Processus de production d'etnangien, **caractérisé par** la fermentation d'un micro-organisme obtenu par manipulation génétique introduisant au moins une séquence codante choisie parmi le groupe contenant la Seq.-ID No 1 à la Seq.-ID No 14, séquences complémentaires Seq.-ID No 15 à la Seq.-ID No 25.

9. Processus selon la revendication 8, **caractérisé par le fait que** le micro-organisme est obtenu par manipulation génétique d'un micro-organisme manquant à l'origine d'une séquence codante codant pour une activité catalytique de la voie de biosynthèse d'etnangien en introduisant une séquence codante choisie parmi le groupe contenant la Seq.-ID No 1 à la Seq.-ID No 14, séquences complémentaires Seq.-ID No 15 à Seq.-ID No 25, la séquence codante codant un produit de traduction complétant l'activité catalytique manquante à l'origine.

10. Procédé selon la revendication 8 ou 9, **caractérisé par le fait que** le micro-organisme porte une mutation dans le gène codant la protéine ribosomique S 12 entraînant un échange d'acides aminés de la lysine à l'arginine au niveau du numéro d'acides aminés 88.
